# EUROPEAN PATENT APPLICATION

(11) **EP 4 122 925 A1**
(43) Date of publication of application: **25.01.2023**
(21) Application number: 21771463.3
(22) Date of filing: 17.03.2021
(51) Int. Cl.: C07D 401/04, C07D 405/04, C07D 401/00, C07D 413/04, C07D 487/00, C07D 491/00, C07D 493/00, A61K 31/445, A61K 31/4525, A61K 31/4523, A61K 31/423, A61P 35/00

(54) **PROTEOLYSIS REGULATOR AND METHOD FOR USING SAME**

(30) Priority: 17.03.2020 CN 202010187846; 01.12.2020 CN 202011400367; 28.12.2020 CN 202011583584; 09.02.2021 CN 202110182231
(71) Applicant: Medshine Discovery Inc., Nanjing, Jiangsu 210032 (CN)
(72) Inventor: LEI, Maoyi, Shanghai 200131 (CN); PENG, Lei, Shanghai 200131 (CN); LUO, Yunfu, Shanghai 200131 (CN); XU, Yu, Shanghai 200131 (CN); LI, Junmiao, Shanghai 200131 (CN); DENG, Xinyuan, Shanghai 200131 (CN); KANG, Zisheng, Shanghai 200131 (CN); GE, Weizhi, Shanghai 200131 (CN); ZHANG, Guoli, Shanghai 200131 (CN); CHEN, Shuhui, Shanghai 200131 (CN)
(74) Representative: D Young & Co LLP
(86) International application number: PCT/CN2021/081375
(87) International publication number: WO 2021/185291

(57) **Abstract**

A proteolysis targeting chimera (PROTAC), and an application thereof in preparation of a drug for treating a related disease. Specifically disclosed are a compound as represented by formula (I) and a pharmaceutically acceptable salt thereof.

PTM-L-ULM (I)

## Description

### REFERENCE TO RELATED APPLICATIONS

This application claims the priority of:
CN202010187846.6, filed on March 17, 2020;
CN202011400367.4, filed on December 01, 2020;
CN202011583584.1, filed on December 28, 2020; and
CN202110182231.9, filed on February 09, 2021.

### FIELD OF THE INVENTION

The present disclosure relates to a class of proteolysis targeting chimeras (PROTAC), and use thereof in the manufacture of a medicament for treating related diseases. Specifically, the present disclosure relates to a compound of formula (I) or a pharmaceutically acceptable salt thereof.

### BACKGROUND OF THE INVENTION

Proteolysis Targeting Chimera (PROTAC) is a technique that utilizes the ubiquitin-proteasome system to target specific proteins and induce their intracellular degradation. The ubiquitin-proteasome system is a major pathway for intracellular protein degradation, and its normal physiological function is mainly responsible for removing denatured, mutated or deleterious proteins from the cell. The degradation of more than 80% of intracellular proteins is dependent on the ubiquitin-proteasome system. PROTAC utilizes a cell's own protein destruction mechanism to remove specific targeted proteins from the cell.

PROTAC molecule is a heterogeneous bifunctional small molecule composed of three moieties: a ligand that binds to the target protein, a ligand that binds to E3 ubiquitin ligase, and a linker that connects the two ligands. PROTAC can bind both the target protein and E3 ubiquitin ligase to form a ternary complex. The mechanism of action is to make the target protein and E3 ligase close enough by binding to the target protein, so that the E3 ligase can ubiquitinate and label the target protein, and then degrade the labeled protein by the proteasome.

Compared with traditional small molecule drugs, PROTAC molecules have many potential advantages: 1) most important of all, changing the target from "undruggable" to "druggable"; most small molecule drugs or macromolecular antibodies require prolonged and high-strength binding to the active site of a target protease to function. However, more than 80% of proteins lack apparent druggable sites on their surface, making it difficult to target these proteins with the current pharmacological strategies. However, PROTAC molecules are different. Theoretically, as long as there are certain cracks or gaps on the surface of proteins, PROTAC molecules can capture and remove these proteins; 2) removing protein accumulation; the binding of drugs to target proteins may cause the accumulation of target proteins; however, PROTAC molecules can directly degrade proteins, thereby reducing the accumulation of proteins in the body; 3) overcoming drug resistance; mutations in specific sites of a target protein usually block the binding of small molecule drugs to the protein, resulting in drug resistance; however, PROTAC molecules degrade proteins through the ubiquitin-proteasome pathway, thereby overcoming drug resistance; 4) reducing toxicity; catalytic amount of PROTAC molecules may exert their efficacy, thereby reducing the large accumulation of drugs in the body, and greatly reducing the generation of off-target toxicity; 5) "event-driven" rather than "placeholder-driven"; both small molecule inhibitors and macromolecular antibodies need to continuously occupy the active site of a target protein to block the function, while PROTAC molecules are "event-driven", which can degrade the protein only by binding to the target protein and then tagging it with ubiquitin. PROTAC molecules can be freed, so that the drug effect can be continuously exerted for a long time.

To date, PROTAC technology has been used to target a wide range of proteins, including transcription factors, backbone proteins, enzymes and regulatory proteins, etc. As a result, PROTAC technology has very broad application prospects in the field of medicine, with therapeutic potential in diseases such as tumors, cardiovascular diseases, degenerative diseases and viral infections.

### SUMMARY OF THE INVENTION

The present disclosure provides a compound of formula (I) or a pharmaceutically acceptable salt thereof,

PTM-L-ULM (I)

wherein
PTM is selected from a drug that binds to a targeted protein or a derivative thereof;
L is a chain connecting PTM and ULM;
ULM is selected from structures represented by formulae (III-1) and (III-2), 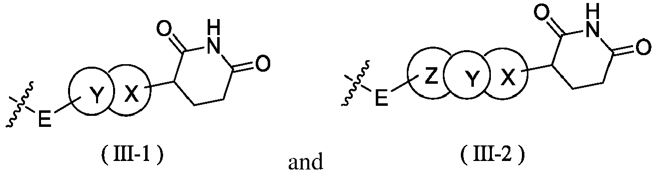
E is selected from a bond, -CH₂-, -NR₁-, -O-, -S-, -S(=O)-, -S(=O)₂-, -C(=O), and -C(=O)NR₂-;
R₁ and R₂ are each independently selected from H and C₁₋₃ alkyl;
Ring X, ring Y, and ring Z are each independently selected from phenyl, thienyl, furyl, triazolyl, oxazolyl, isoxazolyl, pyrrolyl, and pyridyl.
In some embodiments of the present disclosure, the ULM is selected from structures represented by formulae (II-11), (II-12), (II-13), (II-1), (II-2), (II-3), (II-4), (III-21), (III-22), (III-23), and (III-24), 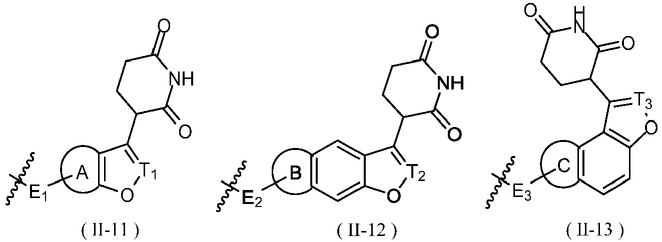 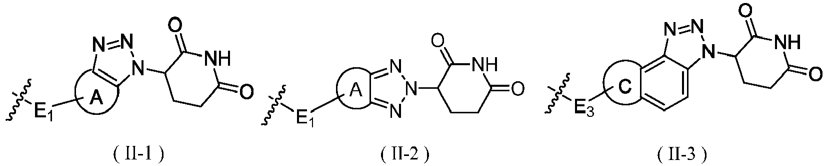 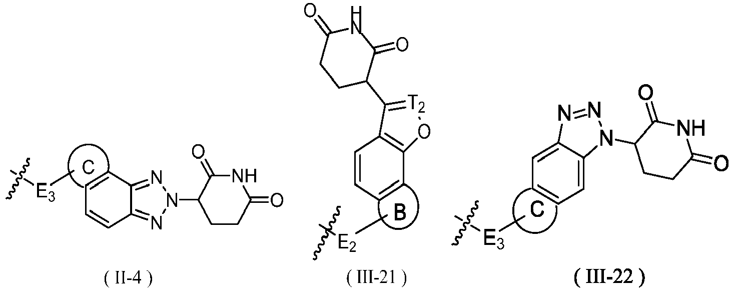 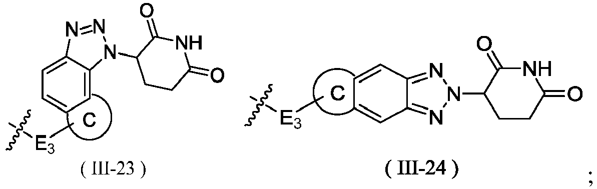
T₁, T₂, and T₃ are each independently selected from CH and N;
E₁, E₂, and E₃ are each independently selected from a bond, -CH₂-, -NR₁-, -O-, -S-, -S(=O)-, -S(=O)₂-, -C(=O), and -C(=O)NR₂-;
R₁ and R₂ are each independently selected from H and C₁₋₃ alkyl;
Ring A, ring B, and ring C are each independently selected from phenyl, thienyl, furyl, pyrrolyl, and pyridyl.

In some embodiments of the present disclosure, the ring A is selected from phenyl and thienyl, and other variables are as defined in this disclosure.

In some embodiments of the present disclosure, the ULM is selected from structures represented by formulae (II-11-1), (II-11-2), (II-1-1), and (II-2-1) 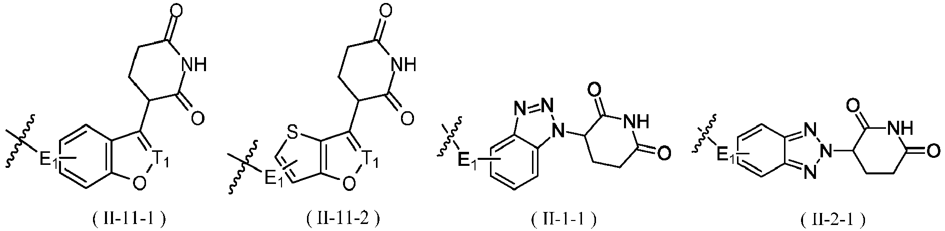 wherein T₁, and E₁ are as defined in this disclosure.

In some embodiments of the present disclosure, the ring B is selected from phenyl, and other variables are as defined in this disclosure.

In some embodiments of the present disclosure, the ULM is selected from structures represented by formulae (II-12-1), and (III-21-1) 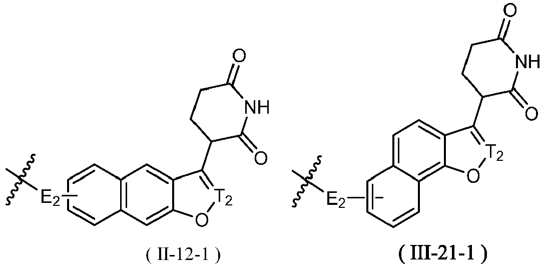 wherein T₂, and E₂ are as defined in this disclosure.

In some embodiments of the present disclosure, the ring C is selected from phenyl, and other variables are as defined in this disclosure.

In some embodiments of the present disclosure, the ULM is selected from structures represented by formulae (II-13-1), (II-3-1), (II-4-2), (III-22-1), (III-23-1), and (III-24-1) 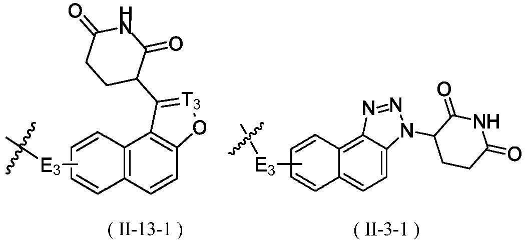 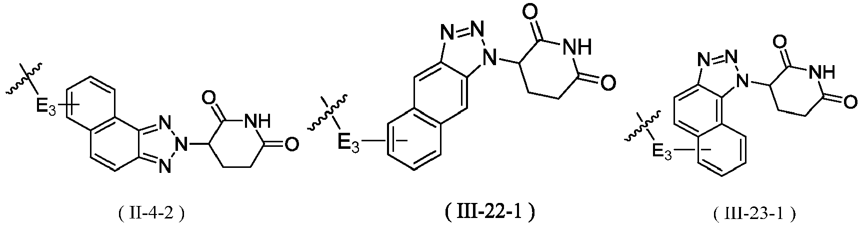 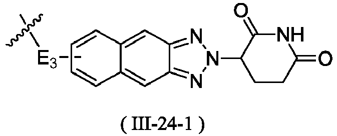 wherein T₃, and E₃ are as defined in this disclosure.

In some embodiments of the present disclosure, the ULM is selected from 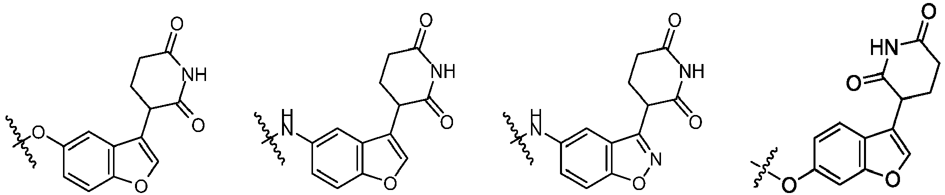 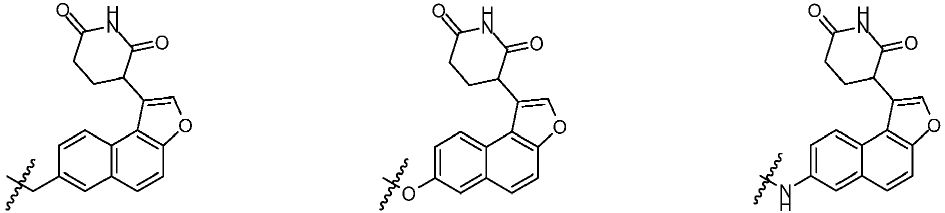 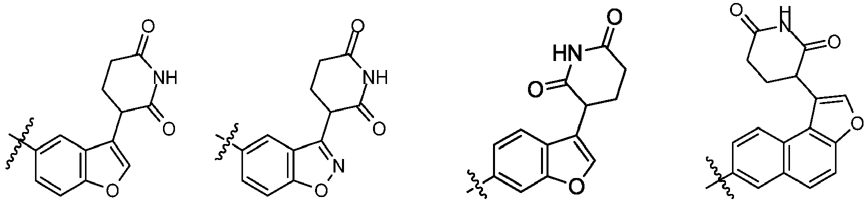 and 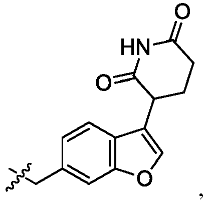 and other variables are as defined in this disclosure.

In some embodiments of the present disclosure, the PTM is selected from drugs that act on ALK, BET, CDK, PARP, EGFR, γ-secretase, CBFβ-SMMHC, WEE1, MEK, BCR-ABL, MET, RAS, BTK, VEGFR, JAK, HER2, HDAC, Akt, PI3K, mTOR, AR, ER, PDEδ, SRC, MDM2, RAF, IRAK4, STAT3, and c-Myc, or derivatives thereof, and other variables are as defined in this disclosure.

In some embodiments of the present disclosure, the PTM is selected from drugs that act on ALK, BRD4, CDK4/6, CDK8, CDK9, PARP, EGFR, γ-secretase, CBFβ-SMMHC, WEE1, MEK, BCR-ABL, MET, KRAS, BTK, VEGFR, HER2, HDAC, Akt, PI3K, mTOR, AR, ER, PDEδ, SRC, JAK, MDM2, RAF, IRAK4, STAT3, and c-Myc, or derivatives thereof, and other variables are as defined in this disclosure.

In some embodiments of the present disclosure, the PTM is selected from drugs that act on ALK, BRD4, CDK4/6, PARP, EGFR, γ-secretase, CBFβ-SMMHC, WEE1, MEK, BCR-ABL, MET, KRAS, EGFR, BTK, AR, ER, PDEδ, JAK, MDM2, and RAF, or derivatives thereof, and other variables are as defined in this disclosure.

In some embodiments of the present disclosure, the PTM is selected from 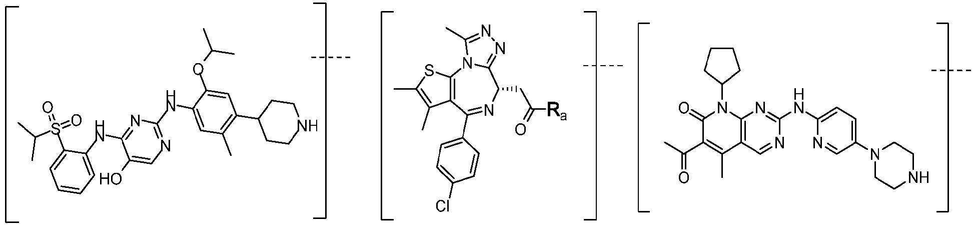 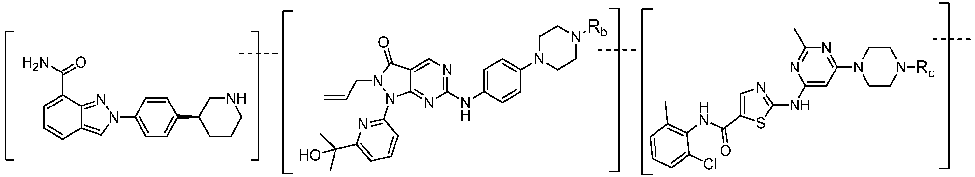 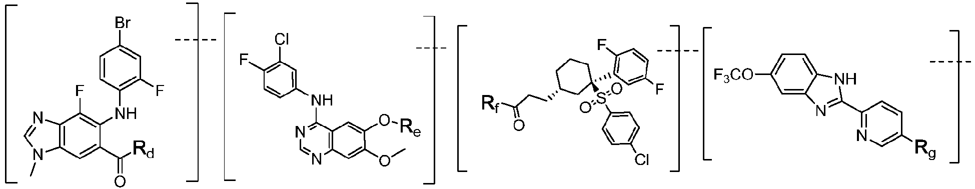 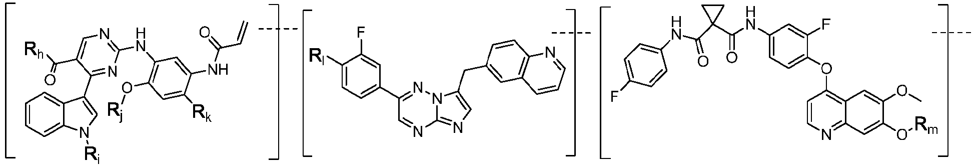 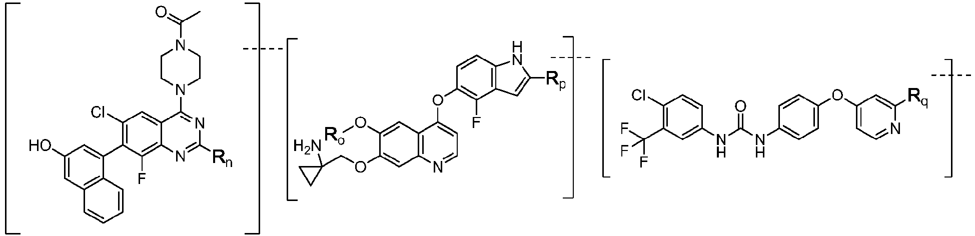 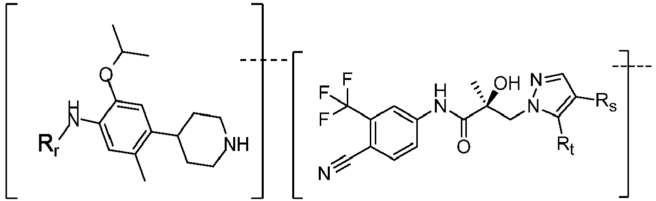 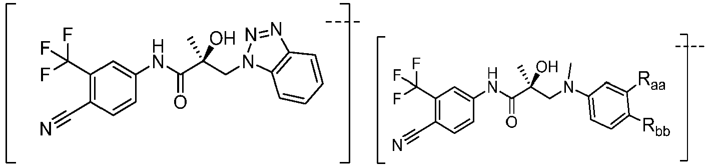 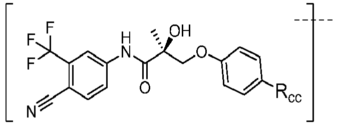 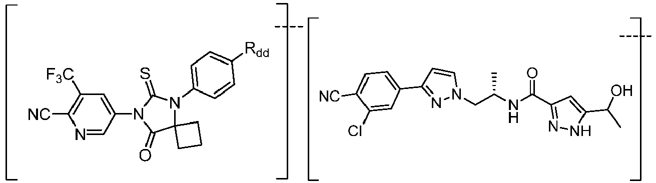 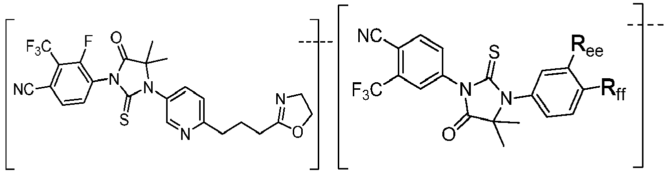 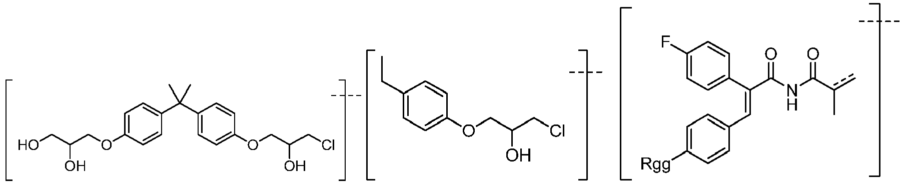 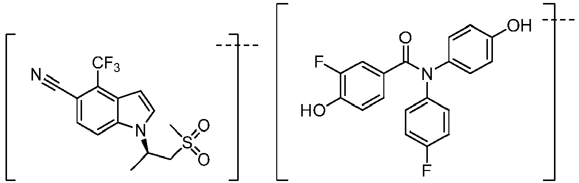 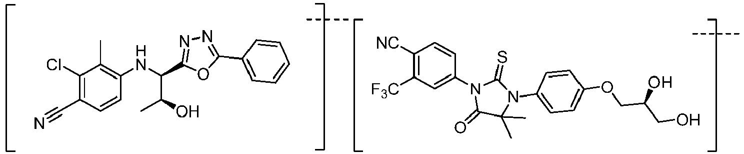 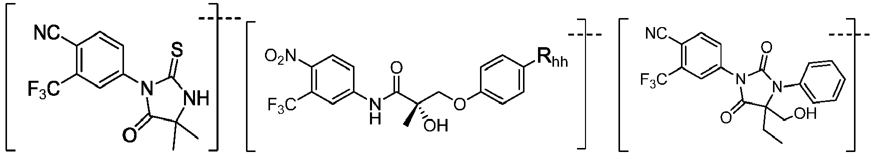 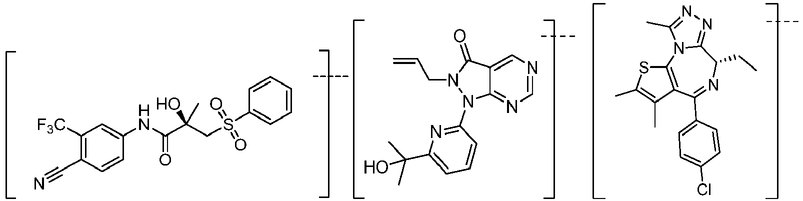 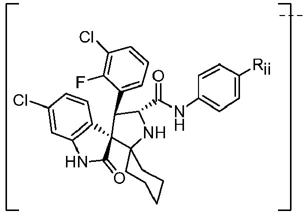 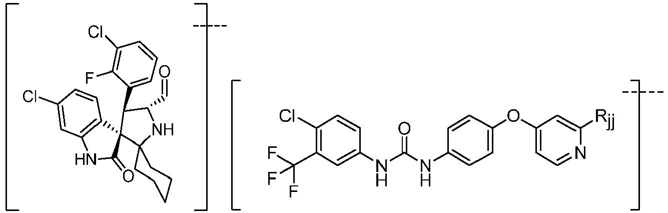 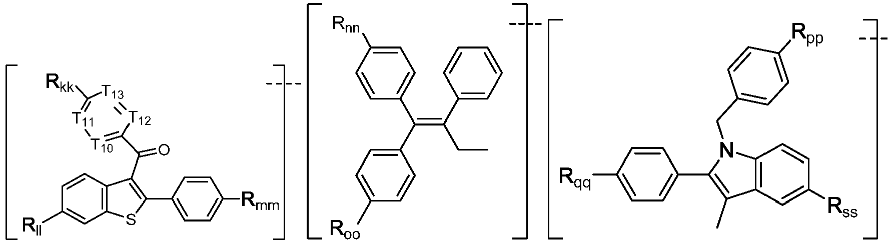 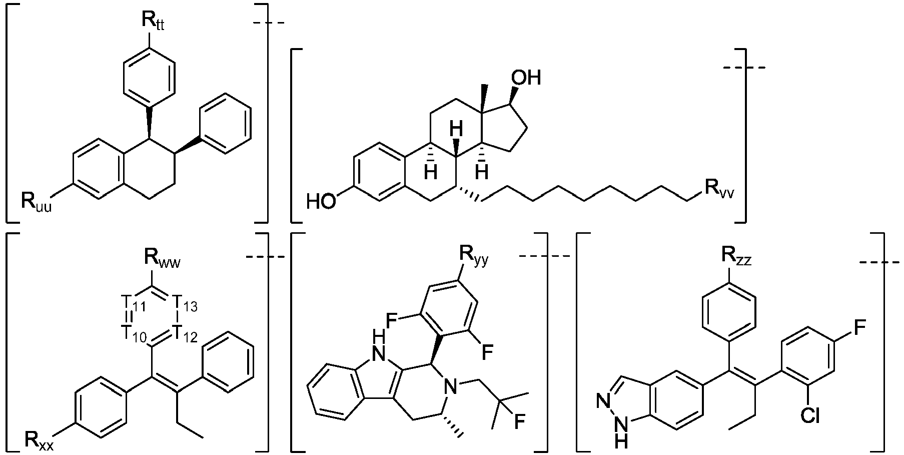 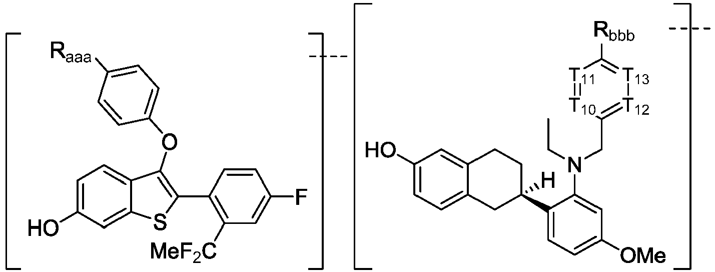 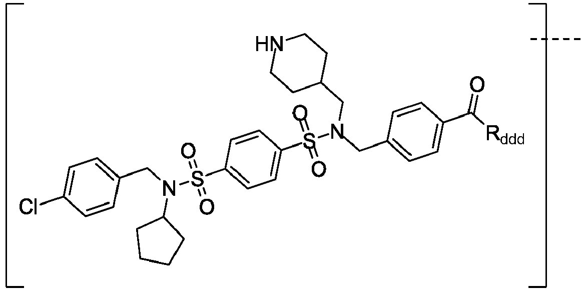 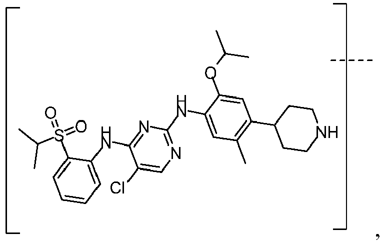 wherein 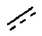
is selected from a single bond and a double bond;
T₁₀, T₁₁, T₁₂, and T₁₃ are each independently selected from N and CR_{ccc}, and at most two of T₁₀, T₁₁, T₁₂, and T₁₃ are selected from N;
Rₐ is selected from H, 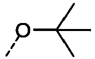 and NH₂;
R_{b} is selected from H and CH₃;
R_{c} is selected from H and 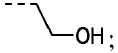
R_{d} is selected from H, NH₂ and 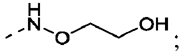
Rₑ is selected from H and 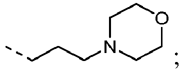
R_{f} is selected from H and OH;
R_{g} is selected from H and OH;
Rₕ is selected from H and 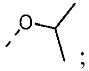
Rᵢ is selected from H and CH₃;
Rⱼ is selected from H and CH₃;
Rₖ is selected from H, NH₂, NHCH₃, and 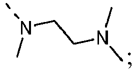
Rₗ is selected from H, 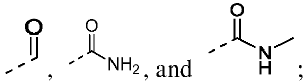
Rₘ is selected from H and 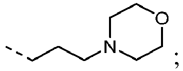
Rₙ is selected from H, NH₂, NHCH₂CH₃, and
Rₒ is selected from H and CH₃;
Rₚ is selected from H and CH₃; 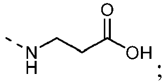
R_{q} is selected from H, 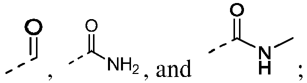
Rᵣ is selected from H and 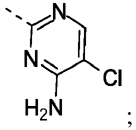
Rₛ is selected from H, F and Cl;
Rₜ is selected from H and Br;
Rₐₐ is selected from H and phenyl;
R_{bb}, and R_{cc} are each independently selected from H and CN;
R_{dd}, R_{ff}, Rₕₕ, Rᵢᵢ, and Rⱼⱼ are each independently selected from H, OCH₃, 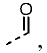 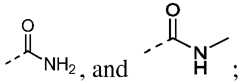
Rₑₑ is selected from H and F;
R_{gg} is selected from H and Cl;
Rₖₖ is selected from H, OH, and 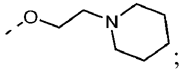
Rₗₗ, and Rₘₘ are each independently selected from H, F, Cl, Br, I, OH, and OCH₃;
Rₙₙ is selected from H, OH and 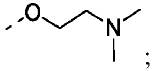
Rₒₒ is selected from H and OH;
Rₚₚ is selected from H, OH and 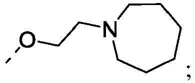
R_{qq}, and Rₛₛ are each independently selected from H, F, Cl, Br, I, OH, and OCH₃;
Rₜₜ is selected from H, OH and 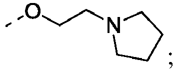
Rᵤᵤ is selected from H, F, Cl, Br, I, OH, and OCH₃;
Rᵥᵥ is selected from H and 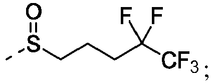
R_{ww} is selected from H and 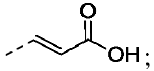
Rₓₓ is selected from H and OH;
R_{yy}, R_{zz}, and Rₐₐₐ are each independently selected from H and 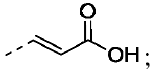
R_{bbb} is selected from H and 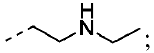
R_{ccc} is selected from H, F, Cl, Br, and I;
R_{ddd} is selected from H and NH₂.

In some embodiments of the present disclosure, the PTM is selected from 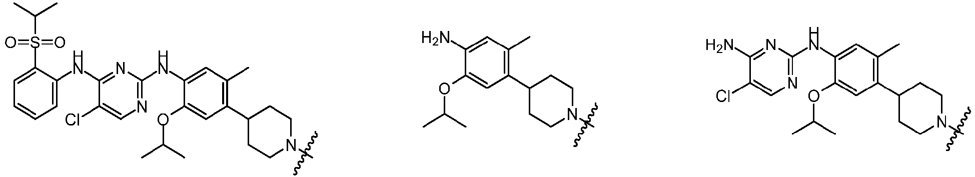 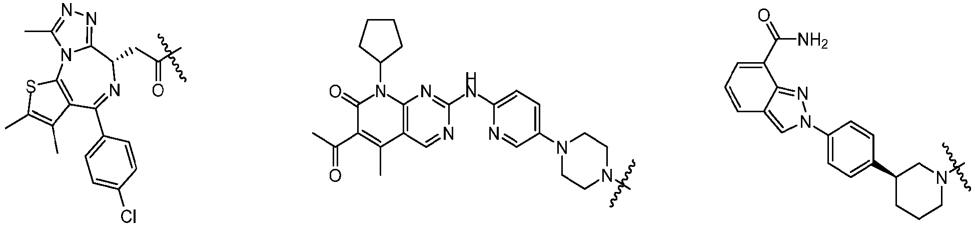 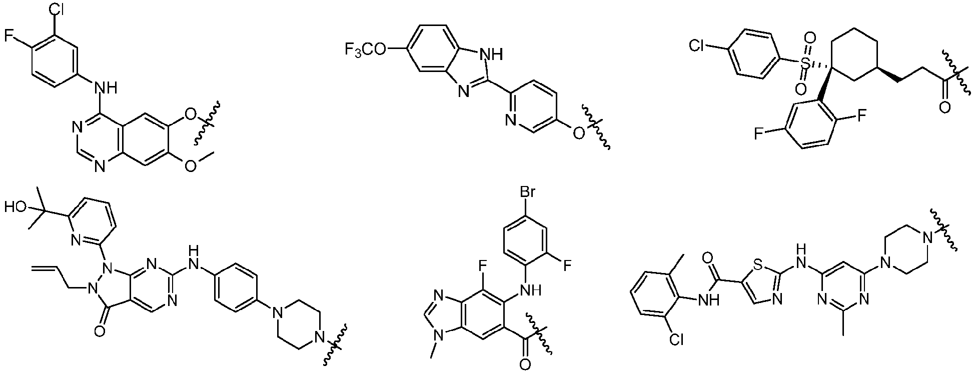 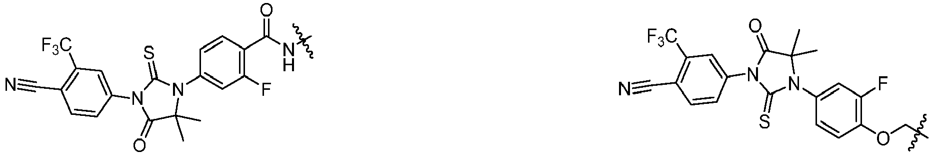 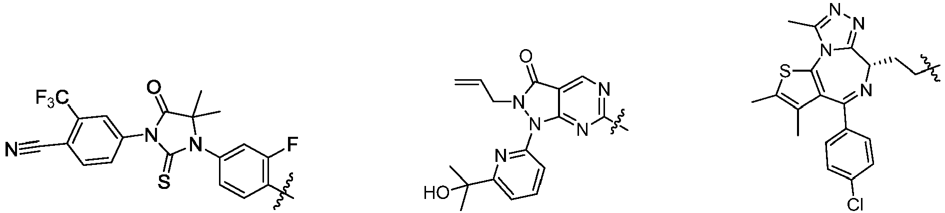 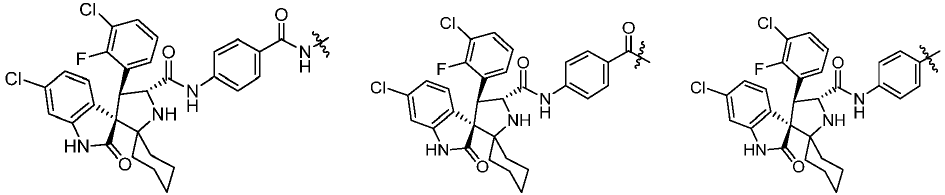 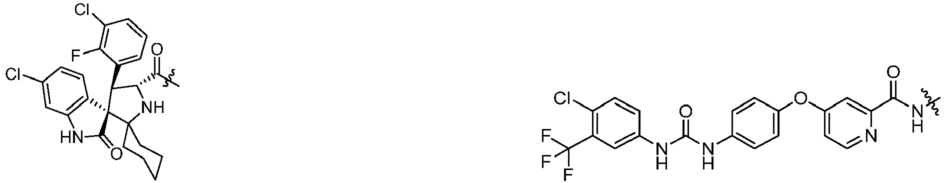 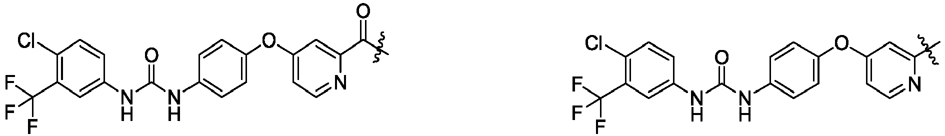 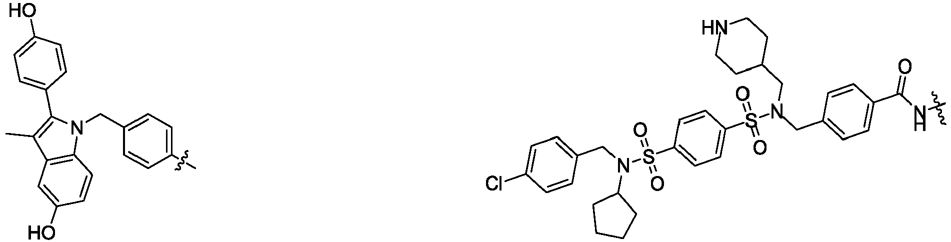 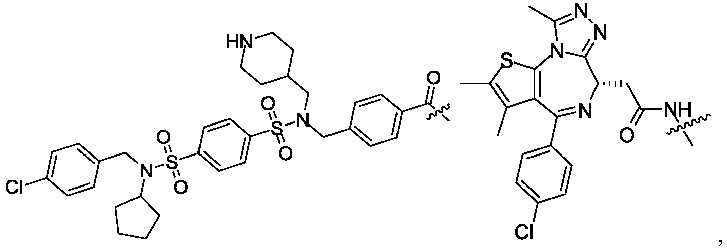 and other variables are as defined in this disclosure.

In some embodiments of the present disclosure, 1, 2 or 3 CH₂ on the L are replaced by cyclopropyl; 1, 2, 3, 4, 5 or 6 CH₂ on the L are optionally replaced by an atom or group selected from -NH-, =N-, -O-, -S-, -C(=O)-, -C(=O)O-, -NHC(=O)-, -NHC(=O)O-, -NHC(=O)NH-, -S(=O)-, -S(=O)₂-, -S(=O)₂NH-, =NO-, -P(=O)(OH)-, -P(=O)(R)-, -P(=O)(NHR)-, -P(=O)(NR₂)-, -P(=O)(R)NH-, C₂₋₄ alkenyl, C₂₋₄ alkynyl, C₆₋₁₂ aryl, 5- to 12-membered heteroaryl, C₃₋₁₄ cycloalkyl, and 3- to 14-membered heterocycloalkyl; L is optionally substituted with 1, 2, 3, 4, 5 or 6 R, wherein R is selected from H, F, Cl, Br, I, OH, NH, CN, C₁₋₃ alkyl, C₆₋₁₂ aryl and C₅₋₁₀ heteroaryl; and other variables are as defined in this disclosure.

In some embodiments of the present disclosure, the L is selected from structures represented by formulae (II-5), (II-6), and (IV-1) 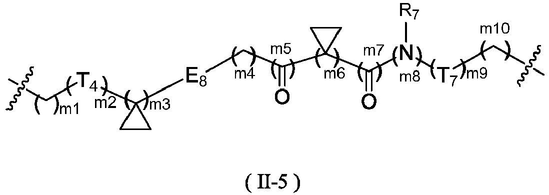 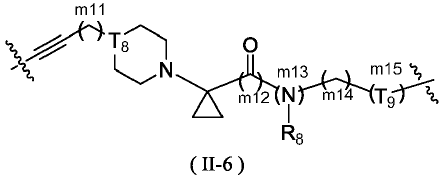 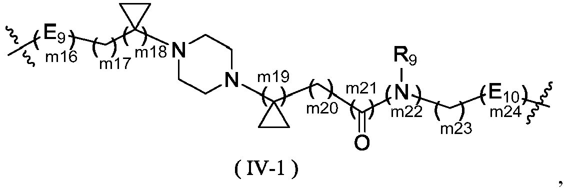 wherein
E₈ is selected from 3- to 8-membered monoheterocycloalkyl, 5- to 14-membered bridged heterocycloalkyl and 5- to 14-membered spiroheterocycloalkyl;
E₉, and E₁₀ are each independently selected from O and NH;
T₄, T₇, Ta, and T₉ are each independently selected from CH and N;
R₇, R₈, and R₉ are each independently selected from H and C₁₋₃ alkyl;
m2, m3, m5, m6, m7, m8 and m9 are each independently selected from 0 or 1;
m1, m4 and m10 are each independently selected from 0 to 15;
and at least one of m1, m2, m3, m4, m5, m6, m7, m8, m9, and m10 is not 0;
and at least one of m3 and m6 is 1;
m12 and m13 are each independently selected from 0 or 1;
m11, m14 and m15 are each independently selected from 0 to 15;
and at least one of m11, m12, m13, m14, and m15 is not 0;
m17, m20 and m23 are each independently selected from 0 to 15;
m16, m18, m19, m21, m22 and m24 are each independently selected from 0 or 1;
and at least one of m16, m17, m18, m19, m20, m21, m22, m23, and m24 is not 0;
and at least one of m18 and m19 is 1.

In some embodiments of the present disclosure, the L is selected from the structure represented by formula (IV-1-1) 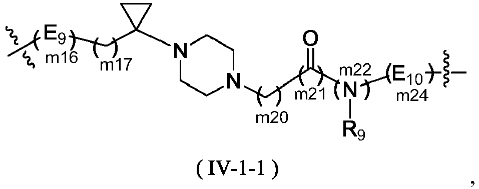 wherein
E₉, and E₁₀ are each independently selected from O and NH;
R₉ is selected from H and CH₃;
m16 is selected from 0 or 1;
m17 is selected from 0, 1, 2, or 3;
m20 is selected from 0, 1, 2, or 3;
m21 and m22 are each independently selected from 0 or 1;
m24 is selected from 0 or 1.

In some embodiments of the present disclosure, the L is selected from structures represented by formulae (I-4), (I-5), (I-6), (II-7), (II-8), (IV-2), (P-1), and (P-2) 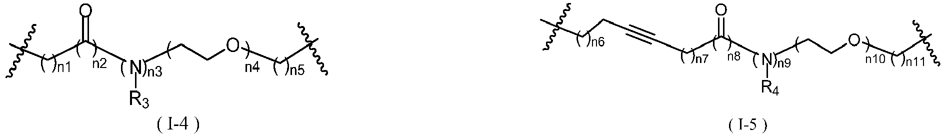 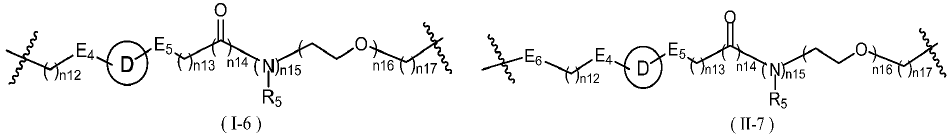 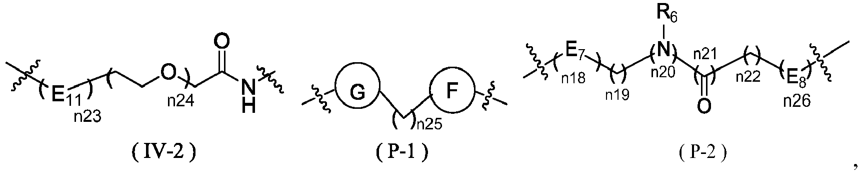 wherein
R₃, R₄, R₅, and R₆ are each independently selected from H and C₁₋₃ alkyl;
n1, n4 and n5 are each independently selected from 0 to 15, n2 and n3 are each independently selected from 0 or 1, and at least one of n1, n2, n3, n4 and n5 is not 0;
n6, n7, n10 and n11 are each independently selected from 0 to 15, n8 and n9 are each independently selected from 0 or 1, and at least one of n6, n7, n8, n9, n10 and n11 is not 0;
n12, n13, n16, and n17 are each independently selected from 0 to 15, n14 and n15 are each independently selected from 0 or 1, and at least one of n12, n13, n14, n15, n16, and n17 is not 0;
n19 and n22 are each independently selected from 0 to 15, n18, n20 and n21 are each independently selected from 0 or 1, and at least one of n18, n19, n20, n21 and n22 is not 0;
E₄ and E₅ are each independently selected from a bond, O, NH, and S(=O)₂;
E₆ and E₇ are each independently selected from O and NH; E₈ is selected from O and NH;
Ring D is selected from phenyl, piperidinyl, piperazinyl, 1,2,3-triazolyl, cyclobutyl and azetidinyl;
E₁₁ is selected from O and NH;
n23 is selected from 0 or 1, n24 is selected from 0 to 15, and at least one of n23 and n24 is not 0;
Ring F and ring G are each independently selected from piperidinyl and piperazinyl;
n25 is selected from 1 to 15;
n26 is selected from 0 and 1.

In some embodiments of the present disclosure, the L is selected from structures represented by formulae (I-4-1), (I-4-3), (I-4-4), (I-4-5), (I-4-6), (II-I-4-7), (IV-2-1), (P-1-4-1), (P-II-8-1), (P-II-8-2), (P-II-8-3), (P-2-1), (P-1-4-2), (P-2-2), and (P-2-3), 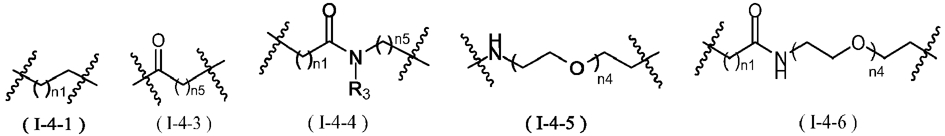 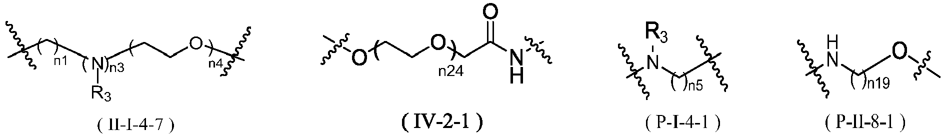 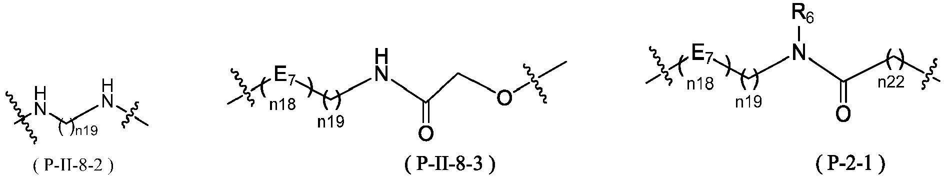 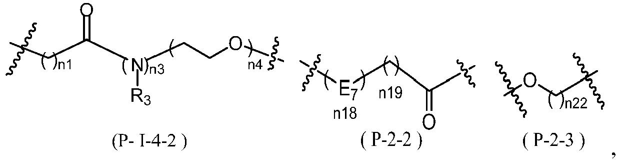 wherein
R₃ is selected from H, CH₃, CH₂CH₃ and CH(CH₃)₂;
R₆ is selected from H and CH₃;
n1 is selected from 0, 1, 2, 3, 4, 5, 6, 7, and 8;
n3 is selected from 0 and 1;
n4 is selected from 0, 1, 2, 3, and 4;
n5 is selected from 0, 1, 2, 3, 4, 5, 6, 7, and 8;
n18 is selected from 0 and 1;
n19 is selected from 0, 1, 2, 3, 4, 5, 6, 7, and 8;
n20 is selected from 0 and 1;
n21 is selected from 0 and 1;
n22 is selected from 0, 1, 2, 3, 4, 5, 6, 7, and 8;
n24 is selected from 0, 1, 2, 3, and 4.

In some embodiments of the present disclosure, the L is selected from structures represented by formulae (I-5-1), (I-5-2), (I-5-3), and (I-5-4), 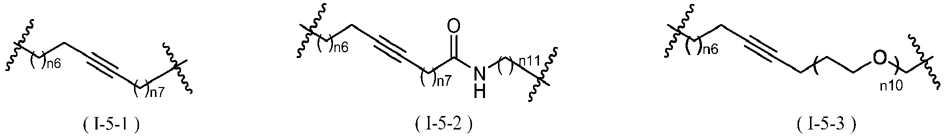 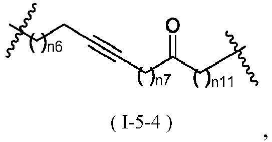 wherein n6, n7, n10 and n11 are as defined in this disclosure.

In some embodiments of the present disclosure, the L is selected from structures represented by formulae (I-6-1), (I-6-2), (I-6-3), (I-6-4), (I-6-5), (I-6-6), (I-6-7), (I-6-8), (II-7-1), (IV-II-7-1), and (P-1-1), 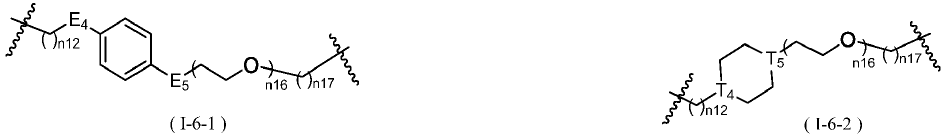 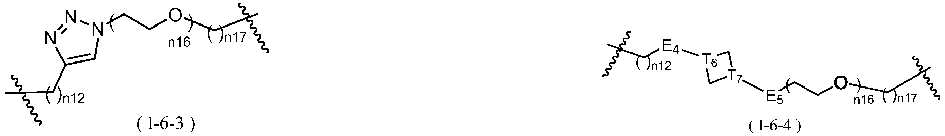 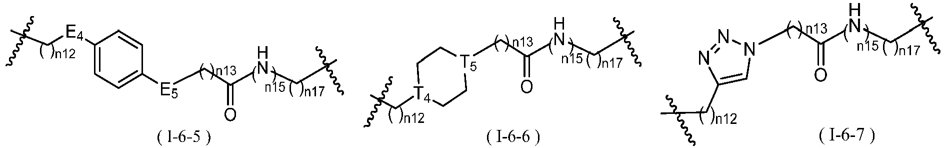 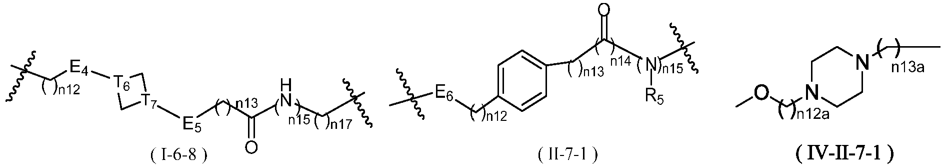 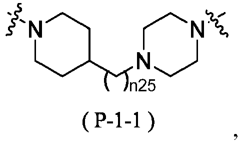 wherein
T₄ and T₅ are each independently selected from CH and N, and one of them must be N;
T₆ and T₇ are each independently selected from CH and N, and one of them must be C;
E₄, E₅, n12, n13, n15, n16, and n17 are as defined in this disclosure;
n12a is selected from 1, 2 and 3;
n12b is selected from 0, 1, 2, 3, and 4;
n25 is selected from 1, 2, 4 and 5.

In some embodiments of the present disclosure, the L is selected from NH, 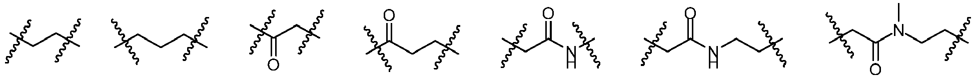 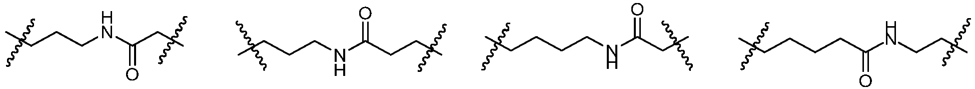 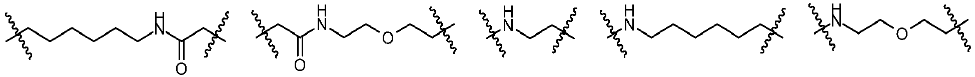 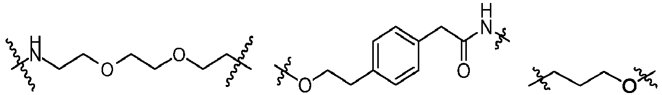 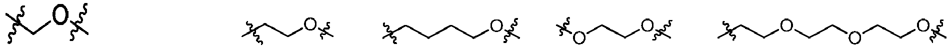 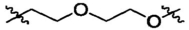 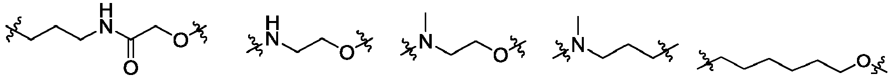 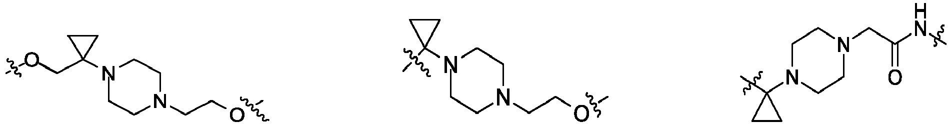 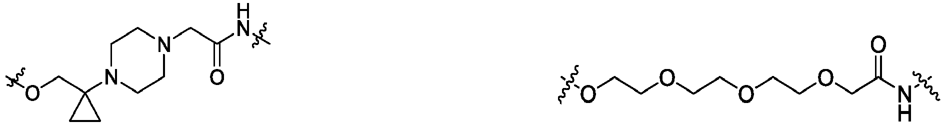 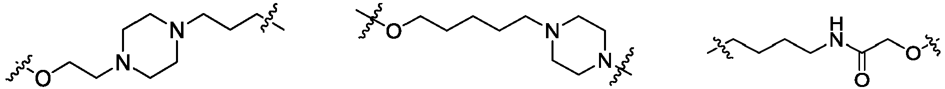 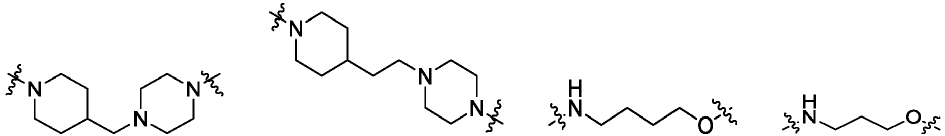 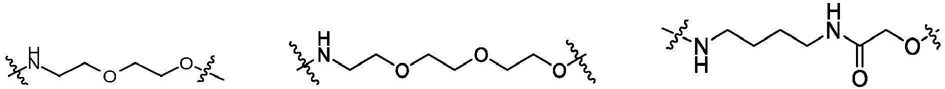 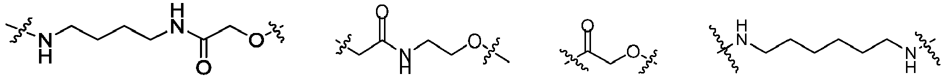 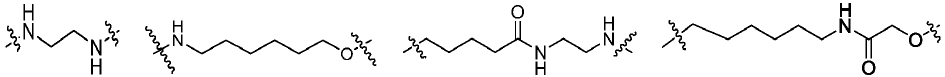 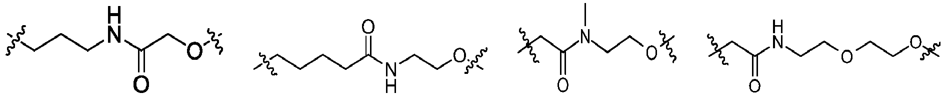 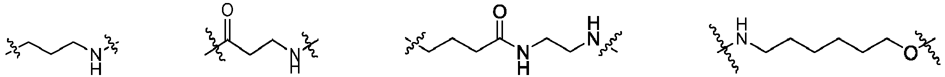 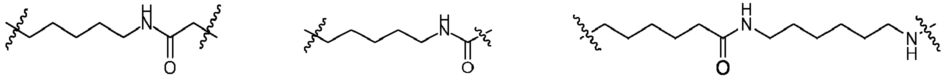 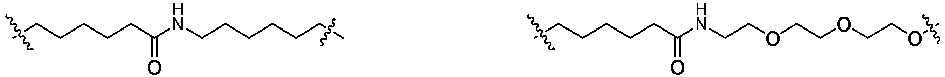 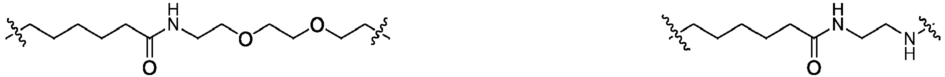 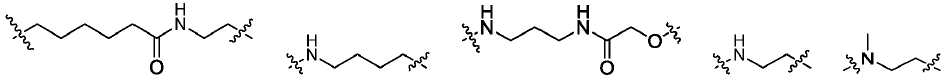 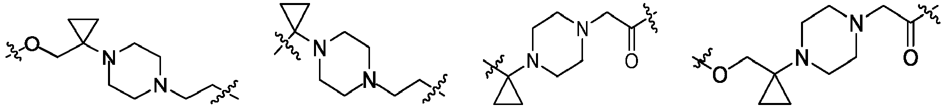 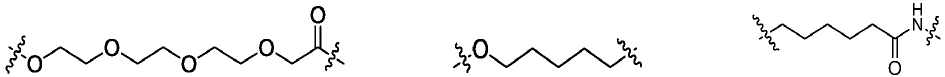 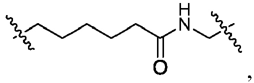 and other variables are as defined in this disclosure.

The present disclosure provides a compound of formula (I) or a pharmaceutically acceptable salt thereof,

PTM-L-ULM (I)

wherein
PTM is selected from a drug that binds to a targeted protein or a derivative thereof;
L is a chain connecting PTM and ULM;
ULM is selected from structures represented by formulae (III-1) and (III-2), 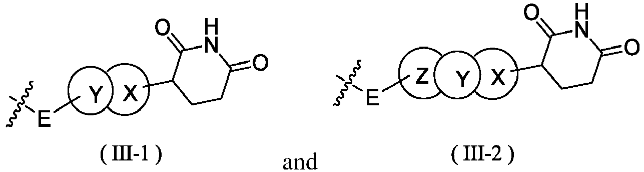
E is selected from a bond, -CH₂-, -NR₁-, -O-, -S-, -S(=O)-, -S(=O)₂-, -C(=O), and -C(=O)NR₂-;
R₁ and R₂ are each independently selected from H and C₁₋₃ alkyl;
Ring X, ring Y, and ring Z are each independently selected from phenyl, thienyl, furyl, triazolyl, oxazolyl, isoxazolyl, pyrrolyl, and pyridyl.

In some embodiments of the present disclosure, ULM in the compound or a pharmaceutically acceptable salt thereof is selected from structures represented by formulae (II-11), (11-12), (II-13), (II-1), (II-2), (II-3), (II-4), (III-21), (III-22), (III-23), and (III-24), 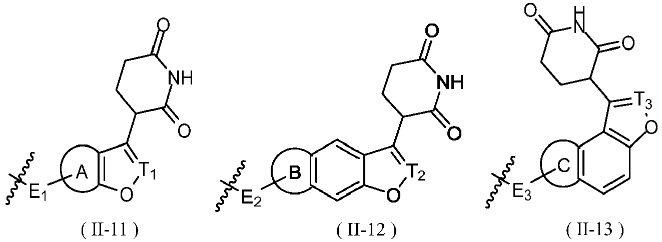 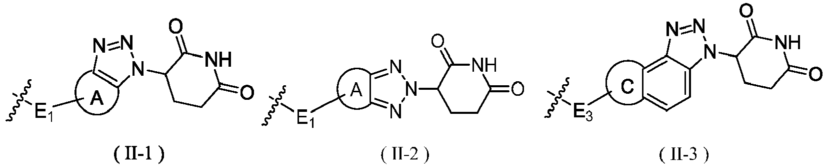 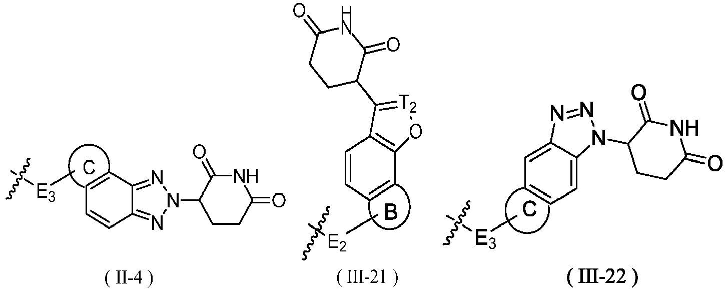 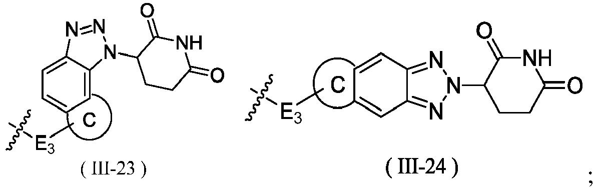
T₁, T₂, and T₃ are each independently selected from CH and N;
E₁, E₂, and E₃ are each independently selected from a bond, -CH₂-, -NR₁-, -O-, -S-, -S(=O)-, -S(=O)₂-, -C(=O), and -C(=O)NR₂-;
R₁ and R₂ are each independently selected from H and C₁₋₃ alkyl;
Ring A, ring B, and ring C are each independently selected from phenyl, thienyl, furyl, pyrrolyl, and pyridyl.

In some embodiments of the present disclosure, the ring A is selected from phenyl and thienyl, and other variables are as defined in this disclosure.

In some embodiments of the present disclosure, the ULM is selected from structures represented by formulae (II-11-1), (II-11-2), (II-1-1), and (II-2-1) 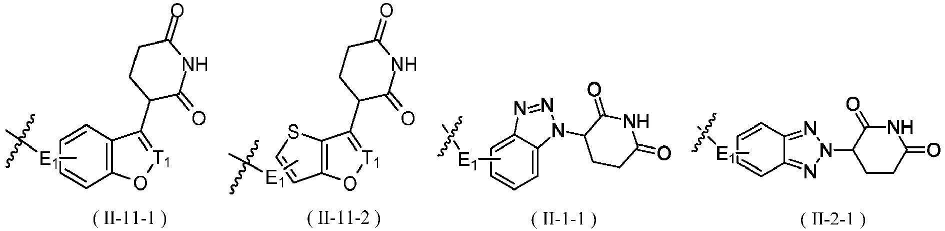 wherein T₁, and E₁ are as defined in this disclosure.

In some embodiments of the present disclosure, the ring B is selected from phenyl, and other variables are as defined in this disclosure.

In some embodiments of the present disclosure, the ULM is selected from structures represented by formulae (II-12-1) and (III-21-1) 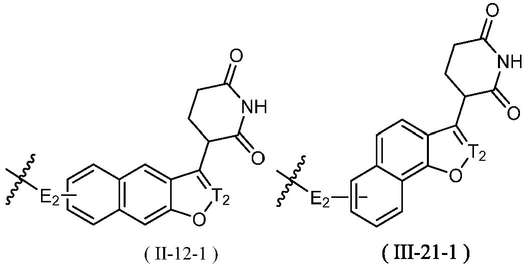 wherein T₂, and E₂ are as defined in this disclosure.

In some embodiments of the present disclosure, the ring C is selected from phenyl, and other variables are as defined in this disclosure.

In some embodiments of the present disclosure, the ULM is selected from structures represented by formulae (II-13-1), (II-3-1), (II-3-2), (III-22-1), (III-23-1), and (III-24-1) 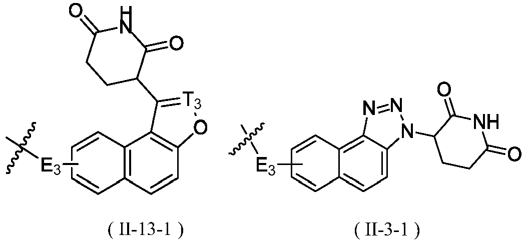 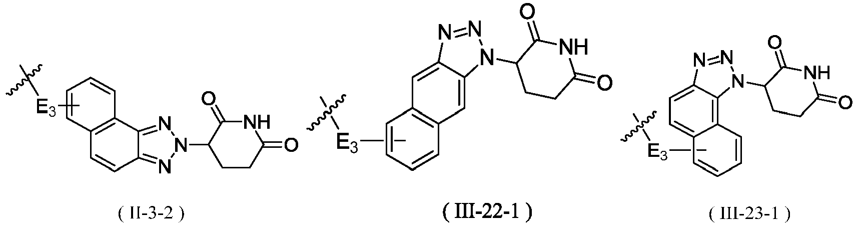 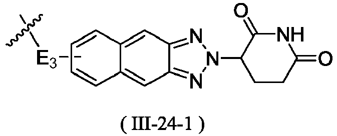 wherein T₃, and E₃ are as defined in this disclosure.

In some embodiments of the present disclosure, the ULM is selected from

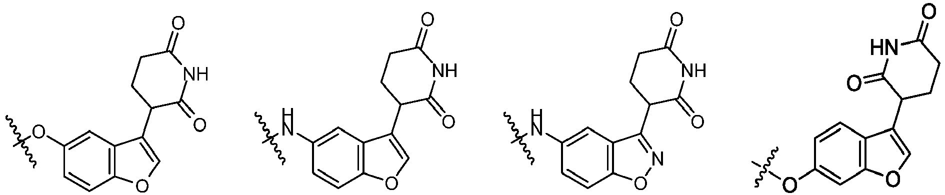

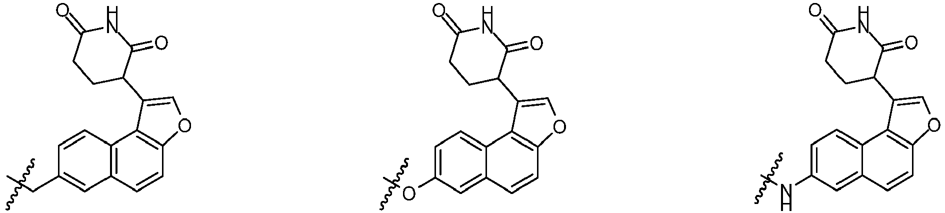

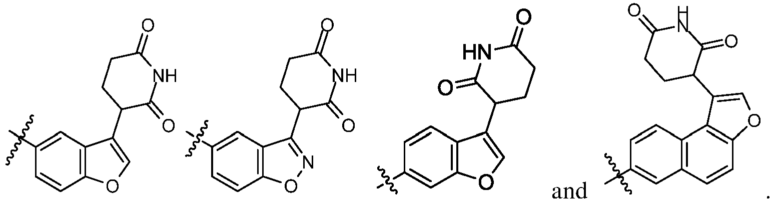

In some embodiments of the present disclosure, the PTM is selected from drugs that act on ALK, BET, CDK, PARP, EGFR, γ-secretase, CBFβ-SMMHC, WEE1, MEK, BCR-ABL, MET, RAS, BTK, VEGFR, JAK, HER2, HDAC, Akt, PI3K, mTOR, AR, ER, SRC, MDM2, RAF, IRAK4, STAT3, and c-Myc, or derivatives thereof, and other variables are as defined in this disclosure.

In some embodiments of the present disclosure, the PTM is selected from drugs that act on ALK, BRD4, CDK4/6, CDK8, CDK9, PARP, EGFR, γ-secretase, CBFβ-SMMHC, WEE1, MEK, BCR-ABL, MET, KRAS, BTK, VEGFR, HER2, HDAC, Akt, PI3K, mTOR, AR, ER, SRC, JAK, MDM2, and RAF, or derivatives thereof, and other variables are as defined in this disclosure.

In some embodiments of the present disclosure, the PTM is selected from drugs that act on ALK, BRD4, CDK4/6, PARP, EGFR, γ-secretase, CBFβ-SMMHC, WEE1, MEK, BCR-ABL, MET, KRAS, EGFR, BTK, AR, ER, JAK, MDM2, and RAF, or derivatives thereof, and other variables are as defined in this disclosure.

In some embodiments of the present disclosure, the PTM is selected from 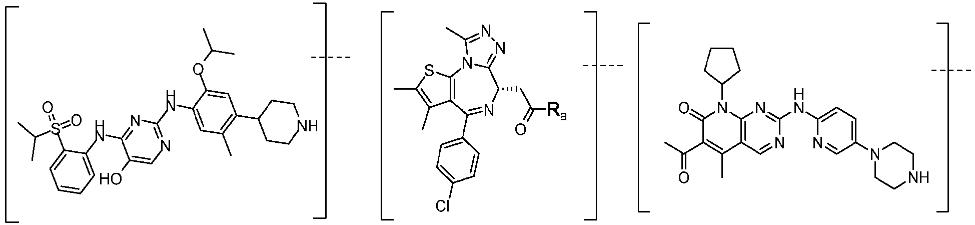 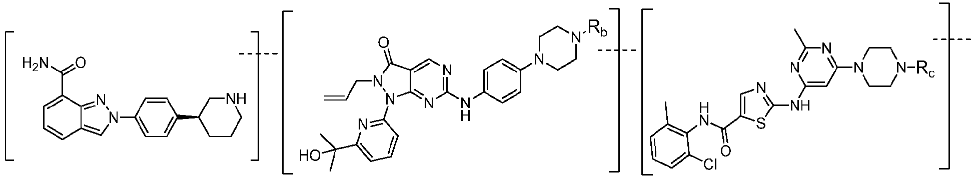 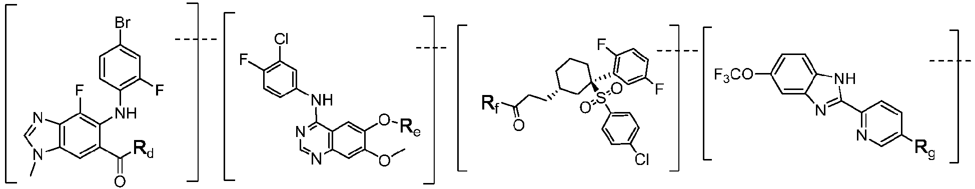 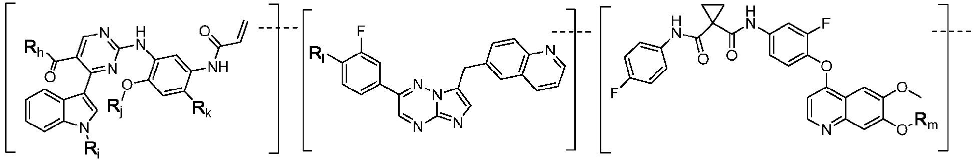 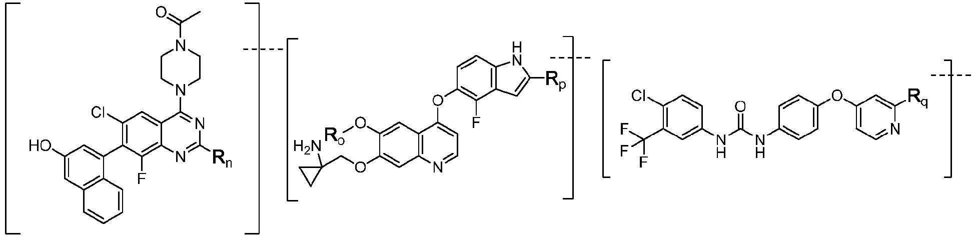 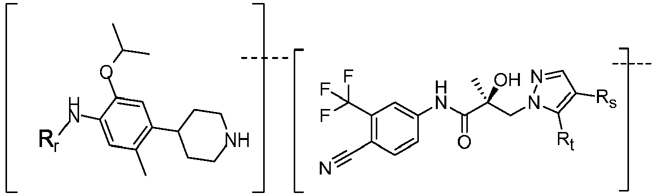 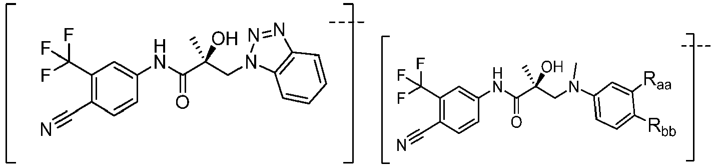 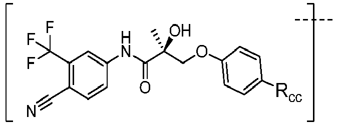 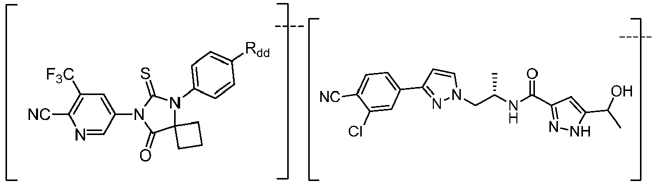 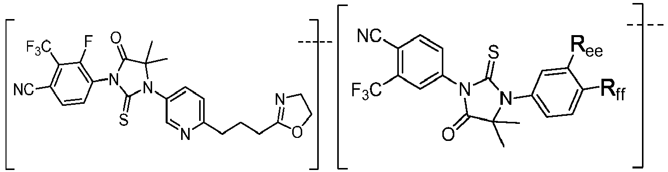 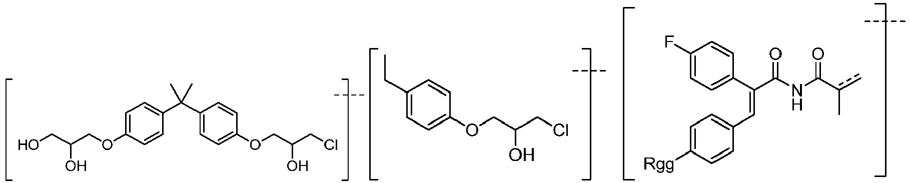 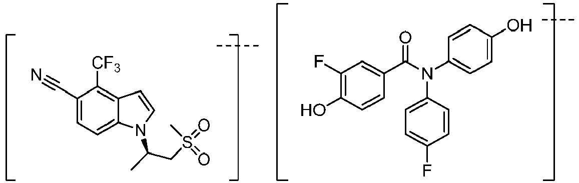 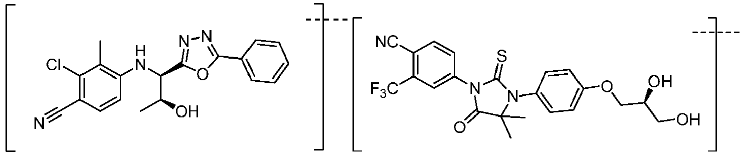 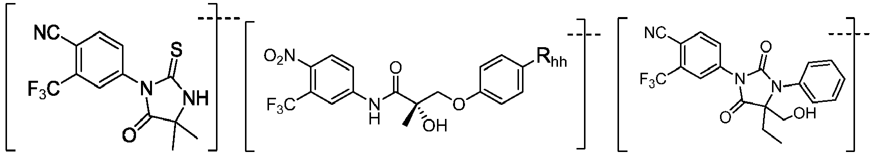 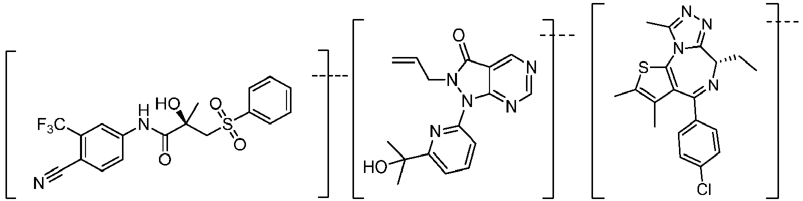 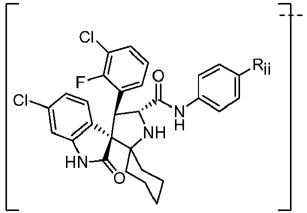 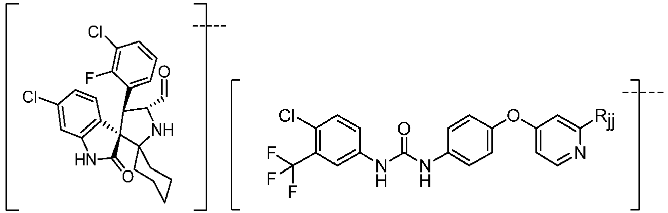 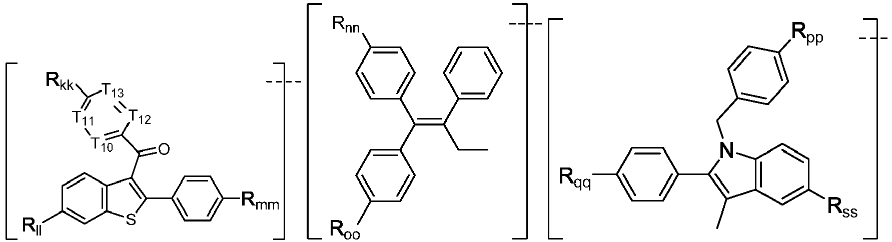 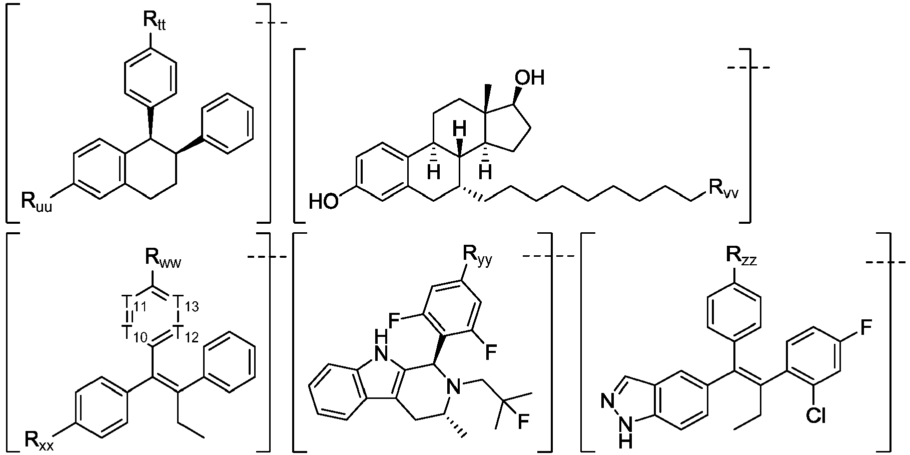 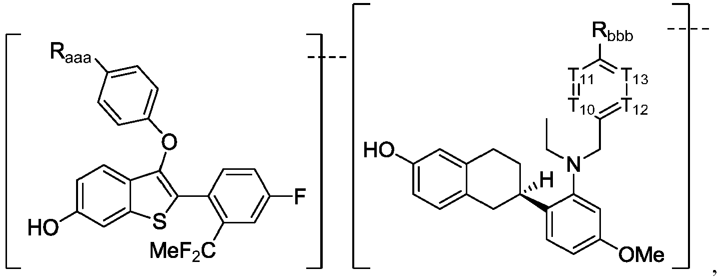 wherein 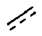
is selected from a single bond and a double bond;
T₁₀, T₁₁, T₁₂, and T₁₃ are each independently selected from N and CR_{ccc}, and at most two of T₁₀, T₁₁, T₁₂, and T₁₃ are selected from N;
Rₐ is selected from H and 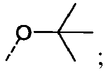
R_{b} is selected from H and CH₃;
R_{c} is selected from H and 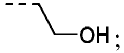
R_{d} is selected from H, NH₂ and 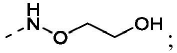
Rₑ is selected from H and 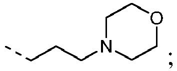
R_{f} is selected from H and OH;
R_{g} is selected from H and OH;
Rₕ is selected from H and 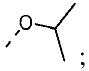
Rᵢ is selected from H and CH₃;
Rⱼ is selected from H and CH₃;
Rₖ is selected from H, NH₂, NHCH₃ and 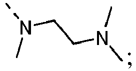
Rₗ is selected from H, 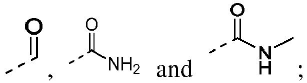
Rₘ is selected from H and 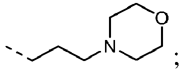
Rₙ is selected from H, NH₂, NHCH₂CH₃ and 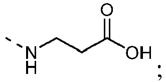
Rₒ is selected from H and CH₃;
Rₚ is selected from H and CH₃;
R_{q} is selected from H, 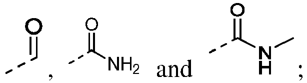
Rᵣ is selected from H and 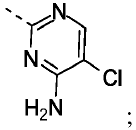
Rₛ is selected from H, F and Cl;
Rₜ is selected from H and Br;
Rₐₐ is selected from H and phenyl;
R_{bb}, and R_{cc} are each independently selected from H and CN;
R_{dd}, Rεε, Rₕₕ, Rᵢᵢ, and Rⱼⱼ are each independently selected from H, OCH₃, 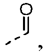 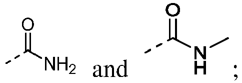
Rₑₑ is selected from H and F;
R_{gg} is selected from H and Cl;
Rₖₖ is selected from H, OH and 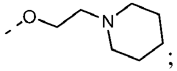
Rₗₗ, and Rₘₘ are each independently selected from H, F, Cl, Br, I, OH, and OCH₃;
Rₙₙ is selected from H, OH and 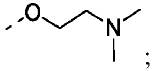
Rₒₒ is selected from H and OH;
Rₚₚ is selected from H, OH and 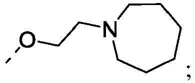
R_{qq}, and Rₛₛ are each independently selected from H, F, Cl, Br, I, OH, and OCH₃;
is selected from H, OH and 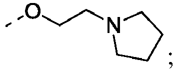
Rᵤᵤ is selected from H, F, Cl, Br, I, OH, and OCH₃;
Rᵥᵥ is selected from H and 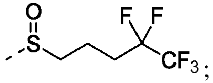
R_{ww} is selected from H and 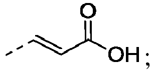
Rₓₓ is selected from H and OH;
R_{yy}, R_{zz}, and Rₐₐₐ are each independently selected from H and 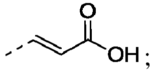
R_{bbb} is selected from H and 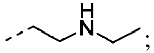
R_{ccc} is selected from H, F, Cl, Br, and I;
other variables are as defined in this disclosure.

In some embodiments of the present disclosure, the PTM is selected from

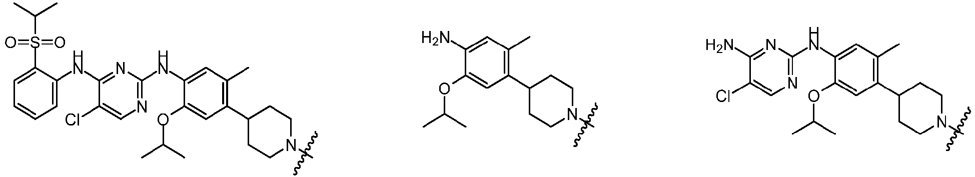

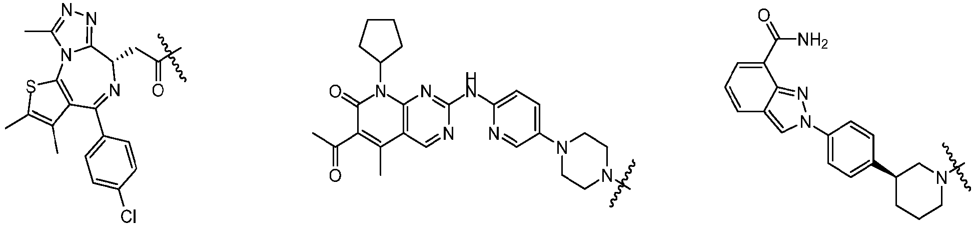

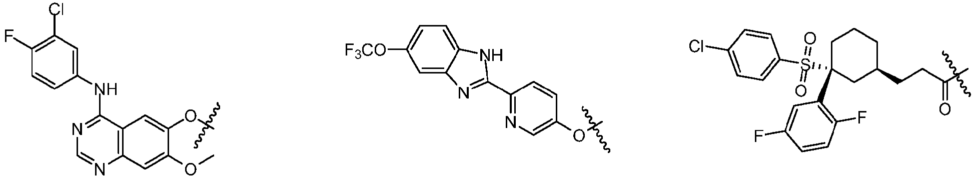

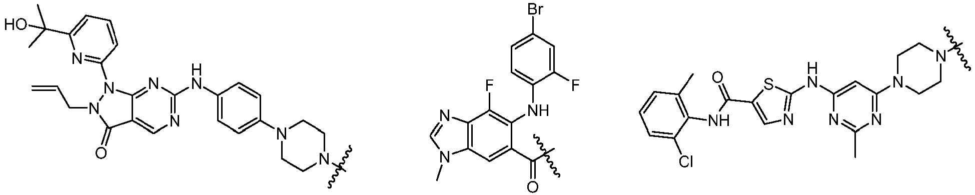

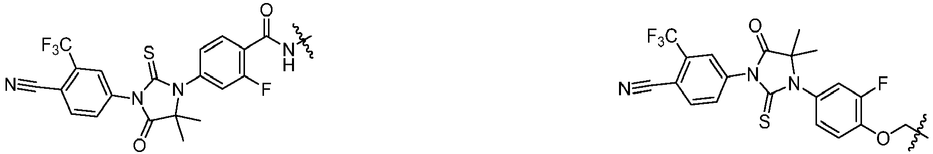

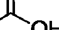

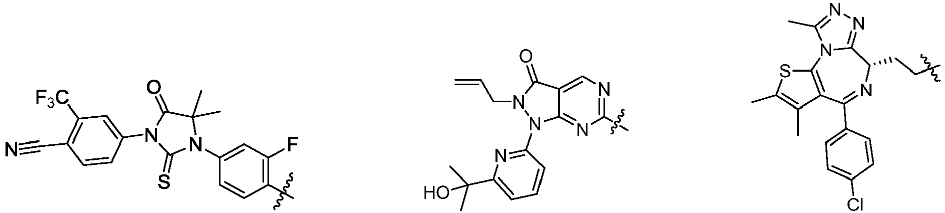

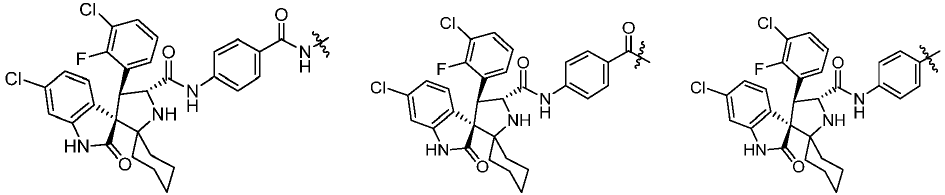

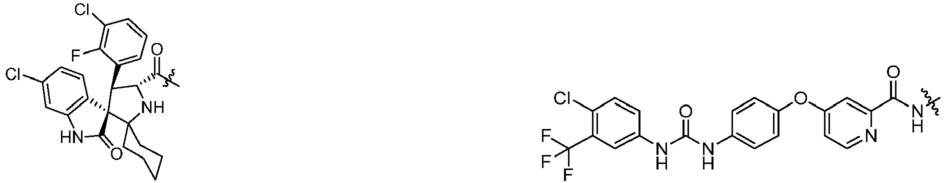

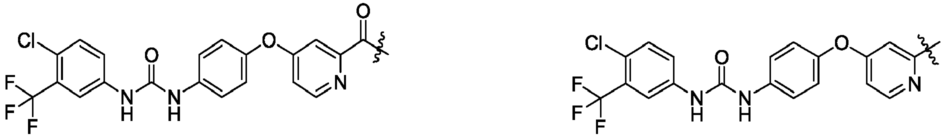

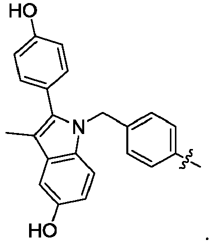

In some embodiments of the present disclosure, the L is selected from C₁₋₂₀ alkyl; 1, 2 or 3 CH₂ on the L are replaced by cyclopropyl; 1, 2, 3, 4, 5 or 6 CH₂ on the L are optionally replaced by an atom or group selected from -NH-, =N-, -O-, -S-, -C(=O)-, -C(=O)O-, -NHC(=O)-, -NHC(=O)O-, -NHC(=O)NH-, -S(=O)-, -S(=O)₂-, -S(=O)₂NH-, =NO-, -P(=O)(OH)-, -P(=O)(R)-, -P(=O)(NHR)-, -P(=O)(NR₂)-, -P(=O)(R)NH-, C₂₋₄ alkenyl, C₂₋₄ alkynyl, C₆₋₁₂ aryl, 5- to 12-membered heteroaryl, C₃₋₁₄ cycloalkyl, and 3- to 14-membered heterocycloalkyl; L is optionally substituted with 1, 2, 3, 4, 5 or 6 R, wherein R is selected from H, F, Cl, Br, I, OH, NH, CN, C₁₋₃ alkyl, C₆₋₁₂ aryl and C₅₋₁₀ heteroaryl; and other variables are as defined in this disclosure.

In some embodiments of the present disclosure, the L is selected from structures represented by formulae (II-5), (II-6), and (IV-1) 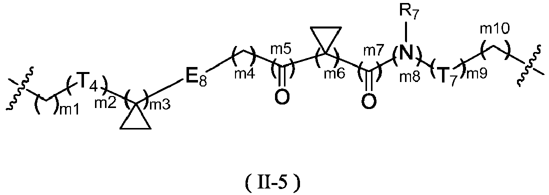 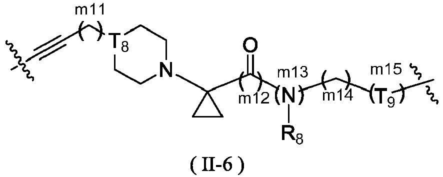 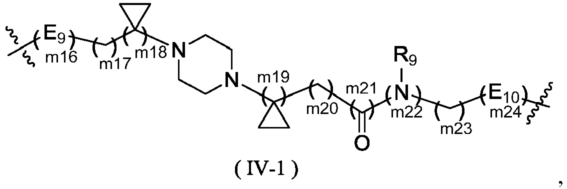 wherein
E₈ is selected from 3- to 8-membered monoheterocycloalkyl, 5- to 14-membered bridged cycloalkyl and 5- to 14-membered spirocycloalkyl;
E₉, and E₁₀ are each independently selected from O and NH;
T₄, T₇, Ta, and T₉ are each independently selected from CH and N;
R₇, R₈, and R₉ are each independently selected from H and C₁₋₃ alkyl;
m2, m3, m5, m6, m7, m8 and m9 are each independently selected from 0 or 1;
m1, m4 and m10 are each independently selected from 0 to 15;
and at least one of m1, m2, m3, m4, m5, m6, m7, m8, m9, and m10 is not 0;
and at least one of m3 and m6 is 1;
m12 and m13 are each independently selected from 0 or 1;
m11, m14 and m15 are each independently selected from 0 to 15;
and at least one of m11, m12, m13, m14, and m15 is not 0;
m17, m20 and m23 are each independently selected from 0 to 15;
m16, m18, m19, m21, m22 and m24 are each independently selected from 0 or 1;
and at least one of m16, m17, m18, m19, m20, m21, m22, m23, and m24 is not 0.

In some embodiments of the present disclosure, the L is selected from the structure represented by formula (IV-1-1) 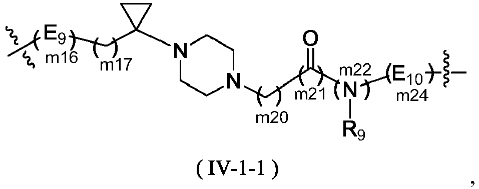 wherein
E₉, and E₁₀ are each independently selected from O and NH;
R₉ is selected from H and CH₃;
m16 is selected from 0 or 1;
m17 is selected from 0, 1, 2, or 3;
m20 is selected from 0, 1, 2, or 3;
m21 and m22 are each independently selected from 0 or 1;
m24 is selected from 0 or 1.

In some embodiments of the present disclosure, the L is selected from structures represented by formulae (I-4), (I-5), (I-6), (II-7), (II-8), and (IV-2) 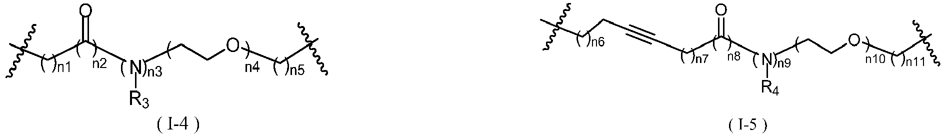 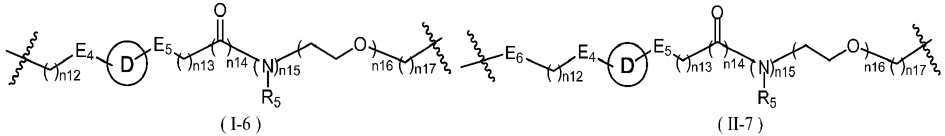 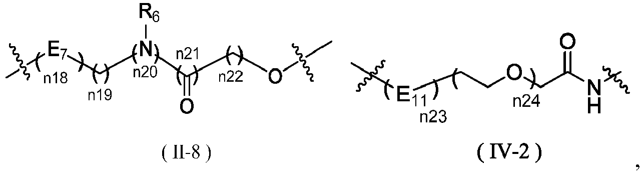 wherein
R₃, R₄, R₅, and R₆ are each independently selected from H and C₁₋₃ alkyl;
n1, n4 and n5 are each independently selected from 0 to 15, n2 and n3 are each independently selected from 0 or 1, and at least one of n1, n2, n3, n4 and n5 is not 0;
n6, n7, n10 and n11 are each independently selected from 0 to 15, n8 and n9 are each independently selected from 0 or 1, and at least one of n6, n7, n8, n9, n10 and n11 is not 0;
n12, n13, n16, and n17 are each independently selected from 0 to 15, n14 and n15 are each independently selected from 0 or 1, and at least one of n12, n13, n14, n15, n16, and n17 is not 0;
n19 and n22 are each independently selected from 0 to 15, n18, n20 and n21 are each independently selected from 0 or 1, and at least one of n18, n19, n20, n21 and n22 is not 0;
E₄ and E₅ are each independently selected from a bond, O, NH, and S(=O)₂;
E₆ and E₇ are each independently selected from O and NH;
Ring D is selected from phenyl, piperidinyl, piperazinyl, 1,2,3-triazolyl, cyclobutyl and azetidinyl;
E₁₁ is selected from O and NH;
n23 is selected from 0 or 1, n24 is selected from 0 to 15, and at least one of n23 and n24 is not 0.

In some embodiments of the present disclosure, the L is selected from structures represented by formulae (I-4-1), (I-4-2), (I-4-3), (I-4-4), (I-4-5), (I-4-6), (II-I-4-7), (II-8-1), and (IV-2-1), 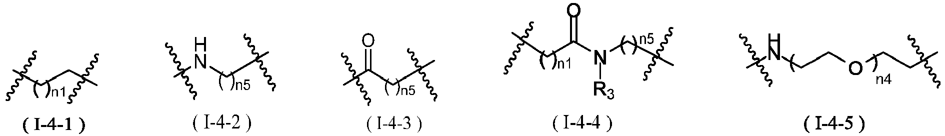 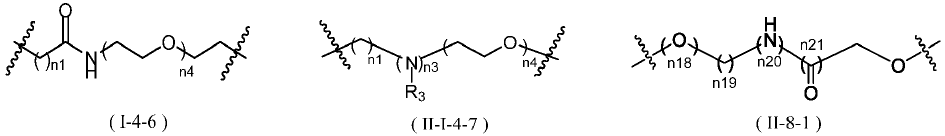 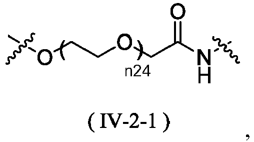 wherein
R₃ is selected from H, CH₃, CH₂CH₃ and CH(CH₃)₂;
n1 is selected from 0, 1, 2, 3, 4, 5, 6, 7, and 8;
n3 is selected from 0 and 1;
n4 is selected from 0, 1, 2, 3, and 4;
n5 is selected from 0, 1, 2, 3, 4, 5, 6, 7, and 8;
n18 is selected from 0 and 1;
n19 is selected from 0, 1, 2, 3, and 4;
n20 is selected from 0 and 1;
n21 is selected from 0 and 1;
n24 is selected from 0, 1, 2, 3, and 4.

In some embodiments of the present disclosure, the L is selected from structures represented by formulae (I-5-1), (I-5-2), (I-5-3), and (I-5-4), 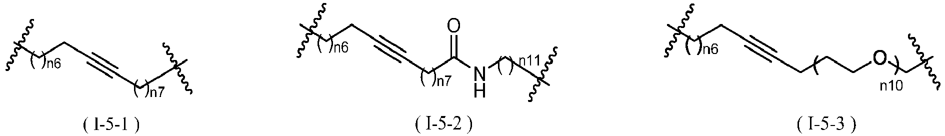 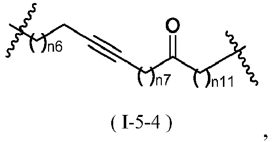 wherein n6, n7, n10 and n11 are as defined in this disclosure.

In some embodiments of the present disclosure, the L is selected from structures represented by formulae (I-6-1), (I-6-2), (I-6-3), (I-6-4), (I-6-5), (I-6-6), (I-6-7), (I-6-8), (11-7-1), and (IV-II-7-1), 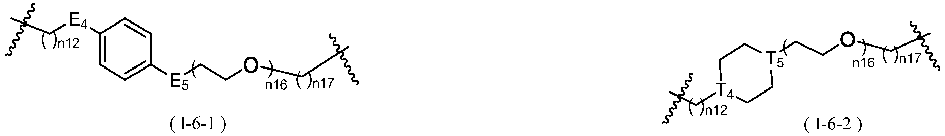 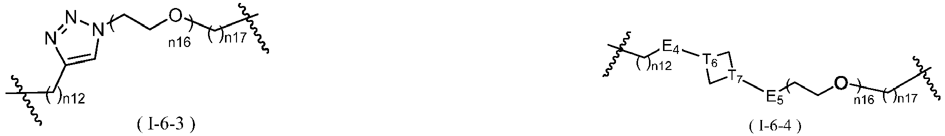 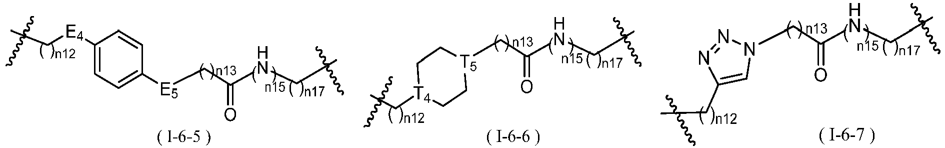 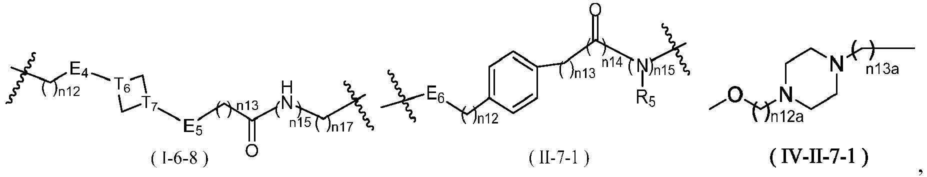 wherein
T₄ and T₅ are each independently selected from CH and N, and one of them must be N;
T₆ and T₇ are each independently selected from CH and N, and one of them must be C;
E₄, E₅, n12, n13, n15, n16, and n17 are as defined in this disclosure;
n12a is selected from 1, 2 and 3;
n12b is selected from 0, 1, 2, 3, and 4.

In some embodiments of the present disclosure, the L is selected from NH,

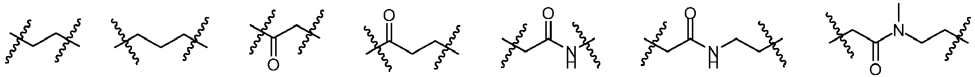

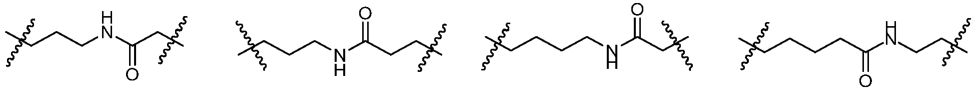

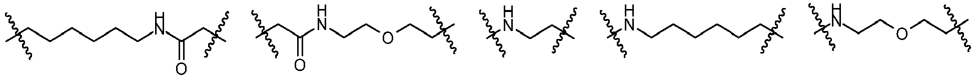

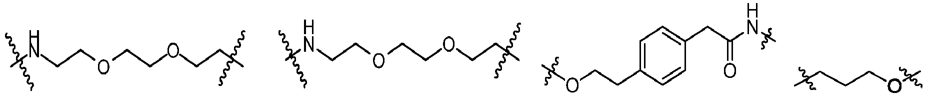

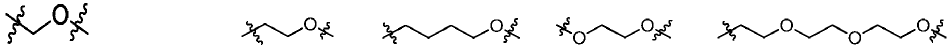

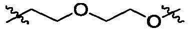

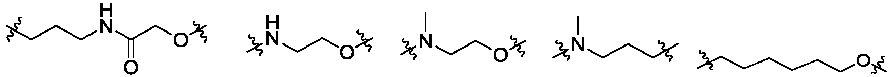

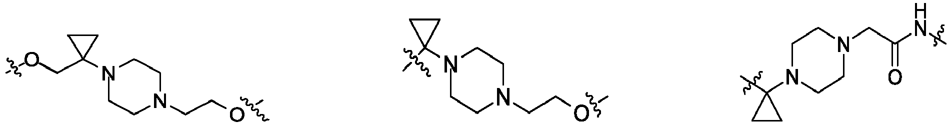

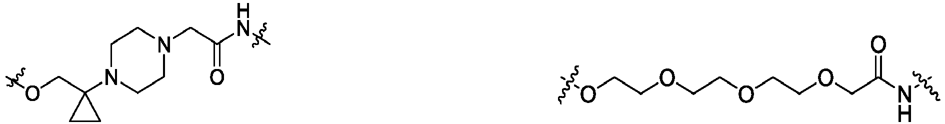

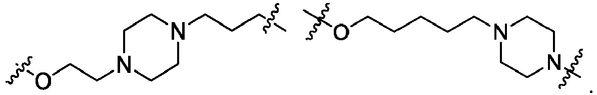

The present disclosure provides a compound of formula (I) or a pharmaceutically acceptable salt thereof,

PTM-L-ULM (I)

wherein
PTM is selected from a drug that binds to a targeted protein or a derivative thereof;
L is a chain connecting PTM and ULM;
ULM is selected from structures represented by formulae (III-1) and (III-2), 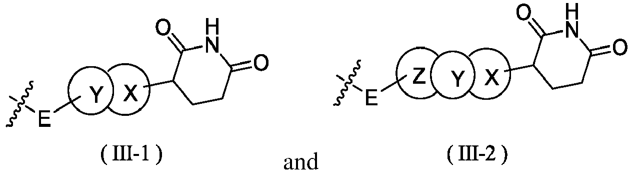
E is selected from a bond, -CH₂-, -NR₁-, -O-, -S-, -S(=O)-, -S(=O)₂-, -C(=O), and -C(=O)NR₂-;
R₁ and R₂ are each independently selected from H and C₁₋₃ alkyl;
Ring X, ring Y, and ring Z are each independently selected from phenyl, thienyl, furyl, triazolyl, oxazolyl, isoxazolyl, pyrrolyl, and pyridyl.

In some embodiments of the present disclosure, ULM in the compound or a pharmaceutically acceptable salt thereof is selected from structures represented by formulae (II-11), (11-12), (II-13), (II-1), (II-2), (II-3), (II-4), (III-21), (III-22), (III-23), and (III-24), 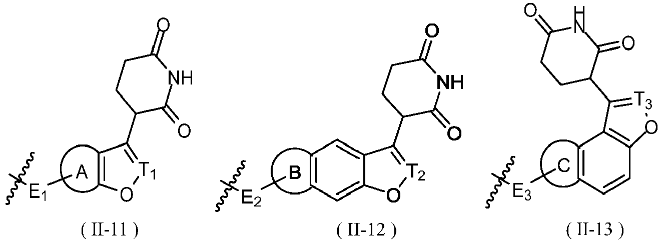 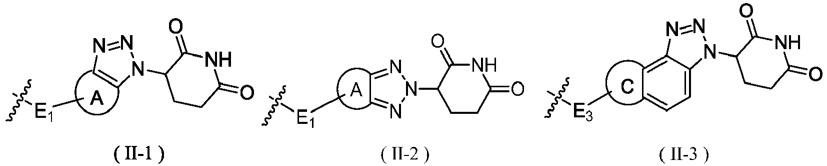 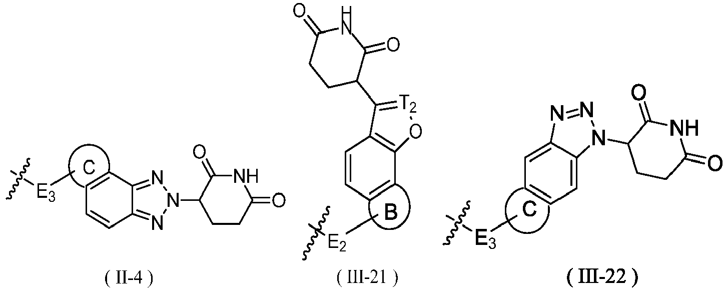 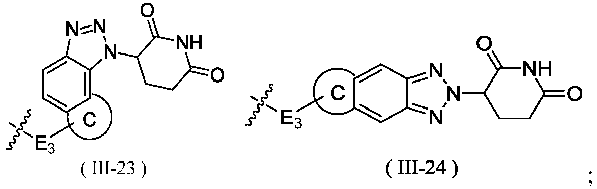
T₁, T₂, and T₃ are each independently selected from CH and N;
E₁, E₂, and E₃ are each independently selected from -CH₂-, -NR₁-, -O-, -S-, -S(=O)-, -S(=O)₂-, -C(=O), and -C(=O)NR₂-;
R₁ and R₂ are each independently selected from H and C₁₋₃ alkyl;
Ring A, ring B, and ring C are each independently selected from phenyl, thienyl, furyl, pyrrolyl, and pyridyl.

In some embodiments of the present disclosure, the ring A is selected from phenyl and thienyl, and other variables are as defined in this disclosure.

In some embodiments of the present disclosure, the ULM is selected from structures represented by formulae (II-11-1), (II-11-2), (II-1-1), and (II-2-1) 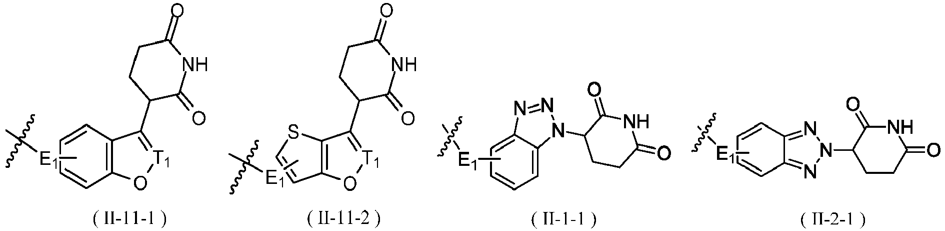 wherein T₁, and E₁ are as defined in this disclosure.

In some embodiments of the present disclosure, the ring B is selected from phenyl, and other variables are as defined in this disclosure.

In some embodiments of the present disclosure, the ULM is selected from structures represented by formulae (II-12-1), and (III-21-1) 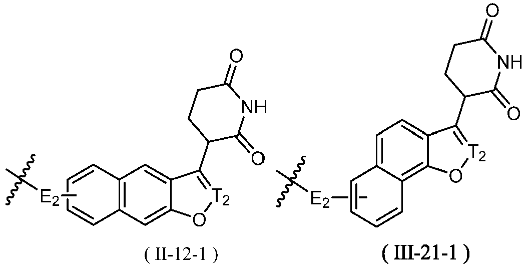 wherein T₂, and E₂ are as defined in this disclosure.

In some embodiments of the present disclosure, the ring C is selected from phenyl, and other variables are as defined in this disclosure.

In some embodiments of the present disclosure, the ULM is selected from structures represented by formulae (II-13-1), (II-3-1), (II-3-2), (III-22-1), (III-23-1), and (III-24-1) 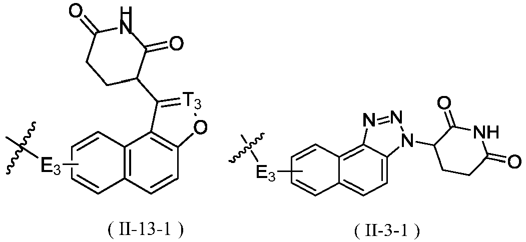 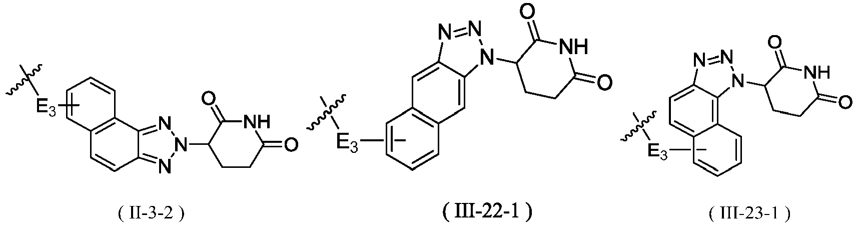 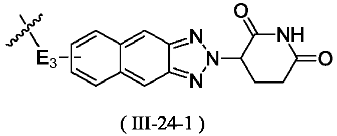 wherein T₃, and E₃ are as defined in this disclosure.

In some embodiments of the present disclosure, the ULM is selected from

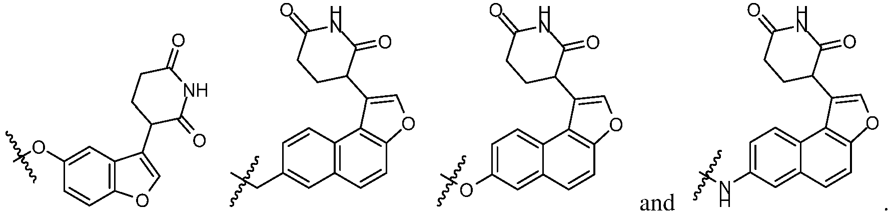

In some embodiments of the present disclosure, the PTM is selected from drugs that act on ALK, BET, CDK, PARP, EGFR, γ-secretase, CBFβ-SMMHC, WEE1, MEK, BCR-ABL, MET, RAS, BTK, VEGFR, JAK, HER2, HDAC, Akt, PI3K, mTOR, AR, ER, SRC, MDM2, and RAF, or derivatives thereof, and other variables are as defined in this disclosure.

In some embodiments of the present disclosure, the PTM is selected from drugs that act on ALK, BRD4, CDK4/6, CDK8, CDK9, PARP, EGFR, γ-secretase, CBFβ-SMMHC, WEE1, MEK, BCR-ABL, MET, KRAS, BTK, VEGFR, HER2, HDAC, Akt, PI3K, mTOR, AR, ER, SRC, JAK, MDM2, and RAF, or derivatives thereof, and other variables are as defined in this disclosure.

In some embodiments of the present disclosure, the PTM is selected from drugs that act on ALK, BRD4, CDK4/6, PARP, EGFR, γ-secretase, CBFβ-SMMHC, WEE1, MEK, BCR-ABL, MET, KRAS, EGFR, BTK, AR, JAK, MDM2, and RAF, or derivatives thereof, and other variables are as defined in this disclosure.

In some embodiments of the present disclosure, the PTM is selected from 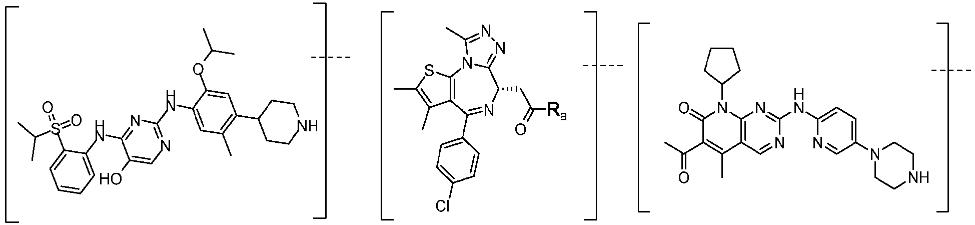 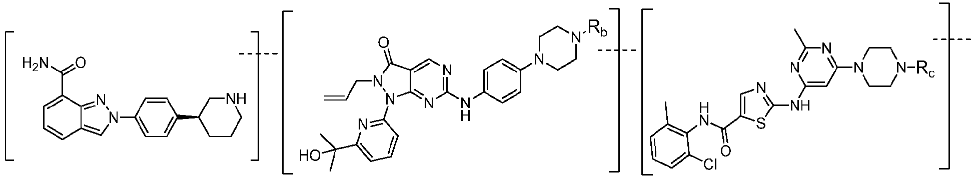 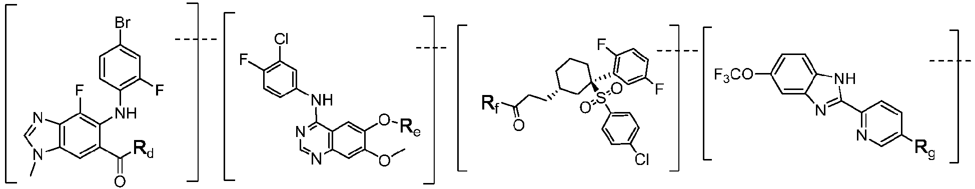 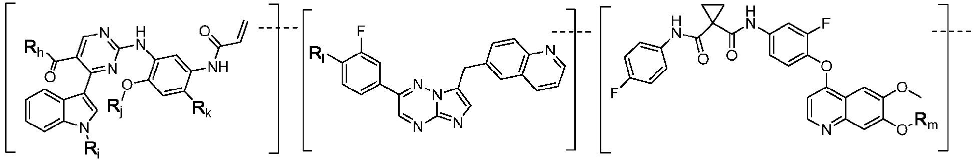 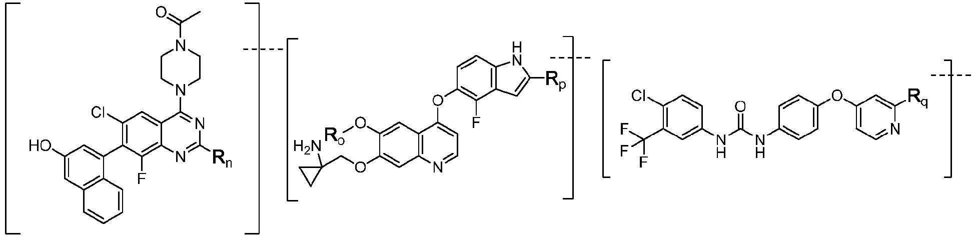 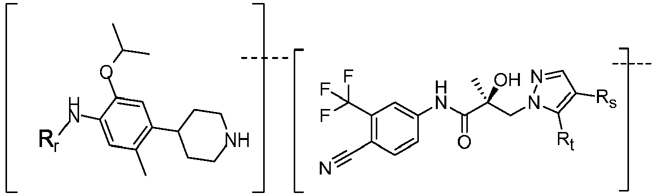 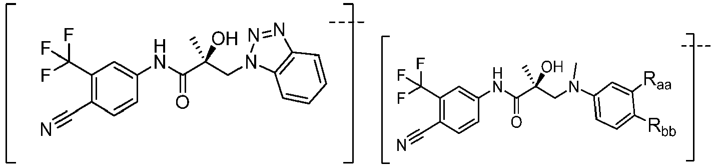 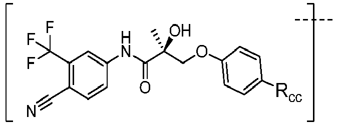 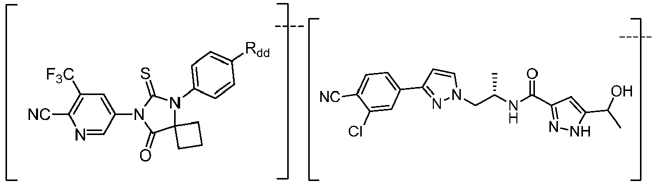 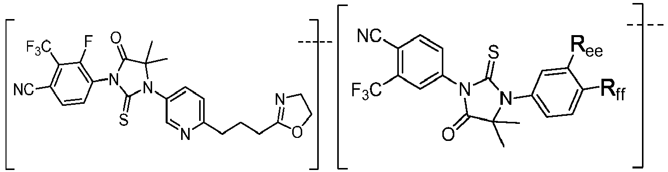 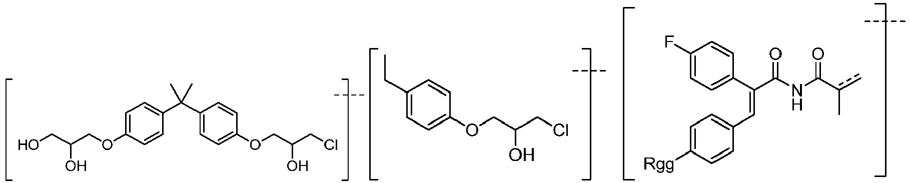 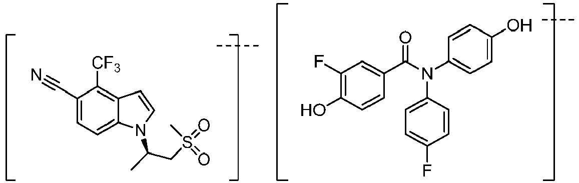 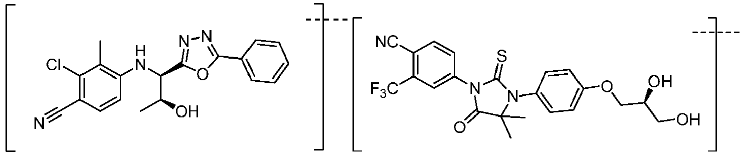 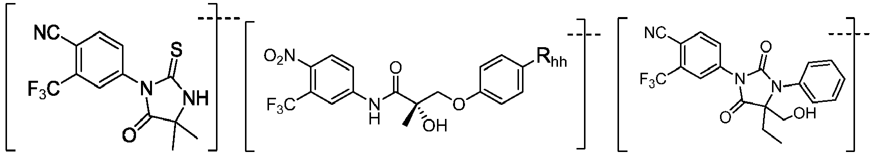 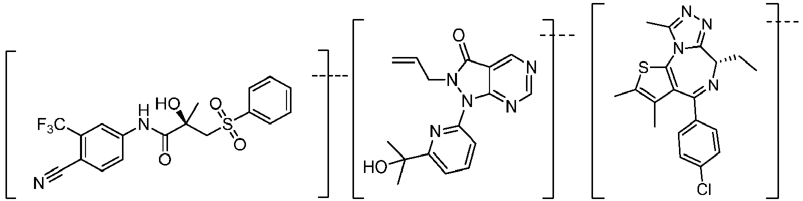 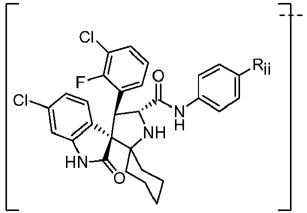 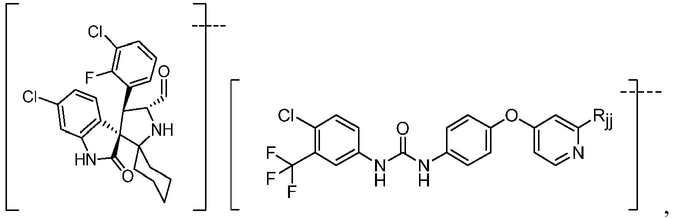 wherein
Rₐ is selected from H and 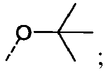
R_{b} is selected from H and CH₃;
R_{c} is selected from H and 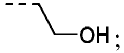
R_{d} is selected from H, NH₂ and 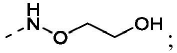
Rₑ is selected from H and 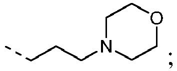
R_{f} is selected from H and OH;
R_{g} is selected from H and OH;
Rₕ is selected from H and 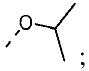
Rᵢ is selected from H and CH₃;
Rⱼ is selected from H and CH₃;
Rₖ is selected from H, NH₂, NHCH₃ and 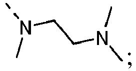
Rₗ is selected from H, 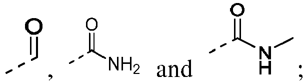
Rₘ is selected from H and 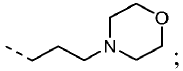
Rₙ is selected from H, NH₂, NHCH₂CH₃ and 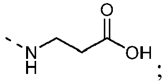
Rₒ is selected from H and CH₃;
Rₚ is selected from H and CH₃;
R_{q} is selected from H, 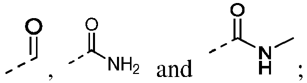
Rᵣ is selected from H and 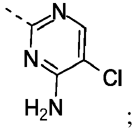
Rₛ is selected from H, F and Cl;
Rₜ is selected from H and Br;
Rₐₐ is selected from H and phenyl;
R_{bb}, and R_{cc} are each independently selected from H and CN;
R_{dd}, Rεε, Rₕₕ, Rᵢᵢ, and Rⱼⱼ are each independently selected from H, OCH₃, 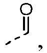 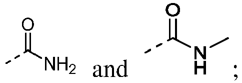
Rₑₑ is selected from H and F;
R_{gg} is selected from H and Cl; 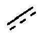
is selected from a single bond and a double bond, and other variables are as defined in this disclosure.

In some embodiments of the present disclosure, the PTM is selected from

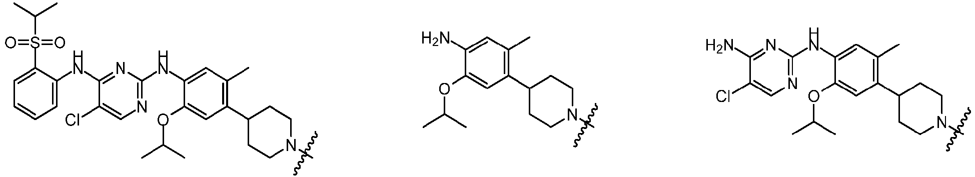

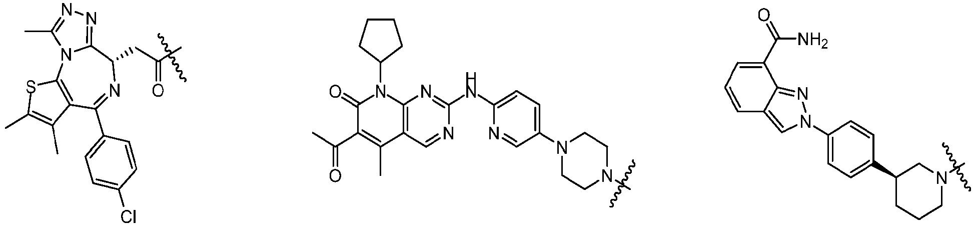

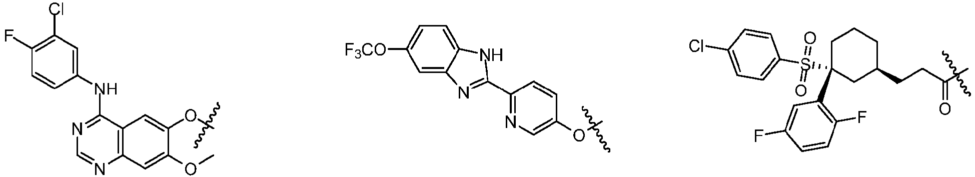

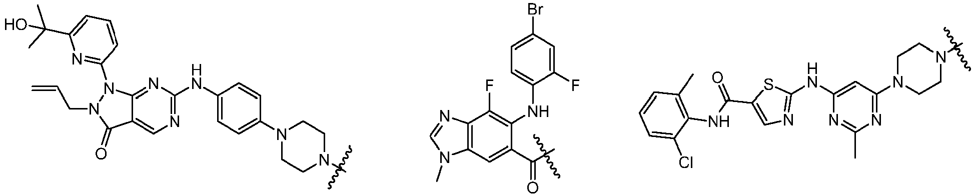

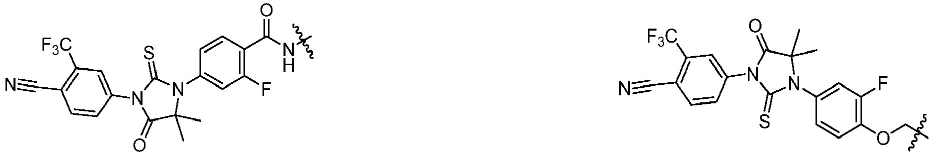

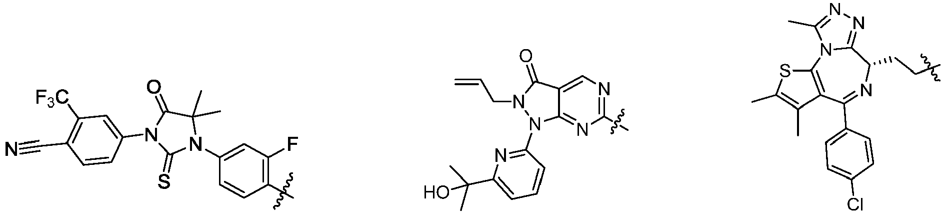

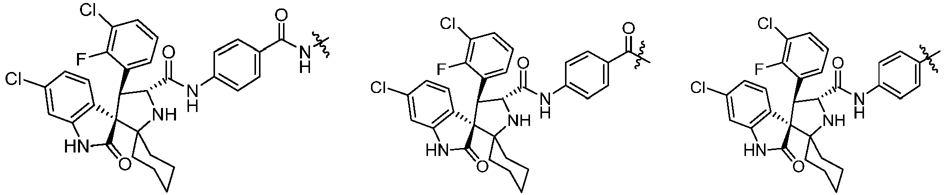

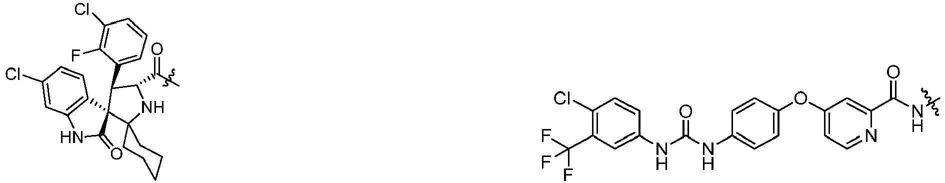

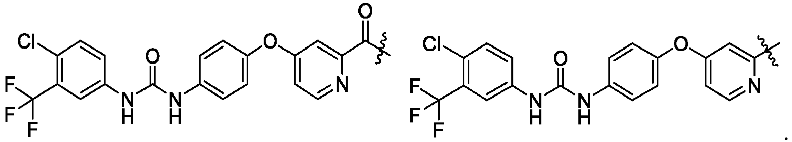

In some embodiments of the present disclosure, the L is selected from C₁₋₂₀ alkyl; 1, 2 or 3 CH₂ on the L are replaced by cyclopropyl; 1, 2, 3, 4, 5 or 6 CH₂ on the L are optionally replaced by an atom or group selected from -NH-, =N-, -O-, -S-, -C(=O)-, -C(=O)O-, -NHC(=O)-, -NHC(=O)O-, -NHC(=O)NH-, -S(=O)-, -S(=O)₂-, -S(=O)₂NH-, =NO-, -P(=O)(OH)-, -P(=O)(R)-, -P(=O)(NHR)-, -P(=O)(NR₂)-, -P(=O)(R)NH-, C₂₋₄ alkenyl, C₂₋₄ alkynyl, C₆₋₁₂ aryl, 5- to 12-membered heteroaryl, C₃₋₁₄ cycloalkyl, and 3- to 14-membered heterocycloalkyl; L is optionally substituted with 1, 2, 3, 4, 5 or 6 R, wherein R is selected from H, F, Cl, Br, I, OH, NH, CN, C₁₋₃ alkyl, C₆₋₁₂ aryl and C₅₋₁₀ heteroaryl; and other variables are as defined in this disclosure.

In some embodiments of the present disclosure, the L is selected from structures represented by formulae (II-5), (II-6) 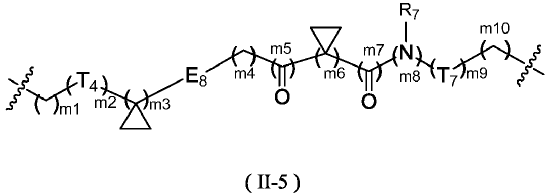 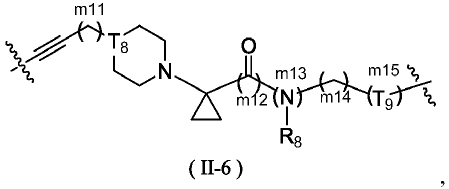 wherein
E₈ is selected from 3- to 8-membered monoheterocycloalkyl, 5- to 14-membered bridged cycloalkyl, 5- to 14-membered spirocycloalkyl;
T₄, T₇, Ta, and T₉ are each independently selected from CH and N;
R₇, and R₈ are each independently selected from H and C₁₋₃ alkyl;
m2, m3, m5, m6, m7, m8 and m9 are each independently selected from 0 or 1;
m1, m4 and m10 are each independently selected from 0 to 15;
and at least one of m1, m2, m3, m4, m5, m6, m7, m8, m9, and m10 is not 0;
and at least one of m3 and m6 is 1;
m12 and m13 are each independently selected from 0 or 1;
m11, m14 and m15 are each independently selected from 0 to 15;
and at least one of m11, m12, m13, m14, and m15 is not 0.

In some embodiments of the present disclosure, the L is selected from structures represented by formulae (I-4), (I-5), (I-6), (II-7), and (II-8) 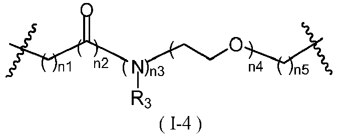 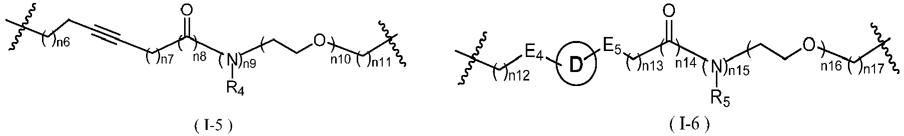 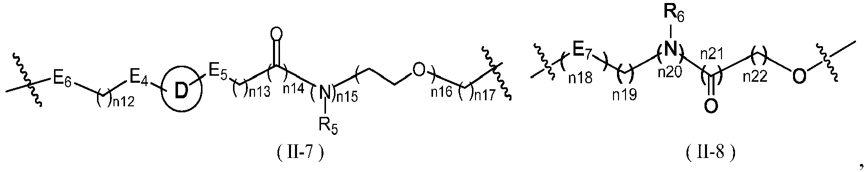 wherein
R₃, R₄, R₅, and R₆ are each independently selected from H and C₁₋₃ alkyl;
n1, n4 and n5 are each independently selected from 0 to 15, n2 and n3 are each independently selected from 0 or 1, and at least one of n1, n2, n3, n4 and n5 is not 0;
n6, n7, n10 and n11 are each independently selected from 0 to 15, n8 and n9 are each independently selected from 0 or 1, and at least one of n6, n7, n8, n9, n10 and n11 is not 0;
n12, n13, n16, and n17 are each independently selected from 0 to 15, n14 and n15 are each independently selected from 0 or 1, and at least one of n12, n13, n14, n15, n16, and n17 is not 0;
n19 and n22 are each independently selected from 0 to 15, n18, n20 and n21 are each independently selected from 0 or 1, and at least one of n18, n19, n20, n21 and n22 is not 0;
E₄ and E₅ are each independently selected from a bond, O, NH, and S(=O)₂;
E₆ and E₇ are each independently selected from O and NH;
Ring D is selected from phenyl, piperidinyl, piperazinyl, 1,2,3-triazolyl, cyclobutyl and azetidinyl.

In some embodiments of the present disclosure, the L is selected from structures represented by formulae (I-4-1), (I-4-2), (I-4-3), (I-4-4), (I-4-5), (I-4-6), (II-I-4-7), and (II-8-1), 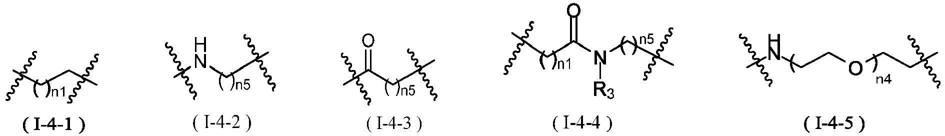 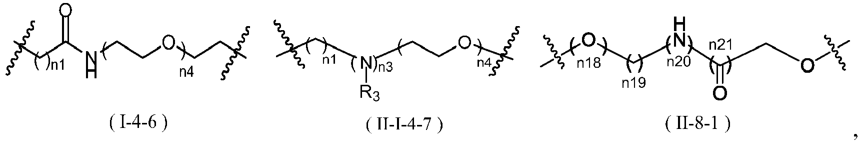 wherein
R₃ is selected from H, CH₃, CH₂CH₃ and CH(CH₃)₂;
n1 is selected from 0, 1, 2, 3, 4, 5, 6, 7, and 8;
n3 is selected from 0 and 1;
n4 is selected from 0, 1, 2, 3, and 4;
n5 is selected from 0, 1, 2, 3, 4, 5, 6, 7, and 8;
n18 is selected from 0 and 1;
n19 is selected from 0, 1, 2, 3, and 4;
n20 is selected from 0 and 1;
n21 is selected from 0 and 1.

In some embodiments of the present disclosure, the L is selected from structures represented by formulae (I-5-1), (I-5-2), (I-5-3), and (I-5-4), 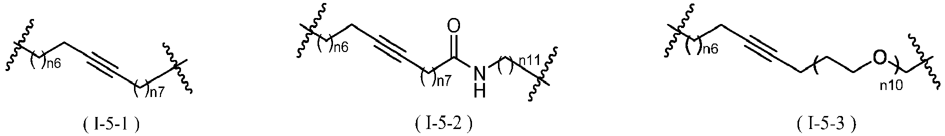 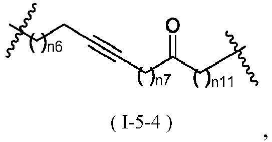 wherein n6, n7, n10 and n11 are as defined in this disclosure.

In some embodiments of the present disclosure, the L is selected from structures represented by formulae (I-6-1), (I-6-2), (I-6-3), (I-6-4), (I-6-5), (I-6-6), (I-6-7), (I-6-8), and (II-7-1), 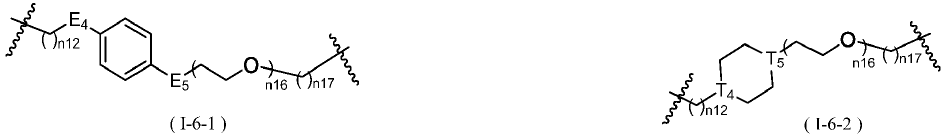 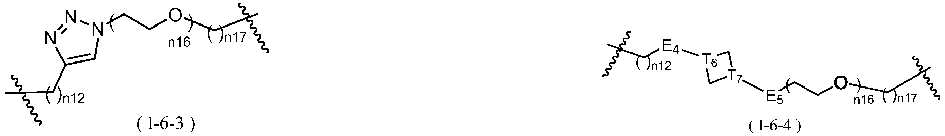 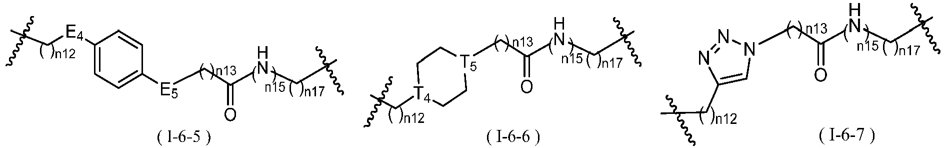 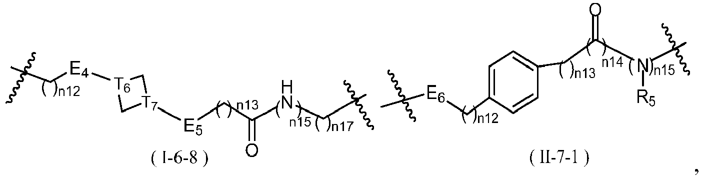 wherein
T₄ and T₅ are each independently selected from CH and N, and one of them must be N;
T₆ and T₇ are each independently selected from CH and N, and one of them must be C;
E₄, E₅, n12, n13, n15, n16, and n17 are as defined in this disclosure.

In some embodiments of the present disclosure, the L is selected from NH,

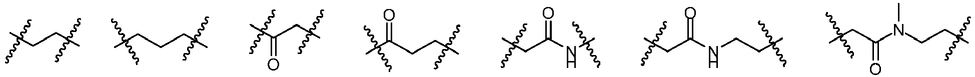

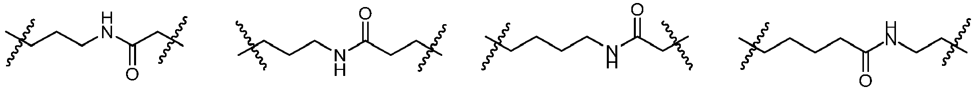

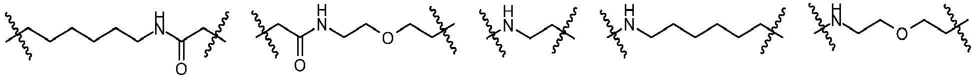

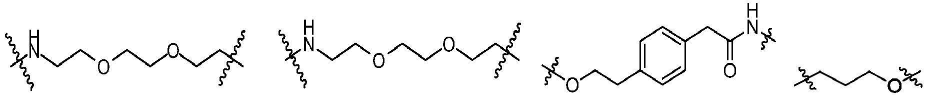

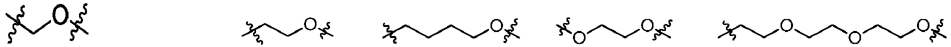

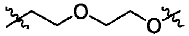

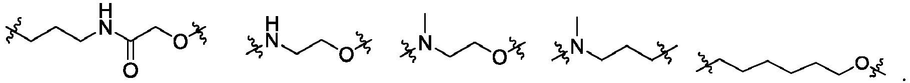

The present disclosure provides a compound of formula (I) or a pharmaceutically acceptable salt thereof,

PTM-L-ULM (I)

wherein
PTM is selected from drugs that act on targeted proteins, or derivatives thereof;
L is a chain connecting PTM and ULM;
ULM is selected from structures represented by formulae (II-11), (II-12), (II-13), (II-1), (II-2), (II-3), and (II-4), 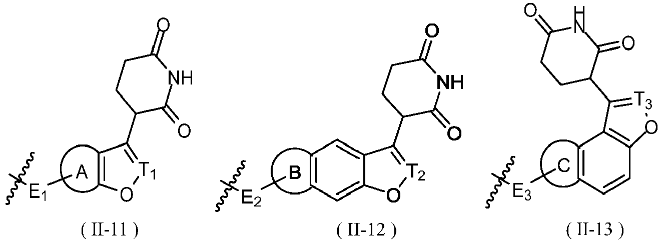 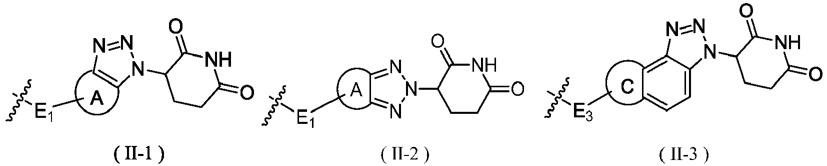 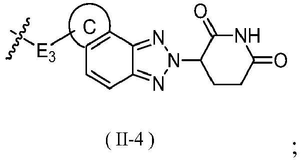
T₁, T₂, and T₃ are each independently selected from CH and N;
E₁, E₂, and E₃ are each independently selected from -CH₂-, -NR₁-, -O-, -S-, -S(=O)-, -S(=O)₂-, -C(=O), and -C(=O)NR₂-;
R₁ and R₂ are each independently selected from H and C₁₋₃ alkyl;
Ring A, ring B, and ring C are each independently selected from phenyl, thienyl, furyl, pyrrolyl, and pyridyl.

In some embodiments of the present disclosure, the ring A is selected from phenyl and thienyl, and other variables are as defined in this disclosure.

In some embodiments of the present disclosure, the ULM is selected from structures represented by formulae (II-11-1), (II-11-2), (II-1-1), and (II-2-1) 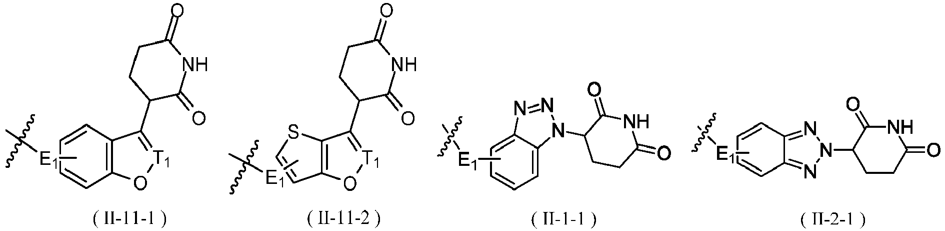 wherein T₁, and E₁ are as defined in this disclosure.

In some embodiments of the present disclosure, the ring B is selected from phenyl, and other variables are as defined in this disclosure.

In some embodiments of the present disclosure, the ULM is selected from the structure represented by formula (II-12-1) 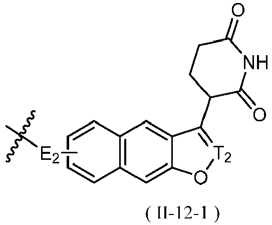 wherein T₂, and E₂ are as defined in this disclosure.

In some embodiments of the present disclosure, the ring C is selected from phenyl, and other variables are as defined in this disclosure.

In some embodiments of the present disclosure, the ULM is selected from structures represented by formulae (II-13-1), (II-3-1), and (II-3-2) 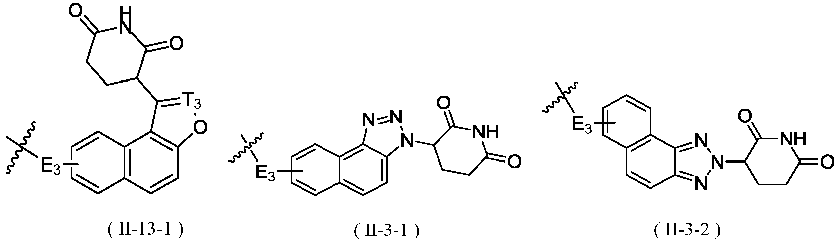 wherein T₃, and E₃ are as defined in this disclosure.

In some embodiments of the present disclosure, the ULM is selected from

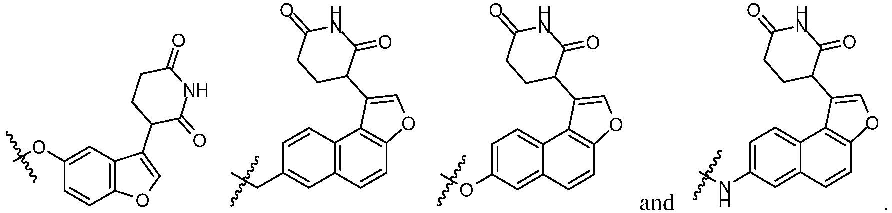

In some embodiments of the present disclosure, the PTM is selected from drugs that act on ALK, BET, CDK, PARP, EGFR, γ-secretase, CBFβ-SMMHC, WEE1, MEK, BCR-ABL, MET, RAS, BTK, VEGFR, JAK, HER2, HDAC, Akt, PI3K, mTOR, AR, ER, SRC, MDM2, and RAF, or derivatives thereof, and other variables are as defined in this disclosure.

In some embodiments of the present disclosure, the PTM is selected from drugs that act on ALK, BRD4, CDK4/6, CDK8, CDK9, PARP, EGFR, γ-secretase, CBFβ-SMMHC, WEE1, MEK, BCR-ABL, MET, KRAS, BTK, VEGFR, HER2, HDAC, Akt, PI3K, mTOR, AR, ER, SRC, JAK, MDM2, and RAF, or derivatives thereof, and other variables are as defined in this disclosure.

In some embodiments of the present disclosure, the PTM is selected from drugs that act on ALK, BRD4, CDK4/6, PARP, EGFR, γ-secretase, CBFβ-SMMHC, WEE1, MEK, BCR-ABL, MET, KRAS, EGFR, BTK, AR, JAK, MDM2, and RAF, or derivatives thereof, and other variables are as defined in this disclosure.

In some embodiments of the present disclosure, the PTM is selected from 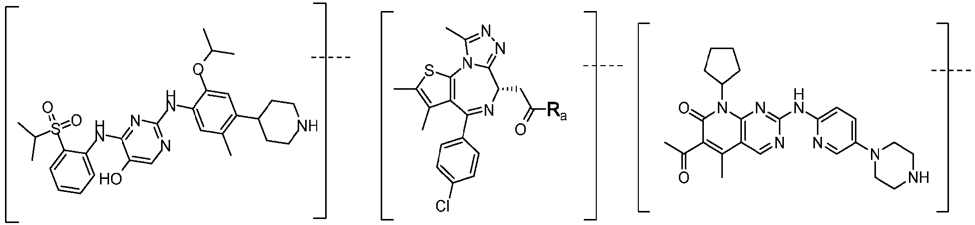 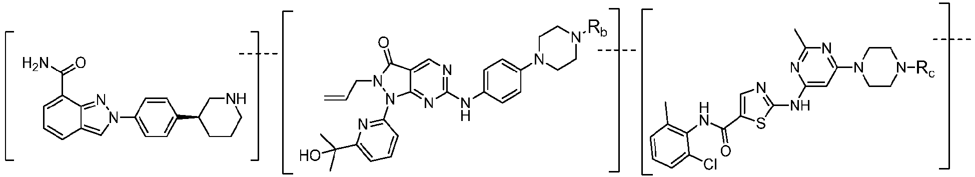 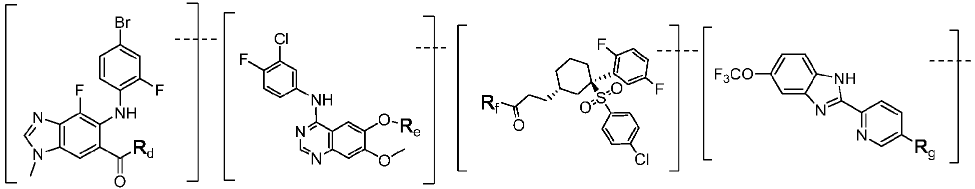 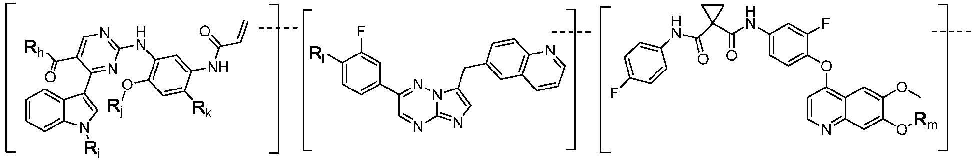 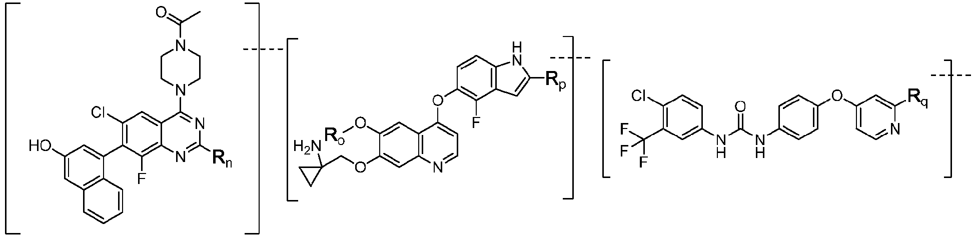 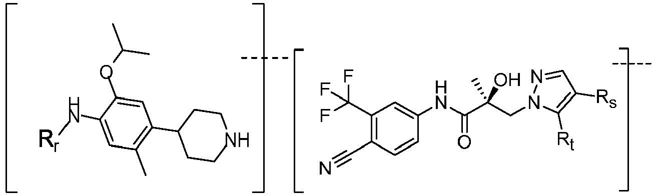 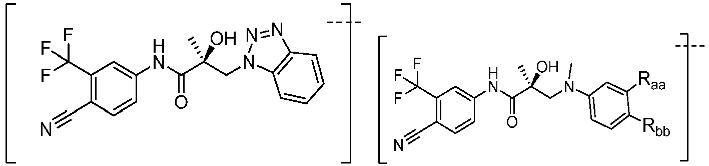 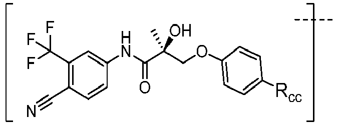 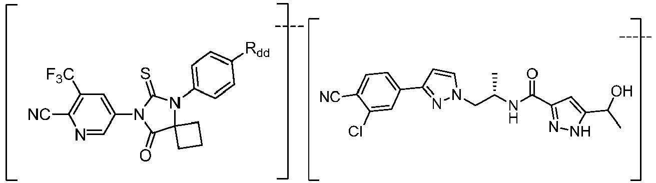 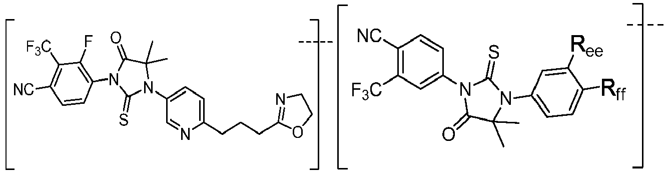 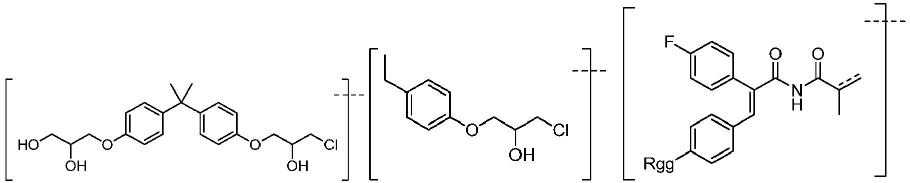 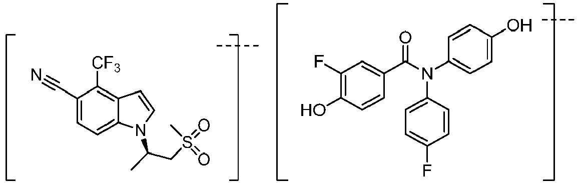 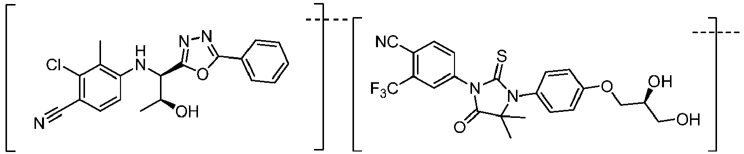 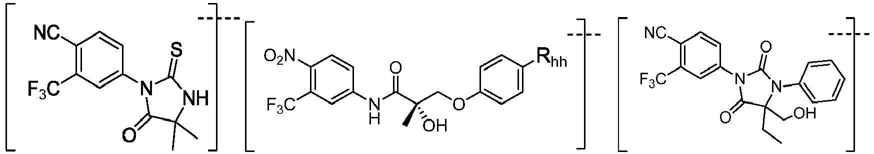 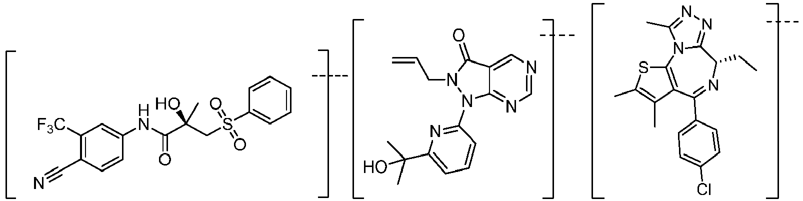 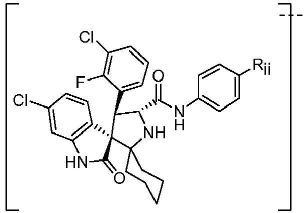 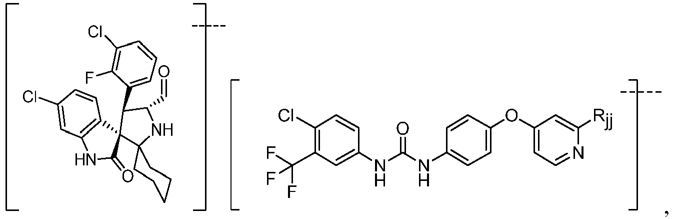 wherein
Rₐ is selected from H and 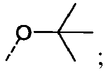
R_{b} is selected from H and CH₃;
R_{c} is selected from H and 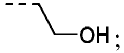
R_{d} is selected from H, NH₂ and 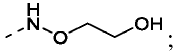
Rₑ is selected from H and 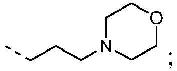
R_{f} is selected from H and OH;
R_{g} is selected from H and OH;
Rₕ is selected from H and 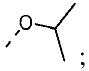
Rᵢ is selected from H and CH₃;
Rⱼ is selected from H and CH₃;
Rₖ is selected from H, NH₂, NHCH₃ and 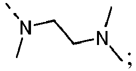
Rₗ is selected from H, 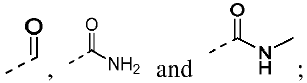
Rₘ is selected from H and 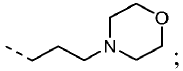
Rₙ is selected from H, NH₂, NHCH₂CH₃ and 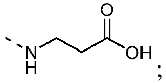
Rₒ is selected from H and CH₃;
Rₚ is selected from H and CH₃;
R_{q} is selected from H, 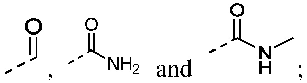
Rᵣ is selected from H and 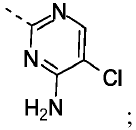
Rₛ is selected from H, F and Cl;
Rₜ is selected from H and Br;
Rₐₐ is selected from H and phenyl;
R_{bb}, and R_{cc} are each independently selected from H and CN;
R_{dd}, Rεε, Rₕₕ, Rᵢᵢ, and Rⱼⱼ are each independently selected from H, OCH₃, 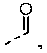 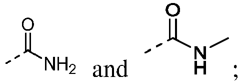
Rₑₑ is selected from H and F;
R_{gg} is selected from H and Cl; 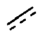
is selected from a single bond and a double bond, and other variables are as defined in this disclosure.

In some embodiments of the present disclosure, the PTM is selected from

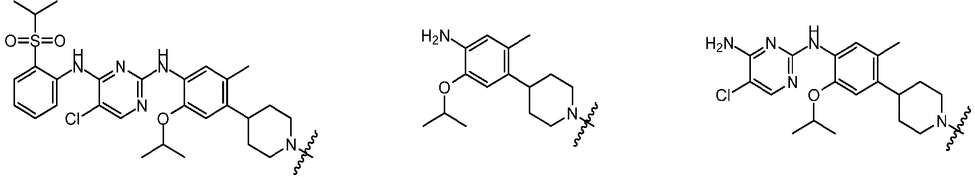

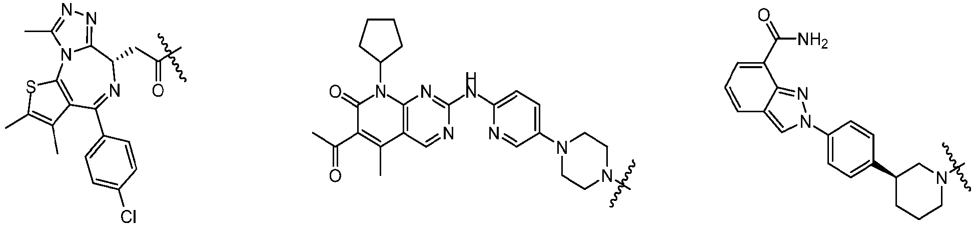

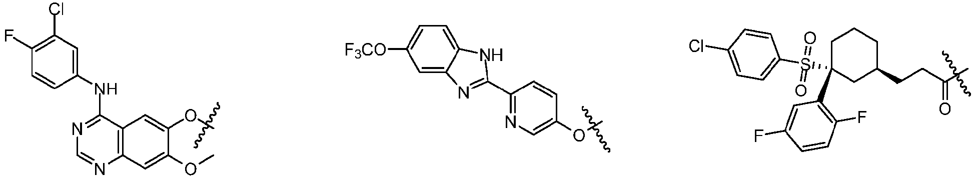

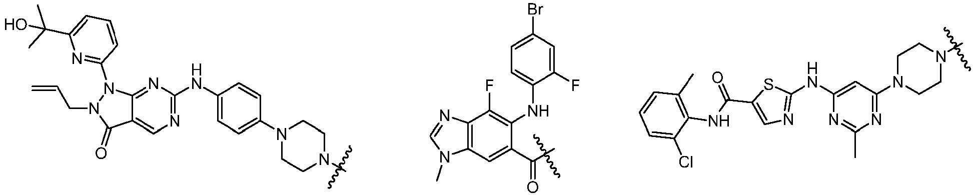

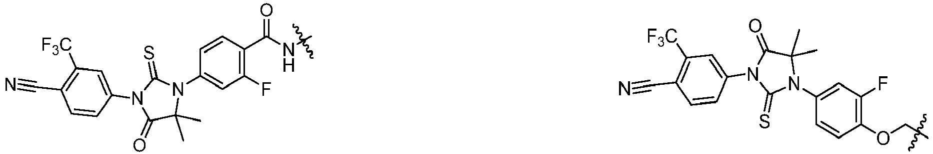

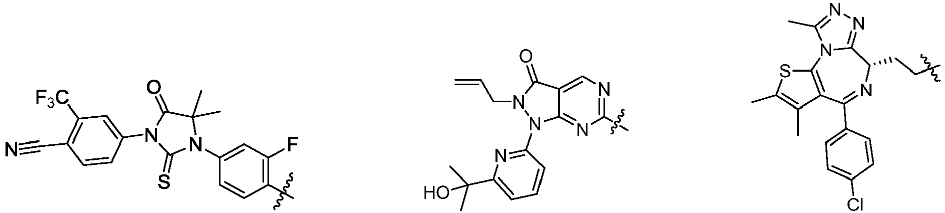

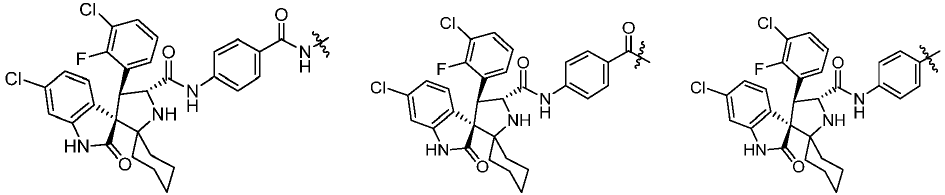

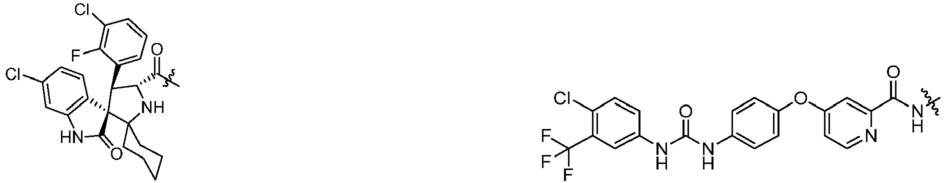

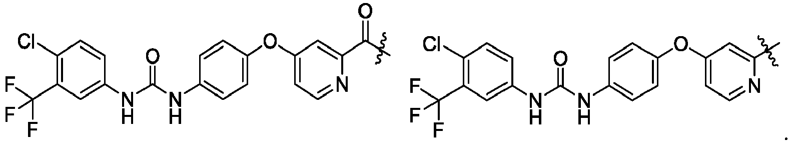

In some embodiments of the present disclosure, the L is selected from C₁₋₂₀ alkyl; CH₂ on the L is replaced by 1, 2 or 3 cyclopropyls; 1, 2 or 3 CH₂ on the L are replaced by cyclopropyl; 1, 2, 3, 4, 5 or 6 CH₂ on the L are optionally replaced by an atom or group selected from -NH-, =N-, -O-, -S-, -C(=O)-, -C(=O)O-, -NHC(=O)-, -NHC(=O)O-, -NHC(=O)NH-, -S(=O)-, -S(=O)₂-, -S(=O)₂NH-, =NO-, -P(=O)(OH)-, -P(=O)(R)-, -P(=O)(NHR)-, -P(=O)(NR₂)-, -P(=O)(R)NH-, C₂₋₄ alkenyl, C₂₋₄ alkynyl, C₆₋₁₂ aryl, 5- to 12-membered heteroaryl, C₃₋₁₄ cycloalkyl, and 3- to 14-membered heterocycloalkyl; L is optionally substituted with 1, 2, 3, 4, 5 or 6 R, wherein R is selected from H, F, Cl, Br, I, OH, NH, CN, C₁₋₃ alkyl, C₆₋₁₂ aryl and C₅₋₁₀ heteroaryl; and other variables are as defined in this disclosure.

In some embodiments of the present disclosure, the L is selected from structures represented by formulae (II-5), (II-6) 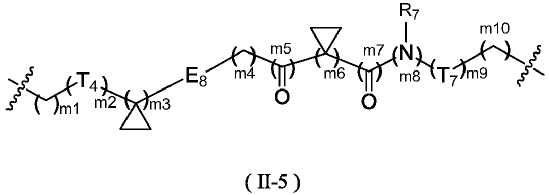 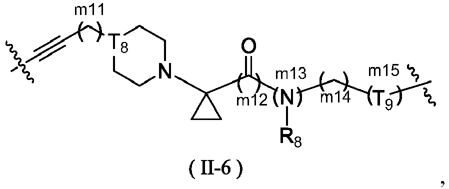 wherein
E₈ is selected from 3- to 8-membered monoheterocycloalkyl, 5- to 14-membered bridged cycloalkyl, 5- to 14-membered spirocycloalkyl;
T₄, T₇, Ta, and T₉ are each independently selected from CH and N;
R₇, and R₈ are each independently selected from H and C₁₋₃ alkyl;
m2, m3, m5, m6, m7, m8 and m9 are each independently selected from 0 or 1;
m1, m4 and m10 are each independently selected from 0 to 15;
and at least one of m1, m2, m3, m4, m5, m6, m7, m8, m9, and m10 is not 0;
and at least one of m3 and m6 is 1;
m12 and m13 are each independently selected from 0 or 1;
m11 and m14 are each independently selected from 0 to 15.

In some embodiments of the present disclosure, the L is selected from structures represented by formulae (I-4), (I-5), (I-6), (II-7), and (II-8) 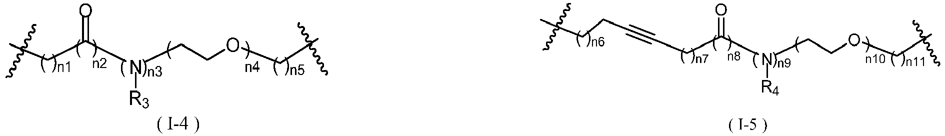 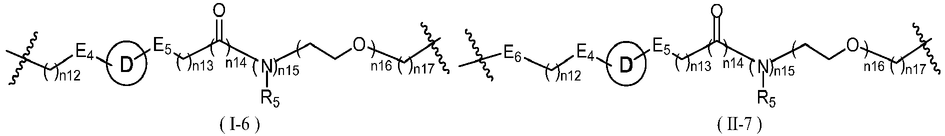 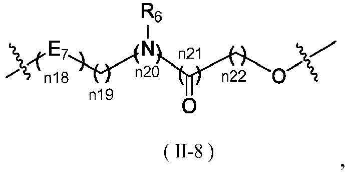 wherein
R₃, R₄, R₅, and R₆ are each independently selected from H and C₁₋₃ alkyl;
n1, n4 and n5 are each independently selected from 0 to 15, n2 and n3 are each independently selected from 0 or 1, and at least one of n1, n2, n3, n4 and n5 is not 0;
n6, n7, n10 and n11 are each independently selected from 0 to 15, n8 and n9 are each independently selected from 0 or 1, and at least one of n6, n7, n8, n9, n10 and n11 is not 0;
n12, n13, n16, and n17 are each independently selected from 0 to 15, n14 and n15 are each independently selected from 0 or 1, and at least one of n12, n13, n14, n15, n16, and n17 is not 0;
n19 and n22 are each independently selected from 0 to 15, n18, n20 and n21 are each independently selected from 0 or 1, and at least one of n18, n19, n20, n21 and n22 is not 0;
E₄ and E₅ are each independently selected from a bond, O, NH, and S(=O)₂;
E₆ and E₇ are each independently selected from O and NH;
Ring D is selected from phenyl, piperidinyl, piperazinyl, 1,2,3-triazolyl, cyclobutyl and azetidinyl.

In some embodiments of the present disclosure, the L is selected from structures represented by formulae (I-4-1), (1-4-2), (I-4-3), (I-4-4), (I-4-5), (I-4-6), (II-I-4-7), and (II-8-1), 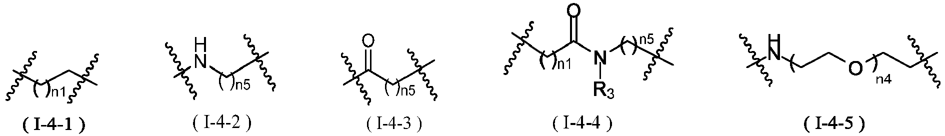 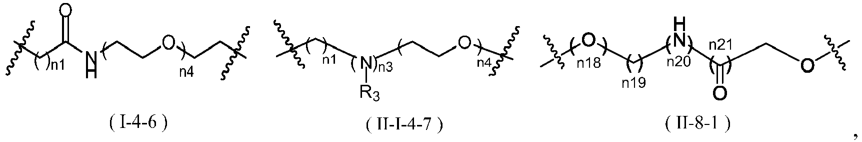 wherein
R₃ is selected from H, CH₃, CH₂CH₃ and CH(CH₃) ₂;
n1 is selected from 0, 1, 2, 3, 4, 5, 6, 7, and 8;
n3 is selected from 0 and 1;
n4 is selected from 0, 1, 2, 3, and 4;
n5 is selected from 0, 1, 2, 3, 4, 5, 6, 7, and 8;
n18 is selected from 0 and 1;
n19 is selected from 0, 1, 2, 3, and 4;
n20 is selected from 0 and 1;
n21 is selected from 0 and 1.

In some embodiments of the present disclosure, the L is selected from structures represented by formulae (I-5-1), (I-5-2), (I-5-3), and (I-5-4), 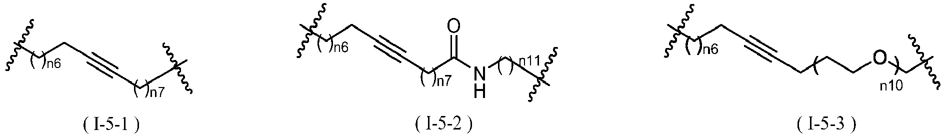 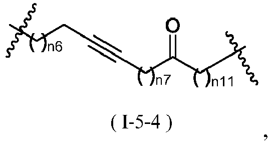 wherein n6, n7, n10 and n11 are as defined in this disclosure.

In some embodiments of the present disclosure, the L is selected from structures represented by formulae (I-6-1), (I-6-2), (I-6-3), (I-6-4), (I-6-5), (I-6-6), (I-6-7), (I-6-8), and (II-7-1), 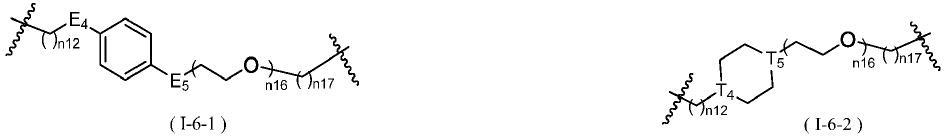 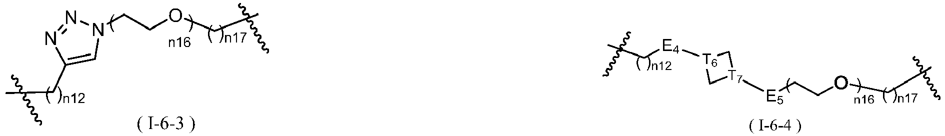 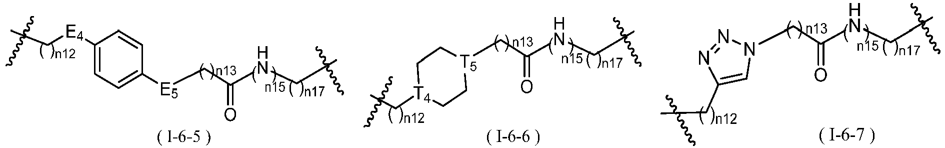 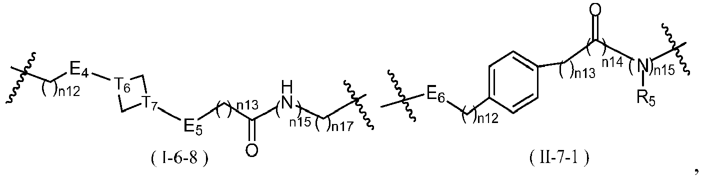 wherein
T₄ and T₅ are each independently selected from CH and N, and one of them must be N;
T₆ and T₇ are each independently selected from CH and N, and one of them must be C;
E₄, E₅, n12, n13, n15, n16, and n17 are as defined in this disclosure.

In some embodiments of the present disclosure, the L is selected from NH,

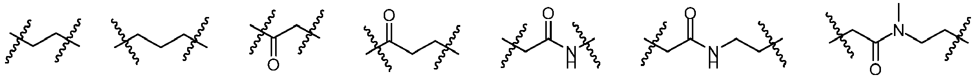

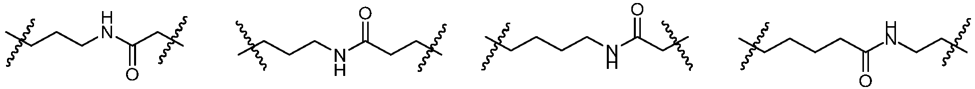

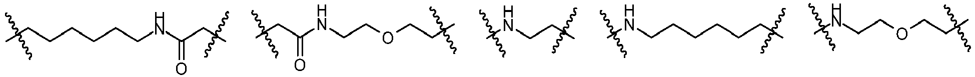

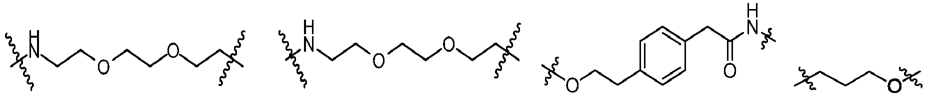

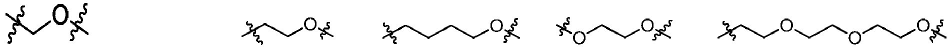

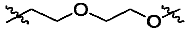

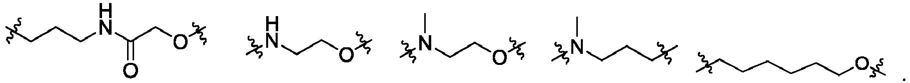

The present disclosure provides a compound of formula (I) or a pharmaceutically acceptable salt thereof,

PTM-L-ULM (I)

wherein
PTM is selected from drugs that act on targeted proteins, or derivatives thereof;
L is a chain connecting PTM and ULM;
ULM is selected from structures represented by formulae (I-1), (I-2), and (I-3), 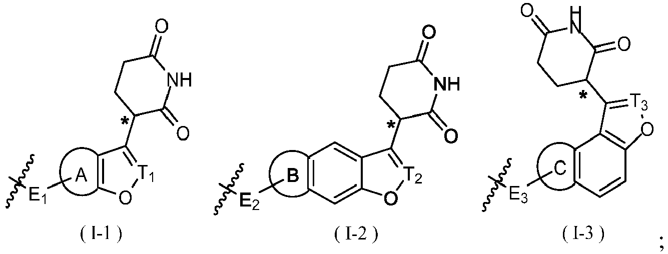
T₁, T₂, and T₃ are each independently selected from CH and N;
E₁, E₂, and E₃ are each independently selected from -CH₂-, -NR₁-, -O-, -S-, -S(=O)-, -S(=O)₂-, -C(=O), and -C(=O)NR₂-;
R₁ and R₂ are each independently selected from H and C₁₋₃ alkyl;
Ring A, ring B, and ring C are each independently selected from phenyl, thienyl, furyl and pyrrolyl;
The carbon atom with "*" is a chiral carbon atom, which exists in the form of single (R) or (S) enantiomer or in an enantiomerically enriched form.

In some embodiments of the present disclosure, the ring A is selected from phenyl and thienyl, and other variables are as defined in this disclosure.

In some embodiments of the present disclosure, the ULM is selected from structures represented by formulae (I-1-1) and (1-1-2), 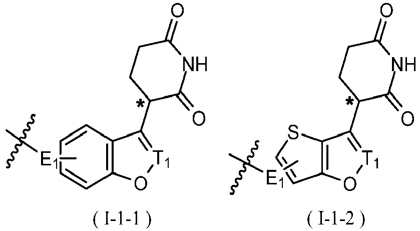 wherein T₁, and E₁ are as defined in this disclosure;

The carbon atom with "*" is a chiral carbon atom, which exists in the form of single (R) or (S) enantiomer or in an enantiomerically enriched form.

In some embodiments of the present disclosure, the ring B is selected from phenyl, and other variables are as defined in this disclosure.

In some embodiments of the present disclosure, the ULM is selected from the structure represented by formula (1-2-1), 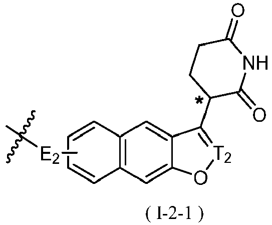 wherein T₂, and E₂ are as defined in this disclosure;

The carbon atom with "*" is a chiral carbon atom, which exists in the form of single (R) or (S) enantiomer or in an enantiomerically enriched form.

In some embodiments of the present disclosure, the ring C is selected from phenyl, and other variables are as defined in this disclosure.

In some embodiments of the present disclosure, the ULM is selected from the structure represented by formula (I-3-1), 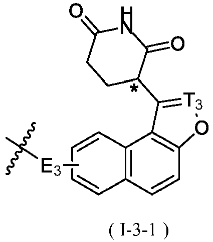 wherein T₃, and E₃ are as defined in this disclosure;

The carbon atom with "*" is a chiral carbon atom, which exists in the form of single (R) or (S) enantiomer or in an enantiomerically enriched form.

In some embodiments of the present disclosure, the ULM is selected from

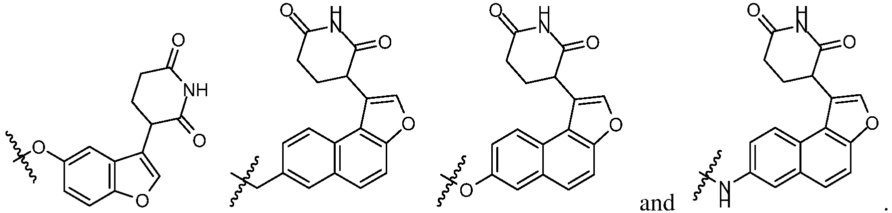

In some embodiments of the present disclosure, the PTM is selected from drugs that act on ALK, BET, CDK, PARP, EGFR, γ-secretase, CBFβ-SMMHC, WEE1, MEK, BCR-ABL, MET, RAS, BTK, VEGFR, JAK, HER2, HDAC, Akt, PI3K, mTOR, AR, ER, and SRC, or derivatives thereof, and other variables are as defined in this disclosure.

In some embodiments of the present disclosure, the PTM is selected from drugs that act on ALK, BRD4, CDK4/6, CDK8, CDK9, PARP, EGFR, γ-secretase, CBFβ-SMMHC, WEE1, MEK, BCR-ABL, MET, KRAS, BTK, VEGFR, HER2, HDAC, Akt, PI3K, mTOR, AR, ER, SRC, and JAK, or derivatives thereof, and other variables are as defined in this disclosure, and other variables are as defined in this disclosure.

In some embodiments of the present disclosure, the PTM is selected from drugs that act on ALK, BRD4, CDK4/6, PARP, EGFR, γ-secretase, CBFβ-SMMHC, WEE1, MEK, BCR-ABL, MET, KRAS, EGFR, BTK, and JAK, or derivatives thereof, and other variables are as defined in this disclosure.

In some embodiments of the present disclosure, the PTM is selected from 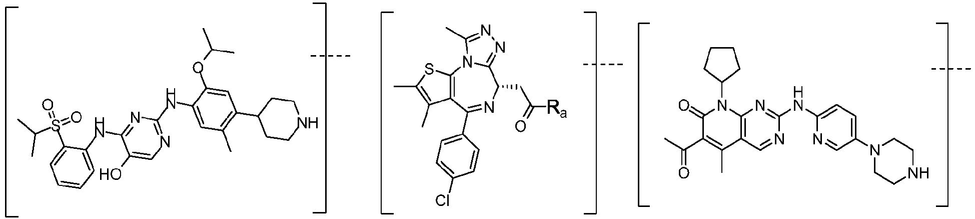 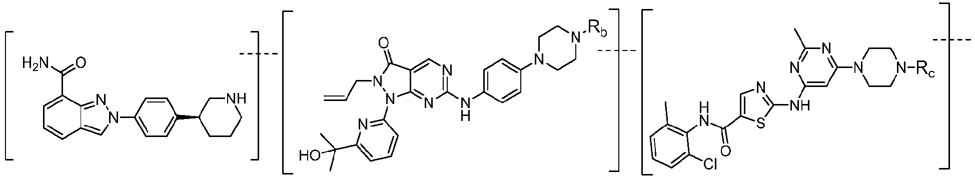 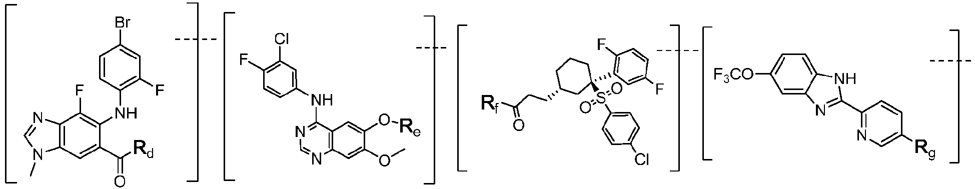 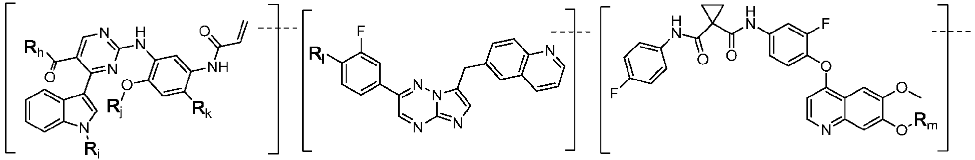 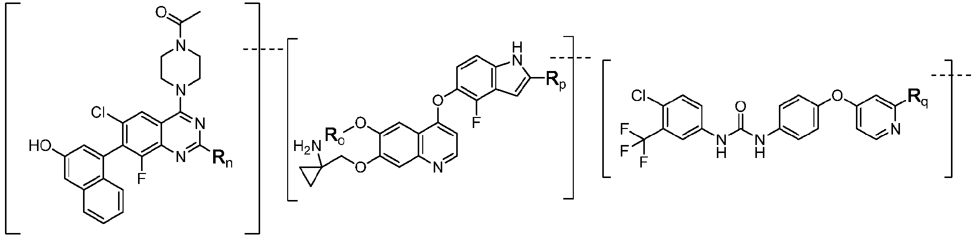 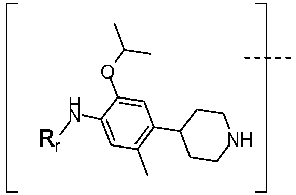 wherein
Rₐ is selected from H and 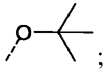
R_{b} is selected from H and CH₃;
R_{c} is selected from H and 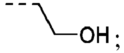
R_{d} is selected from H, NH₂ and 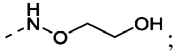
Rₑ is selected from H and 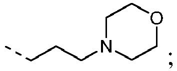
R_{f} is selected from H and OH;
R_{g} is selected from H and OH;
Rₕ is selected from H and 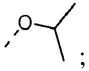
Rᵢ is selected from H and CH₃;
Rⱼ is selected from H and CH₃;
Rₖ is selected from H, NH₂, NHCH₃ and 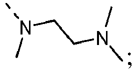
Rₗ is selected from H, 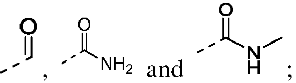
Rₘ is selected from H and 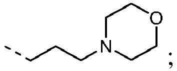
Rₙ is selected from H, NH₂, NHCH₂CH₃ and 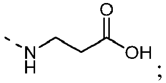
Rₒ is selected from H and CH₃;
Rₚ is selected from H and CH₃;
R_{q} is selected from H, 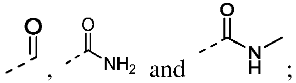
Rᵣ is selected from H and 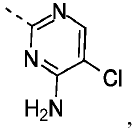 and other variables are as defined in this disclosure.

In some embodiments of the present disclosure, the PTM is selected from

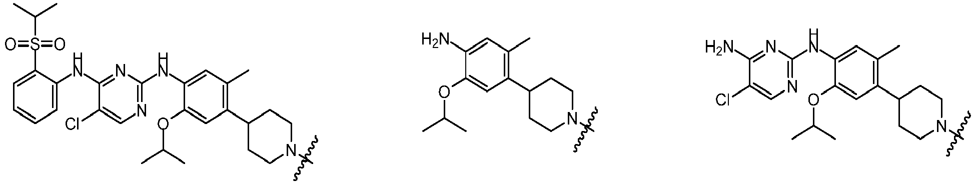

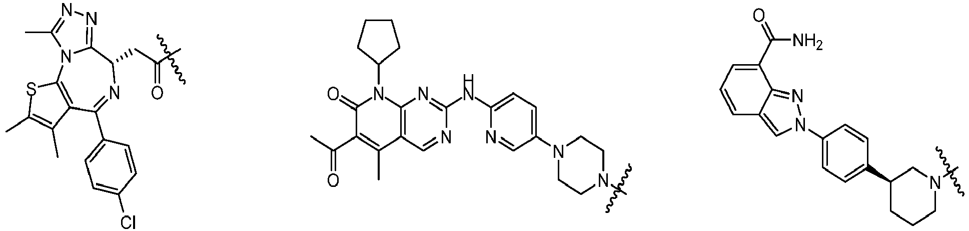

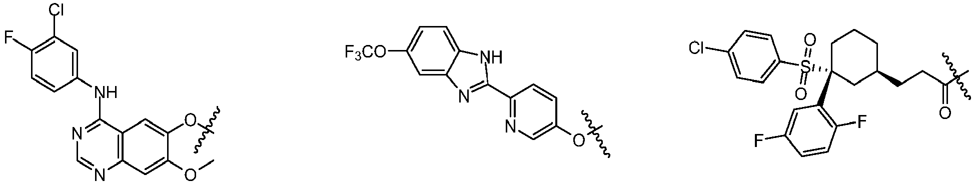

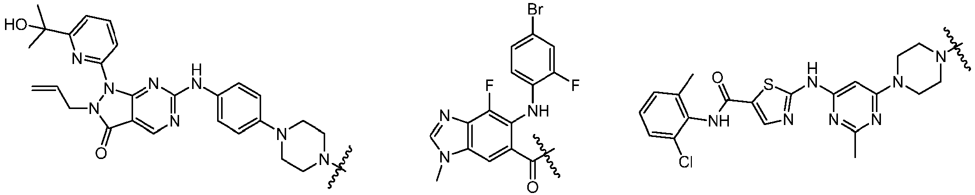

In some embodiments of the present disclosure, the L is selected from structures represented by formulae (I-4), (I-5), (I-6) 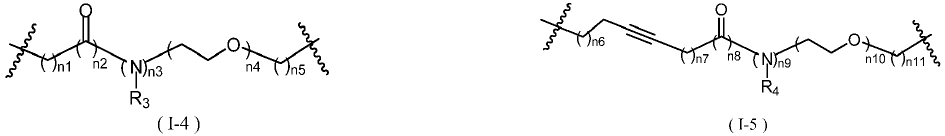 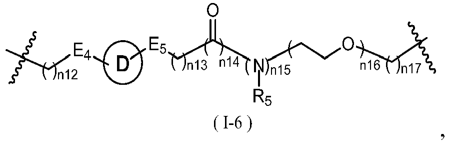 wherein
R₃, R₄, and R₅ are each independently selected from H and C₁₋₃ alkyl;
n1, n4 and n5 are each independently selected from 0 to 15, n2 and n3 are each independently selected from 0 or 1, and at least one of n1, n2, n3, n4 and n5 is not 0;
n6, n7, n10 and n11 are each independently selected from 0 to 15, n8 and n9 are each independently selected from 0 or 1, and at least one of n6, n7, n8, n9, n10 and n11 is not 0;
n12, n13, n16, and n17 are each independently selected from 0 to 15, n14 and n15 are each independently selected from 0 or 1, and at least one of n12, n13, n14, n15, n16, and n17 is not 0;
E₄ and E₅ are each independently selected from a bond, O, NH, and S(=O)₂;
Ring D is selected from phenyl, piperidinyl, piperazinyl, 1,2,3-triazolyl, cyclobutyl and azetidinyl, and other variables are as defined in this disclosure.

In some embodiments of the present disclosure, the L is selected from structures represented by formulae (I-4-1), (I-4-2), (I-4-3), (I-4-4), (I-4-5), and (I-4-6), 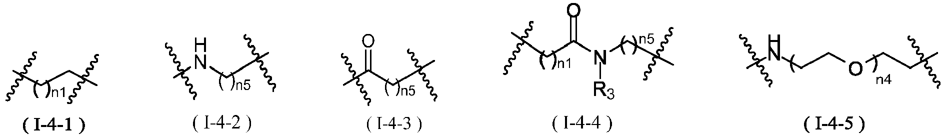 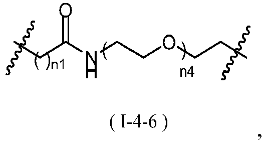 wherein R₃, n1, n4 and n5 are as defined in this disclosure.

In some embodiments of the present disclosure, the L is selected from structures represented by formulae (I-5-1), (I-5-2), (I-5-3), and (I-5-4), 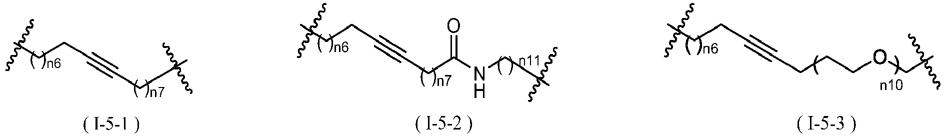 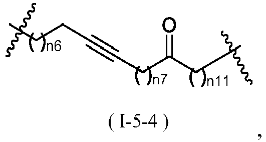 wherein n6, n7, n10 and n11 are as defined in this disclosure.

In some embodiments of the present disclosure, the L is selected from structures represented by formulae (I-6-1), (I-6-2), (I-6-3), (I-6-4), (I-6-5), (I-6-6), (I-6-7), and (I-6-8), 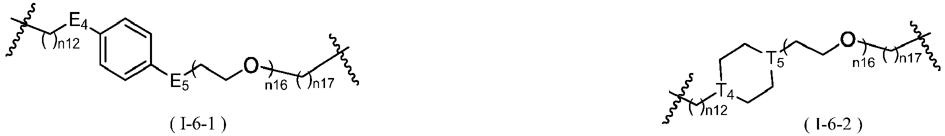 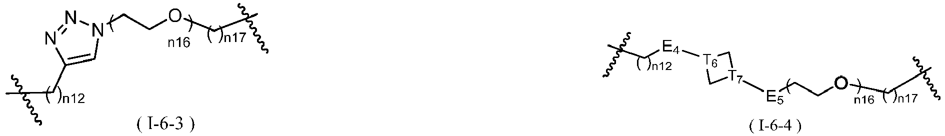 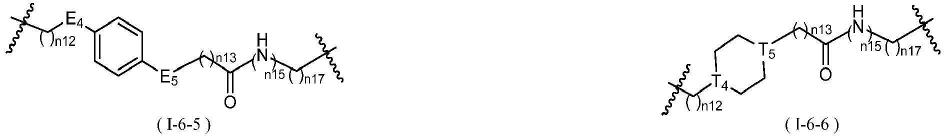 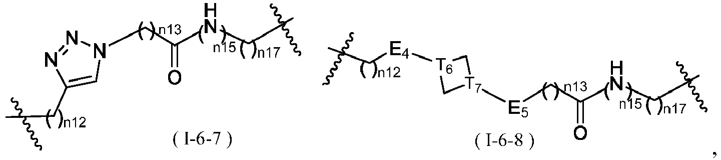 wherein
T₄ and T₅ are each independently selected from CH and N, and one of them must be N;
T₆ and T₇ are each independently selected from CH and N, and one of them must be C;
E₄, E₅, n12, n13, n15, n16, and n17 are as defined in this disclosure.

In some embodiments of the present disclosure, the L is selected from

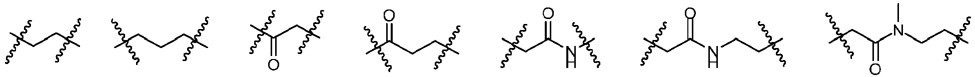

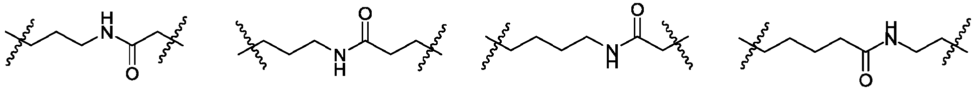

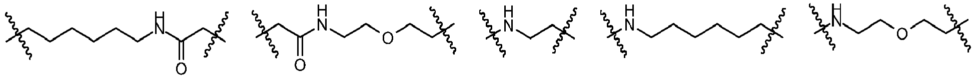

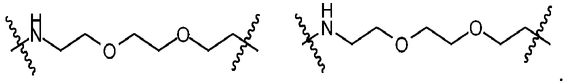

The present disclosure also includes some embodiments obtained from any combination of the above variables.

The present disclosure also provides compounds represented by the following formulae or pharmaceutically acceptable salt thereofs,

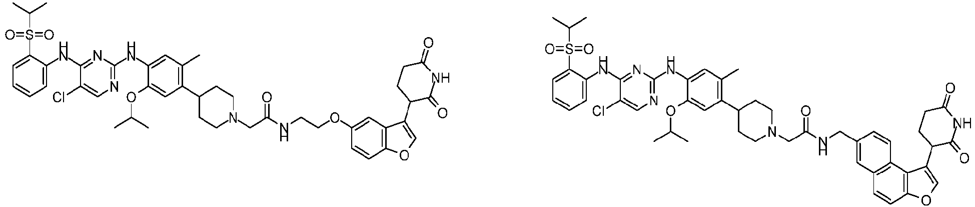

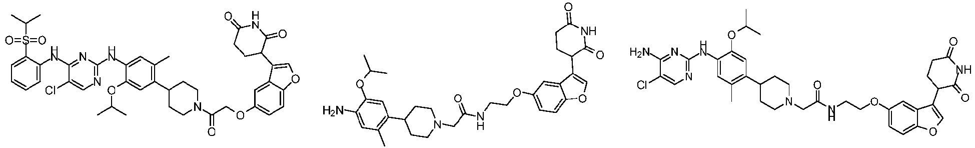

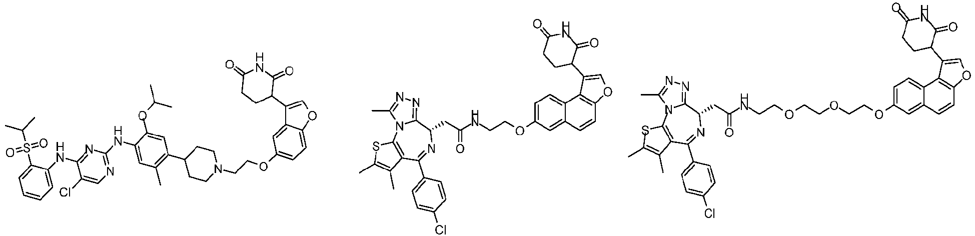

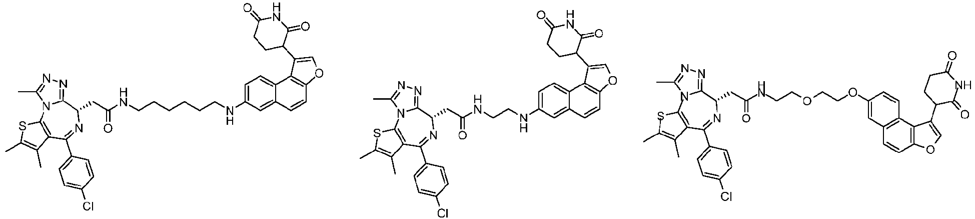

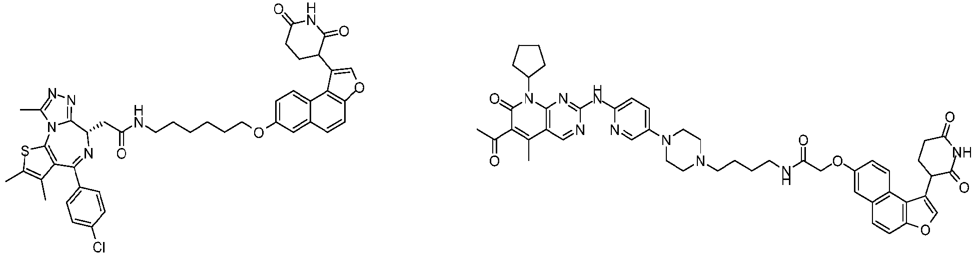

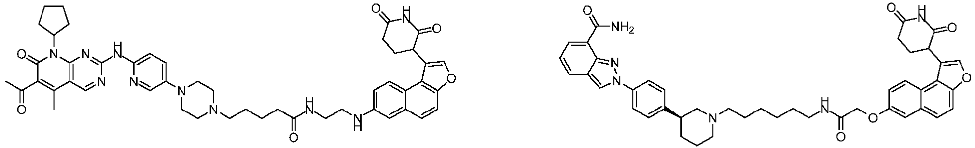

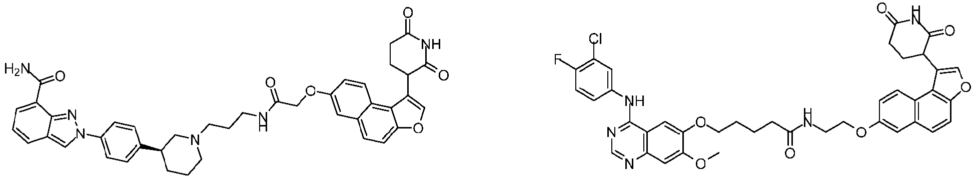

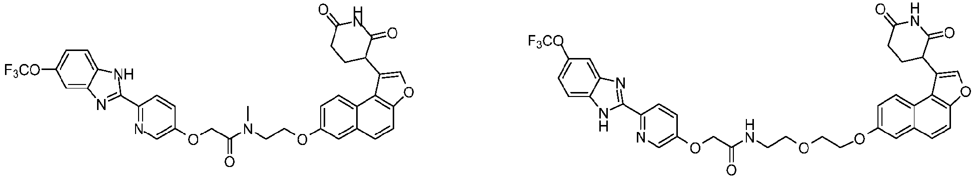

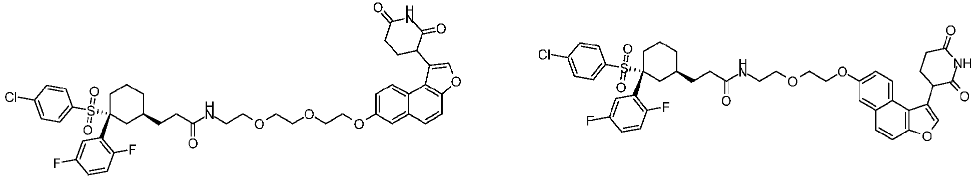

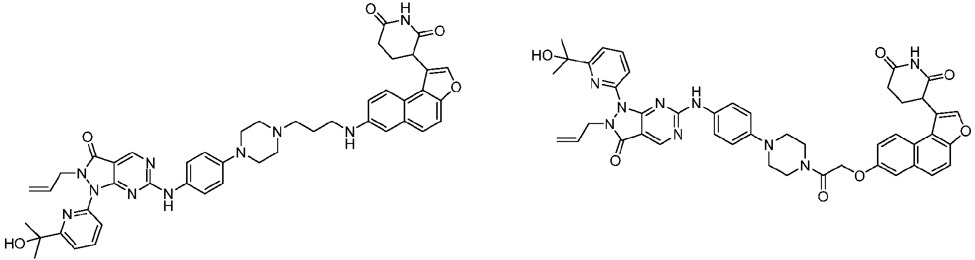

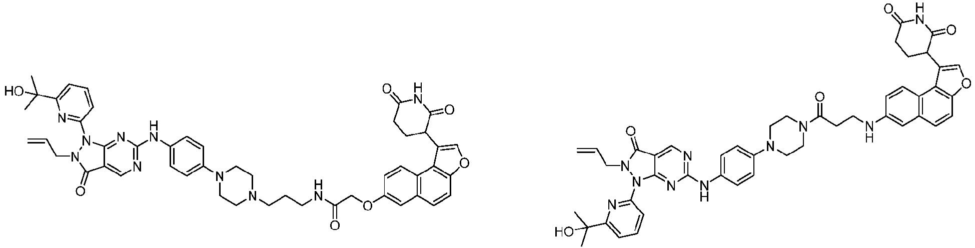

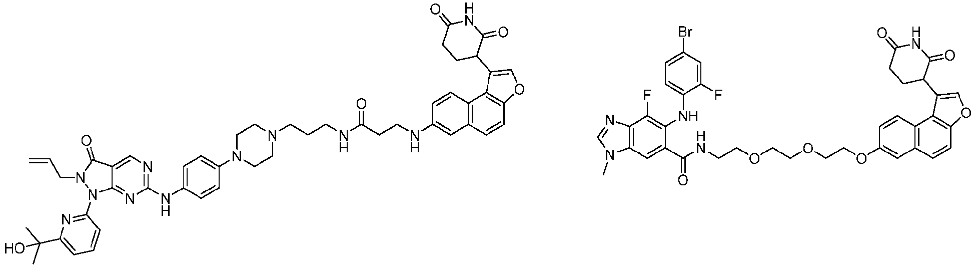

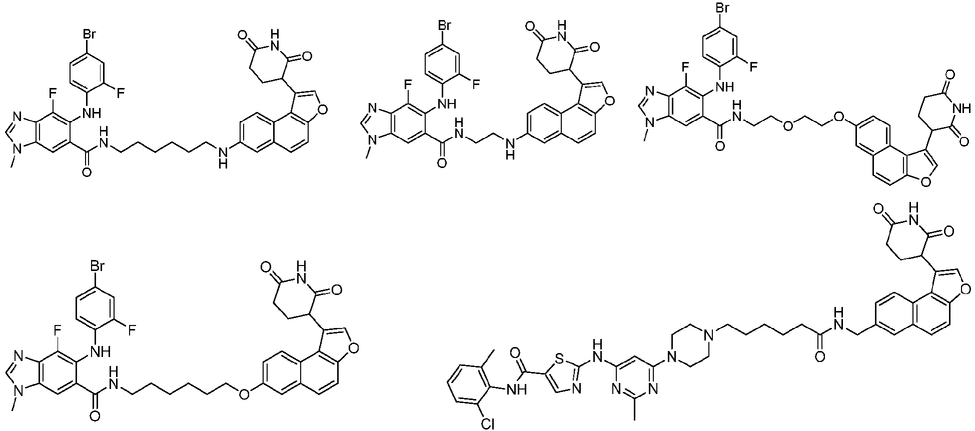

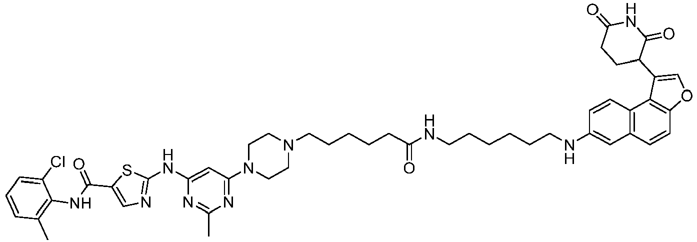

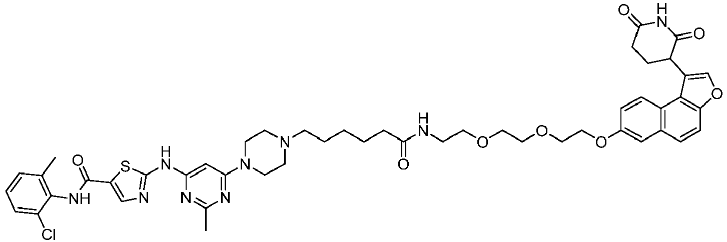

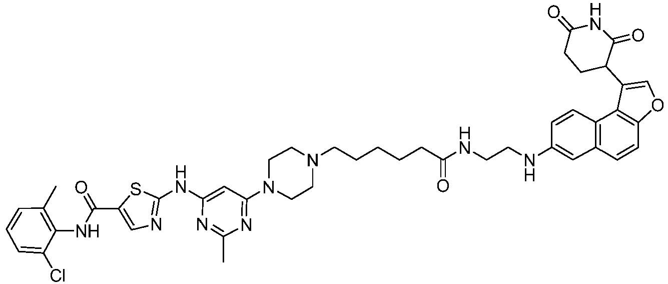

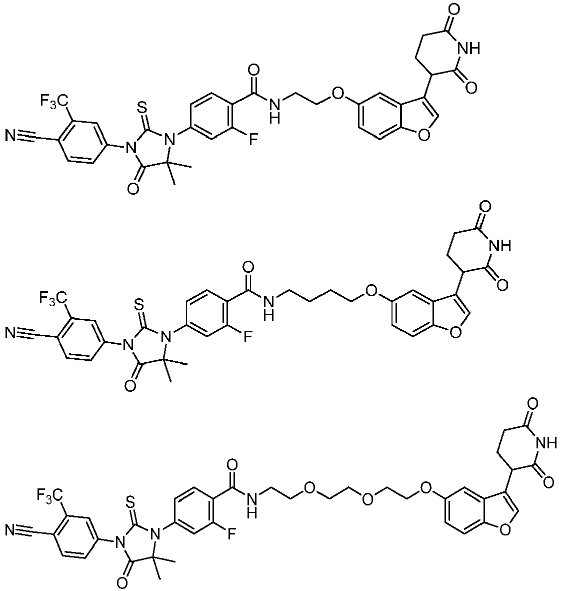

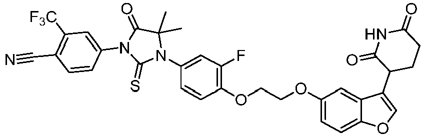

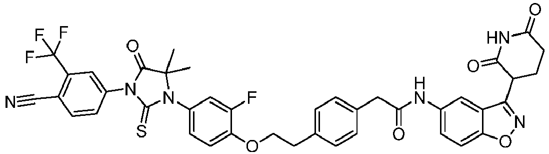

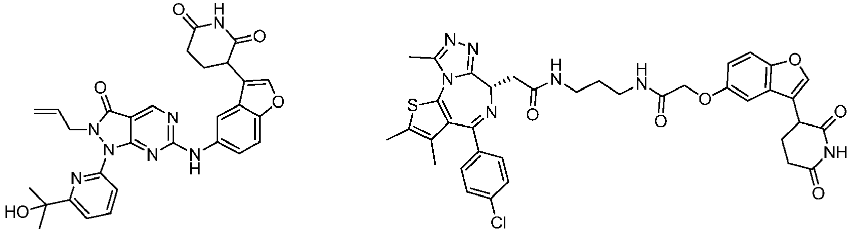

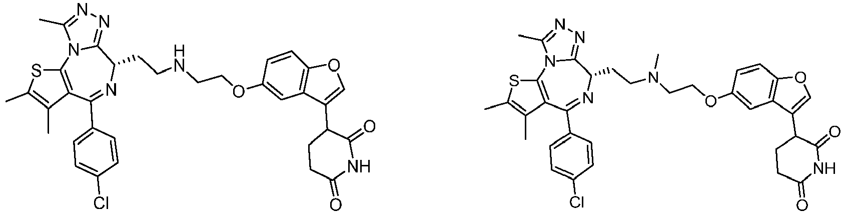

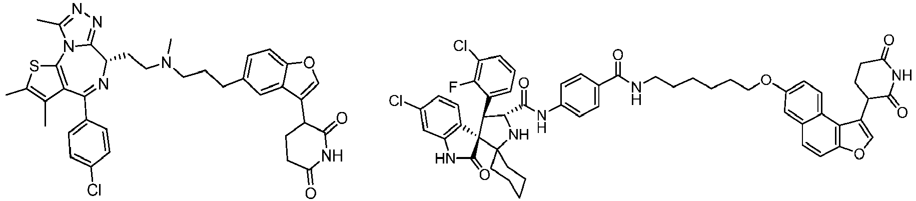

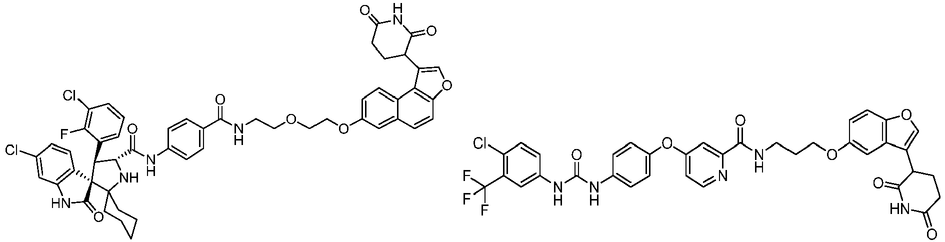

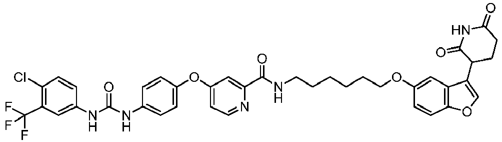

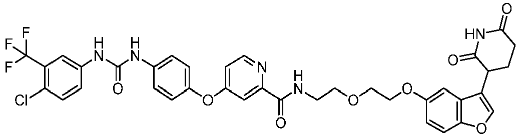

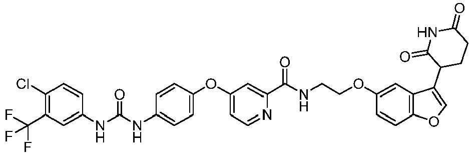

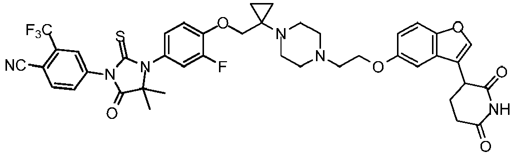

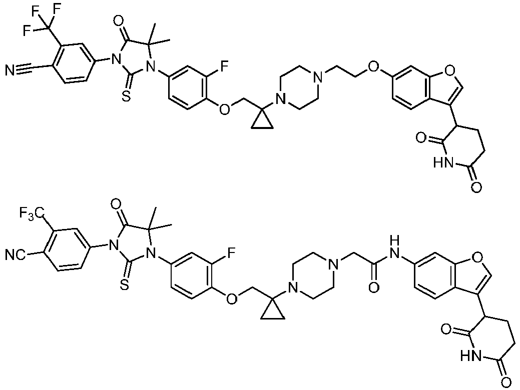

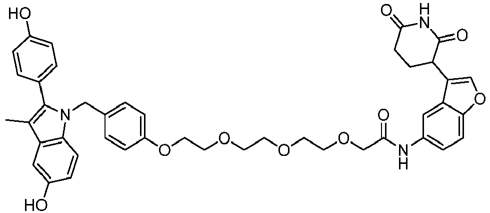

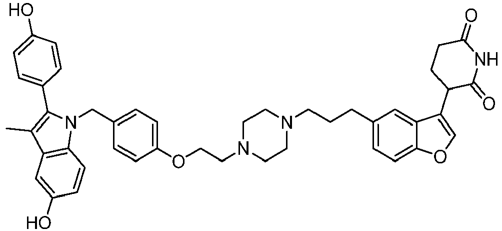

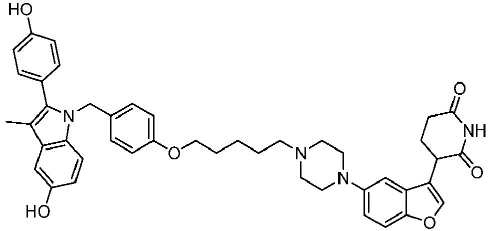

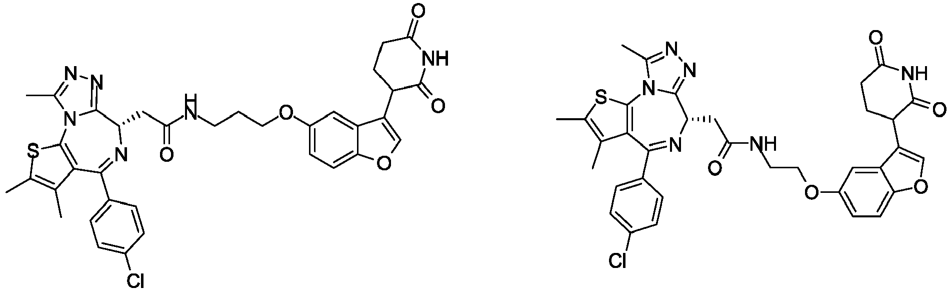

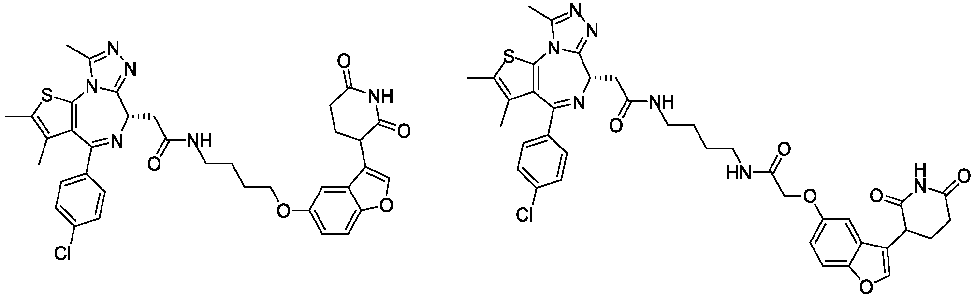

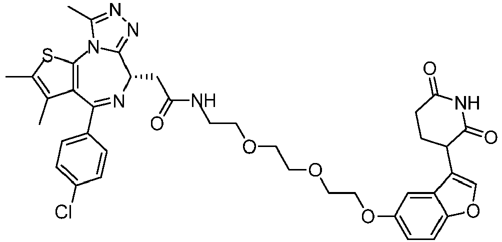

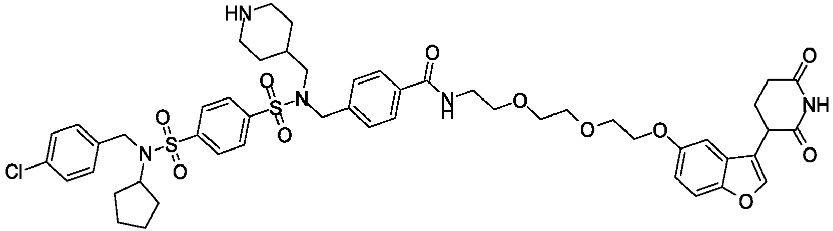

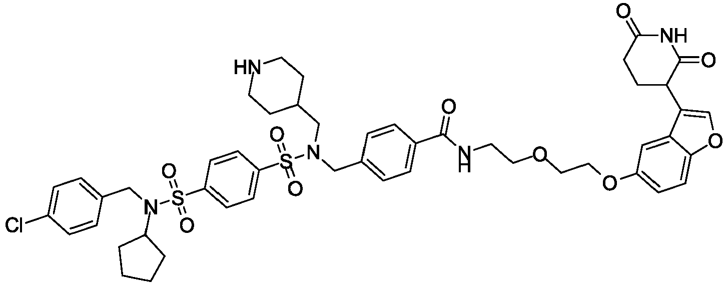

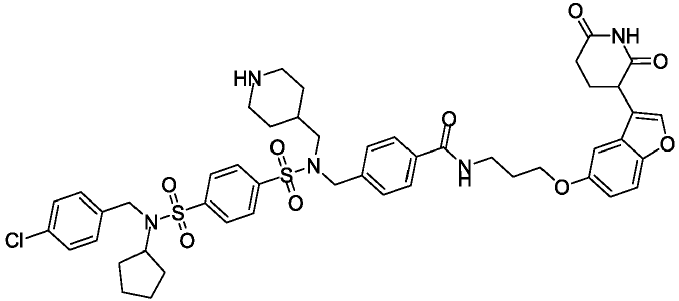

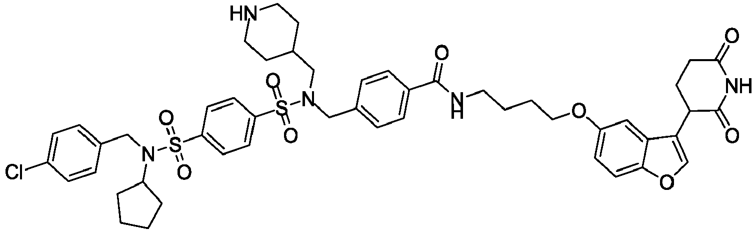

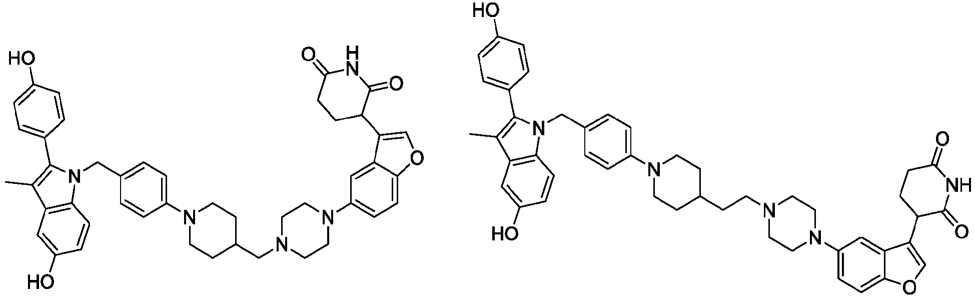

The present disclosure also provides use of the compound or the pharmaceutically acceptable salt thereof in the manufacture of a medicament for treating diseases related to ALK, BET, CDK, PARP, EGFR, γ-secretase, CBFβ-SMMHC, WEE1, MEK, BCR-ABL, MET, RAS, BTK, VEGFR, JAK, HER2, HDAC, Akt, PI3K, mTOR, AR, ER, PDEδ, SRC, MDM2, RAF, IRAK4, STAT3, and c-Myc.

### Technical effect

The compounds of the present disclosure have excellent protein degradation, cell proliferation inhibition and tumor shrinkage effects, and have good pharmacokinetic properties.

### Related Definitions

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" means a salt of compounds disclosed herein that is prepared by reacting the compound having a specific substituent disclosed herein with a relatively non-toxic acid or base. When compounds disclosed herein contain a relatively acidic functional group, a base addition salt can be obtained by bringing the compound into contact with a sufficient amount of base in a pure solution or a suitable inert solvent. The pharmaceutically acceptable base addition salt includes a salt of sodium, potassium, calcium, ammonium, organic amine or magnesium or similar salts. When compounds disclosed herein contain a relatively basic functional group, an acid addition salt can be obtained by bringing the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and an salt of amino acid (such as arginine and the like), and a salt of an organic acid such as glucuronic acid and the like. Certain specific compounds disclosed herein contain both basic and acidic functional groups and can be converted to any base or acid addition salt.

The term "targeted protein" refers to a protein or polypeptide that binds a compound of the present disclosure and is degraded.

The term "drugs or derivatives thereof" includes already developed drugs or derivatives thereof that can bind to a targeted protein and drugs or derivatives thereof developed that can bind to a targeted protein in the future.

The pharmaceutically acceptable salt disclosed herein can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

Compounds disclosed herein may be present in a specific geometric or stereoisomeric form. The present disclosure contemplates all such compounds, including cis and trans isomers, (-)- and (+)-enantiomers, (R)- and (S)-enantiomers, diastereoisomer, (D)-isomer, (L)-isomer, and a racemic mixture and other mixtures, for example, a mixture enriched in enantiomer or diastereoisomer, all of which are encompassed within the scope disclosed herein. The substituent such as alkyl may have an additional asymmetric carbon atom. All these isomers and mixtures thereof are encompassed within the scope disclosed herein.

Compounds disclosed herein may contain an unnatural proportion of atomic isotopes at one or more of the atoms that make up the compounds. For example, a compound may be labeled with a radioisotope such as tritium (³H), iodine-125 (¹²⁵I) or C-14 (¹⁴C). For another example, hydrogen can be replaced by heavy hydrogen to form a deuterated drug. The bond between deuterium and carbon is stronger than that between ordinary hydrogen and carbon. Compared with undeuterated drugs, deuterated drugs have advantages of reduced toxic side effects, increased drug stability, enhanced efficacy, and prolonged biological half-life of drugs. All changes in the isotopic composition of compounds disclosed herein, regardless of radioactivity, are included within the scope of the present disclosure.

The term "optional" or "optionally" means that the subsequent event or condition may occur but not requisite, that the term includes the instance in which the event or condition occurs and the instance in which the event or condition does not occur.

The term "substituted" means one or more than one hydrogen atom(s) on a specific atom are substituted by a substituent, including deuterium and hydrogen variants, as long as the valence of the specific atom is normal and the substituted compound is stable. When the substituent is oxo (i.e., =O), it means two hydrogen atoms are substituted. Positions on an aromatic ring cannot be substituted by oxo. The term "optionally substituted" means an atom can be substituted by a substituent or not, unless otherwise specified, the species and number of the substituent may be arbitrary so long as being chemically achievable.

The term "replaced by" means that a specified atom or group may be replaced with another specified atom or group. For example, CH₂ in CH₃CH₂CH₃ can be replaced by O, S, and NH to obtain CH₃OCH₃, CH₃SCH₃ and CH₃NHCH₃.

When any variable (such as R) occurs in the constitution or structure of the compound more than once, the definition of the variable at each occurrence is independent. Thus, for example, if a group is substituted by 0 to 2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of variables is a single bond, it means that the two groups linked by the single bond are connected directly. For example, when L in A-L-Z represents a single bond, the structure of A-L-Z is actually A-Z.

When an enumerated linking group does not indicate its linking direction, its linking direction is arbitrary. For example, when the linking group L in 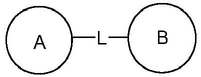 is -M-W-, the -M-W- can be linked to the ring A and the ring B in the same direction as the reading order from left to right to constitute 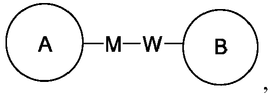 or can be linked to the ring A and the ring B in the reverse direction as the reading order from left to right to constitute 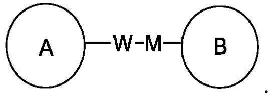 A combination of the linking groups, substituents and/or variants thereof is allowed only when such combination can result in a stable compound.

Unless otherwise specified, when a group has one or more connectable sites, any one or more sites of the group can be connected to other groups through chemical bonds. Where the connection position of the chemical bond is variable, and there is H atom(s) at a connectable site(s), when the connectable site(s) having H atom(s) is connected to the chemical bond, the number of H atom(s) at this site will correspondingly decrease as the number of the connected chemical bond increases, and the group will become a group of corresponding valence. When a group is a condensed ring structure, and the condensed ring structure is connected with other groups through variable chemical bonds, any one or more positions of the condensed ring can be connected with other groups through chemical bonds. The chemical bond between the site and other groups can be represented by a straight solid bond 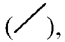 a straight dashed bond 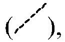 or a wavy line 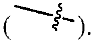 For example, the straight solid bond in -OCH₃ indicates that the group is connected to other groups through the oxygen atom in the group; the straight dashed bond in 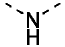 indicates that the group is connected to other groups through two ends of the nitrogen atom in the group; the wavy line in 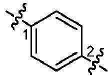 indicates that the group is connected to other groups through the 1- and 2-carbon atoms in the phenyl group; 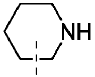 indicates that the piperidinyl group can be connected to other groups through any connectable sites thereon by one chemical bond, including at least four types of linkage, i.e., 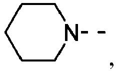 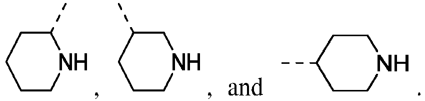 Even though a H atom is drawn on the -N-, 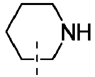 still includes the linkage type of 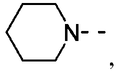 but where one bond is attached, the H at that site is reduced by one correspondingly, leading to a corresponding monovalent piperidyl. 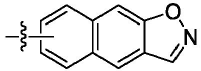 indicates that any connectable site on this naphtho[2,3-d]isoxazolyl can be connected to other groups by one chemical bond, including at least seven types of linkage, i.e.,

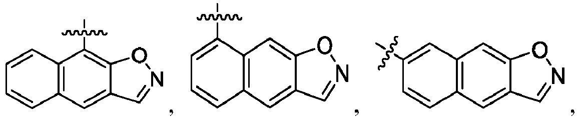

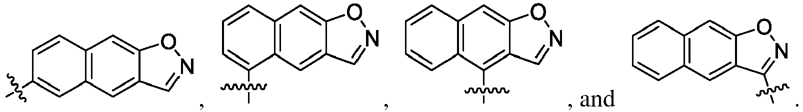

Unless otherwise specified, 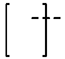 is used to indicate that a hydrogen atom at any site of a group within 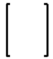 may be substituted.

Unless otherwise specified, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, C₈, C₉, C₁₀, C₁₁ and C₁₂, also includes any range from n to n+m, for example, C₁₋₁₂ includes C₁₋₃, C₁₋₆, C₁₋₉, C₃₋₆, C₃₋₉, C₃₋₁₂, C₆₋₉, C₆₋₁₂ and C₉₋₁₂, etc.; similarly, n membered to n+m membered indicates that the number of atoms on a ring is n to n+m, for example, 3-12 membered ring includes 3 membered ring, 4 membered ring, 5 membered ring, 6 membered ring, 7 membered ring, 8 membered ring, 9 membered ring, 10 membered ring, 11 membered ring, and 12 membered ring, also includes any range from n to n+m, for example, 3-12 membered ring includes 3-6 membered ring, 3-9 membered ring, 5-6 membered ring, 5-7 membered ring, 6-7 membered ring, 6-8 membered ring, and 6-10 membered ring, and the like.

Unless otherwise specified, the term "C₁₋₂₀ alkyl" is used to represent a linear or branched saturated hydrocarbon group composed of 1 to 20 carbon atoms. The C₁₋₂₀ alkyl includes C₁₋₁₉, C₁₋₁₈, C₁₋₁₇, C₁₋₁₆, C₁₋₁₅, C₁₋₁₄, C₁₋₁₃, C₁₋₁₂, C₁₋₁₁, C₁₋₁₀, C₁₋₉, C₁₋₈, C₁₋₇, C₁₋₆, C₁₋₅, C₁₋₄, C₁₋₃, C₁₋₂, C₁₈, C₁₇, C₁₆, C₁₅, C₁₄, C₁₃, C₁₂, C₁₁, C₁₀, C₁₋₉, C₈, C₇, C₆, and C₅ alkyl, etc. It may be monovalent (such as methyl), divalent (such as methylene) or multivalent (such as methenyl). Examples of the C₁₋₈ alkyl include, but are not limited to, methyl (Me), ethyl (Et), propyl (including n-propyl and isopropyl), butyl (including n-butyl, isobutyl, s-butyl and t-butyl), pentyl (including n-pentyl, isopentyl and neopentyl), hexyl, heptyl, octyl, and the like.

Unless otherwise specified, the term "C₁₋₆ alkyl" is used to represent a linear or branched saturated hydrocarbon group composed of 1 to 6 carbon atoms. The C₁₋₆ alkyl includes C₁₋₅, C₁₋₄, C₁₋₃, C₁₋₂, C₂₋₆, C₂₋₄, C₆, and C₅ alkyl, etc. It may be monovalent (such as methyl), divalent (such as methylene) or multivalent (such as methenyl). Examples of the C₁₋₆ alkyl include, but are not limited to, methyl (Me), ethyl (Et), propyl (including n-propyl and isopropyl), butyl (including n-butyl, isobutyl, s-butyl and t-butyl), pentyl (including n-pentyl, isopentyl and neopentyl), hexyl, and the like.

Unless otherwise specified, the term "C₁₋₃ alkyl" is used to represent a linear or branched saturated hydrocarbon group composed of 1 to 3 carbon atoms. The C₁₋₃ alkyl includes C₁₋₂ alkyl, C₂₋₃ alkyl, etc. It may be monovalent (such as methyl), divalent (such as methylene) or multivalent (such as m methenyl). Examples of the C₁₋₃ alkyl include, but are not limited to, methyl (Me), ethyl (Et), propyl (including n-propyl and isopropyl), and the like.

Unless otherwise specified, "C₂₋₄ alkenyl" is used to represent a linear or branched hydrocarbon group composed of 2 to 4 carbon atoms containing at least one carbon-carbon double bond, wherein the carbon-carbon double bond can be located at any position of the group. The C₂₋₄ alkenyl includes C₂₋₃, C₄, C₃, and C₂ alkenyl, etc. It may be monovalent, divalent or multivalent. Examples of the C₂₋₄ alkenyl include, but are not limited to, vinyl, propenyl, butenyl, butadienyl, and the like.

Unless otherwise specified, "C₂₋₄ alkynyl" is used to represent a linear or branched hydrocarbon group composed of 2 to 4 carbon atoms containing at least one carbon-carbon triple bond, wherein the carbon-carbon triple bond can be located at any position of the group. The C₂₋₄ alkynyl includes C₂₋₃, C₄, C₃, and C₂ alkynyl, etc. It may be monovalent, divalent or multivalent. Examples of the C₂₋₄ alkynyl include, but are not limited to, ethynyl, propynyl, butynyl, and the like.

Unless otherwise specified, "C₃₋₁₄ cycloalkyl" represents a saturated cyclic hydrocarbon group composed of 3 to 14 carbon atoms, which comprises monocyclic, bicyclic and tricyclic ring systems, wherein the bicyclic and tricyclic ring systems comprise spiro, fused, and bridged cyclic rings. The C₃₋₁₄ cycloalkyl includes C₃₋₁₂, C₃₋₁₀, C₃₋₈, C₃₋₆, C₃₋₅, C₄₋₁₀, C₄₋₈, C₄₋₆, C₄₋₅, C₅₋₈, and C₅₋₆ cycloalkyl, etc.; It may be monovalent, divalent or multivalent. Examples of the C₃₋₁₄ cycloalkyl include, but are not limited to, cyclopropyl, cyclobutyl, cyclopentyl, cyclohexyl, cycloheptyl, norbornyl, [2.2.2]bicycloocatane, [4.4.0]bicyclodecane, and the like.

Unless otherwise specified, the term "3- to 14-membered heterocycloalkyl" alone or in combination with other terms respectively represents a saturated cyclic group composed of 3 to 14 ring atoms, in which 1, 2, 3 or 4 ring atoms are heteroatoms independently selected from O, S and N, and the remainder is carbon atoms, wherein the nitrogen atom is optionally quaternized, and the nitrogen and sulfur heteroatoms are optionally oxidized (i.e., NO and S(O)ₚ, p is 1 or 2). The ring comprises monocyclic, bicyclic and tricyclic ring systems, wherein the bicyclic and tricyclic ring systems comprise spiro, fused, and bridged cyclic rings. In addition, with respect to the "3- to 14-membered heterocycloalkyl", the heteroatom may be present on the position of attachment of the heterocycloalkyl group to the remainder of a molecule. The 3- to 14-membered heterocycloalkyl includes 3-12 membered, 3-10 membered, 3-8 membered, 3-6 membered, 3-5 membered, 4-6 membered, 5-6 membered, 4 membered, 5 membered, and 6 membered heterocycloalkyl, etc. Examples of the 3- to 14-membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl and tetrahydrothien-3-yl and the like), tetrahydrofuranyl (including tetrahydrofuran-2-yl and the like), tetrahydropyranyl, piperidinyl (including 1-piperidinyl, 2-piperidinyl and 3-piperidinyl and the like), piperazinyl (including 1-piperazinyl and 2-piperazinyl and the like), morpholinyl (including 3-morpholinyl and 4-morpholinyl and the like), dioxanyl, dithianyl, isoxazolidinyl, isothiazolidinyl, 1,2-oxazinyl, 1,2-thiazinyl, hexahydropyridazinyl, homopiperazinyl, homopiperidinyl or dioxepanyl, and the like.

Unless otherwise specified, the term "3- to 8-membered monoheterocycloalkyl" alone or in combination with other terms respectively represents a saturated cyclic group composed of 3 to 8 ring atoms, in which 1, 2, 3 or 4 ring atoms are heteroatoms independently selected from O, S and N, and the remainder is carbon atoms, wherein the nitrogen atom is optionally quaternized, and the nitrogen and sulfur heteroatoms are optionally oxidized (i.e., NO and S(O)ₚ, p is 1 or 2). The ring is a monocyclic ring structure. In addition, with respect to the "3- to 8-membered monoheterocycloalkyl", the heteroatom may be present on the position of attachment of the heterocycloalkyl group to the remainder of a molecule. The 3- to 8-membered monoheterocycloalkyl includes 3-6 membered, 3-5 membered, 4-6 membered, 5-6 membered, 4 membered, 5 membered, and 6 membered heterocycloalkyl, etc. Examples of the 3- to 8-membered monoheterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl and tetrahydrothien-3-yl and the like), tetrahydrofuranyl (including tetrahydrofuran-2-yl and the like), tetrahydropyranyl, piperidinyl (including 1-piperidinyl, 2-piperidinyl and 3-piperidinyl and the like), piperazinyl (including 1-piperazinyl and 2-piperazinyl and the like), morpholinyl (including 3-morpholinyl and 4-morpholinyl and the like), dioxanyl, dithianyl, isoxazolidinyl, isothiazolidinyl, 1,2-oxazinyl, 1,2-thiazinyl, hexahydropyridazinyl, homopiperazinyl, homopiperidinyl or dioxepanyl, and the like.

Unless otherwise specified, the term "5- to 14-membered bridged heterocycloalkyl" alone or in combination with other terms respectively represents a saturated cyclic group composed of 5 to 14 ring atoms, in which 1, 2, 3 or 4 ring atoms are heteroatoms independently selected from O, S and N, and the remainder is carbon atoms, wherein the nitrogen atom is optionally quaternized, and the nitrogen and sulfur heteroatoms are optionally oxidized (i.e., NO and S(O)ₚ, p is 1 or 2). The ring comprises bridged and fused rings of bicyclic and tricyclic ring systems. In addition, with respect to the "5- to 14-membered bridged heterocycloalkyl", the heteroatom may be present on the position of attachment of the heterocycloalkyl group to the remainder of a molecule. The 5- to 14-membered heterocycloalkyl includes 5-12 membered, 5-10 membered, 5-8 membered, 5-6 membered, 5 membered, and 6 membered heterocycloalkyl, etc.

Unless otherwise specified, the term "5- to 14-membered spiroheterocycloalkyl" alone or in combination with other terms respectively represents a saturated cyclic group composed of 5 to 14 ring atoms, in which 1, 2, 3 or 4 ring atoms are heteroatoms independently selected from O, S and N, and the remainder is carbon atoms, wherein the nitrogen atom is optionally quaternized, and the nitrogen and sulfur heteroatoms are optionally oxidized (i.e., NO and S(O)ₚ, p is 1 or 2). The ring comprises spiro rings of bicyclic and tricyclic ring systems. In addition, with respect to the "5- to 14-membered spiroheterocycloalkyl", the heteroatom may be present on the position of attachment of the heterocycloalkyl group to the remainder of a molecule. The 5- to 14-membered heterocycloalkyl includes 5-12 membered, 5-10 membered, 5-8 membered, 5-6 membered, 5 membered, and 6 membered heterocycloalkyl, etc. Unless otherwise specified, the terms "C₆₋₁₂ aromatic ring" and "C₆₋₁₂ aryl" may be used interchangeably in this disclosure. The term"C₆₋₁₂ aromatic ring" or "C₆₋₁₂ aryl" means a cyclic hydrocarbon group having a conjugated pi electron system and consisting of 6 to 12 ring atoms. It may be a monocyclic, fused bicyclic or fused tricyclic ring system, wherein each ring is aromatic. It may be monovalent, divalent or multivalent. The C₆₋₁₂ aryl includes C₆₋₁₀, C₆₋₉, C₆₋₈, C₁₂, C₁₀ and C₆ aryl, etc. Examples of C₆₋₁₂ aryl include, but are not limited to, phenyl, naphthyl (including 1-naphthyl and 2-naphthyl, etc.).

Unless otherwise specified, the terms "5- to 12-membered heteroaromatic ring" and "5- to 12-membered heteroaryl" may be used interchangeably. The term "5- to 12-membered heteroaryl" means a cyclic group having a conjugated pi electron system and consisting of 5 to 12 ring atoms, in which 1, 2, 3 or 4 ring atoms are heteroatoms independently selected from O, S and N, and the remainder is carbon atoms. It may be a monocyclic, fused bicyclic or fused tricyclic ring system, wherein each ring is aromatic, and wherein the nitrogen atom is optionally quaternized and the nitrogen and sulfur heteroatoms are optionally oxidized (i.e., NO and S(O)ₚ, p is 1 or 2). A 5- to 12-membered heteroaryl can be attached to the remainder of the molecule through a heteroatom or a carbon atom. The 5- to 12-membered heteroaryl group includes 5- to 10-membered, 5- to 8-membered, 5- to 7-membered, 5- to 6-membered, 5-membered and 6-membered heteroaryl groups. Examples of the 5-12 membered heteroaryl include, but are not limited to, pyrrolyl (including N-pyrrolyl, 2-pyrrolyl, 3-pyrrolyl, and the like), pyrazolyl (including 2-pyrazolyl and 3-pyrazolyl, and the like), imidazolyl (including N-imidazolyl, 2-imidazolyl, 4-imidazolyl, and 5-imidazolyl, and the like), oxazolyl (including 2-oxazolyl, 4-oxazolyl, and 5-oxazolyl, and the like), triazolyl (1H-1,2,3-triazolyl, 2H-1,2,3-triazolyl, 1H-1,2,4-triazolyl and 4H-1,2,4-triazolyl, and the like), tetrazolyl, isoxazolyl (3-isoxazolyl, 4-isoxazolyl and 5-isoxazolyl, and the like), thiazolyl (including 2-thiazolyl, 4-thiazolyl and 5-thiazolyl, and the like), furyl (including 2-furyl and 3-furyl, and the like), thienyl (including 2-thienyl and 3-thienyl, and the like), pyridyl (including 2-pyridyl, 3-pyridyl and 4-pyridyl, and the like), pyrazinyl or pyrimidinyl (including 2-pyrimidinyl and 4-pyrimidinyl, and the like), benzothiazolyl (including 5-benzothiazolyl, and the like), purinyl, benzimidazolyl (including 2-benzimidazolyl, and the like), benzoxazolyl, indolyl (including 5-indolyl, and the like), isoquinolyl (including 1-isoquinolyl, 5-isoquinolyl, and the like), quinoxalinyl (including 2-quinoxalinyl, 5-quinoxalinyl, and the like) or quinolyl (including 3-quinolyl, 6-quinolyl, and the like).

The term "leaving group" refers to a functional group or atom which can be replaced by another functional group or atom through a substitution reaction (such as nucleophilic substitution reaction). For example, representative leaving groups include triflate; chlorine, bromine and iodine; sulfonate group, such as mesylate, tosylate, p-bromobenzenesulfonate, p-toluenesulfonate and the like; acyloxy, such as acetoxy, trifluoroacetoxy and the like.

The term "protecting group" includes, but is not limited to "amino protecting group", "hydroxy protecting group" or "thio protecting group". The term "amino protecting group" refers to a protecting group suitable for blocking the side reaction on the nitrogen of an amino. Representative amino protecting groups include, but are not limited to: formyl; acyl, such as alkanoyl (e.g. acetyl, trichloroacetyl or trifluoroacetyl); alkoxycarbonyl, such as tert-butoxycarbonyl (Boc); arylmethoxycarbonyl such as benzyloxycarbonyl (Cbz) and 9-fluorenylmethoxycarbonyl (Fmoc); arylmethyl such as benzyl (Bn), trityl (Tr), 1,1-bis-(4'-methoxyphenyl)methyl; silyl such as trimethylsilyl (TMS) and tert-butyldimethylsilyl (TBS) and the like. The term "hydroxy protecting group" refers to a protecting group suitable for blocking the side reaction on hydroxy. Representative hydroxy protecting groups include, but are not limited to: alkyl such as methyl, ethyl and tert-butyl; acyl such as alkanoyl (e.g. acetyl); arylmethyl such as benzyl (Bn), p-methoxybenzyl (PMB), 9-fluorenylmethyl (Fm), and diphenylmethyl (benzhydryl, DPM); silyl such as trimethylsilyl (TMS) and tert-butyl dimethyl silyl (TBS) and the like.

Compounds disclosed herein can be prepared by a variety of synthetic methods well known to those skilled in the art, including the following enumerated embodiment, the embodiment formed by the following enumerated embodiment in combination with other chemical synthesis methods, and equivalent replacement well known to those skilled in the art. Alternative embodiments include, but are not limited to the embodiment disclosed herein.

The structures of compounds disclosed herein can be confirmed by conventional methods well known to those skilled in the art. If the present disclosure relates to an absolute configuration of a compound, the absolute configuration can be confirmed by conventional techniques in the art, such as single crystal X-Ray diffraction (SXRD). In the single crystal X-Ray diffraction (SXRD), the diffraction intensity data of the cultivated single crystal is collected using a Bruker D8 venture diffractometer with a light source of CuKα radiation in a scanning mode of ϕ/ω scan; after collecting the relevant data, the crystal structure is further analyzed by the direct method (Shelxs97) to confirm the absolute configuration.

Solvents used in the present disclosure are commercially available.

Compounds are named according to general naming principles in the art or by ChemDraw^{®} software, and commercially available compounds are named with their vendor directory names.

### BRIEF DESCRIPTION OF THE DRAWINGS

FIG. 1 is a graph of in vitro assaying of ALK protein levels and phosphorylations level thereof in human lung cancer NCI-H2228 cells.
FIG. 2 is a graph of in vitro assaying of BRD4 protein levels and downstream c-Myc levels in human acute myeloid leukemia MV4-11 cells.
FIG. 3 is a graph of in vitro assaying of PDEδ protein levels in human lung cancer H358 cells.

### DETAILED DESCRIPTION OF THE INVENTION

The present disclosure is described in detail below by means of examples. However, it is not intended that these examples have any disadvantageous limitations to the present disclosure. The present disclosure has been described in detail herein, and embodiments are also disclosed herein. It will be apparent to those skilled in the art that various changes and modifications may be made to the embodiments disclosed herein without departing from the spirit and scope disclosed herein.

### Reference example 1

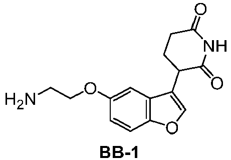

### Synthetic route:

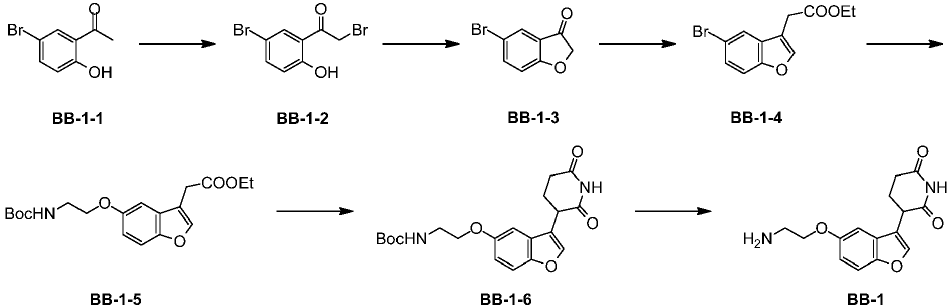

### Step 1: Synthesis of intermediate BB-1-2

Compound **BB-1-1** (100 g, 465.02 mmol) was dissolved in a mixture of trichloromethane (500 mL) and ethyl acetate (500 mL) at room temperature, and copper bromide (207.73 g, 930.04 mmol) was then added. The reaction mixture was heated to 100 °C and stirred to react for 14 hours. After the reaction was completed, the mixture was cooled down to room temperature, and filtered. The solvent was removed from the filtrate under reduced pressure. The resulting residue was added to water (200 mL) and extracted with dichloromethane (200 mL x 3). The organic phases were combined, washed with brine (300 mL×2), dried over anhydrous sodium sulfate, and filtered. The resulting intermediate **BB-1-2** was stored in dichloromethane (600 mL) and used directly in the next reaction step.

### Step 2: Synthesis of intermediate BB-1-3

To the above solution of intermediate **BB-1-2** in dichloromethane (465.02 mmol, 600 mL), triethylamine (47.06 g, 465.02 mmol, 64.73 mL) was added at 0°C, and the reaction mixture was allowed to warm to room temperature and stirred to react for 0.5 hours. After the reaction was completed, water (300 mL) was added to the reaction solution. The layers were separated. The aqueous phase was extracted with dichloromethane (200 mL×3). All organic phases were combined, washed with brine (400 mL×2), dried over anhydrous sodium sulfate, and filtered. The resulting intermediate **BB-1-3** was stored in dichloromethane (1200 mL) and used directly in the next reaction step.

### Step 3: Synthesis of intermediate BB-1-4

To the above solution of intermediate **BB-1-3** in dichloromethane (465.02 mmol, 1200 mL) was added toluene (2000 mL) followed by ethyl(triphenylphosphonium) acetate (194.40 g, 558.02 mmol) at room temperature under nitrogen. The reaction mixture was heated to 130°C and stirred to react for 60 hours. After the reaction was completed, the mixture was cooled down to room temperature, and the solvent was removed under reduced pressure. The resulting residue was separated by column chromatography (eluent: petroleum ether/ethyl acetate = 1/0-70/1, v/v) to give the intermediate **BB-1-4.** ¹H NMR (400 MHz, CDCl₃) δ: 7.72 (d, *J =* 2.0 Hz, 1H), 7.64 (s, 1H), 7.43-7.38 (m, 1H), 7.37-7.33 (m, 1H), 4.21 (q, *J =* 7.2 Hz, 2H), 3.66 (d, *J* = 0.8 Hz, 2H), 1.29 (t, *J* = 7.2 Hz, 3H).

### Step 4: Synthesis of intermediate BB-1-5

Intermediates **BB-1-4** (5.01 g, 17.70 mmol) and tert-butyl N-(2-hydroxyethyl)carbamate (2.85 g, 17.70 mmol) were added to toluene (60 mL) at room temperature under nitrogen, and 2-di-tert-butylphosphino-2',4',6'-triisopropylbiphenyl (751.44 mg, 1.77 mmol), cesium carbonate (11.53 g, 35.39 mmol) and tris(dibenzylideneacetone)dipalladium (1.62 g, 1.77 mmol) were then added sequentially. The reaction mixture was heated to 110 °C and stirred to react for 14 hours. After the reaction was completed, the mixture was cooled down to room temperature. Water (80 mL) was added, and the mixture was extracted with ethyl acetate (60 mL×3). The organic phases were combined, washed with saturated brine (150 mL×2), dried over anhydrous sodium sulfate, and filtered. The solvent was removed from the filtrate under reduced pressure. The resulting residue was separated by column chromatography (eluent: petroleum ether/ethyl acetate = 20/1-10/1, v/v) to give the intermediate **BB-1-5.** ¹H NMR (400 MHz, CDCl₃) δ: 7.61 (s, 1H), 7.50 (d, *J* = 7.6 Hz, 1H), 7.02 (d, *J* = 2.4 Hz, 1H), 6.91 (dd, *J* = 2.4, 8.8 Hz, 1H), 4.20 (q, *J* = 7.1 Hz, 2H), 3.66 (d, *J* = 0.8 Hz, 2H), 3.60-3.53 (m, 2H), 3.43-3.36 (m, 2H), 1.45 (s, 9H), 1.29 (t, *J* = 6.8 Hz, 3H).

### Step 5: Synthesis of intermediate BB-1-6

Intermediate **BB-1-5** (1.37 g, 3.76 mmol) was added to N,N-dimethylformamide (30 mL) followed by potassium tert-butoxide (421.48 mg, 3.76 mmol) at 0°C under nitrogen. The reaction mixture was stirred to react at 0°C for 0.5 h. Subsequently, acrylamide (266.98 mg, 3.76 mmol) was added. The reaction mixture was stirred to react at 0°C for another 1 h. After the reaction was completed, the mixture was allowed to warm to room temperature. Water (50 mL) was added and the mixture was extracted with ethyl acetate (50 mL×3). The organic phases were combined, washed with saturated brine (100 mL×2), dried over anhydrous sodium sulfate, and filtered. The solvent was removed from the filtrate under reduced pressure. The resulting residue was separated by column (eluent: petroleum ether/ethyl acetate = 3/1-2/3, v/v) to give the intermediate **BB-1-6.** MS-ESI *m*/*z*: 411.0 [M+Na]⁺.

### Step 6: Synthesis of intermediate BB-1 hydrochloride

Intermediate **BB-1-6** (227 mg, 533.93 µmol) was added to a solution of hydrochloric acid in ethyl acetate (20 mL, 4 M) at room temperature under nitrogen, and the reaction mixture was stirred to react at room temperature for 14 h. After the reaction was completed, the solvent was directly removed from the reaction solution under reduced pressure to give the intermediate **BB-1** hydrochloride.

### Reference example 2

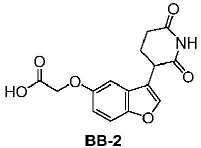

### Synthetic route:

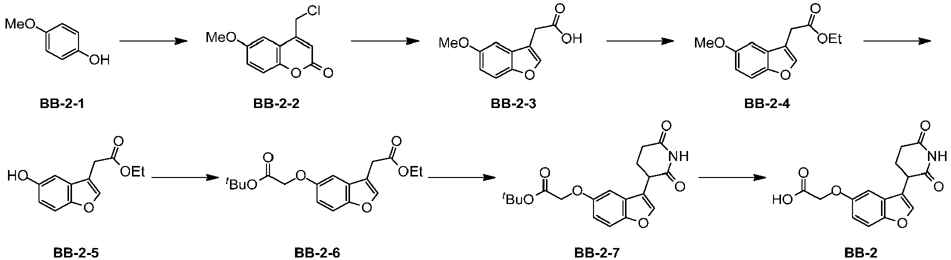

### Step 1: Synthesis of intermediate BB-2-2

To a mixture of compound **BB-2-1** (121.00 g, 974.73 mmol) and ethyl 4-chloroacetoacetate (160.43 g, 974.73 mmol) was added concentrated sulfuric acid (300 mL, purity: 98%) slowly dropwise at 0°C. Then the reaction mixture was warmed up to room temperature and stirred to react for 14 hours. After the reaction was completed, the reaction solution was poured into ice water (1 L) and stirred at room temperature for 1 h. The mixture was filtered, and the filter cake was washed with water (200 mL). The filter cake was collected and dried under vacuum to give the intermediate **BB-2-2.** MS-ESI *m*/*z*: 224.8 [M+H]⁺. ¹H NMR (400 MHz, CDCl₃) δ: 7.30 (d, *J* = 9.2 Hz, 1H), 7.16 (dd, *J* = 2.8, 9.2 Hz, 1H), 7.10 (d, *J*= 2.8 Hz, 1H), 6.59 (s, 1H), 4.66 (d, *J* = 0.8 Hz, 2H), 3.89 (s, 3H).

### Step 2: Synthesis of intermediate BB-2-3

Sodium hydroxide (50.33 g, 1.26 mol) was dissolved in water (500 mL) at room temperature, and intermediate **BB-2-2** (45.66 g, 139.80 mmol) was then added. The reaction mixture was heated to 80°C and stirred to react for 14 hours. After the reaction was completed, the reaction solution was cooled down to room temperature. The reaction solution was extracted with dichloromethane (200 mL×3), and the layers were separated. The organic phase was discarded, and the aqueous phase was collected. The aqueous phase was adjusted to pH 2-3 with 4 M dilute hydrochloric acid solution and extracted with dichloromethane (500 mL×3). The organic phases were combined, washed with saturated brine (1000 mL×2), dried over anhydrous sodium sulfate, and filtered. The solvent was removed from the filtrate under reduced pressure to give the intermediate **BB-2-3.** ¹H NMR (400MHz, CDCl₃) δ: 7.61 (s, 1H), 7.38 (d, *J* = 8.8 Hz, 1H), 7.00 (d, *J* = 2.4 Hz, 1H), 6.92 (dd, *J* = 2.4, 8.8 Hz, 1H), 3.85 (s, 3H), 3.73 (d, *J* = 1.2 Hz, 2H).

### Step 3: Synthesis of intermediate BB-2-4

Intermediate **BB-2-3** (28.90 g, 136.07 mmol) was dissolved in anhydrous ethanol (200 mL) at room temperature, and concentrated sulfuric acid (27.24 g, 272.13 mmol, 14.8 mL, purity: 98%) was then addd. The reaction mixture was heated to 80°C and stirred to react for 14 hours. After the reaction was completed, the mixture was cooled down to room temperature. The reaction solution was slowly added to ice water (300 mL) and extracted with ethyl acetate (100 mL×3). The organic phases were combined, washed with saturated brine (300 mL×2), dried over anhydrous sodium sulfate, and filtered. The solvent was removed from the filtrate under reduced pressure. The resulting residue was separated by preparative column chromatography (eluent: petroleum ether/ethyl acetate = 50/1-30/1, v/v) to give the intermediate **BB-2-4.** ¹H NMR (400MHz, CDCl₃) δ: 7.61 (s, 1H), 7.37 (d, *J* = 8.8 Hz, 1H), 7.02 (d, *J* = 2.4 Hz, 1H), 6.91 (dd, *J* = 2.8, 8.8 Hz, 1H), 4.20 (q, *J* = 7.1 Hz, 2H), 3.85 (s, 3H), 3.67 (d, *J* = 0.8 Hz, 2H), 1.28 (t, *J* = 7.2 Hz, 3H).

### Step 4: Synthesis of intermediate BB-2-5

Intermediate **BB-2-4** (25.63 g, 107.69 mmol) was dissolved in anhydrous dichloromethane (300 mL) at room temperature and the mixture was cooled down to -78 °C. Subsequently, boron tribromide (80.95 g, 323.06 mmol, 31.13 mL) was added and the reaction mixture was warmed up to room temperature and stirred to react for 2 hours. After the reaction was completed, the reaction solution was slowly added to ice water (500 mL) and extracted with dichloromethane (100 mL×3). The organic phases were combined, washed with saturated brine (300 mL×2), dried over anhydrous sodium sulfate, and filtered. The solvent was removed from the filtrate under reduced pressure. The resulting residue was separated by preparative column chromatography (eluent: petroleum ether/ethyl acetate = 10/1-4/1, v/v) to give the intermediate **BB-2-5.** ¹H NMR (400MHz, CDCl₃) δ: 7.59 (s, 1H), 7.30 (d, *J* = 8.8 Hz, 1H), 6.98 (d, *J* = 2.4 Hz, 1H), 6.81 (dd, *J* = 2.4, 8.8 Hz, 1H), 5.64 (s, 1H), 4.20 (q, *J* = 7.1 Hz, 2H), 3.64 (d, *J* = 0.8 Hz, 2H), 1.27 (t, *J* = 7.0 Hz, 3H).

### Step 5: Synthesis of intermediate BB-2-6

Intermediate **BB-2-5** (2.07 g, 9.08 mmol) was dissolved in acetonitrile (100 mL) at room temperature, and tert-butyl bromoacetate (2.66 g, 13.62 mmol) and potassium carbonate (2.51 g, 18.16 mmol) were added sequentially. The reaction mixture was heated to 65°C and stirred to react for 14 hours. After the reaction was completed, the mixture was cooled down to room temperature. The solvent was removed by concentration under reduced pressure. To the resulting residue was added water (60 mL) and the mixture was extracted with ethyl acetate (60 mL×3). The organic phases were combined, washed with saturated brine (100 mL), dried over anhydrous sodium sulfate, and filtered. The solvent was removed from the filtrate under reduced pressure. The resulting residue was separated by column chromatography (eluent: petroleum ether/ethyl acetate = 1011-511, v/v) to give the intermediate **BB-2-6.** ¹H NMR (400 MHz, CDCl₃) δ: 7.61 (s, 1H), 7.37 (d, *J* = 9.2 Hz, 1H), 7.01 (d, *J* = 2.4 Hz, 1H), 6.96 (dd, *J* = 2.8, 8.8 Hz, 1H), 4.55 (s, 2H), 4.19 (q, *J* = 7.2 Hz, 2H), 3.64 (d, *J* = 0.4 Hz, 2H), 1.51 (s, 9H), 1.27 (t, *J* = 7.2 Hz, 3H).

### Step 6: Synthesis of intermediate BB-2-7

Intermediate **BB-2-6** (2.70 g, 8.08 mmol) was dissolved in dimethylformamide (15 mL) at 0°C under nitrogen. Potassium tert-butoxide (996.73 mg, 8.88 mmol) and acrylamide (573.96 mg, 8.08 mmol) were added sequentially, and the reaction mixture was stirred to react at 0°C for 1 h. After the reaction was completed, water (50 mL) was added to the reaction solution and the resulting mixture was extracted with ethyl acetate (50 mL×3). The organic phases were combined, washed with saturated brine (100 mL), dried over anhydrous sodium sulfate, and filtered. The solvent was removed from the filtrate under reduced pressure. The resulting residue was separated by column chromatography (eluent: petroleum ether/ethyl acetate = 1/0-2/1, v/v) to give the intermediate **BB-2-7.** ¹H NMR (400 MHz, CDCl₃) δ: 8.06 (s, 1H), 7.56 (s, 1H), 7.41 (d, *J* = 8.8 Hz, 1H), 6.99-6.95 (m, 2H), 4.55 (s, 2H), 3.96 (t, *J* = 7.2 Hz, 1H), 2.84-2.67 (m, 2H), 2.38-2.32 (m, 2H), 1.50 (s, 9H).

### Step 7: Synthesis of intermediate BB-2

Intermediate **BB-2-7** (198.00 mg, 550.96 µmol) was dissolved in dichloromethane (15 mL) at room temperature, and trifluoroacetic acid (4.02 g, 35.26 mmol) was then added. The reaction mixture was stirred at room temperature for 2 h. After the reaction was completed, the solvent was removed under reduced pressure, and water (50 mL) was added to the resulting residue. The mixture was extracted with ethyl acetate (50 mL×3). The organic phases were combined, washed with saturated brine (100 mL), dried over anhydrous sodium sulfate, and filtered. The solvent was removed from the filtrate under reduced pressure to give the intermediate **BB-2.**

### Reference example 3

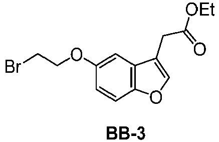

### Synthetic route:

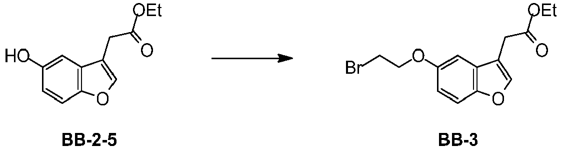

Intermediate **BB-2-5** (2.07 g, 9.08 mmol) was dissolved in toluene (100 mL) at room temperature, and 1,2-dibromoethane (8.53 g, 45.41 mmol), potassium carbonate (3.77 g, 27.25 mmol) and 18-crown-6 (24.00 g, 90.82 mmol) were added sequentially. The reaction mixture was heated to 110°C and stirred to react for 14 hours. After the reaction was completed, the mixture was cooled down to room temperature. The solvent was removed under reduced pressure and water (60 mL) was added to the resulting residue. The mixture was extracted with ethyl acetate (60 mL×3). The organic phases were combined, washed with saturated brine (100 mL), dried over anhydrous sodium sulfate, and filtered. The solvent was removed from the filtrate under reduced pressure. The resulting residue was separated by column chromatography (eluent: petroleum ether/ethyl acetate = 1/0-20/1, v/v) to give the intermediate **BB-3.** ¹H NMR (400 MHz, CDCl₃) δ: 7.62 (s, 1H), 7.38 (d, *J* = 8.8 Hz, 1H), 7.06 (d, *J* = 2.4 Hz, 1H), 6.94 (dd, *J* = 2.4, 8.8 Hz, 1H), 4.34 (t, *J* = 6.4 Hz, 2H), 4.20 (q, *J* = 7.2 Hz, 2H), 3.68-3.65 (m, 4H), 1.29 (t, *J* = 7.2 Hz, 3H).

### Reference example 4

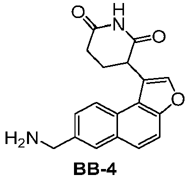

### Synthetic route:

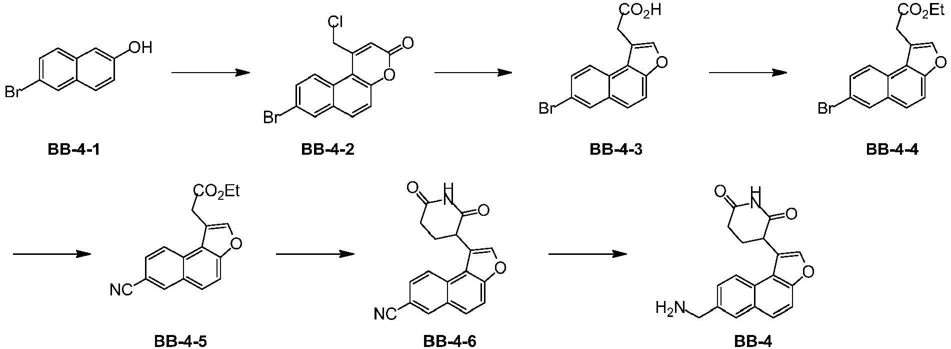

### Step 1: Synthesis of intermediate BB-4-2

Concentrated sulfuric acid (220.80 g, 2.21 mol, 120 mL, purity: 98%) was added dropwise to ice water (40 mL) at room temperature under nitrogen, and compound **BB-4-1** (10 g, 44.83 mmol) was then added. The mixture was cooled down to 5-10 °C. Ethyl 4-chloroacetoacetate (7.38 g, 44.83 mmol) was added slowly dropwise, and the reaction mixture was allowed to warm to room temperature and stirred to react for 16 h. The reaction mixture was then heated to 50 °C and stirred for another 16 h. After the reaction was completed, the mixture was cooled down to room temperature. The mixture was poured into ice water (1 L), and a large amount of solid was precipitated. The mixture was filtered, and the filter cake was collected. Toluene (400 mL) was added to the resulting solid, and the solvent was removed under reduced pressure. Toluene (400 mL) was added again, and the solvent was removed under reduced pressure to give the intermediate **BB-4-2.**

### Step 2: Synthesis of intermediate BB-4-3

Intermediate **BB-4-2** (14.5 g, 44.81 mmol) was dissolved in a solution of sodium hydroxide (8.70 g, 217.52 mmol) in water (150 mL) at room temperature under nitrogen. The reaction mixture was heated to 80°C and stirred to react for 5 hours. After the reaction was completed, the mixture was cooled down to room temperature. Dichloromethane (150 mL) was added for dilution. The organic phase was collected after the layers were separated, and the aqueous phase was extracted with dichloromethane (150 mL×3). The aqueous phase was adjusted to pH 4 with 2 M dilute hydrochloric acid and extracted with ethyl acetate (200 mL×3). The organic phases were combined, washed with saturated brine (50 mL×2), dried over anhydrous sodium sulfate, and filtered. The solvent was removed from the filtrate under reduced pressure to give the intermediate **BB-4-3.**

### Step 3: Synthesis of intermediate BB-4-4

Intermediate **BB-4-3** (11.3 g, 37.03 mmol) was dissolved in ethanol (300 mL) at room temperature under nitrogen, and concentrated sulfuric acid (2.08 g, 20.78 mmol, 1.13 mL, purity: 98%) was then added. The reaction mixture was heated to 80°C and stirred to react for 12 hours. After the reaction was completed, the mixture was cooled down to room temperature and concentrated under reduced pressure to remove the solvent. Water (150 mL) was added and the mixture was extracted with ethyl acetate (150 mL×1, 100 mL×3). The organic phases were combined, washed with saturated brine (50 mL×2), dried over anhydrous sodium sulfate, and filtered. The solvent was removed from the filtrate under reduced pressure. The resulting residue was separated by column chromatography (eluent: petroleum ether/ethyl acetate = 1/0-97/3, v/v) to give the intermediate **BB-4-4.** ¹H NMR (400 MHz, DMSO_*d*₆) δ: 8.35 (d, *J =* 2.0 Hz, 1H), 8.09 (t, *J* = 4.4 Hz, 2H), 7.86 (s, 2H), 7.73 (dd, *J* = 2.0, 8.8 Hz, 1H), 4.18-4.09 (m, 4H), 1.18 (t, *J* = 7.2 Hz, 3H).

### Step 4: Synthesis of intermediate BB-4-5

Intermediate **BB-4-4** (5 g, 15.01 mmol) was dissolved in N,N-dimethylformamide (80 mL) at room temperature under nitrogen, and potassium hexacyanoferrate (II) (1.16 g, 3.15 mmol), sodium carbonate (1.59 g, 15.01 mmol), and palladium acetate (336.92 mg, 1.50 mmol) were added sequentially. The reaction mixture was heated to 140°C and stirred to react for 8 hours. After the reaction was completed, the mixture was cooled down to room temperature. Water (300 mL) was added and the mixture was extracted with ethyl acetate (100 mL×5). The organic phases were combined, dried over anhydrous sodium sulfate, and filtered. The solvent was removed from the filtrate under reduced pressure. The resulting residue was separated by column chromatography (eluent: petroleum ether/ethyl acetate = 1/0-9/1, v/v) to give the intermediate **BB-4-5.** ¹H NMR (400MHz, CDCl₃) δ: 8.32 (d, *J =* 1.2 Hz, 1H), 8.30 (d, *J* = 8.8 Hz, 1H), 7.84 (s, 1H), 7.80-7.74 (m, 2H), 7.72 (dd, *J* = 1.8, 8.6 Hz, 1H), 4.23 (q, *J* = 7.2 Hz, 2H), 4.05 (s, 2H), 1.27 (t, *J* = 7.2 Hz, 3H).

### Step 5: Synthesis of intermediate BB-4-6

Intermediate **BB-4-5** (1.1 g, 3.94 mmol) was dissolved in N,N-dimethylformamide (20 mL) at room temperature, and acrylamide (279.94 mg, 3.94 mmol) and potassium tert-butoxide (441.95 mg, 3.94 mmol) were then added. The reaction mixture was stirred at room temperature for 2 h. After the reaction was completed, water (100 mL) was added and the mixture was extracted with ethyl acetate (30 mL×3). The organic phases were combined, washed with half-saturated brine (20 mL×2), dried over anhydrous sodium sulfate, and filtered. The solvent was removed by concentration under reduced pressure. To the resulting residue was added methanol (5 mL), and the mixture was stirred at room temperature for 5 min. A large amount of solid was precipitated. The mixture was filtered, and the solid was collected. The solvent was removed under reduced pressure to give the intermediate **BB-4-6.** ¹H NMR (400MHz, DMSO_*d*₆) δ: 10.96 (s, 1H), 8.69 (d, *J* = 1.6 Hz, 1H), 8.36 (d, *J* = 8.8 Hz, 1H), 8.12 (s, 1H), 8.02-7.93 (m, 2H), 7.86 (dd, *J* = 1.6, 8.4 Hz, 1H), 4.72 (dd, *J* = 4.4, 12.4 Hz, 1H), 2.95-2.81 (m, 1H), 2.71-2.60 (m, 1H), 2.48-2.38 (m, 1H), 2.34-2.18 (m, 1H).

### Step 6: Synthesis of intermediate BB-4 hydrochloride

Wet palladium on carbon (0.2 g, purity: 10%) was added to N-methylpyrrolidone (3 mL) at room temperature under argon, and intermediate **BB-4-6** (350 mg, 1.15 mmol) and a solution of hydrochloric acid in ethyl acetate (4 M, 2 mL) were then added. The atmosphere was replaced three times with hydrogen, and the reaction mixture was stirred to react at room temperature under hydrogen (15 psi) atmosphere for 12 h. Wet palladium on carbon (0.2 g, purity: 10%) was added supplementally to the above reaction mixture. The atmosphere was replaced three times with hydrogen, and the reaction mixture was stirred to react at room temperature under hydrogen (15 psi) atmosphere for another 12 h. After the reaction was completed, the reaction mixture was filtered directly and the solvent was removed from the filtrate under reduced pressure. The resulting residue was separated by preparative HPLC (mobile phase: acetonitrile/water; acidic system: 0.05% HCl) to give the intermediate **BB-4** hydrochloride. ¹H NMR (400MHz, DMSO_*d*₆) δ:10.95 (s, 1H), 8.57 (s, 3H), 8.23 (d, *J =* 8.4 Hz, 1H), 8.14 (s, 1H), 8.04 (s, 1H), 7.88-7.81 (m, 2H), 7.73 (d, *J* = 8.4 Hz, 1H), 4.71 (dd, *J* = 4.2 12.2 Hz, 1H), 4.25-4.15 (m, 2H), 2.98-2.82 (m, 1H), 2.74-2.59 (m, 1H), 2.45-2.36 (m, 1H), 2.34-2.22 (m, 1H).

### Reference example 5

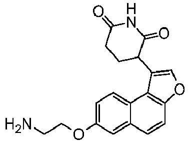

### Synthetic route:

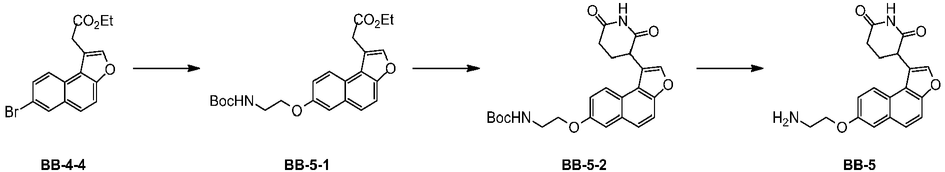

### Step 1: Synthesis of intermediate BB-5-1

Intermediate **BB-4-4** (5 g, 14.58 mmol) was dissolved in toluene (10 mL) at room temperature under nitrogen, and tert-butyl N-(2-hydroxyethyl)carbamate (2.82 g, 17.50 mmol), tris(dibenzylideneacetone)dipalladium (1.34 g, 1.46 mmol), 2-di-tert-butylphosphino-2',4',6'-triisopropylbiphenyl (1.86 g, 4.37 mmol) and cesium carbonate (9.50 g, 29.16 mmol) were then added. The reaction mixture was heated to 110°C and stirred to react for 12 hours. After the reaction was completed, the reaction solution was cooled down to room temperature. Water (100 mL) was added and the mixture was extracted with ethyl acetate (100 mL×3). The organic phases were combined, washed with saturated brine (150 mL×3), dried over anhydrous sodium sulfate, and filtered. The solvent was removed from the filtrate under reduced pressure. The resulting residue was separated by column chromatography (eluent: petroleum ether/ethyl acetate = 1/0-3/1, v/v) to give the intermediate **BB-5-1** (Purity: 83.34%). MS-ESI m/z: 436.1 [M+Na]⁺. ¹H NMR (400 MHz, CDCl₃) δ: 8.15 (d, *J* = 8.8 Hz, 1H), 7.74 (s, 1H), 7.63-7.61 (m, 2H), 7.30 (d, *J* = 2.4 Hz, 1H), 7.24 (dd, *J* = 2.6, 9.0 Hz, 1H), 4.22 (q, *J* = 7.1 Hz, 2H), 4.18-4.15 (m, 2H), 4.04 (s, 2H), 3.65-3.60 (m, 2H), 1.48 (s, 9H), 1.26 (t, *J* = 7.2 Hz, 3H).

### Step 2: Synthesis of intermediate BB-5-2

Intermediate **BB-5-1** (1 g, 2.02 mmol, purity: 83.34%) was dissolved in N,N-dimethylformamide (20 mL) at 0°C, and potassium tert-butoxide (248.79 mg, 2.22 mmol) and acrylamide (143.27 mg, 2.02 mmol) were then added. The reaction mixture was stirred at 0°C for 1 h. After the reaction was completed, water (50 mL) was added to the reaction solution and the mixture was extracted with ethyl acetate (50 mL×3). The organic phases were combined, washed with saturated brine (100 mL×3), dried over anhydrous sodium sulfate, and filtered. The solvent was removed from the filtrate under reduced pressure. The resulting residue was separated by column chromatography (eluent: petroleum ether/ethyl acetate = 1/0-1/1, v/v) to give the intermediate **BB-5-2.** MS-ESI m/z: 338.9 [M-Boc+H]⁺. ¹H NMR (400 MHz, CDCl₃) δ: 8.08 (s, 1H), 7.90 (d, *J* = 9.2 Hz, 1H), 7.68-7.62 (m, 3H), 7.32 (d, *J* = 2.0 Hz, 1H), 7.27-7.24 (m, 1H), 5.05 (br s, 1H), 4.49 (dd, *J* = 5.2, 8.4 Hz, 1H), 4.20-4.15 (m, 2H), 3.65-3.59 (m, 2H), 2.84-2.72 (m, 2H), 2.55-2.45 (m, 2H), 1.48 (s, 9H).

### Step 3: Synthesis of intermediate BB-5 hydrochloride

Intermediate **BB-5-2** (0.22 g, 479.26 µmol) was dissolved in ethyl acetate (10 mL) at room temperature, and a solution of hydrochloric acid in ethyl acetate (4 M, 4.78 mL) was added. The reaction mixture was stirred at room temperature for 12 hours. After the reaction was completed, the solvent was removed by concentration under reduced pressure to give the intermediate **BB-5** hydrochloride. ¹H NMR (400 MHz, DMSO_*d*₆) δ: 10.94 (s, 1H), 8.21 (br s, 3H), 8.14 (br d, *J* = 8.8 Hz, 1H), 7.98 (s, 1H), 7.81-7.75 (m, 2H), 7.57 (d, *J* = 2.4 Hz, 1H), 7.28 (dd, *J* = 2.8, 9.2 Hz, 1H), 4.64 (dd, *J* = 4.2, 11.8 Hz, 1H), 4.34-4.31 (m, 2H), 3.32-3.25 (m, 2H), 2.92-2.83 (m, 1H), 2.68-2.59 (m, 1H), 2.46-2.35 (m, 1H), 2.33-2.23 (m, 1H).

### Reference example 6

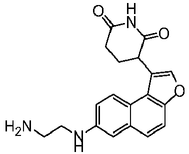

### Synthetic route:

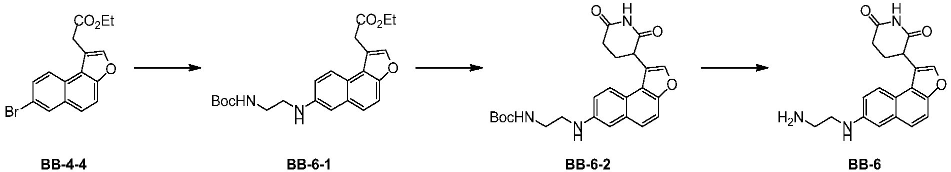

### Step 1: Synthesis of intermediate BB-6-1

Intermediates **BB-4-4** (1.5 g, 4.37 mmol) and N-tert-butoxycarbonylethylenediamine (840.98 mg, 5.25 mmol) were added to a mixture of toluene (20 mL) and water (4 mL) at room temperature under nitrogen, and tris(dibenzylideneacetone)dipalladium (280.39 mg, 306.20 µmol), 2-di-tert-butylphosphino-2',4',6'-triisopropylbiphenyl (260.05 mg, 612.40 µmol) and potassium phosphate (3.71 g, 17.50 mmol) were then added. The reaction mixture was heated to 100°C and stirred to react for 14 hours. After the reaction was completed, the mixture was cooled down to room temperature. Water (30 mL) was added, and the mixture was extracted with ethyl acetate (30 mL×3). The organic phases were combined, washed with saturated brine (50 mL×2), dried over anhydrous sodium sulfate, and filtered. The solvent was removed from the filtrate under reduced pressure. The resulting residue was separated by column chromatography (eluent: petroleum ether/ethyl acetate = 50/1-20/1, v/v) to give the intermediate **BB-6-1.** ¹H NMR (400 MHz, CDCl₃) δ: 8.03 (d, *J* = 9.6 Hz, 1H), 7.70 (s, 1H), 7.57-7.49 (m, 2H), 7.01-6.97 (m, 2H), 4.84 (br s, 1H), 4.22 (q, *J* = 7.1 Hz, 2H), 4.02 (s, 2H), 3.47-3.46 (m, 2H), 3.41-3.35 (m, 2H), 1.48 (s, 9H), 1.26 (t, *J* = 7.2 Hz, 3H).

### Step 2: Synthesis of intermediate BB-6-2

Intermediate **BB-6-1** (1.2 g, 2.91 mmol) was dissolved in N,N-dimethylformamide (20 mL) at 0°C, and potassium tert-butoxide (359.09 mg, 3.20 mmol) and acrylamide (206.78 mg, 2.91 mmol) were added sequentially. The reaction mixture was stirred at 0°C for 1 hour. After the reaction was completed, water (100 mL) was added to the reaction solution and the mixture was extracted with ethyl acetate (50 mL×3). The organic phases were combined, washed with saturated brine (100 mL×3), dried over anhydrous sodium sulfate, and filtered. The solvent was removed from the filtrate under reduced pressure. The resulting residue was added to ethyl acetate (10 mL), and the mixture was stirred at room temperature for 10 min. The mixture was filtered, and the solvent was removed from the resulting filter cake under reduced pressure to give the intermediate **BB-6-2** (purity: 86.70%). MS-ESI m/z: 438.0 [M+H]⁺. ¹H NMR (400 MHz, DMSO_*d*₆) δ: 10.92 (br s, 1H), 7.92-7.82 (m, 2H), 7.62-7.55 (m, 1H), 7.55-7.48 (m, 1H), 7.04-6.87 (m, 3H), 5.79 (br s, 1H), 4.57 (br dd, *J* = 4.2, 11.4 Hz, 1H), 3.22-3.12 (m, 4H), 2.92-2.80 (m, 1H), 2.67-2.55 (m, 1H), 2.40-2.29 (m, 1H), 2.29-2.19 (m, 1H), 1.40 (s, 9H).

### Step 3: Synthesis of intermediate BB-6 hydrochloride

Intermediate **BB-6-2** (0.46 g, 911.62 µmol, purity: 86.70%) was dissolved in ethyl acetate (5 mL) at room temperature, and a solution of hydrochloric acid in ethyl acetate (4 M, 15 mL) was then added. The reaction mixture was stirred at room temperature for 12 h. After the reaction was completed, the solvent was removed directly under reduced pressure to give the intermediate **BB-6** hydrochloride. ¹H NMR (400 MHz, DMSO_*d*₆) δ: 10.93 (s, 1H), 8.27 (br s, 3H), 7.99 (br d, *J* = 8.8 Hz, 1H), 7.91 (s, 1H), 7.69-7.60 (m, 2H), 7.29-7.23 (m, 1H), 7.19 (dd, *J* = 1.8, 9.0 Hz, 1H), 4.60 (br dd, *J* = 4.6, 11.8 Hz, 1H), 3.47 (t, *J* = 6.4 Hz, 2H), 3.14-3.05 (m, 2H), 2.92-2.80 (m, 1H), 2.66-2.57 (m, 1H), 2.43-2.31 (m, 1H), 2.29-2.19 (m, 1H).

### Reference example 7

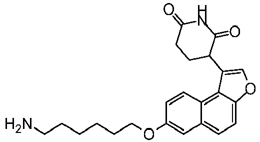

### Synthetic route:

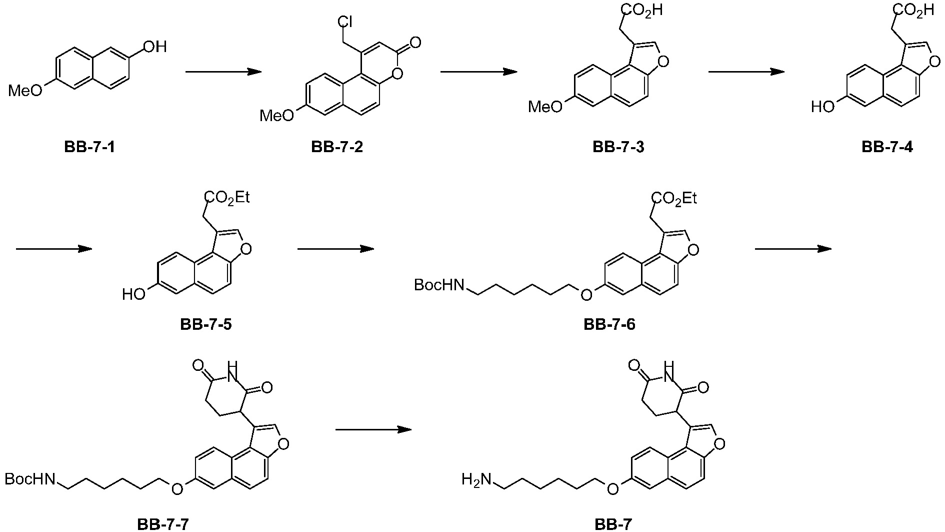

### Step 1: Synthesis of intermediate BB-7-2

Concentrated sulfuric acid (578.09 g, 5.78 mol, 314.18 mL, purity: 98%) was dissolved in ice water (156 mL) at 5-10 °C, and compound **BB-7-1** (25 g, 143.52 mmol) was then added, followed by slow dropwise addition of acetyl ethyl 4-chloroacetoacetate (25.98 g, 157.87 mmol). The reaction mixture was warmed up to room temperature and stirred to react for 12 hours. After the reaction was completed, the reaction mixture was poured into ice water (400 mL) and extracted with ethyl acetate (500 mL×3). The organic phases were combined, dried over anhydrous sodium sulfate, and filtered. The filtrate was concentrated under reduced pressure to remove the solvent. The resulting residue was added to methyl tert-butyl ether (30 mL), and the mixture was stirred at room temperature for 15 min, and then filtered. The solid was collected, and dried under reduced pressure to remove the solvent to give the intermediate **BB-7-2.** ¹H NMR (400MHz, DMSO_*d*₆) δ: 8.42 (d, *J* = 9.6 Hz, 1H), 8.13 (d, *J* = 9.2 Hz, 1H), 7.56-7.49 (m, 2H), 7.36 (dd, *J* = 2.6, 9.4 Hz, 1H), 6.82 (s, 1H), 5.35 (s, 2H), 3.90 (s, 3H).

### Step 2: Synthesis of intermediate BB-7-3

Sodium hydroxide (19.22 g, 480.53 mmol) was dissolved in water (116 mL) at room temperature, and then intermediate **BB-7-2** (12 g, 43.68 mmol) was added. The reaction mixture was heated to 80°C and stirred to react for 12 hours. After the reaction was completed, the mixture was cooled down to room temperature. Water (400 mL) was added and the mixture was extracted with ethyl acetate (100 mL). The organic phase was discarded and the aqueous phase was adjusted to pH 5-6 with 12 M concentrated hydrochloric acid and extracted with ethyl acetate (300 mL×3). The organic phases were combined, dried over anhydrous sodium sulfate, and filtered. The solvent was removed under reduced pressure to give the intermediate **BB-7-3.** MS-ESI *m*/*z*: 257.1 [M+H]⁺.

### Step 3: Synthesis of intermediate BB-7-4

Intermediate **BB-7-3** (8.9 g, 34.73 mmol) was dissolved in dichloromethane (180 mL) at 15 °C under nitrogen and the mixture was cooled down to -60 °C. Subsequently, boron tribromide (23.49 g, 93.77 mmol, 9.04 mL) was added slowly dropwise and the reaction mixture was slowly warmed up to 15 °C and stirred to react for 2 hours. After the reaction was completed, the reaction mixture was poured into ice water (200 mL), and the layers were separated. The organic phase was collected, and the aqueous phase was extracted with ethyl acetate (80 mL×3). The organic phases were combined, dried over anhydrous sodium sulfate, and filtered. The solvent was removed under reduced pressure to give the intermediate **BB-7-4.** MS-ESI m/z: 243.0 [M+H]⁺. ¹H NMR (400MHz, DMSO_*d*₆) δ: 12.51 (br s, 1H), 9.67 (s, 1H), 8.02 (d, *J* = 8.8 Hz, 1H), 7.94 (s, 1H), 7.65 (d, *J* = 9.2 Hz, 1H), 7.60 (d, *J* = 9.2 Hz, 1H), 7.29 (d, *J* = 2.4 Hz, 1H), 7.17 (dd, *J* = 2.6, 9.0 Hz, 1H), 4.00 (s, 2H).

### Step 4: Synthesis of intermediate BB-7-5

Intermediate **BB-7-4** (8 g, 33.03 mmol) was dissolved in ethanol (50 mL) at room temperature, then concentrated sulfuric acid (3.47 g, 34.68 mmol, 1.89 mL, purity: 98%) was added. The reaction mixture was heated to 80°C and stirred to react for 12 hours. After the reaction was completed, the mixture was cooled down to room temperature. The reaction mixture was directly concentrated under reduced pressure to remove the solvent, and the resulting residue was diluted with water (100 mL) and extracted with ethyl acetate (100 mL×3). The organic phases were combined, dried over anhydrous sodium sulfate, and filtered. The solvent was removed under reduced pressure. The resulting residue was separated by column chromatography (eluent: petroleum ether/ethyl acetate = 1/0-4/1, v/v) to give the intermediate **BB-7-5.** MS-ESI m/z: 271.1 [M+H]⁺. ¹H NMR (400 MHz, CDCl₃) δ: 8.12 (d, *J* = 8.4 Hz, 1H), 7.72 (s, 1H), 7.65-7.50 (m, 2H), 7.26 (s, 1H), 7.16 (d, *J* = 8.0 Hz, 1H), 5.12 (s, 1H), 4.22 (q, *J*= 7.2 Hz, 2H), 4.03 (s, 2H), 1.25 (t, *J* = 6.4 Hz, 3H).

### Step 5: Synthesis of intermediate BB-7-6

Tert-butyl (6-hydroxyhexyl)carbamate (5.23 g, 24.05 mmol) and intermediate **BB-7-5** (5 g, 18.50 mmol) were added to anhydrous tetrahydrofuran (120 mL) at 15 °C under nitrogen, and azodicarbonyl dipiperidine (7.00 g, 27.75 mmol) and tributylphosphine (5.61 g, 27.75 mmol, 6.85 mL) were then added sequentially. The mixture was cooled down to 0 °C and stirred for 1 h, then warmed up to 15 °C and stirred for another 12 h. After the reaction was completed, water (200 mL) was added and the reaction mixture was extracted with ethyl acetate (200 mL×2). The organic phases were combined, washed with saturated brine (300 mL×2), dried over anhydrous sodium sulfate, and filtered. The solvent was removed under reduced pressure. The resulting residue was separated by column chromatography (eluent: petroleum ether/ethyl acetate = 1/0-4/1, v/v) to give the intermediate **BB-7-6.** ¹H NMR (400 MHz, CDCl₃) δ: 8.13 (d, *J =* 9.2 Hz, 1H), 7.74 (s, 1H), 7.63-7.59 (m, 2H), 7.29 (d, *J =* 2.4 Hz, 1H), 7.24 (dd, *J* = 2.6, 9.0 Hz, 1H), 4.53 (s, 1H), 4.22 (q, *J* = 7.2 Hz, 2H), 4.09 (t, *J* = 6.4 Hz, 2H), 4.04 (s, 2H), 3.20-3.10 (m, 2H), 1.91-1.81 (m, 2H), 1.60-1.50 (m, 4H), 1.49-1.38 (m, 11H), 1.26 (t, *J* = 7.0 Hz, 3H).

### Step 6: Synthesis of intermediate BB-7-7

Intermediate **BB-7-6** (7.3 g, 15.55 mmol) was dissolved in tetrahydrofuran (140 mL) at 0°C under nitrogen. Acrylamide (1.10 g, 15.55 mmol) and a solution of potassium tert-butoxide in tetrahydrofuran (1 M, 15.55 mL) were added sequentially, and the reaction mixture was warmed up to room temperature and stirred to react for 2 h. A solution of potassium tert-butoxide in tetrahydrofuran (1 M, 3.11 mL) were added again, and the reaction mixture was stirred at room temperature to react for another 2 h. After the reaction was completed, water (200 mL) was added and the reaction mixture was extracted with ethyl acetate (200 mL×1, 150 mL×3). The organic phases were combined, washed with saturated brine (50 mL×2), dried over anhydrous sodium sulfate, and filtered. The solvent was removed from the filtrate under reduced pressure. The resulting residue was separated by column chromatography (eluent: petroleum ether/ethyl acetate = 1/0-4/1, v/v) to give the intermediate **BB-7-7.**

### Step 7: Synthesis of intermediate BB-7 hydrochloride

Intermediate **BB-7-7** (1.5 g, 3.03 mmol) was dissolved in ethyl acetate (10 mL) at room temperature, and a solution of hydrochloric acid in ethyl acetate (4 M, 30 mL) was then added. The reaction mixture was stirred at room temperature for 12 hours. After the reaction was completed, the reaction mixture was directly concentrated under reduced pressure to remove the solvent to give the intermediate **BB-7** hydrochloride. ¹H NMR (400 MHz, DMSO_*d*₆) δ: 10.94 (s, 1H), 8.07 (br d, *J* = 9.2 Hz, 1H), 8.00 (br s, 3H), 7.96 (s, 1H), 7.78-7.70 (m, 2H), 7.50 (d, *J* = 2.4 Hz, 1H), 7.21 (dd, *J* = 2.6, 9.0 Hz, 1H), 4.63 (br dd, *J* = 4.4, 12.0 Hz, 1H), 4.10 (t, *J* = 6.4 Hz, 2H), 2.94-2.83 (m, 1H), 2.82-2.71 (m, 2H), 2.68-2.57 (m, 1H), 2.47-2.34 (m, 1H), 2.33-2.19 (m, 1H), 1.85-1.73 (m, 2H), 1.65-1.55 (m, 2H), 1.53-1.36 (m, 4H).

### Reference example 8

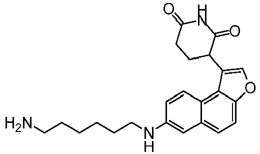

### Synthetic route:

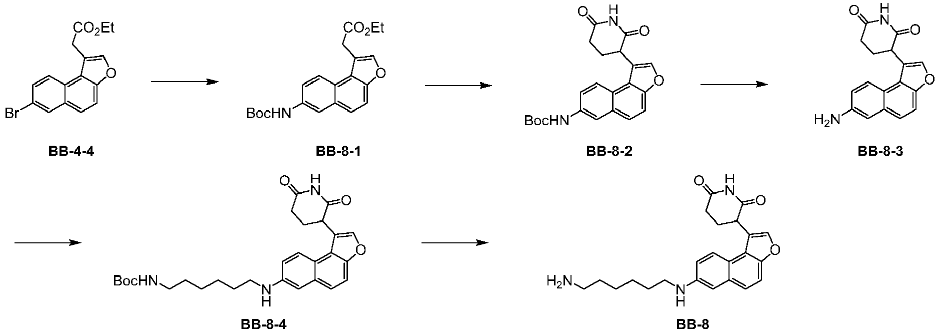

### Step 1: Synthesis of intermediate BB-8-1

Intermediates **BB-4-4** (10 g, 29.16 mmol) and tert-butyl carbamate (10.25 g, 87.49 mmol) were added to a mixture of toluene (100 mL) and water (20 mL) at room temperature under nitrogen, and tris(dibenzylideneacetone)dipalladium (1.87 g, 2.04 mmol), 2-di-tert-butylphosphino-2',4',6'-triisopropylbiphenyl (1.73 g, 4.08 mmol) and potassium phosphate (24.76 g, 116.65 mmol) were then added sequentially. The reaction mixture was heated to 100°C and stirred to react for 14 hours. After the reaction was completed, the mixture was cooled down to room temperature. Water (100 mL) was added, and the mixture was extracted with ethyl acetate (100 mL×3). The organic phases were combined, washed with saturated brine (150 mL×2), dried over anhydrous sodium sulfate, and filtered. The solvent was removed from the filtrate under reduced pressure. The resulting residue was separated by column chromatography (eluent: petroleum ether/ethyl acetate = 30/1-20/1, v/v). The resulting product was added to isopropyl ether (55 mL), and the mixture was heated to 80 °C and stirred for 1.5 h. The mixture was slowly cooled down to room temperature and a large amount of solid was precipitated. The mixture was filtered, and the filter cake was collected. The solvent was removed under reduced pressure to give the intermediate **BB-8-1.** MS-ESI *m*/*z*: 392.1 [M+Na]⁺. ¹H NMR (400 MHz, CDCl₃) δ: 8.15 (d, *J* = 8.8 Hz, 1H), 8.11 (br s, 1H), 7.74 (s, 1H), 7.70-7.58 (m, 2H), 7.45 (dd, *J* = 1.8, 9.0 Hz, 1H), 6.64 (br s, 1H), 4.22 (q, *J* = 7.1 Hz, 2H), 4.04 (s, 2H), 1.57 (s, 9H), 1.26 (t, *J* = 7.0 Hz, 3H).

### Step 2: Synthesis of intermediate BB-8-2

Intermediate **BB-8-1** (3.5 g, 9.29 mmol) was added to N,N-dimethylformamide (50 mL) at 0°C under nitrogen, and potassium tert-butoxide (1.04 g, 9.29 mmol) and acrylamide (659.96 mg, 9.29 mmol) were then added. The reaction mixture was stirred to react at 0°C under nitrogen for 2 h. After the reaction was completed, the reaction mixture was warmed up to room temperature. Water (50 mL) was added and the mixture was extracted with ethyl acetate (50 mL×3). The organic phases were combined, washed with saturated brine (100 mL×2), dried over anhydrous sodium sulfate, and filtered. The solvent was removed from the filtrate under reduced pressure. The resulting residue was added to methanol (25 mL) and the mixture was stirred at room temperature for 1 h. The mixture was then filtered. The filter cake was washed with methanol (10 mL) and collected. The solvent was removed under reduced pressure to give the intermediate **BB-8-2.** MS-ESI *m*/*z*: 339.1 [M+H-56]⁺. ¹H NMR (400 MHz, DMSO_*d*₆) δ: 10.94 (s, 1H), 9.55 (s, 1H), 8.24 (br s, 1H), 8.06 (br d, *J* = 8.8 Hz, 1H), 7.96 (s, 1H), 7.74-7.67 (m, 2H), 7.55 (dd, *J* = 2.0, 9.2 Hz, 1H), 4.63 (br dd, *J* = 4.2, 11.8 Hz, 1H), 2.92-2.81 (m, 1H), 2.66-2.58 (m, 1H), 2.46-2.32 (m, 1H), 2.29-2.21 (m, 1H), 1.52 (s, 9H).

### Step 3: Synthesis of intermediate BB-8-3

Intermediate **BB-8-2** (1.86 g, 4.62 mmol) was added to a solution of hydrochloric acid in ethyl acetate (4 M, 40 mL) at room temperature under nitrogen, and the reaction mixture was stirred to react at room temperature under nitrogen for 4 hours. After the reaction was completed, the solvent was removed from the reaction mixture directly under reduced pressure to give the intermediate **BB-8-3.** ¹H NMR (400 MHz, DMSO_*d*₆) δ: 10.96 (s, 1H), 10.36 (br s, 2H), 8.30 (br d, *J* = 8.4 Hz, 1H), 8.06 (s, 1H), 7.99 (br s, 1H), 7.94-7.85 (m, 2H), 7.56 (br d, *J* = 8.8 Hz, 1H), 4.68 (br dd, *J* = 4.2, 12.2 Hz, 1H), 2.93-2.82 (m, 1H), 2.68-2.60 (m, 1H), 2.46-2.39 (m, 1H), 2.32-2.23 (m, 1H).

### Step 4: Synthesis of intermediate BB-8-4

Intermediate **BB-8-3** (0.5 g, 1.51 mmol) was dissolved in N-methylpyrrolidone (20 mL) at room temperature, and tert-butyl (6-bromohexyl)carbamate (423.56 mg, 1.51 mmol) and N,N-diisopropylethylamine (253.97 mg, 1.97 mmol, 342.28 µL) were then added. The reaction mixture was heated to 110°C and stirred to react for 24 hours. After the reaction was completed, the mixture was cooled down to room temperature. The reaction mixture was diluted with water (100 mL) and extracted with ethyl acetate (50 mL × 3). The organic phases were combined, washed with saturated brine (100 mL×3), dried over anhydrous sodium sulfate, and filtered. The solvent was removed from the filtrate under reduced pressure. The resulting residue was separated by column chromatography (eluent: petroleum ether/ethyl acetate = 1/0-1/2, v/v) to give the intermediate **BB-8-4.** MS-ESI m/z: 494.1 [M+H]⁺. ¹H NMR (400 MHz, CDCl₃) δ: 8.04 (br s, 1H), 7.77 (d, *J* = 9.6 Hz, 1H), 7.59 (s, 1H), 7.57-7.55 (m, 2H), 7.01-6.96 (m, 2H), 4.50-4.45 (m, 1H), 3.24 (t, *J* = 7.0 Hz, 2H), 3.18-3.10 (m, 2H), 2.80-2.70 (m, 2H), 2.52-2.42 (m, 2H), 1.75-1.66 (m, 2H), 1.55-1.48 (m, 4H), 1.46 (s, 9H), 1.44-1.35 (m, 2H).

### Step 5: Synthesis of intermediate BB-8 hydrochloride

Intermediate **BB-8-4** (0.26 g, 498.94 µmol) was dissolved in ethyl acetate (5 mL) at room temperature, and a solution of hydrochloric acid in ethyl acetate (4 M, 10 mL) was then added. The reaction mixture was stirred at room temperature for 12 hours. After the reaction was completed, the reaction mixture was directly concentrated under reduced pressure to remove the solvent to give the intermediate **BB-8** hydrochloride. ¹H NMR (400 MHz, DMSO_*d*₆) δ: 10.95 (s, 1H), 8.30-8.20 (m, 1H), 8.08-8.02 (m, 2H), 7.99 (br s, 3H), 7.89-7.82 (m, 2H), 7.70-7.60 (m, 1H), 4.68 (br dd, *J* = 3.8, 12.2 Hz, 1H), 3.36-3.28 (m, 2H), 2.93-2.83 (m, 1H), 2.79-2.70 (m, 2H), 2.68-2.60 (m, 1H), 2.47-2.37 (m, 1H), 2.33-2.22 (m, 1H), 1.78-1.68 (m, 2H), 1.60-1.51 (m, 2H), 1.44-1.30 (m, 4H).

### Reference example 9

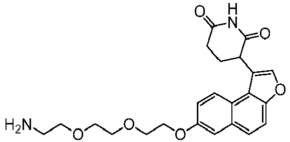

### Synthetic route:

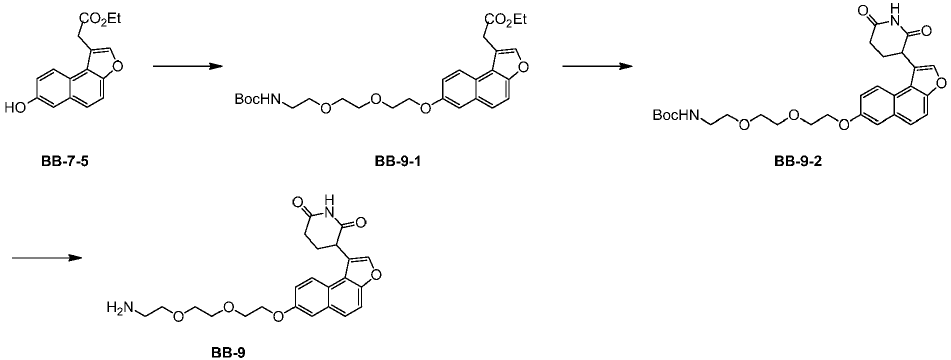

### Step 1: Synthesis of intermediate BB-9-1

Intermediates **BB-7-5** (2.85 g, 10.53 mmol) and tert-butyl (2-(2-(2-hydroxyethoxy)ethoxy)ethyl)carbamate (4.2 g, 16.85 mmol) were added to anhydrous tetrahydrofuran (40 mL) at room temperature under nitrogen, and the mixture was cooled down to 0 °C. Subsequently, azodicarbonyl dipiperidine (4.25 g, 16.85 mmol) and a solution of tributylphosphine (3.41 g, 16.85 mmol) in anhydrous tetrahydrofuran (5 mL) were added sequentially. The reaction mixture was stirred to react at 0°C for 1 h, then warmed up to 15°C and stirred to react for another 11 h. After the reaction was completed, the reaction mixture was quenched by adding saturated citric acid solution (45 mL) and extracted with ethyl acetate (45 mL×4). The organic phases were combined, washed with saturated brine (80 mL×3), dried over anhydrous sodium sulfate, and filtered. The solvent was removed under reduced pressure. The resulting residue was separated by column chromatography (eluent: petroleum ether/ethyl acetate = 1/0-1/4, v/v) to give the intermediate **BB-9-1.** MS-ESI m/z: 502.3 [M +H]⁺.

### Step 2: Synthesis of intermediate BB-9-2

Intermediates **BB-9-1** (3.7 g, 7.38 mmol) and acrylamide (524.33 mg, 7.38 mmol) were dissolved in anhydrous tetrahydrofuran (50 mL) at room temperature under nitrogen. The mixture was cooled down to 0 °C. Subsequently, potassium tert-butoxide (1.08 g, 9.59 mmol) was added and the reaction mixture was stirred to react at 0°C for 1 h. Then the reaction mixture was warmed up to room temperature and stirred to react for another 1 h. After the reaction was completed, the reaction mixture was quenched by adding saturated citric acid solution (50 mL) and extracted with ethyl acetate (55 mL×4). The organic phases were combined, washed with saturated brine (75 mL×3), dried over anhydrous sodium sulfate, and filtered. The solvent was removed under reduced pressure. The resulting residue was separated by column chromatography (eluent: petroleum ether/ethyl acetate = 1/0-1/4, v/v) to give the intermediate **BB-9-2.** ¹H NMR (400MHz, DMSO_*d*₆) δ: 10.94 (s, 1H), 8.09 (d, *J* = 8.4 Hz, 1H), 7.96 (s, 1H), 7.80-7.68 (m, 2H), 7.51 (s, 1H), 7.24 (d, *J* = 8.6 Hz, 1H), 6.77 (s, 1H), 4.70-4.58 (m, 1H), 4.29-4.16 (m, 2H), 3.88-3.75 (m, 2H), 3.69-3.48 (m, 4H), 3.40 (t, *J* = 5.6 Hz, 2H), 3.15-3.00 (m, 2H), 2.95-2.82 (m, 1H), 2.70-2.55 (m, 1H), 2.47-2.35 (m, 1H), 2.32-2.20 (m, 1H), 1.37 (s, 9H).

### Step 3: Synthesis of intermediate BB-9 hydrochloride

Intermediate **BB-9-2** (650 mg, 1.23 mmol) was added to a solution of hydrochloric acid in ethyl acetate (4 M, 15 mL) at room temperature, and the reaction mixture was stirred to react at room temperature for 2 h. After the reaction was completed, the solvent was removed from the reaction solution directly under reduced pressure to give the intermediate **BB-9** hydrochloride.

### Reference example 10

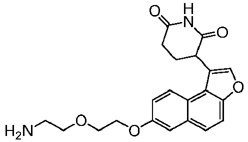

### Synthetic route:

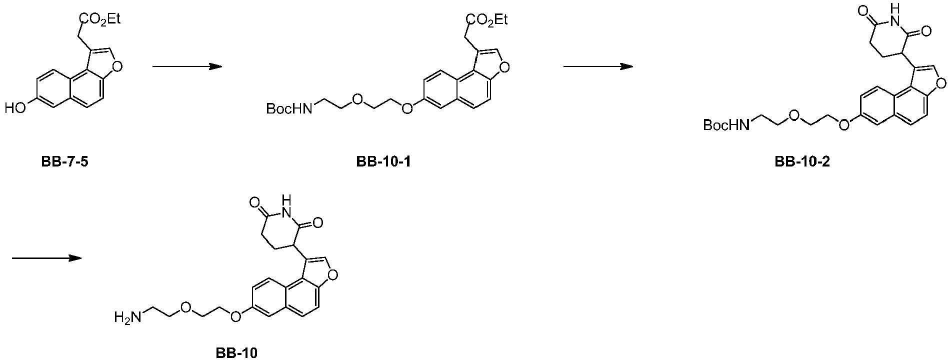

### Step 1: Synthesis of intermediate BB-10-1

Intermediate **BB-7-5** (5.0 g, 18.50 mmol) and 2-(2-tert-butoxycarbonyl-aminoethoxy)ethanol (5.70 g, 27.75 mmol, 63.56 µL) were dissolved in tetrahydrofuran (100 mL), and azodicarbonyl dipiperidine (7.47 g, 29.60 mmol) and tributylphosphine (5.99 g, 29.60 mmol) were added at 0°C. The reaction mixture was stirred to react at 0°C for 1 h, and then stirred to react at 20°C for 11 h. The reaction solution was concentrated under reduced pressure. To the residue, glacial ethanol (5 mL) was added and stirred for 5 min. The mixture was filtered, and the filtrate was concentrated under reduced pressure to remove the solvent. The resulting residue was separated by column chromatography (eluent: petroleum ether/ethyl acetate = 1/0-1/4, v/v) to give the intermediate **BB-10-1.** ¹H NMR (400MHz, CDCl₃) δ: 8.14 (d, *J* = 8.8 Hz, 1H), 7.74 (s, 1H), 7.63-7.60 (m, 2H), 7.33-7.28 (m, 2H), 5.00 (s, 1H), 4.30-4.25 (m, 2H), 4.22 (q, *J* = 7.1 Hz, 2H), 4.04 (d, *J* = 0.8 Hz, 2H), 3.94-3.88 (m, 2H), 3.69-3.63 (m, 2H), 3.43-3.34 (m, 2H), 1.45 (s, 9H), 1.26 (t, *J* = 7.2 Hz, 3H).

### Step 2: Synthesis of intermediate BB-10-2

Intermediate **BB-10-1** (1.02 g, 2.23 mmol) and acrylamide (158.46 mg, 2.23 mmol) were placed in a dry reaction flask, and dissolved by adding anhydrous tetrahydrofuran (20 mL). The atmosphere was replaced three times with nitrogen. The reaction system was cooled down to 0 °C, and potassium tert-butoxide (325.22 mg, 2.90 mmol) was added. The reaction system was stirred at 0°C for 1 h, then warmed up to 20°C and stirred at 20°C for 1 h. The reaction solution was quenched by adding saturated citric acid solution (20 mL), and diluted with water (20 mL) and ethyl acetate (50 mL). The layers were separated, and the organic phase was collected. The aqueous phase was extracted with ethyl acetate (50 mL×3). The organic phases were combined, washed with saturated brine (50 mL×3), dried over anhydrous sodium sulfate, and filtered. The filtrate was concentrated under reduced pressure to give the crude product as an oil. The resulting residue was separated by column chromatography (eluent: petroleum ether/ethyl acetate = 1/0-1/4, v/v) to give the intermediate **BB-10-2.** ¹H NMR (400MHz, DMSO_*d*₆) δ : 10.93 (s, 1H), 8.09 (d, *J =* 8.8 Hz, 1H), 7.96 (s, 1H), 7.77-7.70 (m, 2H), 7.51 (d, J = 2.6 Hz, 1H), 7.24 (dd, *J* = 2.6, 9.2 Hz, 1H), 6.81 (t, *J* = 5.6 Hz, 1H), 4.64 (dd, *J* = 4.4, 11.8 Hz, 1H), 4.28-4.17 (m, 2H), 3.84-3.75 (m, 2H), 3.48 (t, *J* = 6.0 Hz, 2H), 3.16-3.07 (m, 2H), 2.93-2.82 (m, 1H), 2.67-2.56 (m, 1H), 2.46-2.35 (m, 1H), 2.32-2.21 (m, 1H), 1.37 (s, 9H).

### Step 3: Synthesis of intermediate BB-10 hydrochloride

Intermediate **BB-10-2** (380 mg, 787.53 µmol) was added to ethyl acetate (1 mL), and a solution of hydrochloric acid in ethyl acetate (4 M, 5 mL) was added. The mixture was stirred at 15 °C for 2 h. After the reaction was completed, the ethyl acetate was removed by concentration under reduced pressure to give the intermediate **BB-10** hydrochloride. ¹H NMR (400 MHz, DMSO_*d*₆) δ: 10.95 (s, 1H), 8.09 (d, *J* = 8.8 Hz, 1H), 8.02 (br s, 3H), 7.97 (s, 1H), 7.80-7.71 (m, 2H), 7.53 (d, *J* = 2.5 Hz, 1H), 7.24 (dd, *J* = 2.6, 9.2 Hz, 1H), 4.64 (dd, *J* = 4.0, 12.0 Hz, 1H), 4.31-4.23 (m, 2H), 3.92-3.83 (m, 2H), 3.72 (t, *J* = 5.4 Hz, 2H), 3.06-2.96 (m, 2H), 2.94-2.82 (m, 1H), 2.68-2.57 (m, 1H), 2.47-2.34 (m, 1H), 2.33-2.20 (m, 1H).

### Reference example 11

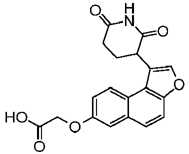

### Synthetic route:

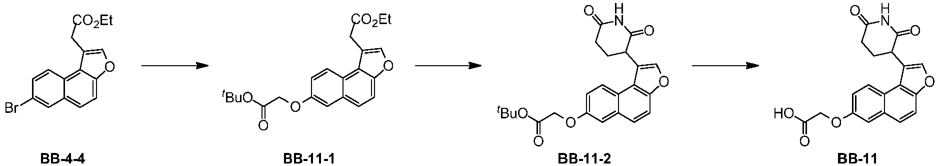

### Step 1: Synthesis of intermediate BB-11-1

Intermediate **BB-4-4** (5 g, 14.58 mmol) was dissolved in toluene (100 mL) at room temperature under nitrogen, and tert-butyl hydroxyacetate (2.31 g, 17.50 mmol), tris(dibenzylideneacetone)dipalladium (1.34 g, 1.46 mmol), 2-di-tert-butylphosphino-2',4',6'-triisopropylbiphenyl (1.86 g, 4.37 mmol) and cesium carbonate (9.50 g, 29.16 mmol, 2 eq) were added sequentially. The reaction mixture was heated to 110°C and stirred to react for 12 hours. After the reaction was completed, the mixture was cooled down to room temperature. The reaction solution was added to water (100 mL) and extracted with ethyl acetate (100 mL×3). The organic phases were combined, washed with saturated brine (150 mL×3), dried over anhydrous sodium sulfate, and filtered. The solvent was removed from the filtrate under reduced pressure. The resulting residue was separated by column chromatography (eluent: petroleum ether/ethyl acetate = 1/0-3/1, v/v) to give the intermediate **BB-11-1** (purity: 68.84%). MS-ESI m/z: 385.4 [M+H]⁺. ¹H NMR (400 MHz, CDCl₃) δ: 8.17 (d, *J* = 9.0 Hz, 1H), 7.75 (s, 1H), 7.64-7.58 (m, 2H), 7.34-7.30 (m, 1H), 7.25 (d, *J =* 2.6 Hz, 1H), 4.65 (s, 2H), 4.22 (q, *J* = 7.2 Hz, 2H), 4.04 (s, 2H), 1.51 (s, 9H), 1.28-1.24 (m, 3H).

### Step 2: Synthesis of intermediate BB-11-2

Intermediate **BB-11-1** (2.9 g, 5.19 mmol, purity: 68.84%) was dissolved in N,N-dimethylformamide (20 mL) at 0°C under nitrogen. Potassium tert-butoxide (641.00 mg, 5.71 mmol) and acrylamide (369.12 mg, 5.19 mmol) were added sequentially, and the reaction solution was stirred to react at 0°C under nitrogen for 1 h. After the reaction was completed, the reaction solution was diluted with water (100 mL) and extracted with ethyl acetate (50 mL×3). The organic phases were combined, washed with saturated brine (100 mL×3), dried over anhydrous sodium sulfate, and filtered. The solvent was removed from the filtrate under reduced pressure. The resulting residue was separated by column chromatography (eluent: petroleum ether/ethyl acetate = 1/0-1/1, v/v). The product was added to ethyl acetate (5 mL), and the mixture was stirred at room temperature for 5 min, and filtered. The solvent was removed from the filter cake under reduced pressure to give the intermediate **BB-11-2** (purity: 68.87%). MS-ESI m/z: 432.0 [M+Na]⁺. ¹H NMR (400 MHz, DMSO_*d*₆) δ: 10.93 (s, 1H), 8.11 (br d, *J* = 8.8 Hz, 1H), 7.97 (s, 1H), 7.77-7.71 (m, 2H), 7.44 (d, *J* = 2.0 Hz, 1H) 7.25 (dd, *J* = 2.2, 9.0, Hz, 1H), 4.78 (s, 2H), 4.69-4.60 (m, 1H), 2.94-2.81 (m, 1H), 2.69-2.57 (m, 1H), 2.39-2.32 (m, 1H), 2.29-2.23 (m, 1H), 1.44 (s, 9H).

### Step 3: Synthesis of intermediate BB-11

Intermediate **BB-11-2** (0.12 g, 201.85 µmol, purity: 68.87%) was dissolved in dichloromethane (10 mL) at room temperature and trifluoroacetic acid (7.70 g, 67.56 mmol, 5 mL) was added. The reaction mixture was stirred to react at room temperature for 12 h. After the reaction was completed, the reaction solution was directly concentrated under reduced pressure to remove the solvent to give the intermediate **BB-11.** ¹H NMR (400 MHz, DMSO_*d*₆) δ: 10.94 (s, 1H), 8.10 (br d, *J* = 9.6 Hz, 1H), 7.97 (s, 1H), 7.77-7.72 (m, 2H), 7.46 (d, *J* = 2.4 Hz, 1H), 7.25 (dd, *J* = 2.8, 9.2 Hz, 1H), 4.81 (s, 2H), 4.64 (br dd, *J* = 4.0, 11.6 Hz, 1H), 2.94-2.81 (m, 1H), 2.69-2.58 (m, 1H), 2.43-2.37 (m, 1H), 2.32-2.21 (m, 1H).

### Reference example 12

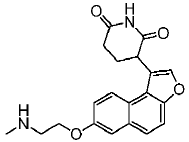

### Synthetic route:

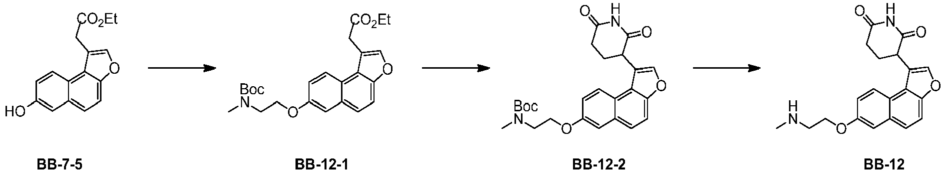

### Step 1: Synthesis of intermediate BB-12-1

N-Boc-N-methyl-2-aminoethanol (1.56 g, 8.88 mmol) was dissolved in tetrahydrofuran (20 mL) at 15 °C under nitrogen. Intermediates **BB-7-5** (2 g, 7.40 mmol) and triphenylphosphine (2.52 g, 9.62 mmol) were added sequentially under stirring. The mixture was cooled down to 0 °C, and diisopropyl azodicarboxylate (1.95 g, 9.62 mmol, 1.87 mL) was added slowly dropwise. The reaction mixture was warmed up to 15 °C and stirred to react for 12 hours. After the reaction was completed, the reaction solution was directly concentrated under reduced pressure to remove the solvent. The resulting residue was separated by column chromatography (eluent: petroleum ether/ethyl acetate = 1/0-4/1, v/v) to give the intermediate **BB-12-1.** ¹H NMR (400 MHz, CDCl₃) δ: 8.15 (d, *J* = 8.8 Hz, 1H), 7.74 (s, 1H), 7.63-7.61 (m, 2H), 7.30 (br s, 1H), 7.24 (dd, *J* = 2.4, 8.8 Hz, 1H), 4.29-4.18 (m, 4H), 4.04 (d, *J* = 0.8 Hz, 2H), 3.73-3.64 (m, 2H), 3.04 (s, 3H), 1.49 (s, 9H), 1.26 (t, *J* = 7.2 Hz, 3H).

### Step 2: Synthesis of intermediate BB-12-2

Intermediate **BB-12-1** (1.9 g, 4.44 mmol) was added to tetrahydrofuran (40 mL) at room temperature under nitrogen. Then acrylamide (315.91 mg, 4.44 mmol) and potassium tert-butoxide (498.72 mg, 4.44 mmol) were added sequentially, and the reaction mixture was stirred at room temperature for 1 hour. After the reaction was completed, the reaction solution was poured into ice water (50 mL) and extracted with ethyl acetate (30 mL×3). The organic phases were combined, washed with saturated brine (30 mL×3), dried over anhydrous sodium sulfate, and filtered. The solvent was removed from the filtrate under reduced pressure. The resulting residue was separated by column chromatography (eluent: petroleum ether/ethyl acetate = 411-111, v/v) to give the intermediate **BB-12-2.** ¹H NMR (400 MHz, CDCl₃) δ: 8.17 (s, 1H), 7.84 (d, *J* = 9.2 Hz, 1H), 7.64-7.55 (m, 3H), 7.27 (s, 1H), 7.24-7.17 (m, 1H), 4.42 (dd, *J* = 5.6, 8.4 Hz, 1H), 4.23-4.10 (m, 2H), 3.68-3.58 (m, 2H), 2.98 (s, 3H), 2.80-2.65 (m, 2H), 2.50-2.34 (m, 2H), 1.43 (s, 9H).

### Step 3: Synthesis of intermediate BB-12 hydrochloride

Intermediate **BB-12-2** (0.8 g, 1.77 mmol) was dissolved in a solution of hydrochloric acid in ethyl acetate (4 M, 15 mL) at 15 °C. The reaction mixture was stirred to react at 15 °C for 20 min, during which a large amount of solid was precipitated. After the reaction was completed, the solvent was removed from the reaction mixture directly under reduced pressure and the residue was washed with ethyl acetate (10 mL×3). The filter cake was collected and the solvent was removed under reduced pressure to give the intermediate **BB-12** hydrochloride. ¹H NMR (400MHz, DMSO_*d*₆) δ : 10.94 (s, 1H), 9.19 (s, 2H), 8.14 (d, *J =* 9.2 Hz, 1H), 7.98 (s, 1H), 7.83-7.70 (m, 2H), 7.58 (d, *J =* 2.0 Hz, 1H), 7.29 (dd, *J* = 2.4, 9.2 Hz, 1H), 4.72-4.57 (m, 1H), 4.47-4.29 (m, 2H), 3.43-3.35 (m, 2H), 2.98-2.79 (m, 1H), 2.67-2.55 (m, 4H), 2.42-2.35 (m, 1H), 2.31-2.20 (m, 1H).

### Reference example 13

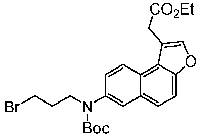

### Synthetic route:

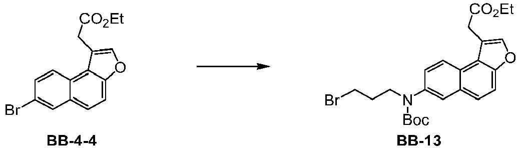

Intermediate **BB-4-4** (1 g, 2.92 mmol) and N-tert-butoxycarbonyl-3-aminopropyl bromide (2.08 g, 8.75 mmol) were added to a mixture of toluene (20 mL) and water (4 mL) at room temperature under nitrogen, and tris(dibenzylideneacetone)dipalladium (186.93 mg, 204.13 µmol), 2-di-tert-butylphosphino-2',4',6'-triisopropylbiphenyl (173.37 mg, 408.26 µmol) and potassium phosphate (2.48 g, 11.66 mmol) were then added sequentially. The reaction mixture was heated to 100 °C and stirred to react for 14 hours. After the reaction was completed, the mixture was cooled down to room temperature. Water (50 mL) was added to the reaction solution, and the mixture was extracted with ethyl acetate (50 mL×3). The organic phases were combined, washed with saturated brine (100 mL×2), dried over anhydrous sodium sulfate, and filtered. The solvent was removed from the filtrate under reduced pressure. The resulting residue was separated by column chromatography (eluent: petroleum ether/ethyl acetate = 50/1-30/1, v/v) to give the intermediate **BB-13.** MS-ESI *m*/*z*: 512.1 [M+Na]⁺. ¹H NMR (400 MHz, CDCl₃) δ: 8.20 (d, *J* = 8.8 Hz, 1H), 7.78 (s, 1H), 7.75 (d, *J* = 1.2 Hz, 1H), 7.71-7.64 (m, 2H), 7.43 (br d, *J* = 8.4 Hz, 1H), 4.23 (q, *J* = 7.1 Hz, 2H), 4.06 (s, 2H), 3.94-3.87 (m, 2H), 3.43 (t, *J* = 6.8 Hz, 2H), 2.23-2.13 (m, 2H), 1.45 (s, 9H), 1.27 (t, *J* = 7.2 Hz, 3H).

### Reference example 14

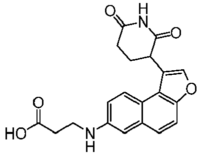

### Synthetic route:

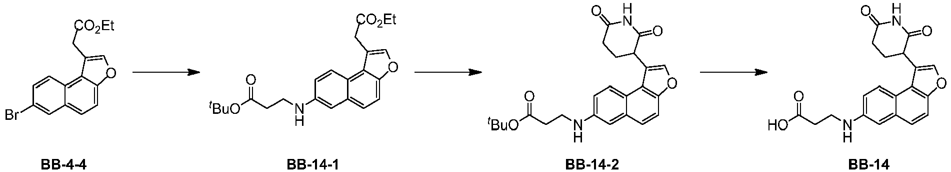

### Step 1: Synthesis of intermediate BB-14-1

Intermediates **BB-4-4** (1.18 g, 3.44 mmol) and tert-butyl 3-aminopropionate (600 mg, 4.13 mmol) were added to a mixture of toluene (20 mL) and water (4 mL) at room temperature under nitrogen, and tris(dibenzylideneacetone)dipalladium (220.73 mg, 241.05 µmol), 2-di-tert-butylphosphino-2',4',6'-triisopropylbiphenyl (204.72 mg, 482.10 µmol) and potassium phosphate (2.92 g, 13.77 mmol) were added sequentially. The reaction mixture was heated to 100°C and stirred to react for 14 hours. After the reaction was completed, the reaction solution was cooled down to room temperature. Water (30 mL) was added and the mixture was extracted with ethyl acetate (30 mL×3). The organic phases were combined, washed with saturated brine (50 mL×2), dried over anhydrous sodium sulfate, and filtered. The solvent was removed from the filtrate under reduced pressure. The resulting residue was separated by column chromatography (eluent: petroleum ether/ethyl acetate = 50/1-30/1, v/v) to give the intermediate **BB-14-1** (purity: 89.86%). MS-ESI m/z: 398.2 [M+H]⁺. ¹H NMR (400 MHz, CDCl₃) δ: 8.04 (d, *J* = 8.4 Hz, 1H), 7.70 (s, 1H), 7.56-7.50 (m, 2H), 7.03-6.97 (m, 2H), 4.25-4.20 (m, 2H), 4.02 (s, 2H), 3.53 (br t, *J* = 6.0 Hz, 2H), 2.62 (t, *J* = 6.2 Hz, 2H), 1.48 (s, 9H), 1.28-1.26 (m, 3H).

### Step 2: Synthesis of intermediate BB-14-2

Intermediate **BB-14-1** (933 mg, 2.11 mmol, purity: 89.86%) was added to N,N-dimethylformamide (15 mL) at 0°C under nitrogen, and potassium tert-butoxide (236.69 mg, 2.11 mmol) and acrylamide (149.93 mg, 2.11 mmol) were then added. The reaction mixture was stirred to react at 0°C under nitrogen for 1.5 hours. After the reaction was completed, the reaction solution was warmed up to room temperature. Water (50 mL) was added and the mixture was extracted with ethyl acetate (50 mL×3). The organic phases were combined, washed with saturated brine (100 mL×2), dried over anhydrous sodium sulfate, and filtered. The solvent was removed from the filtrate under reduced pressure. The resulting residue was separated by column chromatography (eluent: petroleum ether/ethyl acetate = 10/1-1/1, v/v) to give the intermediate **BB-14-2** (purity: 84.31%). MS-ESI m/z: 423.2 [M+H]⁺. ¹H NMR (400 MHz, DMSO_*d*₆) δ: 10.93 (s, 1H), 7.90-7.84 (m, 2H), 7.61-7.53 (m, 2H), 7.02 (dd, J= 2.4, 9.2 Hz, 1H), 6.95 (d, J= 2.0 Hz, 1H), 4.57 (dd, J= 4.6, 11.8 Hz, 1H), 3.39-3.34 (m, 2H), 2.87-2.80 (m, 1H), 2.68-2.60 (m, 1H), 2.59-2.54 (m, 2H), 2.39-2.31 (m, 1H), 2.26-2.22 (m, 1H), 1.41 (s, 9H).

### Step 3: Synthesis of intermediate BB-14

Intermediate **BB-14-2** (150 mg, 299.34 µmol, purity: 84.31%) was added to dichloromethane (5 mL) at room temperature under nitrogen, and trifluoroacetic acid (102.40 mg, 898.03 µmol, 66.49 µL) was then added. The reaction mixture was stirred at room temperature for 14 hours. After the reaction was completed, the solvent was directly removed from the reaction solution under reduced pressure to give the intermediate **BB-14.** ¹H NMR (400 MHz, DMSO_*d*₆) δ: 10.94 (s, 1H), 7.98-7.93 (m, 1H), 7.90 (s, 1H), 7.65-7.60 (m, 2H), 7.16-7.09 (m, 2H), 4.62-4.55 (m, 1H), 3.40 (t, *J* = 7.0 Hz, 2H), 2.87-2.80 (m, 1H), 2.69-2.62 (m, 1H), 2.60 (t, *J* = 6.8 Hz, 2H), 2.41-2.31 (m, 1H), 2.29-2.20 (m, 1H).

### Reference example 15

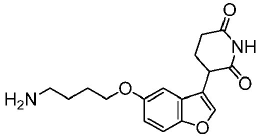

### Synthetic route:

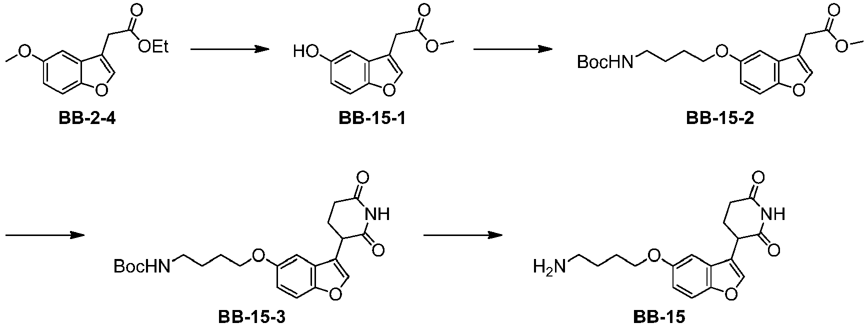

### Step 1: Synthesis of intermediate BB-15-1

Intermediate **BB-2-4** (23.50 g, 98.91 mmol) was dissolved in anhydrous dichloromethane (300 mL) at room temperature, and boron tribromide (74.33 g, 296.72 mmol, 28.59 mL) was then added at -78 °C. The reaction mixture was warmed up to 20 °C and stirred to react for 2 h. After the reaction was completed, the mixture was cooled down to room temperature. Methanol (70 mL) was slowly added at 0°C, and the solvent was removed under reduced pressure. The reaction solution was slowly added to ice water (500 mL) and the mixture was extracted with dichloromethane (100 mL×3). The organic phases were combined, washed with saturated brine (300 mL×2) in turn, dried over anhydrous sodium sulfate, and filtered. The filtrate was concentrated under reduced pressure to remove the solvent, and the residue was separated by preparative column chromatography (eluent: petroleum ether/ethyl acetate = 10/1-4/1, v/v) to give the intermediate **BB-15-1.** ¹H NMR (400MHz, DMSO_*d*₆) δ: 9.21 (s, 1H), 7.80 (s, 1H), 7.34 (d, *J* = 8.8 Hz, 1H), 6.88 (d, *J* = 2.4 Hz, 1H), 6.76 (dd, *J* = 2.4, 8.8 Hz, 1H), 3.72 (s, 2H), 3.64 (s, 3H).

### Step 2: Synthesis of intermediate BB-15-2

Intermediates **BB-15-1** (1.00 g, 3.91 mmol) and 4-(N-Boc-amino)-1-butanol (1.11 g, 5.86 mmol) were dissolved in tetrahydrofuran (100 mL) at 0°C, and azodicarbonyl dipiperidine (1.48 g, 5.86 mmol) and tributylphosphine (1.19 g, 5.86 mmol, 1.45 mL) were then added. The reaction mixture was stirred to react at 20 °C under nitrogen for another 14 h. After the reaction was completed, the mixture was cooled down to room temperature. The mixture was added to water (100 mL) and extracted with ethyl acetate (30 mL×3). The organic phases were combined, washed with saturated brine (100 mL×2), dried over anhydrous sodium sulfate, and filtered. The solvent was removed from the filtrate under reduced pressure. The resulting residue was separated on a preparative column (mobile phase: acetonitrile/water; basic system: NH₄HCO₃) to obtain the target intermediate **BB-15-2.** ¹H NMR (400 MHz, CDCl₃) δ: 7.60 (s, 1H), 7.36 (d, *J =* 8.8 Hz, 1H), 7.00 (d, *J* = 2.8 Hz, 1H), 6.90 (dd, *J* = 2.4, 8.8 Hz, 1H), 4.02 (t, *J* = 6.2 Hz, 2H), 3.74 (s, 3H), 3.68 (s, 2H), 3.22 (br d, *J* = 6.4 Hz, 2H), 1.88-1.81 (m, 2H), 1.74-1.67 (m, 2H), 1.46 (s, 9H).

### Step 2: Synthesis of intermediate BB-15-3

Intermediate **BB-15-2** (720 mg, 1.90 mmol) was dissolved in N,N-dimethylformamide (50 mL) at 0°C, and potassium tert-butoxide (212.92 mg, 1.90 mmol) and acrylamide (134.87 mg, 1.90 mmol) were then added. The reaction mixture was stirred to react at 0°C under nitrogen for another 2 h. After the reaction was completed, the mixture was diluted with water (50 mL) and extracted with ethyl acetate (10 mL×3). The organic phases were combined, washed with saturated brine (50 mL×2), dried over anhydrous sodium sulfate, and filtered. The solvent was removed from the filtrate under reduced pressure. The resulting residue was separated by column chromatography (eluent: petroleum ether/ethyl acetate = 1/0-4/1) to give the compound **BB-15-3.** ¹H NMR (400 MHz, DMSO-*d*₆) δ: 10.87 (s, 1H), 7.83 (s, 1H), 7.45 (d, *J* = 8.8 Hz, 1H), 7.08 (d, *J* = 2.4 Hz, 1H), 6.89 (dd, *J* = 2.8, 8.8 Hz, 1H), 4.10 (dd, *J* = 5.0, 11.8 Hz, 1H), 3.98-3.93 (m, 2H), 2.97 (br d, *J* = 6.4 Hz, 2H), 2.77-2.67 (m, 1H), 2.60-2.54 (m, 1H), 2.39-2.29 (m, 1H), 2.12-2.07 (m, 1H), 1.73-1.66 (m, 2H), 1.57-1.50 (m, 2H), 1.37 (s, 9H).

### Step 3: Synthesis of intermediate BB-15 hydrochloride

Intermediate **BB-15-3** (570 mg, 1.36 mmol) was dissolved in hydrochloric acid/ethyl acetate solution (40 mL, 4 M) at 20 °C. The reaction mixture was stirred to react at 20 °C under nitrogen for another 14 h. After the reaction was completed, the solvent was removed under reduced pressure to give the compound **BB-15** hydrochloride. ¹H NMR (400MHz, DMSO-*d*₆) δ: 10.88 (s, 1H), 7.90 (br s, 3H), 7.84 (s, 1H), 7.46 (d, *J* = 8.8 Hz, 1H), 7.09 (d, *J* = 2.4 Hz, 1H), 6.90 (dd, *J* = 2.5, 8.8 Hz, 1H), 4.11 (dd, *J* = 4.8, 12.0 Hz, 1H), 4.03 - 3.95 (m, 2H), 2.91 - 2.80 (m, 2H), 2.79 - 2.65 (m, 1H), 2.62-2.52 (m, 1H), 2.40-2.27 (m, 1H), 2.14 - 2.05 (m, 1H), 1.85 - 1.65 (m, 4H).

### Reference example 16

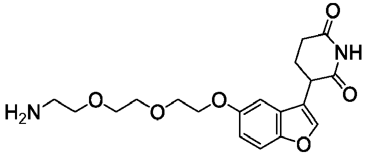

### Synthetic route:

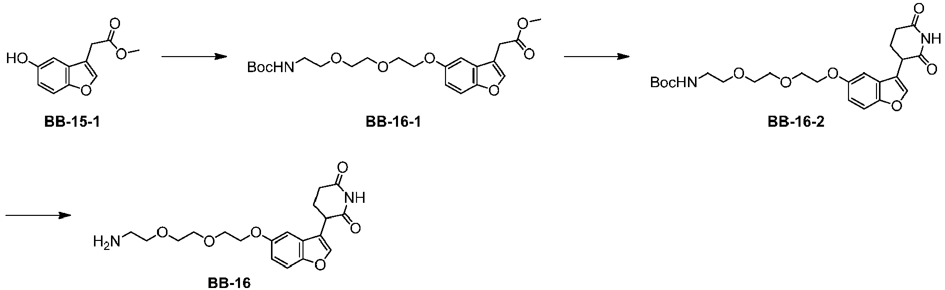

### Step 1: Synthesis of intermediate BB-16-1

Intermediate **BB-15-1** (600 mg, 2.35 mmol) and 2,2-dimethyl-4-oxo-3,8,11-trioxa-5-azetidine-13-4-methylbenzenesulfonate (1.02 g, 2.35 mmol) were dissolved in N,N-dimethylformamide (50 mL) at 20 °C, and cesium carbonate (764.36 mg, 2.35 mmol µmol) was then added. The reaction mixture was stirred to react at 80 °C under nitrogen for another 14 h. After the reaction was completed, the mixture was cooled down to room temperature. Water (50 mL) was added and the mixture was extracted with ethyl acetate (10 mL×3). The organic phases were combined, washed with saturated brine (60 mL×2), dried over anhydrous sodium sulfate, and filtered. The solvent was removed from the filtrate under reduced pressure. The resulting residue was separated by a plate (eluent: petroleum ether/ethyl acetate = 1/1) to give the target intermediate **BB-16-1.** ¹H NMR (400 MHz, CDCl₃) δ: 7.60 (s, 1H), 7.36 (d, *J* = 8.8 Hz, 1H), 7.03 (d, *J* = 2.4 Hz, 1H), 6.95 (dd, *J* = 2.6, 8.8 Hz, 1H), 5.31 (s, 2H), 4.20-4.18(m, 2H), 3.91-3.88 (m, 2H), 3.76-3.75 (m, 1H), 3.74 (s, 3H), 3.68 (s, 2H), 3.67-3.66 (m, 1H), 3.57 (t, *J* = 4.8 Hz, 2H), 3.33 (br d, *J* = 4.4 Hz, 2H), 1.44 (s, 9H).

### Step 2: Synthesis of intermediate BB-16-2

Intermediate **BB-16-1** (275 mg, 556.69 µmol) was dissolved in N,N-dimethylformamide (20 mL) at 0°C, and potassium tert-butoxide (62.47 mg, 556.69 µmol) and acrylamide (39.57 mg, 556.69 µmol) were then added. The reaction mixture was stirred to react at 0°C under nitrogen for another 2 h. After the reaction was completed, the mixture was diluted with water (30 mL) and extracted with ethyl acetate (10 mL×3). The organic phases were combined, washed with saturated brine (60 mL×2), dried over anhydrous sodium sulfate, and filtered. The solvent was removed from the filtrate under reduced pressure. The resulting residue was separated by a plate (eluent: petroleum ether/ethyl acetate = 1/1) to give the compound **BB-16-2.** ¹H NMR (400 MHz, DMSO-*d*₆) δ: 10.88 (s, 1H), 7.84 (s, 1H), 7.46 (d, *J* = 9.2 Hz, 1H), 7.12 (d, *J* = 2.8 Hz, 1H), 6.91 (dd, *J* = 2.6, 8.8 Hz, 1H), 4.12-4.11 (m, 1H), 4.10-4.07 (m, 2H), 3.75 (t, *J* = 4.6 Hz, 2H), 3.60-3.58 (m, 2H), 3.53-3.51 (m, 2H), 3.38 (t, *J* = 6.2 Hz, 2H), 3.06 (br d, *J* = 12.0 Hz, 2H), 2.71-2.67 (m, 1H), 2.59-2.53 (m, 1H), 2.37-2.33 (m, 1H), 2.11-2.07 (m, 1H), 1.36 (s, 9H).

### Step 3: Synthesis of intermediate BB-16 hydrochloride

Intermediate **BB-16-2** (252 mg, 500.23 µmol) was dissolved in hydrochloric acid/ethyl acetate solution (50 mL, 4 M) at 20 °C. The reaction mixture was stirred to react at 20 °C under nitrogen for another 14 h. After the reaction was completed, the solvent was removed under reduced pressure to give the compound **BB-16** hydrochloride. ¹H NMR (400 MHz, DMSO-*d*₆) δ: 10.88 (s, 1H), 7.95-7.93 (m, 3H), 7.85 (s, 1H), 7.47 (d, *J =* 8.8 Hz, 1H), 7.11 (d, *J* = 2.4 Hz, 1H), 6.92 (dd, *J* = 2.2, 9.0 Hz, 1 H), 4.14-4.12 (m, 1H), 4.11-4.07 (m, 2H), 3.77 (t, *J* = 4.6 Hz, 2H), 3.64-3.63 (m, 2H), 3.61 (br s, 2H), 3.60 (d, *J* = 2.0 Hz, 2H), 2.98-2.93 (m, 2H), 2.77-2.71 (m, 1H), 2.59-2.54 (m, 1H), 2.37-2.33 (m, 1H), 2.11-2.07(m, 1H).

### Reference example 17

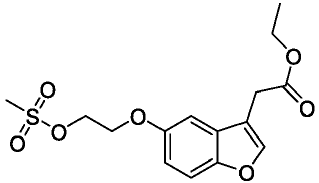

### Synthetic route:

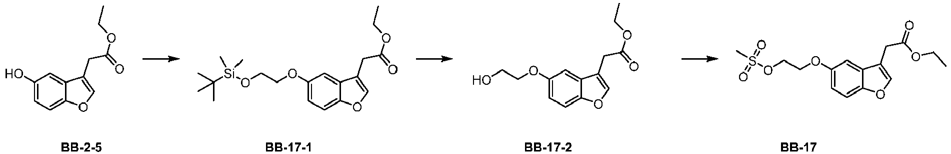

### Step 1: Synthesis of intermediate BB-17-1

2-((tert-butyldimethylsilyl)oxo)ethanol (2.40 g, 13.62 mmol) was dissolved in anhydrous tetrahydrofuran (50 mL) at room temperature under nitrogen, and intermediate **BB-2-5** (2.5 g, 11.35 mmol) and azodicarbonyl dipiperidine (3.72 g, 14.76 mmol) were added sequentially. The reaction system was cooled down to 0°C and tributylphosphine (2.99 g, 14.76 mmol) was added dropwise. The reaction mixture was restored to 25°C and stirred to react for 2 h. After the reaction was completed, the reaction solution was diluted with water (40 mL) and ethyl acetate (30 mL), and the layers were separated. The organic phase was collected, and the aqueous phase was extracted with ethyl acetate (60 mL × 3). The organic phases were combined, washed with saturated brine (40 mL × 3), dried over anhydrous sodium sulfate, and filtered. The filtrate was concentrated under reduced pressure to remove the solvent. The resulting residue was separated by column chromatography (eluent: petroleum ether/ethyl acetate = 1/0-2/3, v/v) to give the intermediate **BB-17-1.**

### Step 2: Synthesis of intermediate BB-17-2

Intermediate **BB-17-1** (3.5 g, 9.25 mmol) was dissolved in tetrahydrofuran (35 mL) at room temperature under nitrogen, and a solution of tetrabutylammonium fluoride in tetrahydrofuran (2 M, 9.25 mL) was added. The reaction system was stirred to react at 25°C for 12 hours. After the reaction was completed, the reaction solution was poured into saturated aqueous ammonium chloride solution (40 mL). The mixture was diluted with ethyl acetate (30 mL), and the layers were separated. The organic phase was collected, and the aqueous phase was extracted with ethyl acetate (50 mL × 3). The organic phases were combined, washed with saturated brine (30 mL × 3), dried over anhydrous sodium sulfate, and filtered. The filtrate was concentrated under reduced pressure to remove the solvent. The resulting residue was separated by column chromatography (eluent: petroleum ether/ethyl acetate = 1/0-2/3, v/v) to give the intermediate **BB-17-2.**

### Step 3: Synthesis of intermediate BB-17

Intermediate **BB-17-2** (403 mg, 1.52 mmol) was dissolved in a mixture of dichloromethane (4 mL) and tetrahydrofuran (4 mL) at room temperature under nitrogen, and 4-dimethylaminopyridine (18.63 mg, 152.49 µmol), methanesulfonyl chloride (262.02 mg, 2.29 mmol), and N,N-diisopropylethylamine (591.26 mg, 4.57 mmol, 796.85 µL) were added at 0°C. The reaction mixture was restored to 20 °C and stirred to react for 1 hr. After the reaction was completed, the reaction solution was poured into saturated aqueous sodium bicarbonate solution (20 mL) at 0°C and extracted with ethyl acetate (30 mL × 3). The organic phases were combined, washed with saturated brine (30 mL × 3), dried over anhydrous sodium sulfate, and filtered. The filtrate was concentrated under reduced pressure to remove the solvent. The resulting residue was separated by column chromatography (eluent: petroleum ether/ethyl acetate = 1/0-3/2, v/v) to give the intermediate **BB-17.** ¹H NMR (400 MHz, DMSO_*d*₆) δ: 7.87 (s, 1H), 7.48 (d, *J* = 8.8 Hz, 1H), 7.17 (d, *J* = 2.4 Hz, 1H), 6.96 (dd, *J* = 2.4, 8.8 Hz, 1H), 4.61-4.47 (m, 2H), 4.36-4.22 (m, 2H), 4.12 (q, *J* = 7.2 Hz, 2H), 3.76 (s, 2H), 3.24 (s, 3H), 1.20 (t, *J* = 7.2 Hz, 3H).

### Reference example 18

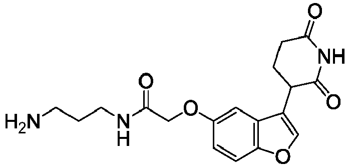

### Synthetic route:

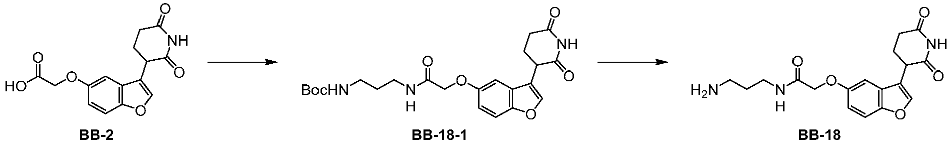

### Step 1: Synthesis of intermediate BB-18-1

Intermediate **BB-2** (500.00 mg, 1.65 mmol) was dissolved in N,N-dimethylformamide (20 mL) at room temperature. To the mixture were added respectively N,N-diisopropylethylamine (1.07 g, 8.24 mmol) and O-(7-azabenzotriazol-1-yl)-N,N,N,N-tetramethyluronium hexafluorophosphate (940.34 mg, 2.47 mmol). The reaction mixture was stirred at room temperature for 30 min. Subsequently, N-tert-butoxycarbonyl-1,3-propanediamine (287.27 mg, 1.65 mmol) was added, and the reaction mixture was stirred to react at room temperature for another 12 h. After the reaction was completed, water (40 mL) was added to the reaction solution and the resulting mixture was extracted with ethyl acetate (40 mL×3). The organic phases were sequentially combined, washed with saturated brine (40 mL×2), dried over anhydrous sodium sulfate, and filtered. The filtrate was concentrated under reduced pressure to remove the solvent. The resulting residue was separated by column chromatography (eluent: petroleum ether/ethyl acetate = 1/1-0/1, v/v) to give the intermediate **BB-18-1.** ¹H NMR (400 MHz, CDCl₃) δ: 7.56 (s, 1H), 7.42 (d, *J* = 9.6 Hz, 1H), 7.04-6.97 (m, 2H), 4.54 (s, 2H), 3.97 (t, *J* = 8.0 Hz, 1H), 3.43-3.38 (m, 2H), 3.19-3.14 (m, 2H), 2.81-2.74 (m, 2H), 2.40-2.33 (m, 2H), 1.69-1.64 (m, 2H), 1.44 (s, 9H).

### Step 2: Synthesis of intermediate BB-18 hydrochloride

Intermediate **BB-18-1** (300.00 mg, 652.90 µmol) was dissolved in ethyl acetate (10 mL) at room temperature. To the mixture was added hydrochloric acid/ethyl acetate solution (4 M, 12.09 mL), and the reaction mixture was stirred to react at room temperature for 12 h. After the reaction was completed, the reaction solution was filtered, and the filter cake was collected, washed with ethyl acetate (10 mL) and dried to give the intermediate **BB-18** hydrochloride. ¹H NMR (400 MHz, DMSO_*d*₆) δ: 10.89 (s, 1H), 8.39 (t, *J* = 6.0 Hz, 1H), 8.06 (s, 3H), 7.86 (s, 1H), 7.49 (d, *J* = 8.8 Hz, 1H), 7.17 (d, *J* = 2.4 Hz, 1H), 7.01 (dd, *J* = 2.4, 8.8 Hz, 1H), 4.50 (d, *J* = 1.2 Hz, 2H), 4.11 (dd, *J* = 4.8, 12.4 Hz, 1H), 3.26-3.17 (m, 2H), 2.80-2.70 (m, 3H), 2.62-2.52 (m, 1H), 2.41-2.28 (m, 1H), 2.13-2.04 (m, 1H), 1.80-1.70 (m, 2H).

### Reference example 19

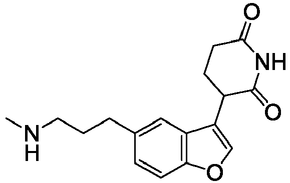

### Synthetic route:

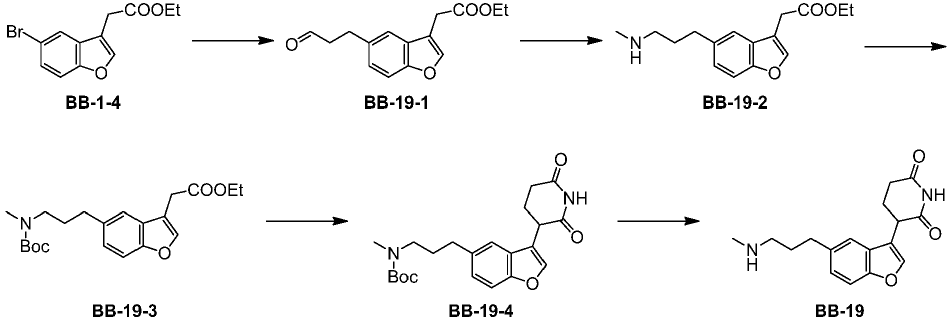

### Step 1: Synthesis of intermediate BB-19-1

Intermediates **BB-1-4** (5 g, 17.66 mmol, 1 eq) and propenol (2.00 g, 34.44 mmol, 2.34 mL, 1.95 eq) were dissolved in 1,4-dioxane (50 mL) at room temperature under nitrogen, and N-cyclohexyl-N-methyl-cyclohexylamine (4.14 g, 21.19 mmol, 4.49 mL, 1.2 eq), tris(dibenzylideneacetone)dipalladium (1.62 g, 1.77 mmol, 0.1 eq) and a solution of tri-tert-butylphosphine (7.15 g, 3.53 mmol, 8.29 mL, 0.2 eq, purity: 10%) in toluene were then added. The reaction mixture was heated to 60 °C and stirred to react under nitrogen for 12 h. After the reaction was completed, the mixture was cooled down to room temperature, and filtered. The filtrate was concentrated under vacuum to remove the solvent. The resulting residue was separated by column chromatography (eluent: petroleum ether/ethyl acetate = 50/1-20/1, v/v) to give the intermediate **BB-19-1.** ¹H NMR (400MHz, DMSO_*d*₆) δ: 9.72 (t, *J* = 1.2 Hz, 1H), 7.87 (s, 1H), 7.46 (d, *J* = 8.8 Hz, 1H), 7.43 (d, *J* = 1.2 Hz, 1H), 7.18 (dd, *J* = 1.8, 8.2 Hz, 1H), 4.11 (q, *J* = 7.1 Hz, 2H), 3.75 (d, *J* = 0.4 Hz, 2H), 3.00-2.91 (m, 2H), 2.83-2.75 (m, 2H), 1.20 (t, *J* = 7.0 Hz, 3H).

### Step 2: Synthesis of intermediate BB-19-2

Intermediate **BB-19-1** (0.7 g, 2.69 mmol, 1 eq) was dissolved in ethanol (15 mL) at room temperature, and methylamine (726.32 mg, 10.76 mmol, 4 eq, hydrochloride), trimethylamine (1.09 g, 10.76 mmol, 1.50 mL, 4 eq) and sodium cyanoborohydride (338.01 mg, 5.38 mmol, 2 eq) were then added. The reaction mixture was stirred to react at room temperature for 12 hours. After the reaction was completed, the reaction solution was concentrated in vacuo to remove the solvent. The resulting residue was diluted with water (50 mL) and extracted with ethyl acetate (30 mL×3). The organic phases were combined, washed with saturated brine (50 mL×2), dried over anhydrous sodium sulfate, and filtered. The filtrate was concentrated under reduced pressure to remove the solvent to give the crude intermediate **BB-19-2.**

### Step 3: Synthesis of intermediate BB-19-3

Intermediate **BB-19-2** (2.69 mmol, 1 eq, crude) was dissolved in dichloromethane (10 mL) at room temperature, and di-tert-butyl dicarbonate (704.51 mg, 3.23 mmol, 741.59 µL, 1.2 eq) and triethylamine (544.40 mg, 5.38 mmol, 748.83 µL, 2 eq) were then added. The reaction mixture was stirred to react at room temperature for 12 hours. After the reaction was completed, the reaction solution was concentrated under reduced pressure to remove the solvent, and the resulting residue was separated by column chromatography (eluent: petroleum ether/ethyl acetate = 20/1-10/1, v/v) to give the intermediate **BB-19-3.** ¹H NMR (400MHz, CDCl₃) δ: 7.61 (s, 1H), 7.40-7.35 (m, 2H), 7.14 (dd, *J* = 1.4, 8.2 Hz, 1H), 4.20 (q, *J* = 7.2 Hz, 2H), 3.69 (s, 2H), 3.35-3.19 (m, 2H), 2.86 (s, 3H), 2.74-2.66 (m, 2H), 1.93-1.83 (m, 2H), 1.45 (s, 9H), 1.28 (t, *J* = 7.2 Hz, 3H).

### Step 4: Synthesis of intermediate BB-19-4

Intermediate **BB-19-3** (0.13 g, 346.24 µmol, 1 eq) was dissolved in N,N-dimethylformamide (5 mL) at 0°C, and potassium tert-butoxide (42.74 mg, 380.87 µmol, 1.1 eq) and acrylamide (27.07 mg, 380.87 µmol, 26.28 µL, 1.1 eq) were then added sequentially. The reaction mixture was stirred to react at 0°C for another 1 hour. After the reaction was completed, the reaction solution was diluted with water (50 mL) and extracted with ethyl acetate (30 mL×3). The organic phases were combined, washed with saturated brine (50 mL×3), dried over anhydrous sodium sulfate, and filtered. The filtrate was concentrated under reduced pressure to remove the solvent. The resulting residue was separated by column chromatography (eluent: petroleum ether/ethyl acetate = 10/1-1/1, v/v) to give the intermediate **BB-19-4.** ¹H NMR (400MHz, CDCl₃) δ: 8.07 (s, 1H), 7.56 (s, 1H), 7.50-7.37 (m, 2H), 7.16 (dd, *J =* 1.6, 8.4 Hz, 1H), 4.03-3.97 (m, 1H), 3.34-3.20 (m, 2H), 2.86 (s, 3H), 2.83-2.75 (m, 2H), 2.74-2.66 (m, 2H), 2.43-2.34 (m, 2H), 1.92-1.82 (m, 2H), 1.46 (s, 9H).

### Step 5: Synthesis of intermediate BB-19 hydrochloride

Intermediate **BB-19-4** (0.05 g, 124.85 µmol, 1 eq) was dissolved in ethyl acetate (5 mL) at room temperature, and a solution of hydrochloric acid in ethyl acetate (10 mL, 4 M) was then added. The reaction mixture was stirred to react at room temperature for 4 h. After the reaction was completed, the reaction solution was concentrated under reduced pressure to remove the solvent to give the crude intermediate **BB-19** hydrochloride.

### Reference example 20

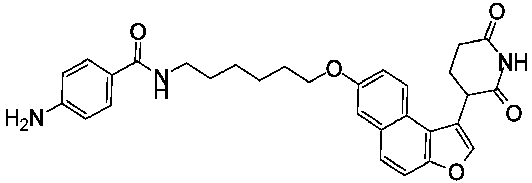

### Synthetic route:

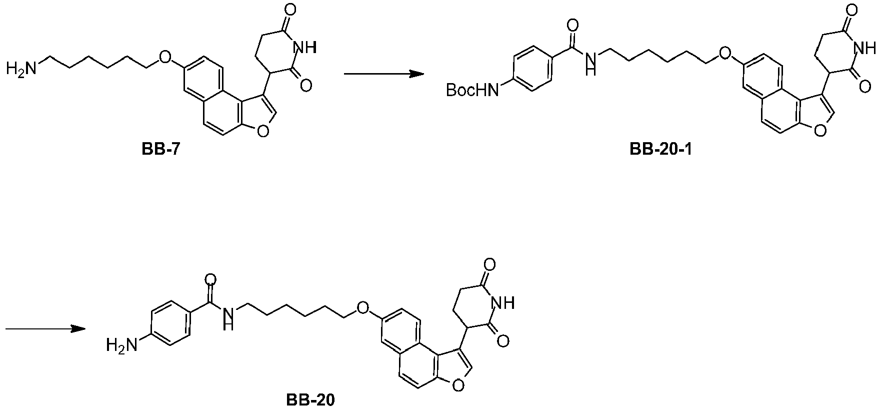

### Step 1: Synthesis of intermediate BB-20-1

4-(Boc-amino)benzoic acid (110.11 mg, 464.12 µmol) was dissolved in N,N-dimethylformamide (2 mL), and 1-(3-dimethylaminopropyl)-3-ethylcarbodiimide hydrochloride (133.46 mg, 696.18 µmol), 1-hydroxybenzotriazole (94.07 mg, 696.18 µmol) and N,N-diisopropylethylamine (209.94 mg, 1.62 mmol, 282.94 µL) were added at 0°C. The mixture was stirred at 0°C for 30 min, and intermediate **BB-7** (200 mg, 464.12 µmol, hydrochloride) was then added. The reaction mixture was warmed up to 15 °C and stirred to react under nitrogen for 16 h. The reaction solution was diluted with ethyl acetate (15 mL) and water (5 mL), and the organic phase was collected after the layers were separated, and the aqueous phase was extracted with ethyl acetate (10 mL×2). The organic phases were combined, dried over anhydrous sodium sulfate, and the solvent was removed by concentration under reduced pressure. The resulting residue was separated by column chromatography (eluent: petroleum ether/ethyl acetate = 8/1-0/1, v/v) to give the intermediate **BB-20-1.**

### Step 2: Synthesis of intermediate BB-20

Intermediate **BB-20-1** (200 mg, 325.89 µmol) was added to ethyl acetate (2 mL), and hydrochloric acid/ethyl acetate solution (4 M, 4.89 mL) was then added. The mixture was reacted at 15°C for 1 hour. After the reaction was completed, the solvent was removed by concentration under reduced pressure to give the intermediate **BB-20.** ¹H NMR (400 MHz, DMSO_*d*₆) δ: 10.94 (s, 1H), 8.41 (s, 1H), 8.07 (d, *J* = 8.8 Hz, 1H), 7.98-7.95 (m, 1H), 7.82 (d, *J* = 8.0 Hz, 2H), 7.77-7.70 (m, 2H), 7.49 (d, *J* = 2.4 Hz, 1H), 7.21 (dd, *J* = 2.4, 9.0 Hz, 1H), 7.16 (d, *J* = 8.0 Hz, 2H), 4.63 (dd, *J* = 4.2, 11.8 Hz, 1H), 4.10 (t, *J* = 6.4 Hz, 2H), 3.29-3.22 (m, 2H), 2.93-2.83 (m, 1H), 2.67-2.56 (m, 1H), 2.45-2.32 (m, 1H), 2.32-2.21 (m, 1H), 1.85-1.74 (m, 2H), 1.60-1.45 (m, 4H), 1.45-1.35 (m, 2H).

### Reference example 21

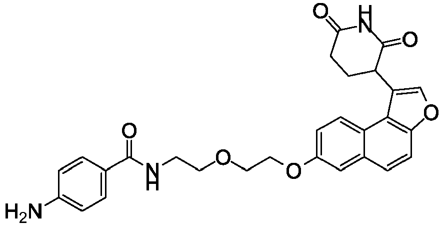

### Synthetic route:

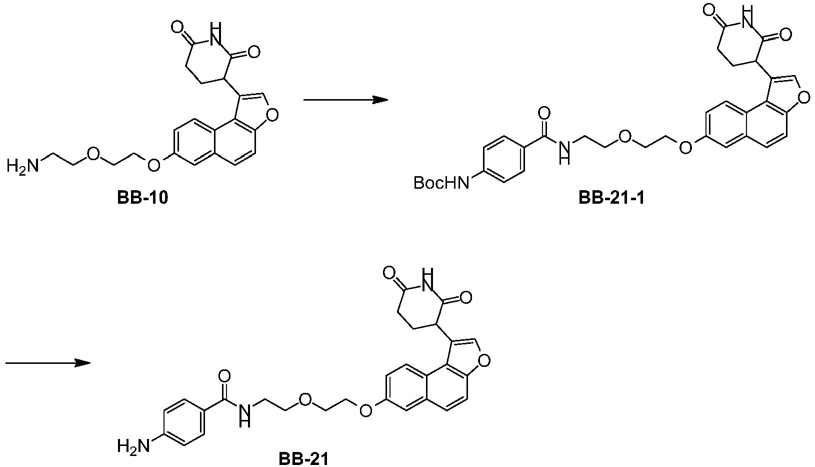

### Step 1: Synthesis of intermediate BB-21-1

4-(Boc-amino)benzoic acid (113.28 mg, 477.48 µmol) was dissolved in N,N-dimethylformamide (2 mL), and 1-ethyl-(3-dimethylaminopropyl)carbodiimide hydrochloride (137.30 mg, 716.21 µmol), 1-hydroxybenzotriazole (96.77 mg, 716.21 µmol) and N,N-diisopropylethylamine (215.98 mg, 1.67 mmol, 291.08 µL) were added at 0°C. The mixture was stirred at 0 °C for 30 min, and intermediate **BB-10** (200 mg, 477.48 µmol, hydrochloride) was added. The reaction mixture was warmed up to 15 °C and reacted with stirring under nitrogen for 16 h. After the reaction was completed, the reaction solution was diluted with ethyl acetate (15 mL) and water (5 mL), and the organic phase was collected after the layers were separated, the aqueous phase was extracted with ethyl acetate (10 mL×2). The organic phases were combined, dried over anhydrous sodium sulfate, and the solvent was removed by concentration under reduced pressure. The resulting residue was separated by column chromatography (eluent: petroleum ether/ethyl acetate = 511-011, v/v) to give the intermediate **BB-21-1.**

### Step 2: Synthesis of intermediate BB-21

Intermediate **BB-21-1** (200 mg, 332.42 µmol) was added to ethyl acetate (2 mL), and hydrochloric acid/ethyl acetate (4 M, 4.98 mL) was then added. The mixture was reacted at 15 °C for 1 h. After the reaction was completed, the mixture was concentrated under reduced pressure to remove ethyl acetate to give the intermediate **BB-21.** ¹H NMR (400 MHz, DMSO_*d*₆) δ: 10.94 (s, 1H), 8.50 (s, 1H), 8.09 (d, *J* = 8.8 Hz, 1H), 7.96 (s, 1H), 7.82 (d, *J* = 8.4 Hz, 2H), 7.74 (s, 2H), 7.51 (d, *J* = 2.4 Hz, 1H), 7.23 (dd, *J* = 2.4, 9.2 Hz, 1H), 7.13 (d, *J* = 8.0 Hz, 2H), 4.64 (dd, *J* = 4.4, 11.6 Hz, 1H), 4.27-4.20 (m, 2H), 3.87-3.80 (m, 2H), 3.66-3.59 (m, 2H), 3.49-3.41 (m, 2H), 2.93-2.83 (m, 1H), 2.67-2.55 (m, 1H), 2.46-2.33 (m, 1H), 2.31-2.20 (m, 1H).

### Reference example 22

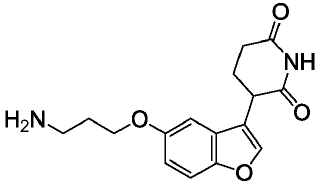

### Synthetic route:

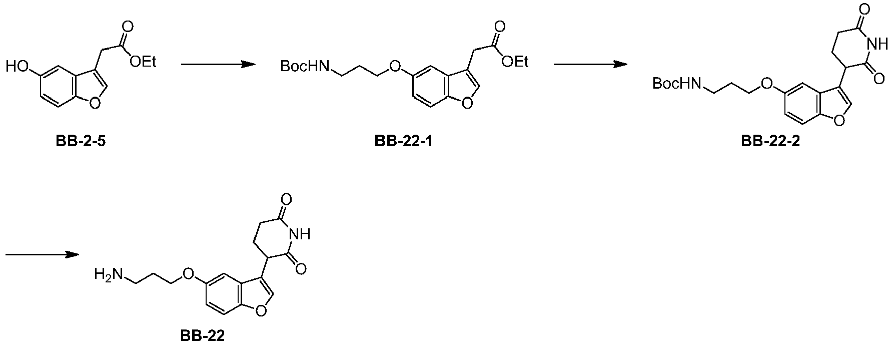

### Step 1: Synthesis of intermediate BB-22-1

Intermediate **BB-2-5** (500.00 mg, 2.27 mmol) was dissolved in tetrahydrofuran (12.5 mL) at room temperature under nitrogen, and 3-tert-butoxycarbonyl-amino-1-propanol (477.41 mg, 2.72 mmol) and azodicarbonyl dipiperidine (744.72 mg, 2.95 mmol) were added sequentially. The atmosphere was replaced three times with nitrogen. Tributylphosphine (728.24 µL, 2.95 mmol) was added dropwise at 0°C and under nitrogen. The reaction mixture was stirred to react at 15 °C for 16 h. After the reaction was completed, the 2 parallel reactions were combined. The reaction system was diluted with water (20 mL) and ethyl acetate (50 mL). The organic phase was collected after the layers were separated, and the aqueous phase was extracted with ethyl acetate (20 mL×2). The organic phases were combined, dried over anhydrous sodium sulfate, and filtered. The filtrate was concentrated under reduced pressure to remove the solvent. The resulting residue was separated by column chromatography (eluent: petroleum ether/ethyl acetate = 1/0-0/1, v/v) to give the intermediate **BB-22-1.** ¹H NMR (400MHz, CDCl₃) δ: 7.60 (s, 1H), 7.36 (d, *J* = 8.8 Hz, 1H), 7.02 (d, *J* = 2.4 Hz, 1H), 6.90 (dd, *J* = 2.4, 9.2 Hz, 1H), 4.84 (s, 1H), 4.22-4.17 (m, 2H), 4.06 (t, *J* = 6.0 Hz, 2H), 3.66 (s, 2H), 3.37-3.34 (m, 2H), 2.00 (m, 2H), 1.46 (s, 9H), 1.32-1.26 (t, *J* = 7.2 Hz, 3H).

### Step 2: Synthesis of intermediate BB-22-2

Intermediate **BB-22-1** (1.00 g, 2.65 mmol) was dissolved in tetrahydrofuran (20 mL) at room temperature under nitrogen. The atmosphere was replaced three times with nitrogen. Acrylamide (188.32 mg, 2.65 mmol) and potassium tert-butoxide (356.77 mg, 3.18 mmol) were added sequentially under nitrogen, and the reaction mixture was stirred to react at 15 °C for 1 h. After the reaction was completed, the reaction solution was slowly poured into ice water (50 mL) and extracted with ethyl acetate (100 mL×3). The organic phases were combined, dried over anhydrous sodium sulfate, and filtered. The filtrate was concentrated under reduced pressure to remove the solvent. The resulting residue was separated by column chromatography (eluent: petroleum ether/ethyl acetate = 1/0-0/1, v/v) to give the intermediate **BB-22-2.** ¹H NMR (400MHz, CDCl₃) δ: 8.11 (s, 1H), 7.55 (s, 1H), 7.40 (d, *J* = 9.6 Hz, 1H), 6.96-6.90 (m, 2H), 4.80 (s, 1H), 4.05 (t, *J* = 6.0 Hz, 2H), 3.97 (t, *J* = 7.6 Hz, 1H), 3.37-3.33 (m, 2H), 2.85-2.68 (m, 2H), 2.40-2.32 (m, 2H), 2.03-1.96 (m, 2H), 1.45 (s, 9H).

### Step 3: Synthesis of intermediate BB-22 hydrochloride

Intermediate **BB-22-2** (380.24 mg, 944.84 µmol) was dissolved in ethyl acetate (8 mL) at room temperature under nitrogen, and hydrochloric acid/ethyl acetate solution (4 M, 8 mL) was added. The reaction mixture was stirred at 25 °C for 1 h. After the reaction was completed, the reaction solution was concentrated under reduced pressure to remove the solvent to give the crude intermediate **BB-22** hydrochloride.

### Reference example 23

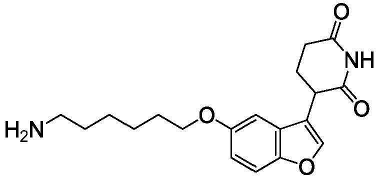

### Synthetic route:

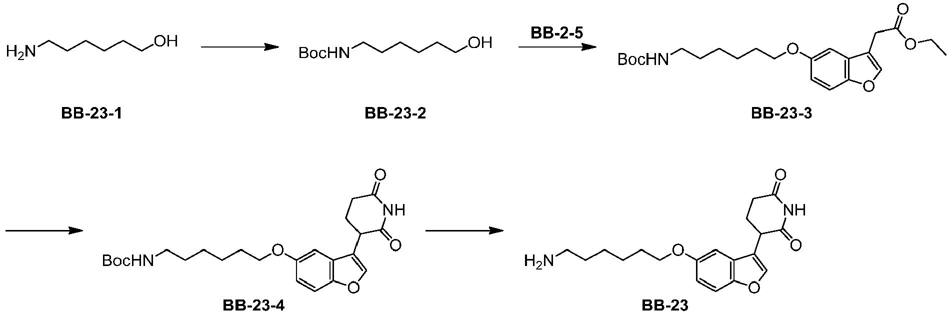

### Step 1: Synthesis of intermediate BB-23-2

Intermediate **BB-23-1** (10.00 g, 85.33 mmol) and triethylamine (17.27 g, 170.66 mmol, 23.75 mL) were dissolved in dichloromethane (100 mL) at room temperature under nitrogen, and di-tert-butyl dicarbonate (22.35 g, 102.40 mmol) dissolved in dichloromethane (10 mL) was slowly added at 0°C. The reaction mixture was warmed up to 18°C to react for 12 hours. After the reaction was completed, water (50 mL) was added to the reaction solution. The organic phase was separated and the aqueous phase was extracted with dichloromethane (100 mL). The organic phases were combined, dried over anhydrous sodium sulfate, and filtered. The filtrate was concentrated under reduced pressure to remove the solvent. The resulting residue was separated by column chromatography (eluent: petroleum ether/ethyl acetate = 10/1-0/1, v/v) to give the intermediate **BB-23-2.** ¹H NMR (400MHz, CDCl₃) δ: 4.54 (s, 1H), 3.63 (t, *J* = 6.4 Hz, 2H), 3.11 (t, *J* = 6.8 Hz, 2H), 1.61-1.55 (m, 2H), 1.54-1.41 (m, 11H), 1.41-1.30 (m, 4H).

### Step 2: Synthesis of intermediate BB-23-3

Intermediate **BB-2-5** (500.00 mg, 2.27 mmol) was dissolved in anhydrous tetrahydrofuran (12.5 mL) at room temperature under nitrogen atmosphere, and intermediate **BB-23-2** (591.94 mg, 2.72 mmol), N,N,N',N'-tetramethylazodicarbonamide (508.12 mg, 2.95 mmol) were added sequentially. The atmosphere was replaced three times with nitrogen. Tributylphosphine (597.04 mg, 2.95 mmol, 728.09 µL) was added dropwise at 0°C and the reaction solution was slowly warmed up to 15 °C and stirred for 16 h. After the reaction was completed, the reaction system was diluted with water (20 mL) and ethyl acetate (50 mL). The organic phase was collected after the layers were separated, and the aqueous phase was extracted with ethyl acetate (20 mL×2). The organic phases were combined, dried over anhydrous sodium sulfate, and filtered. The filtrate was concentrated under reduced pressure to remove the solvent. The resulting residue was separated by column chromatography (eluent: petroleum ether/ethyl acetate = 1/0-1/5, v/v) to give the intermediate **BB-23-3.** ¹H NMR (400MHz, CDCl₃) δ: 7.60 (s, 1H), 7.35 (d, *J* = 8.8 Hz, 1H), 7.01 (d, *J* = 2.4 Hz, 1H), 6.90 (dd, *J* = 2.4, 8.8 Hz, 1H), 4.54 (s, 1H), 4.22-1.17 (m, 2H), 3.99 (t, *J* = 6.4 Hz, 2H), 3.66 (s, 2H), 3.13 (t, *J* = 6.4 Hz 2H), 1.87-1.76 (m, 2H), 1.58-1.48 (m, 4H), 1.48-1.39 (m, 11H), 1.30-1.25 (m, 3H).

### Step 3: Synthesis of intermediate BB-23-4

Intermediate **BB-23-3** (1.10 g, 2.62 mmol) was dissolved in anhydrous tetrahydrofuran (24 mL) at room temperature under nitrogen, and the atmosphere was replaced three times with nitrogen. Acrylamide (186.37 mg, 2.62 mmol) and potassium tert-butoxide (353.08 mg, 3.15 mmol) were added sequentially. The reaction mixture was stirred to react at 15 °C for 1 h. After the reaction was completed, the 2 parallel reactions were combined and the reaction solution was slowly poured into ice water (50 mL) and extracted with ethyl acetate (100 mL×3). The organic phases were combined, dried over anhydrous sodium sulfate, and filtered. The filtrate was concentrated under reduced pressure to remove the solvent. The resulting residue was separated by column chromatography (eluent: petroleum ether/ethyl acetate = 1/0-0/1, v/v) to give the intermediate **BB-23-4.** ¹H NMR (400MHz, CDCl₃) δ: 8.10 (s, 1H), 7.54 (s, 1H), 7.39 (d, *J* = 9.6 Hz, 1H), 6.95-6.91 (m, 2H), 4.55 (s, 1H), 4.02-3.94 (m, 3H), 3.20-3.08 (m, 2H), 2.86-2.68 (m, 2H), 2.42-2.33 (m, 2H), 1.85-1.76 (m, 2H), 1.55-1.37 (m, 15H).

### Step 4: Synthesis of intermediate BB-23 hydrochloride

Intermediate **BB-23-4** (0.60 g, 1.35 mmol) was dissolved in ethyl acetate (6 mL) at room temperature under nitrogen, and hydrochloric acid/ethyl acetate solution (4 M, 6.00 mL) was then added. The reaction solution was stirred at 20 °C for 1 h. After the reaction was completed, the reaction solution was concentrated under reduced pressure to remove the solvent to give the crude intermediate **BB-23** hydrochloride.

### Reference example 24

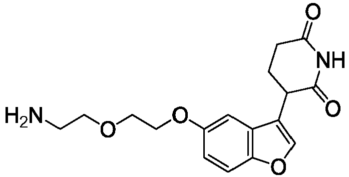

### Synthetic route:

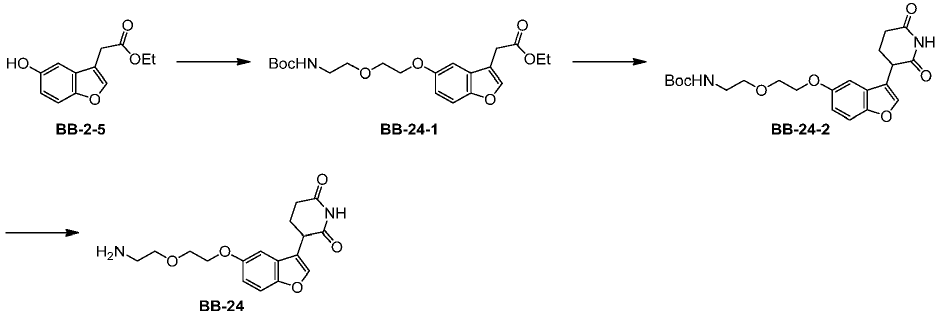

### Step 1: Synthesis of intermediate BB-24-1

2-[2-[(tert-butoxycarbonyl)amino]ethoxy]ethanol (559.21 mg, 2.72 mmol) and intermediate **BB-2-5** (500.00 mg, 2.27 mmol) were dissolved in tetrahydrofuran (12.5 mL) at room temperature under nitrogen, and N,N,N',N'-tetramethylazodicarbonamide (508.22 mg, 2.95 mmol) was added. The reaction system was purged three times with nitrogen. Tributylphosphine (728.24 µL, 2.95 mmol) was added and stirred at 15°C for 12 h. After the reaction was completed, the reactions were combined and quenched by the addition of saturated brine (50 mL). The mixture was diluted with ethyl acetate (80 mL), and the layers were separated. The organic phase was collected and the aqueous phase was extracted with ethyl acetate (80 mL×3). The organic phases were sequentially combined, washed with saturated brine (50 mL×3), dried over anhydrous sodium sulfate, and filtered. The filtrate was concentrated under reduced pressure to remove the solvent. The resulting residue was separated by column chromatography (eluent: petroleum ether/ethyl acetate = 1/0-1/1, v/v) to give the intermediate **BB-24-1.**

### Step 2: Synthesis of intermediate BB-24-2

Intermediate **BB-24-1** (530.00 mg, 1.30 mmol) was dissolved in anhydrous tetrahydrofuran (5 mL) at 0°C under nitrogen. Potassium tert-butoxide (175.15 mg, 1.56 mmol) and acrylamide (92.45 mg, 1.30 mmol) were added, and the reaction system was stirred at 15°C for 2 hours. After the reaction was completed, the reaction solution was quenched by pouring the reaction solution into ice water (15 mL). The mixture was diluted with ethyl acetate (35 mL), and the layers were separated. The organic phase was collected, and the aqueous phase was extracted with ethyl acetate (35 mL × 3). The organic phases were sequentially combined, washed with saturated brine (45 mL×2), dried over anhydrous sodium sulfate, and filtered. The filtrate was concentrated under reduced pressure to remove the solvent. The resulting residue was separated by column chromatography (eluent: petroleum ether/ethyl acetate = 1/0-1/4, v/v) to give the intermediate **BB-24-2.**

### Step 3: Synthesis of intermediate BB-24 hydrochloride

Intermediate **BB-24-2** (260.00 mg, 601.20 µmol) was dissolved in ethyl acetate (3 mL) at room temperature under nitrogen, and hydrochloric acid/ethyl acetate solution (4 M, 3 mL) was added at 25°C. The reaction system was stirred at 25°C for 1 hour. After the reaction was completed, the reaction solution was concentrated under reduced pressure to remove the solvent to give crude intermediate **BB-24** hydrochloride.

### Reference example 25

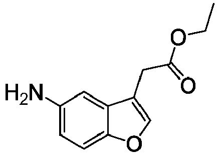

### Synthetic route:

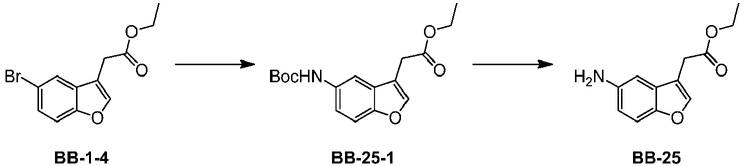

### Step 1: Synthesis of intermediate BB-25-1

Intermediate **BB-1-4** (2.49 g, 5.37 mmol) and tert-butyl carbamate (1.89 g, 16.10 mmol) were added to a mixture of toluene (40 mL) and water (8 mL) at room temperature under nitrogen, and potassium phosphate (4.56 g, 21.46 mmol), tris(dibenzylideneacetone)dipalladium (491.33 mg, 536.55 µmol) and 2-di-tert-butylphosphino-2',4',6'-triisopropylbiphenyl (455.69 mg, 1.07 mmol) were added sequentially. The reaction mixture was stirred to react at 105 °C under nitrogen for 14 h. After the reaction was completed, the mixture was cooled down to room temperature. Water (30 mL) was added to the reaction solution and the resulting mixture was extracted with ethyl acetate (30 mL×3). The organic phases were combined, washed with saturated brine (30 mL×3), dried over anhydrous sodium sulfate, and filtered. The filtrate was concentrated under reduced pressure to remove the solvent. The resulting residue was separated by column chromatography (eluent: petroleum ether/ethyl acetate = 20/1-5/1, v/v) to give the intermediate **BB-25-1.** ¹H NMR (400 MHz, CDCl₃) δ: 7.71 (br s, 1H), 7.62 (s, 1H), 7.37 (d, *J* = 8.8 Hz, 1H), 7.15 (dd, *J* = 2.0, 8.8 Hz, 1H), 6.60 (br s, 1H), 4.20 (q, *J* = 7.2 Hz, 2H), 3.67 (d, *J* = 0.8 Hz, 2H), 1.53 (s, 9H), 1.29 (s, 3H).

### Step 2: Synthesis of intermediate BB-25 hydrochloride

Intermediate **BB-25-1** (500.00 mg, 1.13 mmol) was dissolved in ethyl acetate (10 mL) at room temperature. To the mixture was added hydrochloric acid/ethyl acetate solution (4 M, 2.82 mL), and the reaction mixture was stirred to react at room temperature for 12 h. After the reaction was completed, the mixture was filtered, and the filter cake was washed with ethyl acetate (50 mL), and dried to give the intermediate **BB-25** hydrochloride. ¹H NMR (400 MHz, DMSO_*d*₆) δ: 10.49 (br s, 3H), 8.04 (s, 1H), 7.70 (d, *J* = 8.4 Hz, 1H), 7.61 (d, *J =* 2.0 Hz, 1H), 7.36 (dd, *J* = 2.0, 8.8 Hz, 1H), 4.12 (q, *J* = 7.1 Hz, 2H), 3.81 (s, 2H), 1.21 (t, *J* = 7.2 Hz, 3H).

### Reference example 26

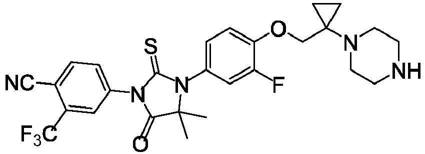

### Synthetic route:

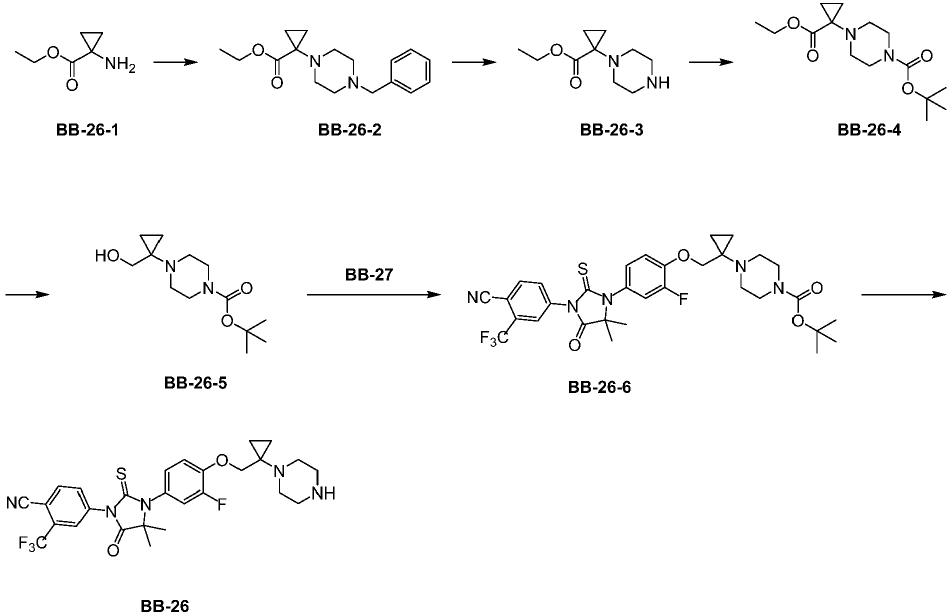

### Step 1: Synthesis of intermediate BB-26-2

To a pre-dried reaction flask were added N-benzyl-biso-chloroethylamino hydrochloride (24.33 g, 90.57 mmol), ethanol (150 mL), compound **BB-26-1** (24.33 g, 90.57 mmol) and *N*,*N*-diisopropylethylamine (117.05 g, 905.70 mmol, 157.75 mL), and the reaction system was heated to 90 °C and stirred for 16 h. After the reaction was completed, the solvent was removed by concentration under reduced pressure and water (100 mL) was added to the residue. The mixture was extracted with dichloromethane (100 mL×3), and the organic phases were combined, washed with saturated brine (300 mL×2), dried over anhydrous sodium sulfate, and filtered. The filtrate was concentrated under reduced pressure to remove the solvent. The resulting residue was purified by column chromatography (eluent: petroleum ether/ethyl acetate = 5011-1011, v/v) to give the compound **BB-26-2.** MS-ESI m/z: 289.3 [M+H]⁺. ¹H NMR (400MHz, CDCl₃) δ: 7.29-7.18 (m, 5H), 4.11-4.06 (q, *J* = 6.8 Hz, 2H), 3.45 (s, 2H), 2.92 (s, 4H), 2.31 (s, 4H), 1.23-1.19 (m, 5H), 0.85-0.83 (m, 2H).

### Step 2: Synthesis of intermediate BB-26-3 hydrochloride

Compound **BB-26-2** (8 g, 27.74 mmol) was dissolved in dichloromethane (80 mL), and chloroethyl chloroformate (5.79 g, 40.50 mmol) was added at 0°C. The reaction system was warmed up to 20 °C and stirred for 1 hour. The reaction was completed and the reaction solution was concentrated under reduced pressure to remove the solvent. The residue was redissolved in ethanol (80 mL) and reacted at 85 °C for another 16 h. After the reaction was completed, the reaction solution was concentrated under reduced pressure to remove the solvent. Ethyl acetate (80 mL) was added to the residue, and the mixture was stirred for 20 min and then filtered. The filter cake was collected to give compound **BB-26-3** hydrochloride. ¹H NMR (400 MHz, CD₃OD) δ: 4.22 - 4.11 (m, 2H), 3.59 - 3.35 (m, 4H), 3.31 - 3.12 (m, 4H), 1.57 - 1.27 (m, 4H), 1.27 - 1.18 (m, 3H).

### Step 3: Synthesis of intermediate BB-26-4

Compound **BB-26-3** (3.5 g, 14.91 mmol, hydrochloride) was dissolved in a mixture of dioxane (30 mL) and water (10 mL), and the reaction system was cooled down to 0 °C. Sodium bicarbonate (3.76 g, 44.73 mmol, 1.74 mL) and tert-butyl carbonate (3.25 g, 14.91 mmol, 3.43 mL) were added, and the reaction system was restored to 20°C and stirred for 12 hours. The organic phases were combined, washed with saturated brine (30 mL), dried over anhydrous sodium sulfate, and filtered. The filtrate was concentrated under reduced pressure. The resulting residue was purified by column chromatography (eluent: petroleum ether/ethyl acetate = 0/1-25/2, v/v) to give the compound **BB-26-4.** ¹H NMR (400MHz, CDCl₃) δ: 4.12 (q, *J* = 7.1 Hz, 2H), 3.31 (br s, 4H), 2.90 (br s, 4H), 1.47 (s, 9H), 1.32 - 1.27(m, 2H), 1.25 (t, *J* = 7.2 Hz, 3H), 0.94 (q, *J* = 3.7 Hz, 2H).

### Step 4: Synthesis of intermediate BB-26-5

Compound **BB-26-4** (3.1 g, 10.39 mmol) was dissolved in tetrahydrofuran (35 mL) and the atmosphere was replaced three times with nitrogen. The mixture was cooled down to -65 °C, and lithium tetrahydroaluminum (1 M in tetrahydrofuran, 31.17 mL) was slowly added and stirred at 0°C for 1 h. After the reaction was completed, sodium sulfate decahydrate (30 g) was slowly added to the reaction solution at 0°C. The mixture was filtered, and the filter cake was washed with dichloromethane, the filtrate was concentrated under reduced pressure to give compound **BB-26-5.** ¹H NMR (400MHz, CDCl₃) δ: 3.58 (s, 2H), 3.35 (t, *J* = 4.8 Hz, 4H), 2.69 (t, *J* = 4.8 Hz, 4H), 1.45 (s, 9H), 0.71-0.68 (m, 2H), 0.55-0.52 (m, 2H).

### Step 5: Synthesis of intermediate BB-26-6

Compound **BB-27** (2.39 g, 5.66 mmol) was dissolved in tetrahydrofuran (50 mL) under nitrogen, then compound **BB-26-5** (2.9 g, 11.31 mmol) and triphenylphosphine (2.97 g, 11.31 mmol) were added. The reaction system was cooled down to 0 °C. Diisopropyl azodicarboxylate (2.29 g, 11.31 mmol, 2.20 mL) was added dropwise. The mixture was heated to 40°C and stirred for 12 hours. After the reaction was completed, water (20 mL) and ethyl acetate (50 mL) were added to the reaction solution. The organic phase was separated, and the aqueous phase was extracted with ethyl acetate (50 mL×3). The organic phases were combined, washed with saturated brine (50 mL×3), dried over anhydrous sodium sulfate, and filtered. The filtrate was concentrated under reduced pressure to remove the solvent. The resulting residue was purified by column chromatography (eluent: petroleum ether/ethyl acetate = 1/0-4/5, v/v) to give the compound **BB-26-6.** ¹H NMR (400MHz, CDCl₃) δ: 8.00-7.96 (m, 2H), 7.85-7.82 (m, 1H), 7.08-7.02 (m, 3H), 4.05 (s, 2H), 2.81 (s, 4H), 2.69 (s, 4H), 1.59 (s, 6H), 1.46 (s, 9H), 0.75-0.70 (m, 4H).

### Step 5: Synthesis of intermediate BB-26 hydrochloride

Compound **BB-26-6** (3.2 g, 4.84 mmol) was dissolved in ethyl acetate (5 mL) and hydrochloric acid/ethyl acetate (30 mL, 4 M) was added. The mixture was reacted at 15 °C for 2 h. The reaction solution was concentrated under reduced pressure to remove the solvent, and methyl tert-butyl ether (10 mL) was added. The mixture was stirred for 5 min, and filtered. The filter cake was washed with methyl tert-butyl ether (2 mL×2), collected and dried under reduced pressure to remove the solvent to give compound **BB-26** hydrochloride.

### Reference example 27

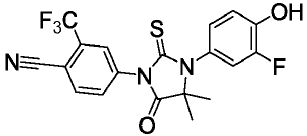

### Synthetic route:

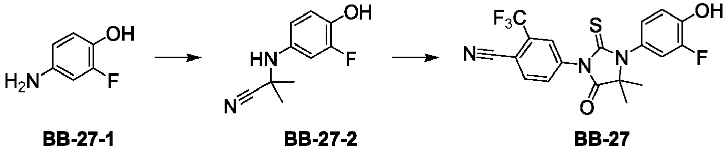

### Step 1: Synthesis of intermediate BB-27-2

Compound **BB-27-1** (10 g, 78.67 mmol) was dissolved in dichloromethane (120 mL) and acetone (60 mL) at 0-5°C under nitrogen, and cyanotrimethylsilane (12.45 g, 125.50 mmol, 15.70 mL) and trimethylsilyl trifluoromethanesulfonate (820.00 mg, 3.69 mmol, 666.67 µL) were added slowly dropwise sequentially. The reaction mixture was stirred to react at 25 °C for 2 h. After the reaction was completed, the mixture was cooled down to 0-5 °C, diluted with water (200 mL) and extracted with ethyl acetate (200 mL × 3). The combined organic phases were washed with saturated brine (100 mL × 2), dried over anhydrous sodium sulfate, and filtered. The solvent was removed from the filtrate under reduced pressure. The resulting residue was separated by column chromatography (eluent: petroleum ether/ethyl acetate = 10/1-5/1, v/v) to give target compound **BB-27-2.** ¹H NMR (400MHz, CDCl₃) δ: 6.83 (t, *J* = 9.0 Hz, 1H), 6.74 (dd, *J* = 2.7, 12.1 Hz, 1H), 6.66 - 6.59 (m, 1H), 1.65 (s, 6H).

### Step 2: Synthesis of compound BB-27

Compound **BB-27-2** (8 g, 45.40 mmol) was dissolved in N,N-dimethylacetamide (150 mL) at room temperature under nitrogen, and 4-thioisocyanato-2-(trifluoromethyl)benzonitrile (10.36 g, 45.40 mmol) was added to the reaction solution in batches. The reaction mixture was stirred at 25°C for 12 h. Methanol (60 mL) and dilute hydrochloric acid (2M, 60 mL) were added. The reaction mixture was stirred at 70°C for 3 h. After the reaction was completed, the mixture was cooled down to room temperature, diluted with water (500 mL) and extracted with ethyl acetate (200 mL × 3). The organic phases were combined, washed with saturated brine (100 mL × 2), dried over anhydrous sodium sulfate, and filtered. The solvent was removed from the filtrate under reduced pressure. The resulting residue was separated by column chromatography (eluent: petroleum ether/ethyl acetate = 311-111, v/v) to give target compound **BB-27.** ¹H NMR (400MHz,CDCl₃) δ: 7.97 (s, 1H), 7.85 (dd, *J =* 1.8, 8.3 Hz, 1H), 7.18 - 7.10 (m, 2H), 6.99 - 6.88 (m, 2H), 6.06 (br s, 1H), 1.58 (s, 6H).

### Reference example 28

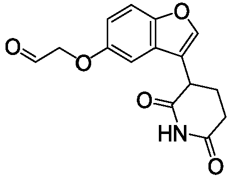

### Synthetic route:

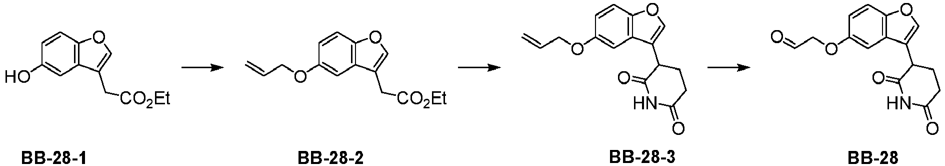

### Step 1: Synthesis of intermediate BB-28-2

Compound **BB-28-1** (2 g, 9.08 mmol), allyl bromide (1.21 g, 9.99 mmol) and potassium carbonate (2.51 g, 18.16 mmol) were dissolved in N,N-dimethylformamide (20 mL), and the mixture was heated to 50 °C to react for 15 hours. After the reaction was completed, the reaction solution was allowed to cool down to room temperature. Water (150 mL) was added and the mixture was extracted with ethyl acetate (30 mL×3). The organic phases were combined, washed with saturated brine (50 mL×3), dried over anhydrous sodium sulfate, and filtered. The filtrate was concentrated under reduced pressure to remove the solvent to give the compound **BB-28-2.**

### Step 2: Synthesis of intermediate BB-28-3

Compound **BB-28-2** (2 g, 7.68 mmol) was dissolved in *N*,*N*-dimethylformamide (20 mL), and acrylamide (546.15 mg, 7.68 mmol, 530.20 µL) and potassium tert-butoxide (1.12 g, 9.99 mmol) were added sequentially. The mixture was reacted at 20 °C for 1 hour. After the reaction was completed, the reaction solution was adjusted to pH of 5-6 with 0.5 N dilute hydrochloric acid, and extracted with ethyl acetate (20 mL×2). The organic phase was washed with saturated brine (50 mL), dried over anhydrous sodium sulfate, and filtered. The filtrate was concentrated under reduced pressure to remove the solvent. The resulting residue was purified by column chromatography (eluent: petroleum ether/ethyl acetate = 3/1-1/1, v/v) to give the compound **BB-28-3.** ¹H NMR (400MHz, CDCl₃) δ: 8.05 (s, 1H), 7.55 (s, 1H), 7.45-7.40 (m, 1H), 6.99-6.96 (m, 2H), 6.13-6.06 (m, 1H), 5.47-5.42 (m,1H), 5.33-5.30 (m, 1H), 4.58-4.56 (m, 2H), 3.99-3.96 (m, 1H), 2.84-2.73 (m, 2H), 2.39-2.36 (m, 2H).

### Step 3: Synthesis of intermediate BB-28

Compound **BB-28-3** (0.1 g, 350.52 µmol) was dissolved in tetrahydrofuran (3 mL) and water (1 mL), and the mixture was cooled down to 0 °C. Sodium periodate (299.89 mg, 1.40 mmol, 77.69 µL) and potassium osmate dihydrate (12.91 mg, 35.05 µmol) were added, and the mixture was reacted at 25 °C for 3 h. After the reaction was completed, water (5 mL) and ethyl acetate (10 mL) were added to the reaction solution. The organic phase was separated, and the aqueous phase was extracted with ethyl acetate (10 mL×3). The organic phases were combined, washed with saturated brine (10 mL×3), dried over anhydrous sodium sulfate, and filtered. The filtrate was concentrated under reduced pressure to give the compound **BB-28** as a crude product, which was directly used in the next step.

### Reference example 29

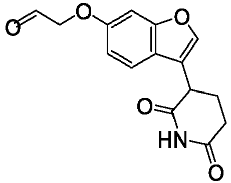

### Synthetic route:

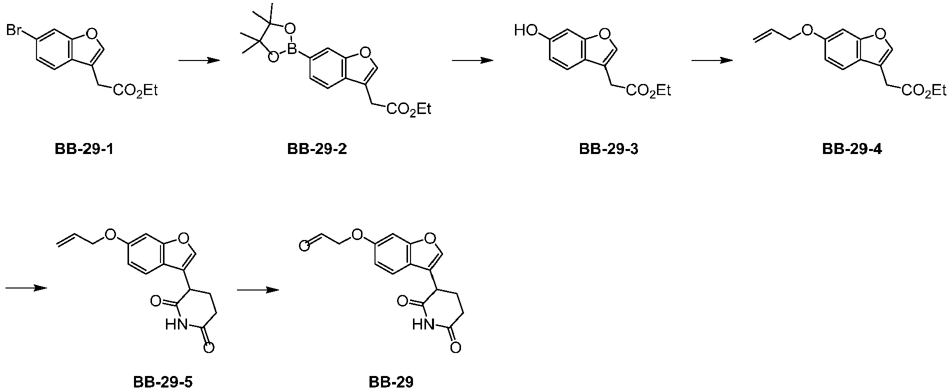

### Step 1: Synthesis of compound BB-29-2

Compound **BB-29-1** (3 g, 10.60 mmol), bis(pinacolato)diboron (3.23 g, 12.72 mmol), potassium acetate (4.16 g, 42.39 mmol), and [1,1-bis(diphenylphosphino)ferrocene]palladium dichloride dichloromethane (865.33 mg, 1.06 mmol) were dissolved in dioxane (50 mL) at room temperature under nitrogen. The mixture was heated to 100 °C and stirred for 5 h. After the reaction was completed, the reaction solution was allowed to cool down to room temperature, and filtered directly through pad of celite. The filter cake was washed with dichloromethane (30 mL×3), and the filtrate was concentrated under reduced pressure to remove the solvent. The resulting residue was purified by column chromatography (eluent: petroleum ether/ethyl acetate = 30/1, v/v) to give the compound **BB-29-2.**

### Step 2: Synthesis of compound BB-29-3

Compound **BB-29-2** (2.5 g, 7.57 mmol) and sodium bicarbonate (1.27 g, 15.14 mmol, 588.95 µL) were dissolved in a mixture of tetrahydrofuran (30 mL) and water (15 mL) at room temperature under nitrogen. The mixture was cooled down to 0 °C, and hydrogen peroxide (6.59 g, 58.12 mmol, 5.58 mL, purity: 30%) was added dropwise. The mixture was stirred for 2 h. 15% aqueous sodium sulfite (50 mL) was added and the mixture was stirred for 10 min. The mixture was adjusted to pH of 5 to 6 with 1N hydrochloric acid, and extracted with ethyl acetate (50 mL×3). The organic phase was washed with saturated brine (50 mL), dried over anhydrous sodium sulfate, and filtered. The filtrate was concentrated under reduced pressure to remove the solvent. The resulting residue was purified by column chromatography (eluent: petroleum ether/ethyl acetate = 6/1, v/v) to give the compound **BB-29-3.**

### Step 3: Synthesis of compound BB-29-4

Compound **BB-29-3** (0.85 g, 3.86 mmol) was added to N,N-dimethylformamide (20 mL) at room temperature under nitrogen, and allyl bromide (513.63 mg, 4.25 mmol) and potassium carbonate (1.07 g, 7.72 mmol) were added. The reaction system was heated to 50°C to react for 15 hours. After the reaction was completed, water (100 mL) was added to the reaction solution. The organic phase was separated, and the aqueous phase was extracted with ethyl acetate (20 mL×3). The organic phases were combined, washed with saturated brine (50 mL), dried over anhydrous sodium sulfate, and filtered. The filtrate was concentrated under reduced pressure to remove the solvent to give the compound **BB-29-4.**

### Step 4: Synthesis of compound BB-29-5

Compound **BB-29-4** (0.9 g, 3.46 mmol) was dissolved in N,N-dimethylformamide (10 mL) at room temperature under nitrogen, and acrylamide (245.77 mg, 3.46 mmol, 238.61 µL) and potassium tert-butoxide (504.9 mg, 4.50 mmol) were added sequentially. The mixture was reacted at 20 °C for 1 h. After the reaction was completed, the reaction solution was adjusted to pH of 5 to 6 with 0.5 N dilute hydrochloric acid, and extracted with ethyl acetate (30 mL×2). The organic phase was washed with saturated brine (50 mL), dried over anhydrous sodium sulfate, and filtered. The filtrate was concentrated under reduced pressure to remove the solvent. Methyl tert-butyl ether (5 mL) was added to the resulting residue, and the mixture was stirred for 5 min, and then filtered. The filter cake was washed with methyl tert-butyl ether (2 mL × 2) and collected to give the compound **BB-29-5.**

### Step 5: Synthesis of compound BB-29

Compound **BB-29-5** (0.1 g, 350.52 µmol) was dissolved in tetrahydrofuran (3 mL) and water (1 mL) at room temperature under nitrogen. The mixture was cooled down to 0 °C, and sodium periodate (299.89 mg, 1.40 mmol, 77.69 µL) and potassium osmate dihydrate (12.91 mg, 35.05 µmol) were added. The mixture was reacted at 25 °C for 3 hours. After the reaction was completed, water (5 mL) and ethyl acetate (10 mL) were added to the reaction solution. The organic phase was separated, and the aqueous phase was extracted with ethyl acetate (10 mL×3). The organic phases were combined, washed with saturated brine (10 mL×3), dried over anhydrous sodium sulfate, and filtered. The filtrate was concentrated under reduced pressure to remove the residual solvent to give compound **BB-29.**

### Reference example 30

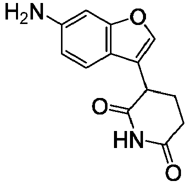

### Synthetic route:

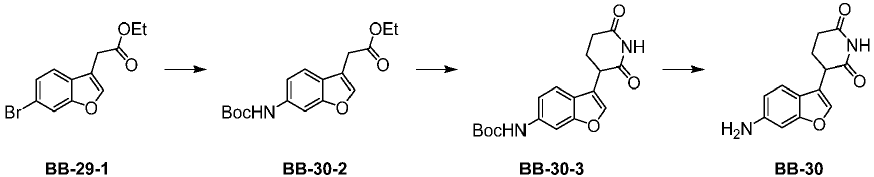

### Step 1: Synthesis of intermediate BB-30-2

Compound **BB-29-1** (1 g, 3.53 mmol) and tert-butyl carbamate (2.07 g, 17.66 mmol) were added to toluene (15 mL) and water (1.5 mL), and tris(dibenzylideneacetone)dipalladium (226.41 mg, 247.25 µmol), 2-di-tert-butylphosphino-2',4',6'-triisopropylbiphenyl (209.98 mg, 494.49 µmol) and potassium phosphate (3.00 g, 14.13 mmol) were sequentially added slowly to the reaction solution. The mixture was purged three times with nitrogen, then heated to 100 °C, and stirred for 12 h. The reaction solution was cooled down to room temperature and most of the organic solvent was rotary-evaporated under reduced pressure. The residue was diluted with ethyl acetate (30 mL). The organic phase was washed twice with water (30 mL × 2), dried over anhydrous sodium sulfate, and filtered. The filtrate was rotary-evaporated to dryness under reduced pressure. The resulting residue was separated by column chromatography (eluent: petroleum ether/ethyl acetate = 1/0-3/2, v/v) to give the intermediate **BB-30-2.** ¹H NMR (400MHz, CDCl₃) δ ppm:7.83 - 7.73 (m, 1H), 7.57 (s, 1H), 7.47 - 7.40 (m, 1H), 7.14 - 7.05 (m, 1H), 6.81 (br d, *J* = 1.0 Hz, 1H), 4.19 (q, *J* = 7.0 Hz, 2H), 3.67 (d, *J* = 1.3 Hz, 2H), 1.49 - 1.42 (m, 9H), 1.32 - 1.23 (m, 3H).

### Step 2: Synthesis of intermediate BB-30-3

Compound **BB-30-2** (300 mg, 939.40 µmol) and acrylamide (73.45 mg, 1.03 mmol, 71.31 µL) were added to tetrahydrofuran (5 mL) at 0°C. Potassium tert-butoxide (158.12 mg, 1.41 mmol) was slowly added to the reaction. The mixture was purged three times with nitrogen and then stirred at 0°C for 1 h. The reaction solution was slowly added to saturated aqueous ammonium chloride solution (20 mL), and extracted twice with ethyl acetate (20 mL × 2). The organic phase was washed once with saturated brine (20 mL), dried over anhydrous sodium sulfate, and filtered. The filtrate was rotary-evaporated to dryness under reduced pressure. The resulting residue was separated by column chromatography (eluent: petroleum ether/ethyl acetate = 1/0-3/1, volumetric ratio) to give the intermediate **BB-30-3.**

### Step 3: Synthesis of intermediate BB-30 hydrochloride

Compound **BB-30-3** (40 mg, 116.16 µmol) was added to ethyl acetate (2 mL) and hydrochloric acid/dioxane (4 M, 1 mL) was added slowly dropwise to the reaction mixture. The mixture was purged three times with nitrogen and then stirred at 25 °C for 12 h. The reaction solution was directly rotary-evaporated to dryness to give the intermediate **BB-30** hydrochloride.

### Reference example 31

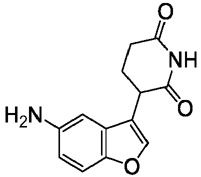

### Synthetic route:

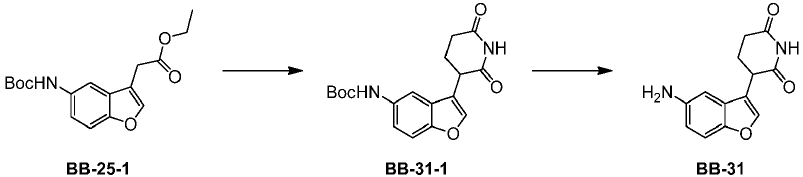

### Step 1: Synthesis of intermediate BB-31-1

Intermediate **BB-25-1** (12 g, 37.58 mmol) was dissolved in tetrahydrofuran (240 mL) at room temperature under nitrogen, and the atmosphere was replaced three times with nitrogen. Acrylamide (2.67 g, 37.58 mmol) and potassium tert-butoxide (5.48 g, 48.85 mmol) were added sequentially under nitrogen, and the reaction mixture was stirred to react at 20°C for 1 h. After the reaction was completed, water (12 mL) was added to the reaction solution, and then ethyl acetate (1.5 L) was added. The mixture was stirred for 3 min, then dried over anhydrous sodium sulfate, and filtered. The filtrate was concentrated under reduced pressure to remove the solvent. The resulting residue was separated by column chromatography (eluent: petroleum ether/ethyl acetate = 0/1-1/1, v/v) to give the intermediate **BB-31-1.**

### Step 2: Synthesis of intermediate BB-31 hydrochloride

Hydrochloric acid/ethyl acetate (4 M, 200 mL) was added to intermediate **BB-31-1** (8.3 g, 24.10 mmol) at 20 °C and the mixture was stirred for 1 h. After the reaction was completed, the reaction mixture was concentrated under reduced pressure to remove the solvent to give the intermediate **BB-31** hydrochloride.

### Reference example 32

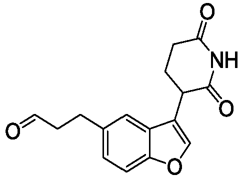

### Synthetic route:

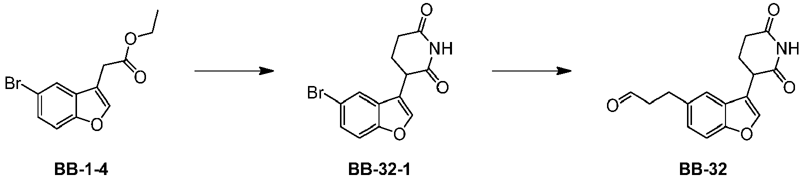

### Step 1: Synthesis of intermediate BB-32-1

**BB-1-4** (5.00 g, 17.66 mmol) was dissolved in N,N-dimethylformamide (30 mL) at 0°C under nitrogen. Potassium tert-butoxide (2.18 g, 19.43 mmol) and acrylamide (1.38 g, 19.43 mmol) were added respectively, and the reaction mixture was stirred to react at 0°C for 1 hour. After the reaction was completed, water (50 mL) was added to the reaction solution and the resulting mixture was extracted with ethyl acetate (50 mL×3). The organic phases were sequentially combined, washed with saturated brine (50 mL×3), dried over anhydrous sodium sulfate, and filtered. The filtrate was concentrated under reduced pressure to remove the solvent. Ethyl acetate (5 mL) was added to the resulting residue and the mixture was stirred at room temperature for 10 min and purified by slurrying to give the intermediate **BB-32-1.** ¹H NMR (400 MHz, DMSO_*d*₆) δ: 10.90 (s, 1H), 7.96 (s, 1H), 7.86 (d, *J =* 2.0 Hz, 1H), 7.56 (d, *J* = 8.8 Hz, 1H), 7.45 (dd, *J* = 1.8, 8.6 Hz, 1H), 4.16 (dd, *J* = 4.8, 12.4 Hz, 1H), 2.79-2.66 (m, 1H), 2.63-2.53 (m, 1H), 2.42-2.29 (m, 1H), 2.15-2.03 (m, 1H).

### Step 2: Synthesis of intermediate BB-32

Intermediates **BB-32-1** (2.00 g, 6.49 mmol) and propenol (2.45 g, 42.18 mmol) were dissolved in 1,4-dioxane (30 mL) at room temperature. To the mixture were added separately tris(dibenzylideneacetone)dipalladium (594.38 mg, 649.08 µmol), N,N-dicyclohexylmethylamine (1.52 g, 7.79 mmol) and tri-tert-butylphosphine (10% in toluene) (2.63 g, 1.30 mmol). The reaction mixture was stirred to react at room temperature under nitrogen for 12 h. After the reaction was completed, water (100 mL) was added to the reaction solution and the resulting mixture was extracted with ethyl acetate (100 mL×3). The organic phases were sequentially combined, washed with saturated brine (100 mL×3), dried over anhydrous sodium sulfate, and filtered. The filtrate was concentrated under reduced pressure to remove the solvent. The resulting residue was separated by column chromatography (eluent: petroleum ether/ethyl acetate = 1/0-1/1, v/v) to give the intermediate **BB-32.** ¹H NMR (400 MHz, DMSO_*d*₆) δ: 10.89 (s, 1H), 9.72 (t, *J* = 1.4 Hz, 1H), 7.85 (s, 1H), 7.51-7.42 (m, 2H), 7.17 (dd, *J* = 1.6, 8.4 Hz, 1H), 4.11 (dd, *J* = 4.8, 12.4 Hz, 1H), 2.99-2.90 (m, 2H), 2.83-2.68 (m, 3H), 2.63-2.53 (m, 1H), 2.40-2.26 (m, 1H), 2.14-2.05 (m, 1H).

### Reference example 33

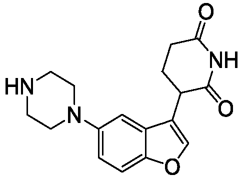

### Synthetic route:

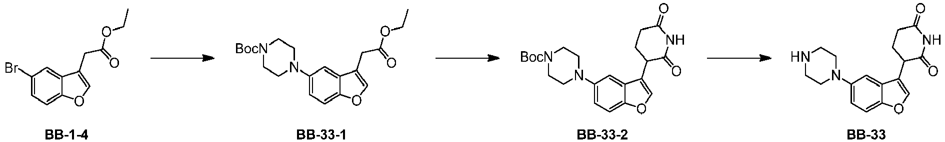

### Step 1: Synthesis of intermediate BB-33-1

Intermediate **BB-1-4** (3 g, 10.60 mmol) was dissolved in a mixture of toluene (70 mL) and water (14 mL) at room temperature under nitrogen, and tris(dibenzylideneacetone)dipalladium (485.16 mg, 529.81 µmol), 2-di-tert-butylphosphino-2',4',6'-triisopropylbiphenyl (449.96 mg, 1.06 mmol), potassium phosphate (6.75 g, 31.79 mmol) and 1-tert-butoxycarbonylpiperazine (3.95 g, 21.19 mmol) were then added sequentially. The reaction mixture was stirred to react at 100 °C under nitrogen for 12 h. After the reaction was completed, the mixture was cooled down to room temperature. Water (100 mL) was added to the reaction solution and the resulting mixture was extracted with ethyl acetate (50 mL×3). The organic phases were combined, washed with saturated brine (50 mL×3), dried over anhydrous sodium sulfate, and filtered. The solvent was removed from the filtrate under reduced pressure. The resulting residue was separated by column chromatography (eluent: petroleum ether/ethyl acetate = 40/1, v/v) to give the intermediate **BB-33-1.** ¹H NMR (400 MHz, CDCl₃) δ: 7.60 (s, 1H), 7.39 (d, *J* = 8.8 Hz, 1H), 7.08 (s, 1H), 7.01 (d, *J* = 8.4 Hz, 1H), 4.20 (q, *J* = 7.2 Hz, 2H), 3.66 (s, 2H), 3.63 (br s, 4H), 3.11 (br s, 4H), 1.50 (s, 9H), 1.28 (t, *J* = 7.2 Hz, 3H).

### Step 2: Synthesis of intermediate BB-33-2

Intermediate **BB-33-1** (1 g, 2.57 mmol) was dissolved in N,N-dimethylformamide (10 mL) at 0°C under nitrogen. Then potassium tert-butoxide (433.29 mg, 3.86 mmol) and acrylamide (219.57 mg, 3.09 mmol) were added sequentially and the reaction mixture was stirred to react at 0°C under nitrogen for 1 h. After the reaction was completed, water (100 mL) was added to the reaction solution and the resulting mixture was extracted with ethyl acetate (50 mL×3). The organic phases were combined, washed with saturated brine (50 mL×3), dried over anhydrous sodium sulfate, and filtered. The solvent was removed from the filtrate under reduced pressure. The resulting residue was separated by column chromatography (eluent: petroleum ether/ethyl acetate = 3/1-1/1, v/v) to give the intermediate **BB-33-2.** MS-ESI *m*/*z*: 413.9 [M+H]⁺.

### Step 3: Synthesis of intermediate BB-33 hydrochloride

Intermediate **BB-33-2** (1.04 mmol) was added to ethyl acetate (3 mL) at room temperature under nitrogen, and hydrochloric acid/ethyl acetate (25 mL, 100.00 mmol,) was then added. The reaction mixture was stirred to react at room temperature under nitrogen for 12 hours. After the reaction was completed, the reaction solution was directly concentrated under reduced pressure to remove the solvent to give the intermediate **BB-33** hydrochloride. MS-ESI *m*/*z:* 313.9 [M+H]⁺.

### Reference example 34

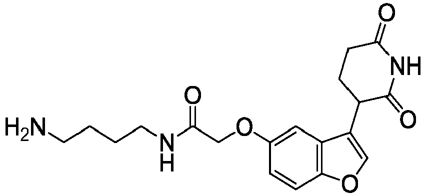

### Synthetic route:

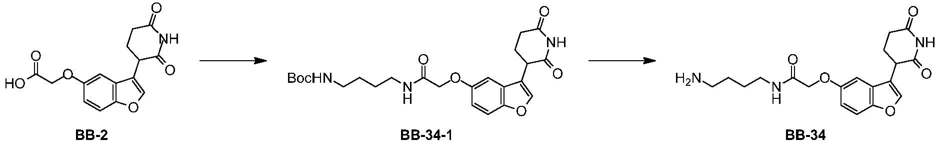

### Step 1: Synthesis of intermediate BB-34-1

Intermediate **BB-2** (500.00 mg, 1.65 mmol) was dissolved in N,N-dimethylformamide (20 mL) at room temperature. To the mixture were added separately N,N-diisopropylethylamine (1.07 g, 8.24 mmol) and O-(7-azabenzotriazol-1-yl)-N,N,N,N-tetramethyluronium hexafluorophosphate (940.34 mg, 2.47 mmol). The mixture solution was stirred for half an hour at room temperature. Subsequently, N-tert-butoxycarbonyl-1,4-butanediamine (310.40 mg, 1.65 mmol) was added and the reaction mixture was stirred to react at room temperature for 12 h. After the reaction was completed, water (40 mL) was added to the reaction solution and the resulting mixture was extracted with ethyl acetate (30 mL×3). The organic phases were combined, washed successively with saturated brine (40 mL×2), dried over anhydrous sodium sulfate, and filtered. The filtrate was concentrated under reduced pressure to remove the solvent. The resulting residue was separated by column chromatography (eluent: petroleum ether/ethyl acetate = 1/1-0/1, v/v) to give the intermediate **BB-34-1.** ¹H NMR (400 MHz, CDCl₃) δ: 7.55 (s, 1H), 7.41 (d, *J* = 8.8 Hz, 1H), 6.98 (d, *J* = 2.4 Hz, 1H), 6.95 (dd, *J* = 2.4, 8.8 Hz, 1H), 4.52 (s, 2H), 3.99-3.90 (m, 1H), 3.43-3.27 (m, 2H), 3.15-3.05 (m, 2H), 2.85-2.68 (m, 2H), 2.39-2.27 (m, 2H), 1.60-1.45 (m, 4H), 1.43 (s, 9H).

### Step 5: Synthesis of intermediate BB-34 hydrochloride

Intermediate **BB-34-1** (400.00 mg, 844.74 µmol) was dissolved in ethyl acetate (10 mL) at room temperature. To the mixture was added hydrochloric acid/ethyl acetate solution (4M, 15.64 mL). The reaction mixture was stirred to react at room temperature for 12 hours. After the reaction was completed, the mixture was filtered, and the filter cake was washed with ethyl acetate (10 mL). The filter cake was collected and dried to give the intermediate **BB-34** hydrochloride. ¹H NMR (400 MHz, DMSO_*d*₆) δ: 10.89 (s, 1H), 8.22 (t, *J* = 5.8 Hz, 1H), 7.98 (br s, 3H), 7.87 (s, 1H), 7.50 (d, *J* = 8.8 Hz, 1H), 7.17 (d, *J* = 2.4 Hz, 1H), 7.00 (dd, *J* = 2.8, 8.8 Hz, 1H), 4.47 (d, *J* = 3.2 Hz, 2H), 4.11 (dd, *J* = 4.8, 12.0 Hz, 1H), 3.19-3.12 (m, 2H), 2.80-2.71 (m, 3H), 2.62-2.53 (m, 1H), 2.41-2.28 (m, 1H), 2.13-2.04 (m, 1H), 1.59-1.45 (m, 4H).

### Example 1

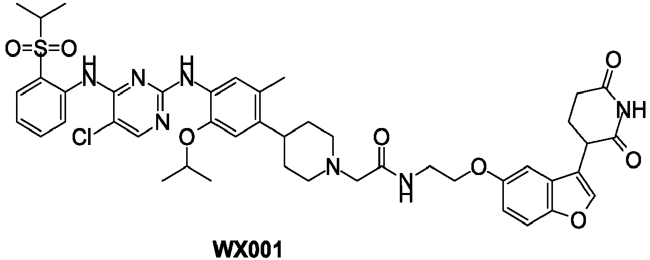

### Synthetic route:

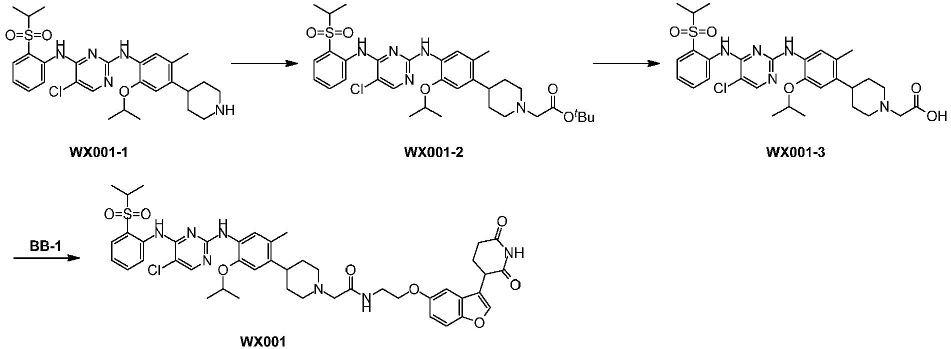

### Step 1: Synthesis of intermediate WX001-2

Compound **WX001-1** (1 g, 1.79 mmol) was dissolved in ethanol (15 mL) at room temperature, and tert-butyl bromoacetate (454.32 mg, 2.33 mmol, 344.18 µL) and N,N-diisopropyl ethylamine (463.13 mg, 3.58 mmol, 624.16 µL) were then added sequentially. The reaction mixture was heated to 50°C and stirred to react for 12 hours. After the reaction was completed, the mixture was cooled down to room temperature. The reaction solution was directly concentrated under reduced pressure to remove the solvent. The resulting residue was separated by column chromatography (eluent: petroleum ether/ethyl acetate = 511-111, v/v) to give the intermediate **WX001-2.** ¹H NMR (400MHz, CDCl₃) δ: 9.49 (s, 1H), 8.58 (d, *J* = 8.4 Hz, 1H), 8.146 (s, 1H), 7.99 (s, 1H), 7.93 (dd, *J* = 1.4, 8.2 Hz, 1H), 7.62 (td, *J* = 1.6, 8.6 Hz, 1H), 7.54 (s, 1H), 7.26 (t, *J* = 7.0 Hz, 1H), 6.82 (s, 1H), 4.58-4.46 (m, 1H), 3.32-3.22 (m, 1H), 3.18 (s, 2H), 3.14-3.05 (m, 2H), 2.74-2.61 (m, 1H), 2.32 (t, *J* = 11.8 Hz, 2H), 2.16 (s, 3H), 1.93-1.79 (m, 2H), 1.78-1.70 (m, 2H), 1.49 (s, 9H), 1.36 (d, *J* = 6.0 Hz, 6H), 1.31 (d, *J* = 6.4 Hz, 6H).

### Step 2: Synthesis of intermediate WX001-3

Intermediate **WX001-2** (0.5 g, 743.74 µmol) was dissolved in dichloromethane (5 mL) at room temperature, and trifluoroacetic acid (7.70 g, 67.53 mmol, 5 mL) was then added. The reaction mixture was stirred to react at room temperature for 2 hours. After the reaction was completed, the reaction solution was directly concentrated under reduced pressure to remove the solvent. The resulting residue was diluted with water (20 mL) and extracted with ethyl acetate (20 mL×4). The organic phases were combined, dried over anhydrous sodium sulfate, filtered, and concentrated under reduced pressure to remove the solvent to give the intermediate **WX001-3.** ¹H NMR (400MHz, CDCl₃) δ : 10.47 (s, 2H), 8.40 (d, *J* = 8.4 Hz, 1H), 8.01 (s, 1H), 7.93-7.89 (m, 1H), 7.47-7.30 (m, 3H), 6.90 (s, 1H), 4.62-4.47 (m, 1H), 4.02-3.80 (m, 4H), 3.24-3.17 (m, 1H), 3.17-2.97 (m, 3H), 2.44-2.25 (m, 2H), 2.18 (s, 3H), 2.02-1.91 (m, 2H), 1.31 (d, *J* = 6.8 Hz, 6H), 1.25 (d, *J* = 6.0 Hz, 6H).

### Step 3: Synthesis of WX001

Intermediate **WX001-3** (149.76 mg, 227.86 µmol) and intermediate **BB-1** (74 mg, 227.86 µmol, hydrochloride) were added to N,N-dimethylformamide (5 mL) at room temperature under nitrogen, and O-(7-azabenzotriazol-1-yl)-N,N,N,N-tetramethyluronium hexafluorophosphate (129.96 mg, 341.79 µmol) and triethylamine (69.17 mg, 683.58 µmol, 95.15 µL) were then added. The reaction mixture was stirred to react at room temperature under nitrogen for 14 h. After the reaction was completed, water (30 mL) was added to the reaction solution and the resulting mixture was extracted with ethyl acetate (30 mL×3). The organic phases were combined, washed with saturated brine (50 mL×2), dried over anhydrous sodium sulfate, and filtered. The solvent was removed from the filtrate under reduced pressure. The resulting residue was separated by preparative HPLC (mobile phase: acetonitrile/water; acidic system: 0.05% HCl) to give the title compound **WX001.** MS-ESI m/z: 886.3 [M+H]⁺. ¹H NMR (400 MHz, CD₃OD) δ: 8.25 (br s, 2H), 8.02 (d, *J* = 8.0 Hz, 1H), 7.79-7.69 (m, 2H), 7.56 (t, *J* = 8.0 Hz, 1H), 7.39 (d, *J* = 9.2 Hz, 1H), 7.34 (br s, 1H), 7.15 (d, *J* = 2.0 Hz, 1H), 7.00-6.92 (m, 2H), 4.71-4.63 (m, 1H), 4.20-4.09 (m, 3H), 3.99 (s, 2H), 3.74-3.61 (m, 4H), 3.43-3.37 (m, 1H), 3.27-3.21 (m, 1H), 3.16-3.07 (m, 1H), 2.88-2.69 (m, 2H), 2.50-2.38 (m, 1H), 2.31-2.23 (m, 1H), 2.19 (s, 3H), 2.14-2.05 (m, 2H), 2.02-1.88 (m, 3H), 1.31 (d, *J* = 6.0 Hz, 6H), 1.25 (d, *J* = 6.8 Hz, 6H).

### Example 2

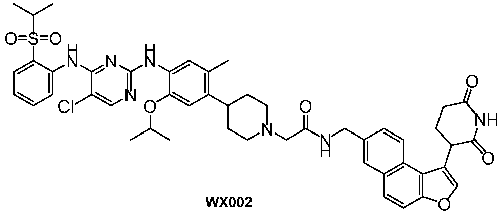

### Synthetic route:

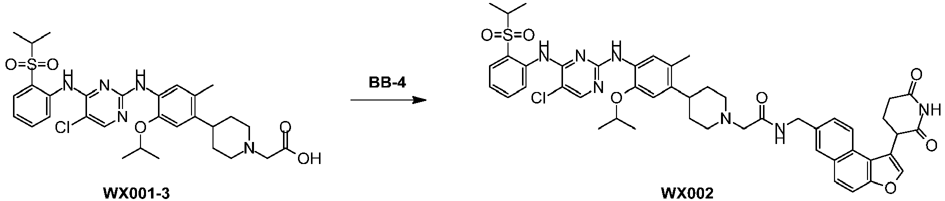

Intermediate **WX001-3** (160 mg, 259.67 µmol) was dissolved in N,N-dimethylformamide (5 mL) at 0°C under nitrogen. Subsequently, O-(7-azabenzotriazol-1-yl)-N,N,N,N-tetramethyluronium hexafluorophosphate (148.10 mg, 389.50 µmol) and triethylamine (78.83 mg, 779.01 µmol, 108.43 µL) were added sequentially. The reaction mixture was stirred at 0°C for 15 min, and then intermediate **BB-4** (98.48 mg, 285.64 µmol, hydrochloride) was added. The reaction mixture was allowed to warm up naturally to room temperature and stirred to react for another 12 h. After the reaction was completed, water (30 mL) was added to the reaction solution and the mixture was extracted with ethyl acetate (20 mL×3). The organic phases were combined, dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX002** hydrochloride. MS-ESI *m*/*z*: 906.3 [M+H]⁺. ¹H NMR (400MHz, DMSO_*d*₆) δ: 10.96 (s, 1H), 9.73 (s, 1H), 9.62 (s, 1H), 9.25 (t, *J* = 5.2 Hz, 1H), 8.37 (d, *J* = 8.8 Hz, 2H), 8.31 (s, 1H), 8.15 (d, *J* = 7.2 Hz, 1H), 8.02 (s, 1H), 7.96 (s, 1H), 7.86 (dd, *J* = 1.4, 7.8 Hz, 1H), 7.82 (d, *J* = 3.2 Hz, 2H), 7.64 (t, *J* = 7.4 Hz, 1H), 7.54 (dd, *J* = 1.4, 8.6 Hz, 1H), 7.48 (s, 1H), 7.40 (t, *J* = 7.6 Hz, 1H), 6.80 (s, 1H), 4.66 (dd, *J* = 4.2, 8.6, Hz, 1H), 4.57 (d, *J* = 5.6 Hz, 2H), 4.54-4.46 (m, 1H), 4.08 (d, *J* = 4.4 Hz, 2H), 3.58 (d, *J* = 12.8 Hz, 1H), 3.34-3.20 (m, 2H), 3.07-2.82 (m, 3H), 2.73-2.59 (m, 2H), 2.46-2.37 (m, 1H), 2.32-2.21 (m, 1H), 2.12 (s, 3H), 2.10-2.03 (m, 2H), 1.88 (d, *J* = 13.6 Hz, 2H), 1.25 (d, *J* = 6.0 Hz, 6H), 1.15 (d, *J* = 6.8 Hz, 6H).

### Example 3

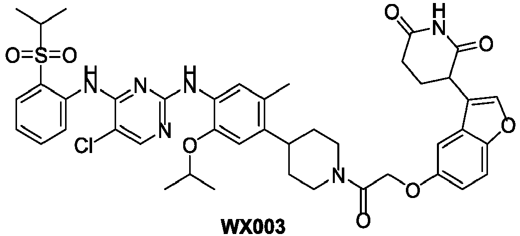

### Synthetic route:

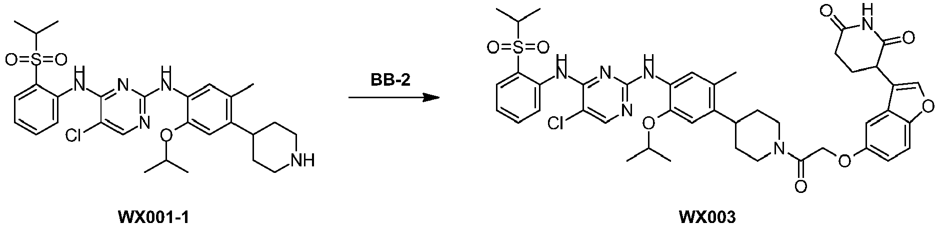

Intermediate **BB-2** (156.94 mg, 517.50 µmol) was dissolved in N,N-dimethylformamide (15 mL) at room temperature. Subsequently, triethylamine (157.10 mg, 1.55 mmol), O-(7-azabenzotriazol-1-yl)-N,N,N,N-tetramethyluronium hexafluorophosphate (295.15 mg, 776.25 µmol) and **WX001-1** (288.83 mg, 517.50 µmol) were added sequentially. The reaction mixture was stirred to react at room temperature for 2 hours. After the reaction was completed, the reaction solution was directly concentrated under reduced pressure to remove the solvent. Water (40 mL) was added to the resulting residue and the mixture was extracted with ethyl acetate (40 mL×3). The organic phases were sequentially combined, washed with saturated brine (100 mL), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX003.** MS-ESI *m*/*z*: 843.2 [M+H]⁺. ¹H NMR (400 MHz, DMSO_*d*₆) δ: 10.89 (s, 1H), 9.82 (s, 1H), 8.66 (s, 1H), 8.37 (s, 1H), 8.26 (d, *J =* 8.0 Hz, 1H), 7.89 (dd, *J* = 1.2, 8.0 Hz, 1H), 7.87 (s, 1H), 7.69 (t, *J* = 7.4 Hz, 1H), 7.48 (d, *J* = 9.2 Hz, 1H), 7.46 (t, *J* = 7.6 Hz, 1H), 7.39 (s, 1H), 7.15 (d, *J* = 2.8 Hz, 1H), 6.96 (dd, *J* = 2.6, 9.0 Hz, 1H), 6.78 (d, *J* = 3.2 Hz, 1H), 4.83 (s, 2H), 4.58-4.47 (m, 2H), 4.09 (dd, *J* = 4.8, 11.6 Hz, 1H), 4.02 (d, *J* = 13.2 Hz, 1H), 3.48-3.43 (m, 1H), 3.18 (t, *J* = 7.0 Hz, 1H), 2.92 (t, *J* = 11.2 Hz, 1H), 2.78-2.67 (m, 2H), 2.62-2.52 (m, 1H), 2.38-2.27 (m, 1H), 2.15-2.10 (m, 1H), 2.09 (s, 3H), 1.73-1.59 (m, 3H), 1.55-1.42 (m, 1H), 1.18 (d, *J* = 6.0 Hz, 6H), 1.14 (d, *J =* 6.8 Hz, 6H).

### Example 4

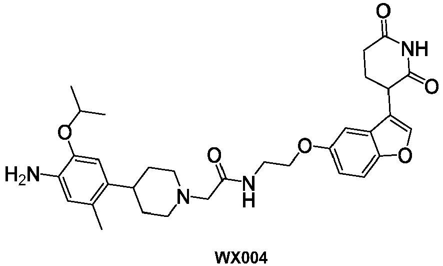

### Synthetic route:

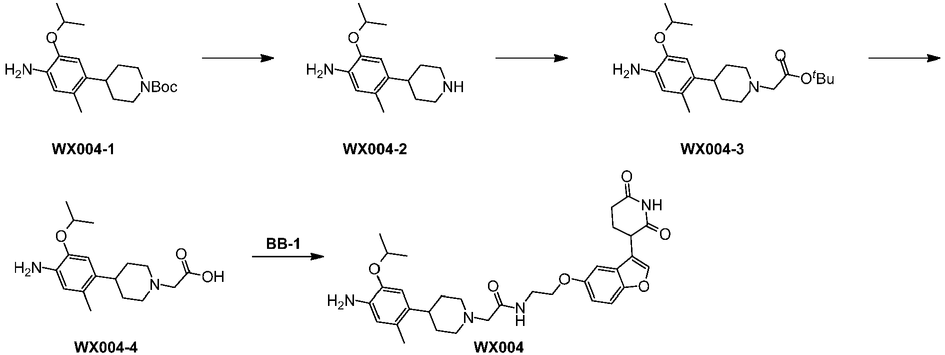

### Step 1: Synthesis of intermediate WX004-2

**WX004-1** (0.6 g, 1.72 mmol) was dissolved in ethyl acetate (20 mL) at room temperature. A solution of hydrochloric acid in ethyl acetate (4 M, 10 mL) was added, and the reaction solution was stirred to react at room temperature for 12 hours. After the reaction was completed, the reaction solution was directly concentrated under reduced pressure to remove the solvent. Saturated aqueous sodium bicarbonate solution was added to the resulting residue, and the mixture was extracted with dichloromethane (20 mL x 4). The organic phases were combined, washed with saturated brine (30 mL×3), dried over anhydrous sodium sulfate, and filtered. The filtrate was concentrated under reduced pressure to remove the solvent to give the intermediate **WX004-2.** MS-ESI *m*/*z*: 249.0 [M+H]⁺. ¹H NMR (400 MHz, CDCl₃) δ: 6.71 (s, 1H), 6.54 (s, 1H), 4.49-4.43 (m, 1H), 3.63 (br s, 1H), 3.25-3.22 (m, 2H), 2.81-2.71 (m, 3H), 2.21 (s, 3H), 1.78-1.74 (m, 2H), 1.71-1.59 (m, 2H), 1.34 (d, *J* = 6.0 Hz, 6H).

### Step 2: Synthesis of intermediate WX004-3

**WX004-2** (0.34 g, 1.36 mmol) was dissolved in N,N-dimethylformamide (10 mL) at 0°C, and potassium carbonate (188.54 mg, 1.36 mmol) and tert-butyl bromoacetate (266.09 mg, 1.36 mmol) were added sequentially. The reaction mixture was stirred to react at 0°C for 1 h. After the reaction was completed, the reaction solution was diluted with water (20 mL) and extracted with ethyl acetate (20 mL×3). The organic phases were combined, washed with saturated brine (30 mL×3), dried over anhydrous sodium sulfate, and filtered. The filtrate was concentrated under reduced pressure to remove the solvent. The resulting residue was separated by thin layer chromatography (developing solvent: petroleum ether/ethyl acetate = 2/1, v/v) to give the intermediate **WX004-3** (purity: 89.45%). MS-ESI *m*/*z:* 363.1 [M+H]⁺. ¹H NMR (400 MHz, CDCl₃) δ: 6.73 (s, 1H), 6.53 (s, 1H), 4.48-4.39 (m, 1H), 3.17 (s, 2H), 3.09-3.06 (m, 2H), 2.65-2.57 (m, 1H), 2.33-2.26 (m, 2H), 2.19 (s, 3H), 1.88-1.78 (m, 2H), 1.74-1.71 (m, 2H), 1.49 (s, 9H), 1.32 (d, *J =* 6.0 Hz, 6H).

### Step 3: Synthesis of intermediate WX004-4

Intermediate **WX004-3** (0.16 g, 394.81 µmol, purity: 89.45%) was dissolved in dichloromethane (10 mL) at room temperature under nitrogen, and trifluoroacetic acid (7.70 g, 67.53 mmol, 5 mL) was added. The reaction solution was stirred to react at room temperature for 12 hours. After the reaction was completed, the reaction solution was directly concentrated under reduced pressure to remove the solvent to give the intermediate **WX004-4** (crude).

### Step 4: Synthesis of WX004

Intermediate **BB-1** (0.08 g, 246.34 µmol, hydrochloride) and **WX004-4** (crude, 354.60 µmol) were dissolved in N,N-dimethylformamide (10 mL) at room temperature. Subsequently, triethylamine (124.63 mg, 1.23 mmol, 171.43 µL) and O-(7-azabenzotriazol-1-yl)-N,N,N,N-tetramethyluronium hexafluorophosphate (140.50 mg, 369.50 µmol) were added sequentially. The reaction solution was stirred to react at room temperature for 2 hours. After the reaction was completed, the reaction solution was diluted with water (30 mL) and extracted with ethyl acetate (20 mL×3). The organic phases were combined, washed with saturated brine (30 mL×3), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX004.** MS-ESI *m*/*z*: 577.2 [M+H]⁺. ¹H NMR (400 MHz, DMSO_*d*₆) δ: 10.88 (s, 1H), 8.89 (t, *J =* 5.4 Hz, 1H), 7.86 (s, 1H), 7.48 (d, *J =* 8.8 Hz, 1H), 7.12 (d, *J* = 2.4 Hz, 1H), 6.92 (dd, *J* = 2.6, 9.0 Hz, 1H), 6.85 (s, 1H), 6.77 (s, 1H), 4.60-4.54 (m, 1H), 4.13-4.04 (m, 3H), 3.97 (s, 2H), 3.59-3.55 (m, 2H), 3.53-3.50 (m, 2H), 3.19 (t, *J =* 11.6 Hz, 2H), 2.97-2.89 (m, 1H), 2.78-2.66 (m, 1H), 2.61-2.55 (m, 1H), 2.40-2.29 (m, 1H), 2.19 (s, 3H), 2.13-2.06 (m, 1H), 2.05-1.97 (m, 2H), 1.83-1.80 (m, 2H), 1.30 (d, *J* = 6.0 Hz, 6H).

### Example 5

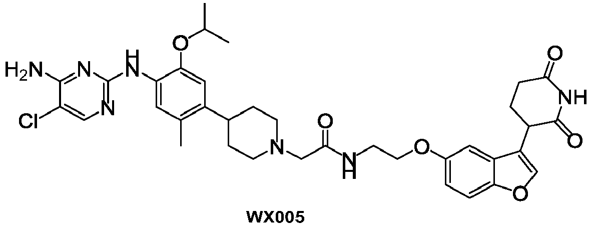

### Synthetic route:

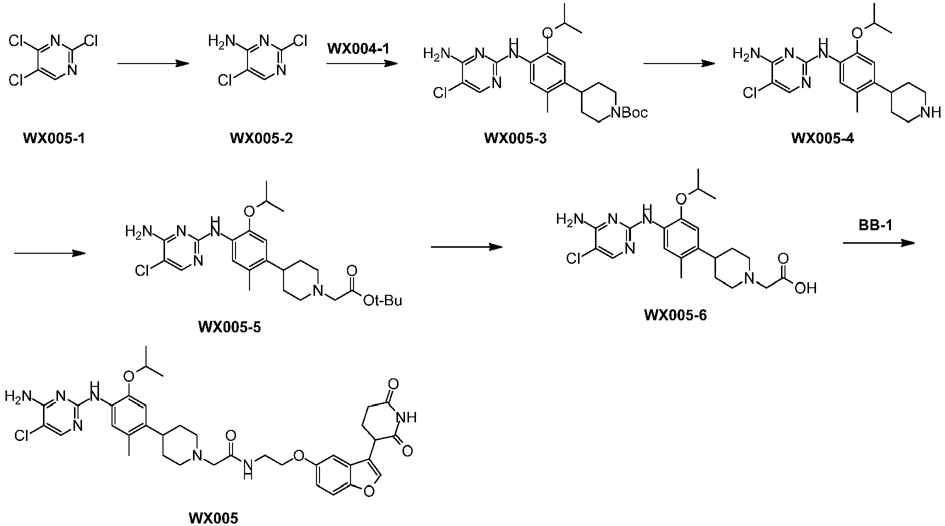

### Step 1: Synthesis of intermediate WX005-2

**WX005-1** (20 g, 109.04 mmol) was dissolved in a solution of ammonia in methanol (7 M, 100 mL) at room temperature. The reaction solution was stirred to react at room temperature for 12 hours. After the reaction was completed, the reaction solution was directly concentrated under reduced pressure to remove the solvent. The resulting residue was added to ethyl acetate (30 mL). The mixture was filtered, and the filter cake was washed with ethyl acetate (10 mL) and dried under vacuum to give the intermediate **WX005-2.** MS-ESI *m*/*z*: 163.7 [M+H]⁺. ¹H NMR (400 MHz, CDCl₃) δ: 8.15 (s, 1H), 5.70 (br s, 2H).

### Step 2: Synthesis of intermediate WX005-3

**WX005-2** (498.78 mg, 2.98 mmol) was dissolved in 1,4-dioxane (20 mL) at room temperature, and **WX004-1** (1.25 g, 3.58 mmol) and p-toluenesulfonic acid (769.85 mg, 4.47 mmol) were added. The reaction mixture was heated to 85°C and stirred to react for 12 hours. After the reaction was completed, the mixture was cooled down to room temperature. The mixture was diluted with water (40 mL) and extracted with ethyl acetate (20 mL×3). The organic phases were combined, washed with saturated brine (30 mL×3), dried over anhydrous sodium sulfate, and filtered. The filtrate was concentrated under reduced pressure to remove the solvent. The resulting residue was separated by column chromatography (eluent: petroleum ether/ethyl acetate = 1/0-5/1, v/v) to give the intermediate **WX005-3.** ¹H NMR (400 MHz, CDCl₃) δ: 8.15 (s, 1H), 8.01 (s, 1H), 7.35 (s, 1H), 6.70 (s, 1H), 5.57 (br s, 2H), 4.58-4.50 (m, 1H), 4.30-4.23 (m, 2H), 2.85-2.77 (m, 3H), 2.32 (s, 3H), 1.76-1.30 (m, 2H), 1.54-1.51 (m, 2H), 1.50 (s, 9H), 1.35 (d, *J* = 6.0 Hz, 6H).

### Step 3: Synthesis of intermediate WX005-4 hydrochloride

**WX005-3** (0.45 g, 945.36 µmol) was dissolved in ethyl acetate (15 mL) at room temperature and a solution of hydrochloric acid in ethyl acetate (4 M, 5 mL) was then added. The reaction mixture was stirred to react at room temperature for 12 hours. After the reaction was completed, the reaction solution was directly concentrated under reduced pressure to remove the solvent to give the intermediate WX005-4 hydrochloride. MS-ESI *m*/*z*: 376.1 [M+H]⁺.

### Step 4: Synthesis of intermediate WX005-5

**WX005-4** (0.4 g, 970.04 µmol, hydrochloride) was dissolved in N,N-dimethylformamide (20 mL) at 0°C and potassium carbonate (201.10 mg, 1.46 mmol) and tert-butyl bromoacetate (189.21 mg, 970.04 µmol) were added sequentially. The reaction mixture was stirred to react at 0°C for 1 h. After the reaction was completed, the reaction solution was diluted with water (30 mL) and extracted with ethyl acetate (20 mL×3). The organic phases were combined, washed with saturated brine (30 mL×3), dried over anhydrous sodium sulfate, and filtered. The filtrate was concentrated under reduced pressure to remove the solvent. The resulting residue was separated by column chromatography (eluent: petroleum ether/ethyl acetate = 1/0-5/1, v/v) to give the intermediate **WX005-5.** MS-ESI *m*/*z*: 490.2 [M+H]⁺. ¹H NMR (400 MHz, CDCl₃) δ: 8.12 (s, 1H), 8.00 (s, 1H), 7.35 (s, 1H), 6.80 (s, 1H), 5.13 (s, 2H), 4.54-4.48 (m, 1H), 3.18 (s, 2H), 3.10-3.07 (m, 2H), 2.71-2.65 (m, 1H), 2.34-2.29 (m, 5H), 1.90-1.80 (m, 2H), 1.76-1.73 (m, 2H), 1.49 (s, 9H), 1.34 (d, *J* = 6.0 Hz, 6H).

### Step 5: Synthesis of intermediate WX005-6 trifluoroacetate

Intermediate **WX005-5** (0.18 g, 358.17 µmol) was dissolved in dichloromethane (10 mL) at room temperature under nitrogen, and trifluoroacetic acid (7.70 g, 67.53 mmol, 5 mL) was added. The reaction mixture was stirred to react at room temperature for 12 hours. After the reaction was completed, the reaction solution was directly concentrated under reduced pressure to remove the solvent to give the intermediate **WX005-6** trifluoroacetate. MS-ESI *m*/*z*: 434.1 [M+H]⁺.

### Step 6: Synthesis of WX005

Intermediate **BB-1** (80 mg, 246.34 µmol, hydrochloride) and **WX005-6** (358.17 µmol, trifluoroacetate) were dissolved in N,N-dimethylformamide (10 mL) at room temperature. Triethylamine (124.63 mg, 1.23 mmol, 171.44 µL) and O-(7-azabenzotriazol-1-yl)-N,N,N,N-tetramethyluronium hexafluorophosphate (140.50 mg, 369.50 µmol) were added sequentially. The reaction mixture was stirred to react at room temperature for 12 hours. After the reaction was completed, the reaction solution was diluted with water (20 mL) and extracted with ethyl acetate (10 mL×3). The organic phases were combined, washed with saturated brine (20 mL×3), dried over anhydrous sodium sulfate, and filtered. The filtrate was concentrated under reduced pressure to remove the solvent. The resulting residue was separated by preparative HPLC (mobile phase: acetonitrile/water; neutral system: 0.05% NH₄HCO₃) to give the title compound **WX005.** MS-ESI *m*/*z*: 704.2 [M+H]⁺. ¹H NMR (400 MHz, DMSO_*d*₆) δ: 10.88 (s, 1H), 9.65 (br s, 1H), 8.90 (t, *J* = 5.2 Hz, 1H), 8.13 (s, 1H), 7.89-7.85 (m, 2H), 7.48 (d, *J* = 8.8 Hz, 1H), 7.12 (d, *J* = 2.4 Hz, 1H), 6.92 (dd, *J* = 2.4, 8.8 Hz, 1H), 6.80 (s, 1H), 4.60-4.53 (m, 1H), 4.13-4.05 (m, 3H), 3.98 (s, 2H), 3.60-3.50 (m, 4H), 3.25-3.15 (m, 2H), 3.00-2.93 (m, 1H), 2.79-2.65 (m, 1H), 2.62-2.55 (m, 1H), 2.40-2.32 (m, 1H), 2.27 (s, 3H), 2.15-2.08 (m, 1H), 2.06-1.96 (m, 2H), 1.85-1.80 (m, 2H), 1.28 (d, *J* = 6.0 Hz, 6H).

### Example 6

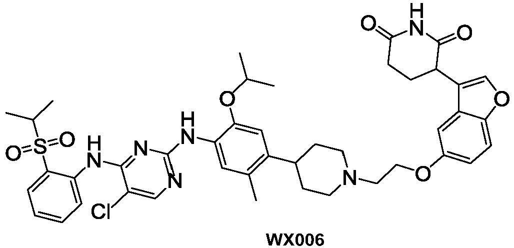

### Synthetic route:

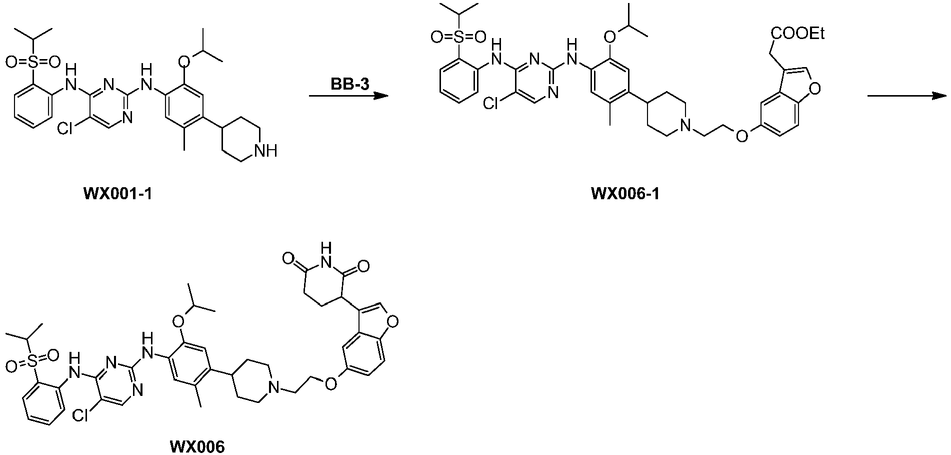

### Step 1: Synthesis of intermediate WX006-1

Intermediate **BB-3** (300 mg, 916.95 µmol) was dissolved in dimethylformamide (20 mL) at room temperature. Subsequently, potassium carbonate (190.10 mg, 1.38 mmol) and **WX001-1** (511.78 mg, 916.95 µmol) were added sequentially. The reaction mixture was heated to 80°C and stirred to react for 14 hours. After the reaction was completed, the mixture was cooled down to room temperature. Water (50 mL) was added to the reaction solution and the resulting mixture was extracted with ethyl acetate (50 mL×3). The organic phases were combined, washed with saturated brine (100 mL), dried over anhydrous sodium sulfate, and filtered. The filtrate was concentrated under reduced pressure to remove the solvent. The resulting residue was separated by column chromatography (eluent: petroleum ether/ethyl acetate = 1/0-2/1, v/v) to give the intermediate **WX006-1.** ¹H NMR (400 MHz, CDCl₃) δ: 9.22 (s, 1H), 8.31 (d, *J* = 8.4 Hz, 1H), 7.88 (s, 1H), 7.73 (s, 1H), 7.66 (dd, *J* = 1.6, 8.0 Hz, 1H), 7.38-7.35 (m, 1H), 7.33 (s, 1H), 7.09 (d, *J* = 8.8 Hz, 1H), 7.00-6.97 (m, 2H), 6.77 (d, *J* = 2.4 Hz, 1H), 6.67 (dd, *J* = 2.4, 8.8 Hz, 1H), 6.55 (s, 1H), 4.29-4.23 (m, 1H), 3.95-3.90 (m, 4H), 3.38 (d, *J* = 0.8 Hz, 2H), 3.03-2.96 (m, 1H), 2.93-2.90 (m, 2H), 2.65-2.61 (m, 2H), 2.47-2.38 (m, 1H), 2.05-1.97 (m, 2H), 1.89 (s, 3H), 1.56-1.53 (m, 4H), 1.08 (d, *J* = 6.0 Hz, 6H), 1.05 (d, *J* = 6.8 Hz, 6H), 1.01 (t, *J* = 7.2 Hz, 3H).

### Step 2: Synthesis of WX006

**WX006-1** (200.00 mg, 245.28 µmol) was dissolved in N,N-dimethylformamide (10 mL) at 0°C under nitrogen. Subsequently, potassium tert-butoxide (30.27 mg, 269.81 µmol) and acrylamide (17.73 mg, 245.28 µmol) were added sequentially. The reaction mixture was stirred to react at 0°C for 1 h. After the reaction was completed, water (40 mL) was added to the reaction solution and the resulting mixture was extracted with ethyl acetate (40 mL×3). The organic phases were combined, washed with saturated brine (100 mL), dried over anhydrous sodium sulfate, and filtered. The filtrate was concentrated under reduced pressure to remove the solvent. The resulting residue was separated by preparative HPLC (mobile phase: acetonitrile/water; neutral system: 10 mM NH₄HCO₃) to give the title compound **WX006.** MS-ESI *m*/*z*: 829.3 [M+H]⁺. ¹H NMR (400 MHz, DMSO_*d*₆) δ: 10.88 (s, 1H), 9.45 (s, 1H), 8.46 (d, *J* = 8.4 Hz, 1H), 8.24 (s, 1H), 8.05 (s, 1H), 7.85 (s, 1H), 7.84-7.81 (m, 1H), 7.62 (t, *J* = 7.8 Hz, 1H), 7.50 (s, 1H), 7.46 (d, *J* = 9.2 Hz, 1H), 7.38-7.32 (m, 1H), 7.12 (d, *J* = 2.4 Hz, 1H), 6.92 (dd, *J* = 2.8, 8.8 Hz, 1H), 6.85 (s, 1H), 4.60-4.54 (m, 1H), 4.13-4.09 (m, 3H), 3.47-3.40 (m, 1H), 3.10-3.07 (m, 2H), 2.77-2.52 (m, 6H), 2.37-2.32 (m, 1H), 2.23-2.15 (m, 2H), 2.12 (s, 3H), 1.71-1.64 (m, 4H), 1.21 (d, *J* = 6.0 Hz, 6H), 1.15 (d, *J* = 6.8 Hz, 6H).

### Example 7

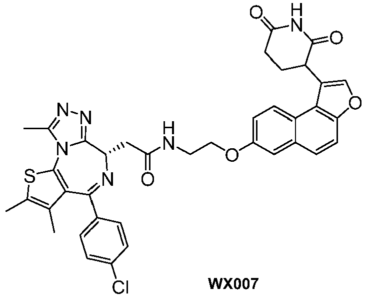

### Synthetic route:

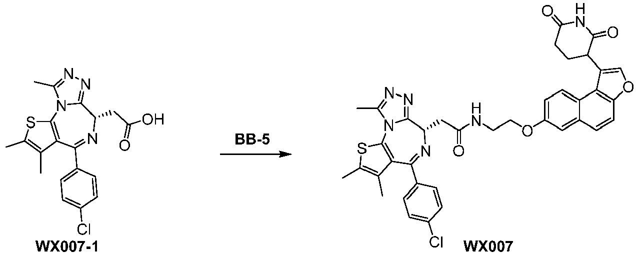

**WX007-1** (0.06 g, 149.67 µmol) was dissolved in N,N-dimethylformamide (5 mL) at room temperature. Subsequently, O-(7-azabenzotriazol-1-yl)-N,N,N,N-tetramethyluronium hexafluorophosphate (85.36 mg, 224.51 µmol) and N,N-diisopropylethylamine (77.38 mg, 598.68 µmol, 104.28 µL) were added sequentially. The reaction mixture was stirred at room temperature for 30 minutes, and then intermediate **BB-5** (61.71 mg, 164.64 µmol, hydrochloride) was added. The reaction mixture was stirred to react at room temperature for another 12 hours. After the reaction was completed, the reaction solution was diluted with water (30 mL) and extracted with ethyl acetate (20 mL×3). The organic phases were combined, washed with saturated brine (50 mL×3), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX007.** MS-ESI *m*/*z*: 721.0 [M+H]⁺. ¹H NMR (400 MHz, DMSO_*d*₆) δ: 10.93 (s, 1H), 8.60-8.55 (m, 1H), 8.15-8.05 (m, 1H), 7.97 (s, 1H), 7.77-7.72 (m, 2H), 7.56 (br s, 1H), 7.37-7.32 (m, 2H), 7.30-7.25 (m, 2H), 7.25-7.20 (m, 1H), 4.67-4.60 (m, 1H), 4.55-4.50 (m, 1H), 4.20-4.15 (m, 2H), 3.70-3.60 (m, 1H), 3.55-3.50 (m, 1H), 3.36-3.30 (m, 1H), 3.25-3.17 (m, 1H), 2.91-2.81 (m, 1H), 2.68-2.61 (m, 1H), 2.59 (s, 3H), 2.40 (s, 3H), 2.35-2.30 (m, 1H), 2.30-2.20 (m, 1H), 1.59 (d, *J =* 5.6 Hz, 3H).

### Example 8

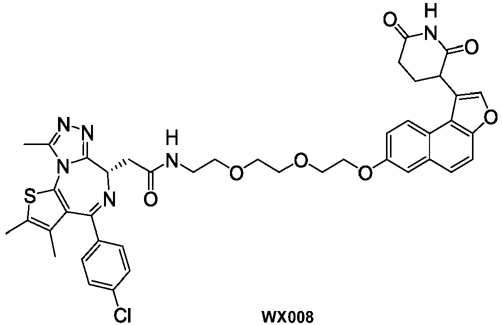

### Synthetic route:

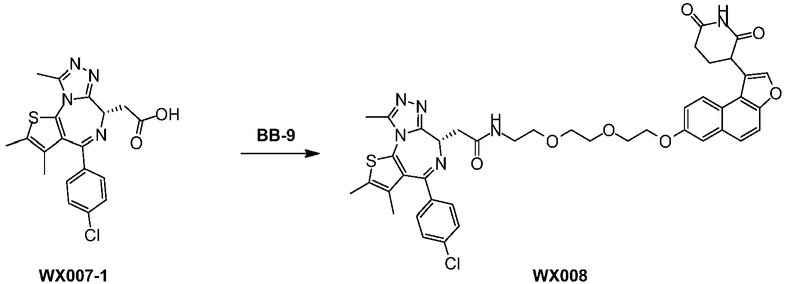

**WX007-1** (0.05 g, 124.73 µmol) was dissolved in N,N-dimethylformamide (5 mL) at room temperature. Subsequently, O-(7-azabenzotriazol-1-yl)-N,N,N,N-tetramethyluronium hexafluorophosphate (71.14 mg, 187.09 µmol) and N,N-diisopropylethylamine (64.48 mg, 498.90 µmol, 86.90 µL) were added sequentially. The reaction mixture was stirred at room temperature for 30 minutes, and then intermediate **BB-9** (68.97 mg, 137.20 µmol, hydrochloride) was added. The reaction mixture was stirred to react at room temperature for another 12 hours. After the reaction was completed, the reaction solution was diluted with water (30 mL) and extracted with ethyl acetate (20 mL×3). The organic phases were combined, washed with saturated brine (50 mL×3), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX008.** MS-ESI *m*/*z*: 809.2 [M+H]⁺. ¹H NMR (400 MHz, DMSO_*d*₆) δ: 10.93 (s, 1H), 8.30 (t, *J* = 5.4 Hz, 1H), 8.08 (br d, *J* = 9.2 Hz, 1H), 7.96 (s, 1H), 7.75-7.70 (m, 2H), 7.51 (d, *J* = 2.4 Hz, 1H), 7.49-7.46 (m, 2H), 7.44-7.39 (m, 2H), 7.23 (dd, *J* = 2.8, 9.2 Hz, 1H), 4.63 (br dd, *J* = 4.2, 11.8 Hz, 1H), 4.55-4.50 (m, 1H), 4.23 (t, *J* = 4.6 Hz, 2H), 3.83 (t, *J* = 4.4 Hz, 2H), 3.67-3.63 (m, 2H), 3.62-3.58 (m, 2H), 3.49 (t, *J* = 5.8 Hz, 2H), 3.36-3.16 (m, 4H), 2.92-2.82 (m, 1H), 2.69-2.61 (m, 1H), 2.60 (s, 3H), 2.46-2.40 (m, 1H), 2.39 (s, 3H), 2.30-2.21 (m, 1H), 1.60 (s, 3H).

### Example 9

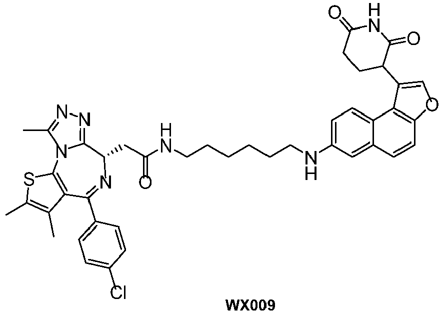

### Synthetic route:

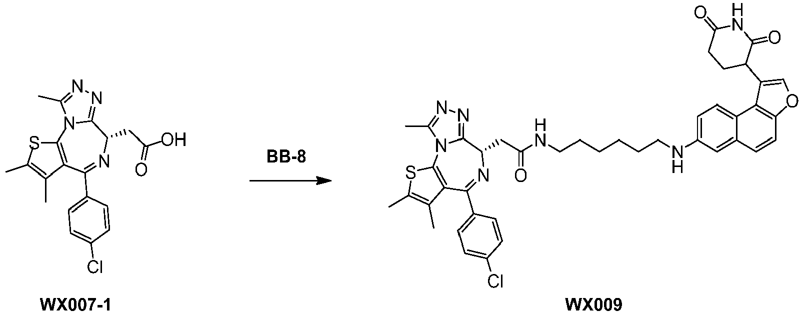

**WX007-1** (0.05 g, 124.73 µmol) was dissolved in N,N-dimethylformamide (5 mL) at room temperature. Subsequently, O-(7-azabenzotriazol-1-yl)-N,N,N,N-tetramethyluronium hexafluorophosphate (71.14 mg, 187.09 µmol) and N,N-diisopropylethylamine (64.48 mg, 498.90 µmol, 86.90 µL) were added sequentially. The reaction mixture was stirred at room temperature for 30 minutes, and then intermediate **BB-8** (58.99 mg, 137.20 µmol, hydrochloride) was added. The reaction mixture was stirred to react at room temperature for another 12 hours. After the reaction was completed, the reaction solution was diluted with water (30 mL) and extracted with ethyl acetate (20 mL×3). The organic phases were combined, washed with saturated brine (50 mL×3), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX009.** MS-ESI *m*/*z:* 776.2 [M+H]⁺. ¹H NMR (400 MHz, DMSO_*d*₆) δ: 10.95 (s, 1H), 8.29 (br d, *J* = 8.8 Hz, 1H), 8.22 (br t, *J* = 5.6 Hz, 1H), 8.09 (br s, 1H), 8.06 (s, 1H), 7.91-7.83 (m, 2H), 7.72-7.65 (m, 1H), 7.53-7.48 (m, 2H), 7.44-7.39 (m, 2H), 4.69 (br dd, *J* = 4.4, 12.0 Hz, 1H), 4.55 (t, *J* = 7.2 Hz, 1H), 3.39-3.32 (m, 2H), 3.30-3.17 (m, 2H), 3.14-3.07 (m, 2H), 2.93-2.82 (m, 1H), 2.69-2.64 (m, 1H), 2.62 (s, 3H), 2.46-2.40 (m, 1H), 2.39 (s, 3H), 2.31-2.22 (m, 1H), 1.76-1.67 (m, 2H), 1.58 (d, *J* = 3.6 Hz, 3H), 1.49-1.30 (m, 6H).

### Example 10

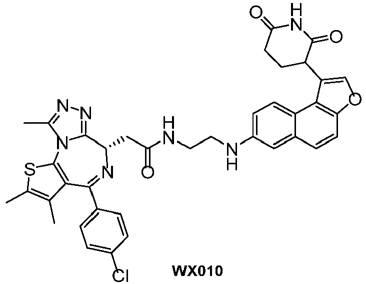

### Synthetic route:

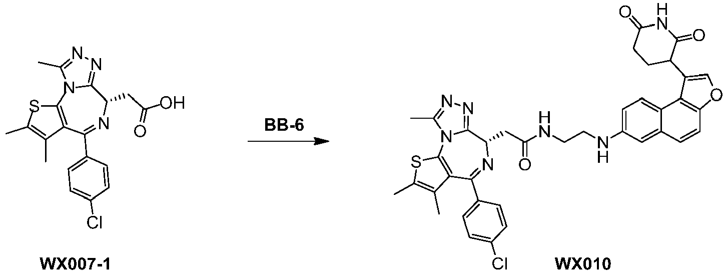

**WX007-1** (0.05 g, 124.73 µmol) was dissolved in N,N-dimethylformamide (5 mL) at room temperature. Subsequently, O-(7-azabenzotriazol-1-yl)-N,N,N,N-tetramethyluronium hexafluorophosphate (71.14 mg, 187.09 µmol) and N,N-diisopropylethylamine (64.48 mg, 498.92 µmol, 86.90 µL) were added sequentially. The reaction mixture was stirred at room temperature for 30 minutes, and then intermediate **BB-6** (51.29 mg, 137.20 µmol, hydrochloride) was added. The reaction mixture was stirred to react at room temperature for another 12 hours. After the reaction was completed, the reaction solution was diluted with water (30 mL) and extracted with ethyl acetate (20 mL×3). The organic phases were combined, washed with saturated brine (50 mL×3), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX010.** MS-ESI *m*/*z:* 720.2 [M+H]⁺. ¹H NMR (400 MHz, DMSO_*d*₆) δ: 10.95 (s, 1H), 8.65 (t, *J* = 4.8 Hz, 1H), 8.21 (br d, *J* = 8.4 Hz, 1H), 8.02 (s, 1H), 7.90 (s, 1H), 7.84-7.79 (m, 2H), 7.58 (br d, *J* = 9.2 Hz, 1H), 7.49-7.39 (m, 4H), 7.37-7.29 (m, 1H), 4.69-4.59 (m, 2H), 3.56-3.46 (m, 4H), 3.37-3.30 (m, 2H), 2.94-2.79 (m, 1H), 2.67 (s, 3H), 2.65-2.58 (m, 1H), 2.42 (s, 3H), 2.40-2.31 (m, 1H), 2.31-2.21 (m, 1H), 1.61 (s, 3H).

### Example 11

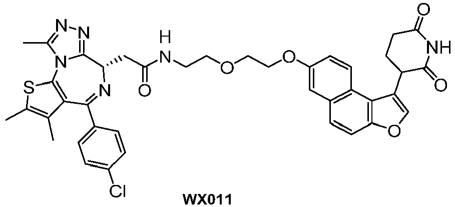

### Synthetic route:

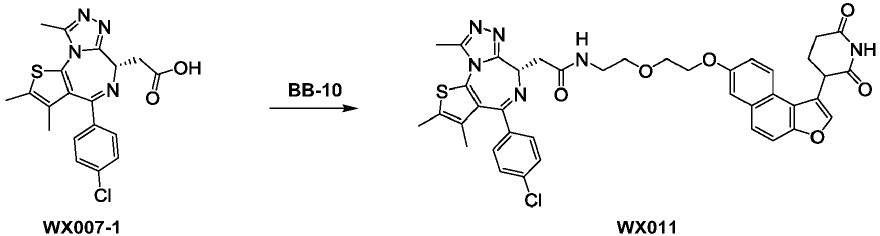

**WX007-1** (0.06 g, 149.67 µmol) was dissolved in N,N-dimethylformamide (5 mL) at room temperature. Subsequently, O-(7-azabenzotriazol-1-yl)-N,N,N,N-tetramethyluronium hexafluorophosphate (85.36 mg, 224.51 µmol) and N,N-diisopropylethylamine (77.37 mg, 598.68 µmol, 104.28 µL) were added sequentially. The reaction mixture was stirred at room temperature for 30 minutes, and then intermediate **BB-10** (62.96 mg, 164.64 µmol, hydrochloride) was added. The reaction mixture was stirred to react at room temperature for another 12 hours. After the reaction was completed, the reaction solution was diluted with water (30 mL) and extracted with ethyl acetate (20 mL×3). The organic phases were combined, washed with saturated brine (50 mL×3), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX011.** MS-ESI *m*/*z*: 765.3 [M+H]⁺. ¹H NMR (400 MHz, DMSO_*d*₆) δ: 10.92 (s, 1H), 8.36 (brt, J= 5.4 Hz, 1H), 8.08 (br d, J= 9.2 Hz, 1H), 7.96 (s, 1H), 7.77-7.69 (m, 2H), 7.52 (d, *J* = 2.4 Hz, 1H), 7.50-7.46 (m, 2H), 7.45-7.39 (m, 2H), 7.24 (dd, *J* = 2.6, 9.0 Hz, 1H), 4.63 (br dd, *J* = 4.4, 12.0 Hz, 1H), 4.60-4.54 (m, 1H), 4.25 (t, *J* = 4.6 Hz, 2H), 3.85 (t, *J* = 4,4 Hz, 2H), 3.57 (t, *J* = 5.8 Hz, 2H), 3.42-3.17 (m, 4H), 2.94-2.81 (m, 1H), 2.64 (s, 3H), 2.68-2.54 (m, 1H), 2.40 (s, 3H), 2.39-2.30 (m, 1H), 2.30-2.21 (m, 1H), 1.60 (s, 3H).

### Example 12

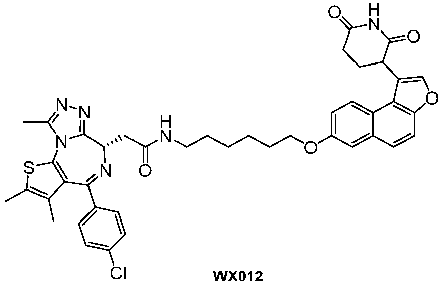

### Synthetic route:

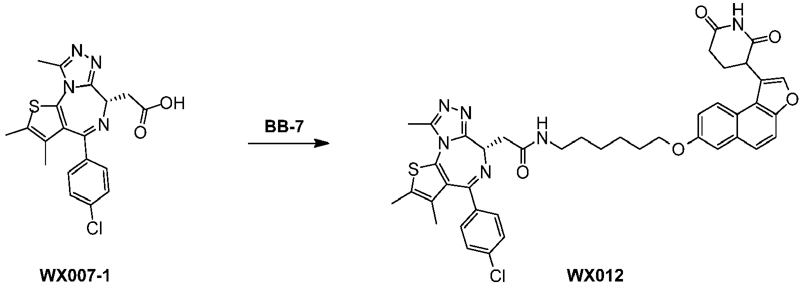

**WX007-1** (0.06 g, 149.67 µmol) was dissolved in N,N-dimethylformamide (5 mL) at room temperature. Subsequently, O-(7-azabenzotriazol-1-yl)-N,N,N,N-tetramethyluronium hexafluorophosphate (85.36 mg, 224.51 µmol) and N,N-diisopropylethylamine (77.37 mg, 598.68 µmol, 104.28 µL) were added sequentially. The reaction mixture was stirred at room temperature for 30 minutes, and then intermediate **BB-7** (64.94 mg, 164.64 µmol, hydrochloride) was added. The reaction mixture was stirred to react at room temperature for another 12 hours. After the reaction was completed, the reaction solution was diluted with water (30 mL) and extracted with ethyl acetate (20 mL×3). The organic phases were combined, washed with saturated brine (50 mL×3), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX012.** MS-ESI *m*/*z*: 777.3 [M+H]⁺. ¹H NMR (400 MHz, DMSO_*d*₆) δ: 10.93 (s, 1H), 8.22 (brt, J= 5.6 Hz, 1H), 8.07 (br d, *J* = 8.8 Hz, 1H), 7.95 (s, 1H), 7.76-7.69 (m, 2H), 7.53-7.47 (m, 3H), 7.46-7.40 (m, 2H), 7.21 (dd, *J* = 2.4, 9.2 Hz, 1H), 4.63 (br dd, *J* = 4.6, 11.8 Hz, 1H), 4.60-4.53 (m, 1H), 4.09 (br t, *J* = 6.4 Hz, 2H), 3.32-3.05 (m, 4H), 2.93-2.82 (m, 1H), 2.68-2.57 (m, 1H), 2.62 (s, 3H), 2.46-2.40 (m, 1H), 2.39 (s, 3H), 2.30-2.20 (m, 1H), 1.83-1.72 (m, 2H), 1.60 (s, 3H), 1.55-1.44 (m, 4H), 1.44-1.36 (m, 2H).

### Example 13

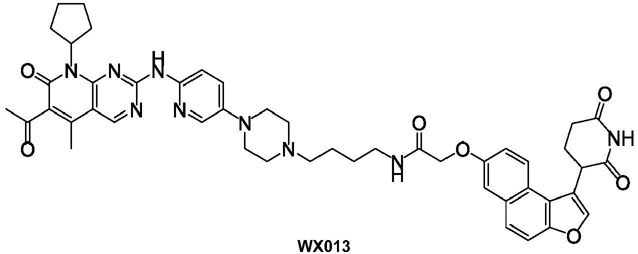

### Synthetic route:

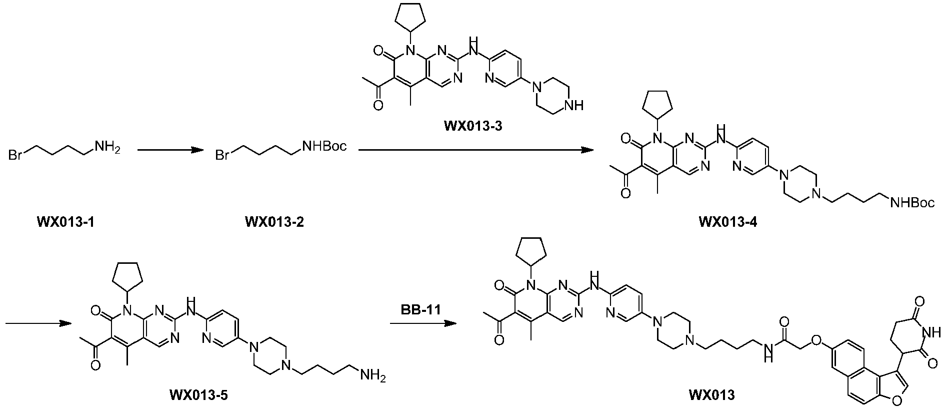

### Step 1: Synthesis of intermediate WX013-2

Intermediate **WX013-1** (1 g, 6.58 mmol, hydrobromide) was dissolved in dichloromethane (20 mL) at room temperature under nitrogen. Subsequently, triethylamine (1.33 g, 13.16 mmol, 1.83 mL) and di-tert-butyl dicarbonate (1.58 g, 7.24 mmol, 1.66 mL) were added sequentially. The reaction mixture was stirred to react at room temperature for 2 hours. After the reaction was completed, dichloromethane (20 mL) was added to the reaction solution and the mixture was washed with dilute hydrochloric acid solution (2 M, 50 mL×3). The organic phase was dried over anhydrous sodium sulfate, and filtered. The filtrate was concentrated under reduced pressure to remove the solvent. The resulting residue was separated by column chromatography (eluent: petroleum ether/ethyl acetate = 20/1-2/1, v/v) to give the intermediate **WX013-2.** ¹H NMR (400 MHz, CDCl₃) δ: 4.53 (br s, 1H), 3.43 (t, *J* = 6.8 Hz, 2H), 3.20-3.10 (m, 2H), 1.95-1.86 (m, 2H), 1.70-1.60 (m, 2H), 1.45 (s, 9H).

### Step 2: Synthesis of intermediate WX013-4

**WX013-3** (0.05 g, 111.72 µmol) was dissolved in N,N-dimethylformamide (2 mL) at room temperature. Subsequently, intermediate **WX013-2** (28.17 mg, 111.72 µmol) and sodium bicarbonate (18.77 mg, 223.45 µmol) were added sequentially. The reaction mixture was stirred to react at room temperature for 12 hours. Potassium iodide (9.27 mg, 55.86 µmol) was then added and the reaction mixture was heated to 80 °C to react with stirring for another 6 hours. After the reaction was completed, the mixture was cooled down to room temperature. The reaction solution was diluted with water (30 mL) and extracted with ethyl acetate (20 mL×3). The organic phases were combined, washed with saturated brine (50 mL×3), dried over anhydrous sodium sulfate, and filtered. The filtrate was concentrated under reduced pressure to remove the solvent. The resulting residue was separated by thin layer chromatography (developing agent: dichloromethane/methanol = 10/1, v/v) to give the intermediate **WX013-4.** ¹H NMR (400 MHz, CDCl₃) δ: 8.81 (s, 1H), 8.16 (br d, *J* = 8.8 Hz, 1H), 8.07 (br s, 1H), 8.04 (br d, *J* = 2.4 Hz, 1H), 7.34 (dd, *J* = 2.4, 8.8 Hz, 1H), 5.94-5.82 (m, 1H), 5.20 (br s, 1H), 3.24-3.20 (m, 4H), 3.20-3.12 (m, 2H), 2.70-2.60 (m, 4H), 2.56 (s, 3H), 2.48-2.41 (m, 2H), 2.38 (s, 3H), 2.14-2.00 (m, 2H), 1.95-1.83 (m, 2H), 1.75-1.65 (m, 4H), 1.62-1.55 (m, 4H), 1.44 (s, 9H).

### Step 3: Synthesis of intermediate WX013-5 hydrochloride

Intermediate **WX013-4** (0.055 g, 88.89 µmol) was dissolved in ethyl acetate (5 mL) at room temperature, and a solution of hydrochloric acid in ethyl acetate (4 M, 3 mL) was then added. The reaction mixture was stirred to react at room temperature for 2 hours. After the reaction was completed, the reaction solution was directly concentrated under reduced pressure to remove the solvent to give the intermediate **WX013-5** hydrochloride. MS-ESI *m*/*z*: 519.1 [M+H]⁺.

### Step 4: Synthesis of WX013

Intermediate **BB-11** (100.9 µmol) was dissolved in N,N-dimethylformamide (5 mL) at room temperature, and O-(7-azabenzotriazol-1-yl)-N,N,N,N-tetramethyluronium hexafluorophosphate (57.54 mg, 151.35 µmol) and N,N-diisopropylethylamine (52.15 mg, 403.60 µmol) were added. The reaction mixture was stirred at room temperature for 30 minutes, and then **WX013-5** (88.89 µmol, hydrochloride) was added. The reaction mixture was stirred to react at room temperature for another 12 hours. After the reaction was completed, the reaction solution was diluted with water (30 mL) and extracted with ethyl acetate (20 mL×3). The organic phases were combined, washed with saturated brine (50 mL×3), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX013.** MS-ESI *m*/*z:* 854.1 [M+H]⁺. ¹H NMR (400 MHz, DMSO_*d*₆) δ: 11.42 (br s, 1H), 10.94 (s, 1H), 9.01 (s, 1H), 8.38 (t, *J* = 5.2 Hz, 1H), 8.18-8.10 (m, 1H), 8.07 (s, 1H), 7.97 (s, 1H), 7.93-7.87 (m, 1H), 7.85-7.77 (m, 1H), 7.77-7.71 (m, 2H), 7.51 (s, 1H), 7.36-7.30 (m, 1H), 5.90-5.78 (m, 1H), 4.71-4.66 (m, 1H), 4.64 (s, 2H), 3.80-3.72 (m, 1H), 3.28-3.17 (m, 5H), 3.12-2.99 (m, 4H), 2.95-2.83 (m, 1H), 2.70-2.56 (m, 2H), 2.43 (s, 3H), 2.33 (s, 3H), 2.30-2.15 (m, 4H), 2.00-1.87 (m, 2H), 1.85-1.68 (m, 5H), 1.63-1.56 (m, 2H), 1.55-1.47 (m, 2H).

### Example 14

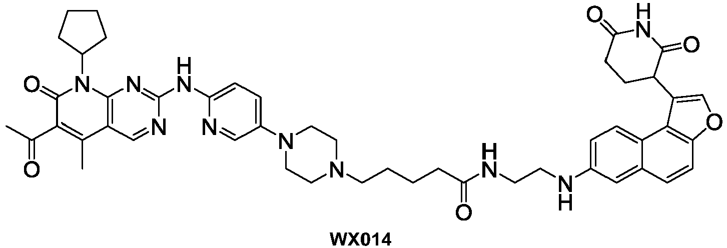

### Synthetic route:

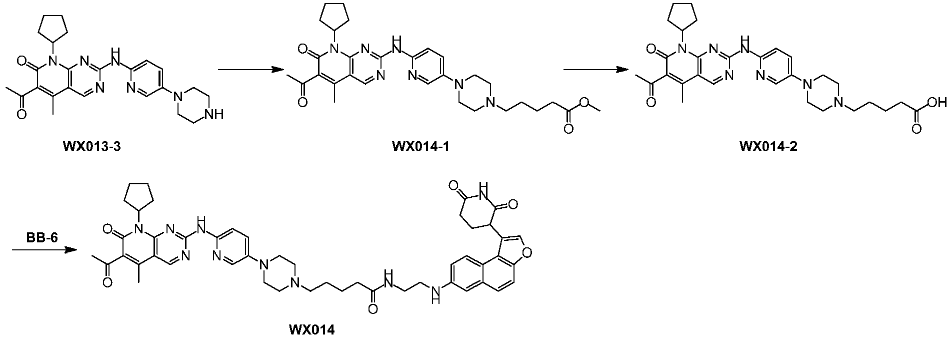

### Step 1: Synthesis of intermediate WX014-1

**WX013-3** (0.1 g, 223.45 µmol) was dissolved in N,N-dimethylformamide (10 mL) at room temperature. Subsequently, methyl 5-bromovalerate (65.38 mg, 335.17 µmol), sodium bicarbonate (37.54 mg, 446.90 µmol) and potassium iodide (18.55 mg, 111.73 µmol) were added sequentially. The reaction mixture was heated to 80°C and stirred to react for 12 hours. After the reaction was completed, the mixture was cooled down to room temperature. The reaction solution was diluted with water (50 mL) and extracted with ethyl acetate (30 mL×3). The organic phases were combined, washed with saturated brine (50 mL×3), dried over anhydrous sodium sulfate, and filtered. The filtrate was concentrated under reduced pressure to remove the solvent. The resulting residue was separated by thin layer chromatography (developing agent: dichloromethane/methanol = 10/1, v/v) to give the intermediate **WX014-1.** MS-ESI *m*/*z:* 562.2 [M+H]⁺. ¹H NMR (400 MHz, CDCl₃) δ: 8.82 (s, 1H), 8.23-8.11 (m, 2H), 8.06 (br d, *J* = 2.8 Hz, 1H), 7.33 (dd, *J* = 3.0, 9.4 Hz, 1H), 5.95-5.82 (m, 1H), 3.69 (s, 3H), 3.26-3.16 (m, 4H), 2.68-2.58 (m, 4H), 2.56 (s, 3H), 2.47-2.40 (m, 2H), 2.40-2.31 (m, 7H), 2.15-2.00 (m, 2H), 1.96-1.83 (m, 2H), 1.75-1.66 (m, 4H), 1.62-1.55 (m, 2H).

### Step 2: Synthesis of intermediate WX014-2

Intermediate **WX014-1** (0.104 g, 183.90 µmol) was dissolved in methanol (10 mL) at room temperature. Subsequently, a solution of sodium hydroxide (22.07 mg, 551.71 µmol) in water (1 mL) was added. The reaction mixture was stirred to react at room temperature for 12 hours. Then a solution of sodium hydroxide (73.56 mg, 1.84 mmol) in water (3 mL) was added to the above reaction mixture and the reaction mixture was stirred to react at room temperature for another 4 h. After the reaction was completed, the reaction solution was directly concentrated under reduced pressure to remove the solvent. The resulting residue was added to water (20 mL) and the pH was adjusted to 5-6 with 2 N dilute hydrochloric acid. The solvent was removed by concentration under reduced pressure to give the intermediate **WX014-2** (crude). MS-ESI *m*/*z:* 548.1 [M+H]⁺.

### Step 3: Synthesis of WX014

**WX014-2** (183.90 µmol, crude) was dissolved in N,N-dimethylformamide (5 mL) at room temperature. Subsequently, O-(7-azabenzotriazol-1-yl)-N,N,N,N-tetramethyluronium hexafluorophosphate (104.89 mg, 275.85 µmol) and N,N-diisopropylethylamine (95.07 mg, 735.60 µmol) were added sequentially. The reaction mixture was stirred at room temperature for 30 min, and intermediate BB-6 (68.75 mg, 183.91 µmol, hydrochloride) was then added. The reaction mixture was stirred to react at room temperature for another 2 h. After the reaction was completed, the reaction solution was diluted with water (30 mL) and extracted with ethyl acetate (20 mL×3). The organic phases were combined, washed with saturated brine (50 mL×3), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX014.** MS-ESI *m*/*z:* 867.3 [M+H]⁺. ¹H NMR (400 MHz, DMSO_*d*₆) δ: 11.22 (br s, 1H), 11.03 (br s, 1H), 10.94 (s, 1H), 9.02 (s, 1H), 8.26 (t, *J* = 4.6 Hz, 1H), 8.10-8.00 (m, 2H), 7.93 (s, 1H), 7.92-7.88 (m, 1H), 7.81 (d, *J* = 9.2 Hz, 1H), 7.72-7.62 (m, 2H), 7.50-7.37 (m, 1H), 7.34-7.26 (m, 1H), 5.91-5.79 (m, 1H), 4.62 (br dd, *J =* 4.0, 12.0 Hz, 1H), 3.58-3.52 (m, 2H), 3.42-3.35 (m, 3H), 3.35-3.29 (m, 2H), 3.28-3.18 (m, 3H), 3.16-3.06 (m, 4H), 2.93-2.79 (m, 1H), 2.70-2.57 (m, 1H), 2.44 (s, 3H), 2.42-2.37 (m, 1H), 2.35 (s, 3H), 2.29-2.15 (m, 5H), 2.00-1.90 (m, 2H), 1.86-1.67 (m, 4H), 1.65-1.50 (m, 4H).

### Example 15

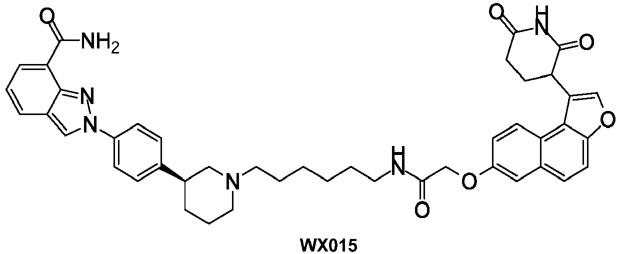

### Synthetic route:

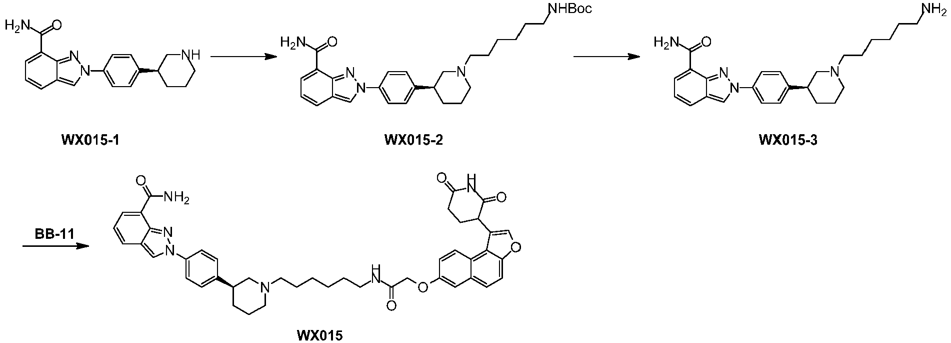

### Step 1: Synthesis of intermediate WX015-2

**WX015-1** (300.00 mg, 936.37 µmol) was dissolved in dimethylformamide (15 mL) at room temperature. Subsequently, N-Boc-6-bromohexylamine (314.84 mg, 1.12 mmol) and sodium bicarbonate (235.99 mg, 2.81 mmol) were added sequentially. The reaction mixture was heated to 40°C and stirred to react for 12 hours. After the reaction was completed, the mixture was cooled down to room temperature, and concentrated under reduced pressure to remove the solvent. Water (50 mL) was added to the resulting residue and the mixture was extracted with ethyl acetate (50 mL×3). The organic phases were combined, washed with saturated brine (100 mL), dried over anhydrous sodium sulfate, and filtered. The filtrate was concentrated under reduced pressure to remove the solvent. The resulting residue was separated by column chromatography (eluent: dichloromethane/methanol = 1/0-20/1, v/v) to give the intermediate **WX015-2.** ¹H NMR (400 MHz, CDCl₃) δ: 9.05 (s, 1H), 8.51 (s, 1H), 8.31 (d, *J* = 6.8 Hz, 1H), 7.91 (d, J = 8.4 Hz, 1H), 7.83 (d, *J* = 8.4 Hz, 2H), 7.43 (d, *J* = 8.4 Hz, 2H), 7.29-7.25 (m, 2H), 6.02 (s, 1H), 3.11-2.96 (m, 5H), 2.56-2.41 (m, 2H), 2.12-2.08 (m, 2H), 2.05-2.01 (m, 2H), 1.94-1.87 (m, 2H), 1.60-1.57 (m, 2H), 1.52-1.48 (m, 2H), 1.44 (s, 9H), 1.39-1.29 (m, 4H).

### Step 2: Synthesis of intermediate WX015-3 hydrochloride

Intermediate **WX015-2** (320.00 mg, 615.77 µmol) was dissolved in ethyl acetate (15 mL) at room temperature, and a solution of hydrochloric acid in ethyl acetate (4 M, 20 mL) was then added. The reaction mixture was stirred to react at room temperature for 12 hours. After the reaction was completed, the solvent was removed by concentration under reduced pressure to give the intermediate **WX015-3** hydrochloride. ¹H NMR (400 MHz, DMSO_*d*₆) δ: 11.10 (s, 1H), 9.34 (s, 1H), 8.55 (s, 1H), 8.15 (d, *J* = 8.0 Hz, 2H), 8.10 (s, 3H), 8.06 (d, *J* = 6.8 Hz, 1H), 8.02 (d, *J* = 8.4 Hz, 1H), 7.90 (s, 1H), 7.53 (d, *J* = 8.0 Hz, 2H), 7.29-7.25 (m, 1H), 3.51-3.48 (m, 2H), 3.41-3.35 (m, 1H), 3.20-3.12 (m, 1H), 3.04-3.02 (m, 2H), 2.96-2.93 (m, 1H), 2.75-2.67 (m, 2H), 2.14-2.03 (m, 1H), 1.98-1.90 (m, 2H), 1.82-1.71 (m, 3H), 1.58-1.50 (m, 2H), 1.40-1.23 (m, 4H).

### Step 3: Synthesis of WX015 hydrochloride

Intermediate **BB-11** (35.65 mg, 100.90 µmol) was dissolved in dimethylformamide (10 mL) at room temperature. Subsequently, O-(7-azabenzotriazol-1-yl)-N,N,N,N-tetramethyluronium hexafluorophosphate (57.55 mg, 151.35 µmol) and N,N-diisopropylethylamine (65.20 mg, 504.50 µmol) were added sequentially. The reaction mixture was stirred to react at room temperature for half an hour. Then **WX015-3** (46.89 mg, 102.12 µmol, hydrochloride) was added to the reaction solution. The reaction mixture was stirred to react at room temperature for another 12 hours. After the reaction was completed, the solvent was removed by concentration under reduced pressure. Water (40 mL) was added to the resulting residue and the mixture was extracted with ethyl acetate (40 mL×3). The organic phases were combined, washed with saturated brine (100 mL), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX015** hydrochloride. MS-ESI m/z: 755.1 [M+H]⁺. ¹H NMR (400 MHz, DMSO_*d*₆) δ: 10.93 (s, 1H), 10.00 (s, 1H), 9.31 (s, 1H), 8.54 (s, 1H), 8.16-8.11 (m, 4H), 8.06 (d, *J =* 6.4 Hz, 1H), 8.02 (d, *J* = 8.0 Hz, 1H), 7.97 (s, 1H), 7.89 (s, 1H), 7.76-7.71 (m, 2H), 7.54-7.52 (m, 2H), 7.47 (d, *J* = 2.4 Hz, 1H), 7.32-7.26 (m, 2H), 4.65-4.60 (m, 3H), 3.53-3.45 (m, 2H), 3.21-3.09 (m, 3H), 3.02-3.00 (m, 2H), 2.90-2.84 (m, 2H), 2.67-2.60 (m, 2H), 2.38-2.35 (m, 2H), 2.27-2.23 (m, 2H), 1.98-1.91 (m, 2H), 1.70-1.64 (m, 2H), 1.50-1.44 (m, 2H), 1.30-1.21 (m, 4H).

### Example 16

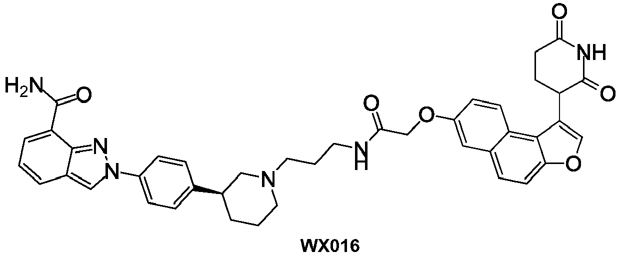

### Synthetic route:

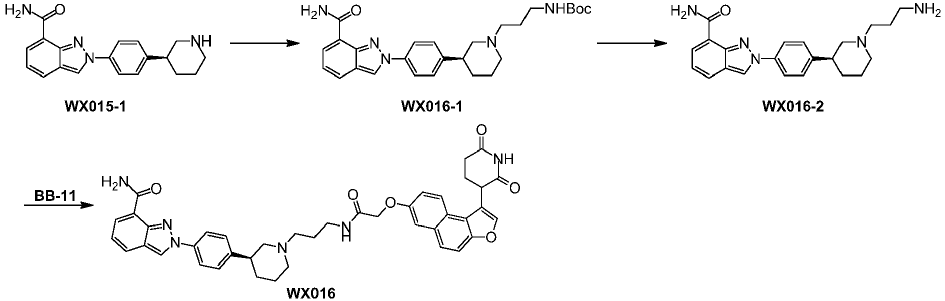

### Step 1: Synthesis of intermediate WX016-1

**WX015-1** (400.00 mg, 1.25 mmol) was dissolved in dimethylformamide (15 mL) at room temperature. Subsequently, N-Boc-3-aminopropyl bromide (314.84 mg, 1.12 mmol) and sodium bicarbonate (314.66 mg, 3.75 mmol) were added sequentially. The reaction mixture was heated to 40°C and stirred to react for 12 hours. After the reaction was completed, the mixture was cooled down to room temperature, and concentrated under reduced pressure to remove the solvent. Water (30 mL) was added to the resulting residue and the mixture was extracted with ethyl acetate (30 mL×3). The organic phases were combined, washed with saturated brine (50 mL), dried over anhydrous sodium sulfate, and filtered. The filtrate was concentrated under reduced pressure to remove the solvent. The resulting residue was separated by column chromatography (eluent: dichloromethane/methanol = 1/0-20/1, v/v) to give the intermediate **WX016-1.** ¹H NMR (400 MHz, CDCl₃) δ: 9.06 (s, 1H), 8.52 (s, 1H), 8.31 (d, *J* = 6.8 Hz, 1H), 7.91 (d, *J* = 8.0 Hz, 1H), 7.83 (d, *J* = 8.0 Hz, 2H), 7.43 (d, *J* = 8.4 Hz, 2H), 7.29-7.25 (m, 1H), 6.19 (s, 1H), 5.50 (s, 1H), 3.31-3.23 (m, 3H), 3.10-3.03 (m, 2H), 2.57-2.42 (m, 2H), 2.08-2.05 (m, 2H), 2.03-2.00 (m, 2H), 1.88-1.81 (m, 2H), 1.75-1.72 (m, 2H), 1.46 (s, 9H).

### Step 2: Synthesis of intermediate WX016-2 hydrochloride

Intermediate **WX016-1** (450.00 mg, 942.22 µmol) was dissolved in ethyl acetate (15 mL) at room temperature, and a solution of hydrochloric acid in ethyl acetate (4 M, 10 mL) was then added. The reaction mixture was stirred to react at room temperature for 12 hours. After the reaction was completed, the solvent was removed by concentration under reduced pressure to give the intermediate **WX016-2** hydrochloride. ¹H NMR (400 MHz, DMSO_*d*₆) δ: 11.12 (s, 1H), 9.36-9.30 (m, 1H), 8.55 (s, 1H), 8.25 (br s, 3H), 8.16-8.13 (m, 2H), 8.06-8.01 (m, 2H), 7.89 (s, 1H), 7.60-7.50 (m, 2H), 7.30-7.24 (m, 1H), 3.51-3.48 (m, 2H), 3.43-3.37 (m, 1H), 3.29-3.15 (m, 3H), 2.99-2.88 (m, 3H), 2.19-2.04 (m, 3H), 1.98-1.94 (m, 2H), 1.80-1.73 (m, 1H).

### Step 3: Synthesis of WX016 hydrochloride

Intermediate **BB-11** (70.00 mg, 198.12 µmol) was dissolved in dimethylformamide (10 mL) at room temperature. To the mixture were added separately O-(7-azabenzotriazol-1-yl)-N,N,N,N-tetramethyluronium hexafluorophosphate (113.00 mg, 297.18 µmol) and N,N-diisopropylethylamine (128.02 mg, 990.59 µmol). The reaction mixture was stirred to react at room temperature for half an hour. **WX016-2** (82.01 mg, 198.12 µmol, hydrochloride) was then added to the reaction solution and the mixture was stirred to react at room temperature for another 12 hours. After the reaction was completed, the solvent was removed by concentration under reduced pressure. Water (40 mL) was added to the resulting residue and the mixture was extracted with ethyl acetate (30 mL×3). The organic phases were sequentially combined, washed with saturated brine (60 mL), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX016** hydrochloride. MS-ESI *m*/*z:* 713.2 [M+H]⁺. ¹H NMR (400 MHz, DMSO_*d*₆) δ: 10.93 (s, 1H), 10.31 (s, 1H), 9.31 (s, 1H), 8.55 (s, 1H), 8.39 (t, *J =* 5.4 Hz, 1H), 8.16-8.10 (m, 3H), 8.06 (d, *J* = 7.2 Hz, 1H), 8.02 (d, *J* = 8.4 Hz, 1H), 7.97 (s, 1H), 7.91 (s, 1H), 7.78-7.71 (m, 2H), 7.50 (d, J = 2.4 Hz, 1H), 7.47-7.45 (m, 2H), 7.32 (dd, *J* = 2.4,8.8 Hz, 1H), 7.30-7.26 (m, 1H), 4.67-4.65 (m, 3H), 3.51-3.44 (m, 2H), 3.27-3.23 (m, 2H), 3.04-2.91 (m, 3H), 2.88-2.78 (m, 2H), 2.67-2.59 (m, 2H), 2.44-2.33 (m, 1H), 2.25-2.21 (m, 1H), 2.01-1.87 (m, 5H), 1.68-1.52 (m, 1H).

### Example 17

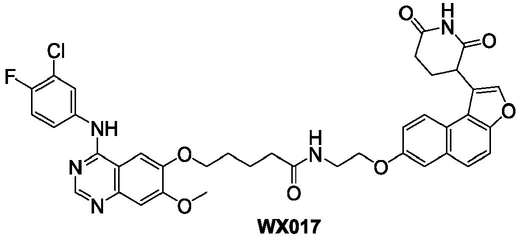

### Synthetic route:

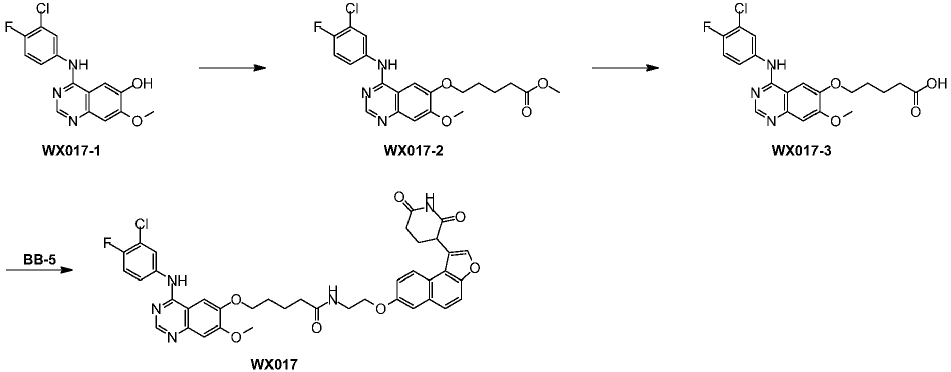

### Step 1: Synthesis of intermediate WX017-2

Compound **WX017-1** (500 mg, 1.56 mmol) was dissolved in N,N-dimethylformamide (30 mL) at room temperature. Subsequently, methyl 5-bromovalerate (457.56 mg, 2.35 mmol) and potassium carbonate (432.27 mg, 3.13 mmol) were added sequentially. The reaction mixture was heated to 50°C and stirred to react for 10 hours. After the reaction was completed, the reaction solution was added to water (50 mL) and extracted with dichloromethane (10 mL×3). The organic phases were combined, washed with saturated brine (40 mL), dried over anhydrous sodium sulfate, and filtered. The filtrate was concentrated under reduced pressure to remove the solvent. The resulting residue was purified by column chromatography (eluent: dichloromethane/methanol = 1/0, v/v) to give the intermediate **WX017-2.** MS-ESI *m*/*z:* 434.0 [M+H]⁺. ¹H NMR (400 MHz, CDCl₃) δ: 8.71 (s, 1H), 8.70 (br s, 1H), 8.08 (s, 1H), 7.99 (dd, *J =* 2.4, 6.6 Hz, 1H), 7.78-7.66 (m, 1H), 7.52 (s, 1H), 7.22-7.13 (m, 1H), 4.13 (br t, *J* = 6.8 Hz, 2H), 4.02 (s, 3H), 3.73 (s, 3H), 2.49 (t, *J* = 7.0 Hz, 2H), 1.99-1.90 (m, 2H), 1.87-1.78 (m, 2H).

### Step 2: Synthesis of intermediate WX017-3

Intermediate **WX017-2** (500 mg, 1.15 mmol) was dissolved in a mixture of methanol (15 mL) and water (3 mL) at room temperature. Subsequently, sodium hydroxide (230.47 mg, 5.76 mmol) was added and the reaction mixture was stirred to react at room temperature for 3 h. After the reaction was completed, the reaction solution was adjusted to pH of 3 with 1 M dilute hydrochloric acid. The solvent was removed by direct concentration under reduced pressure to give the intermediate **WX017-3.** MS-ESI *m*/*z:* 420.0 [M+H]⁺.

### Step 3: Synthesis of WX017

Intermediate **BB-5** (56 mg, 149.41 µmol, hydrochloride) and intermediate **WX017-3** (188.18 mg, 448.22 µmol) were dissolved in N,N-dimethylformamide (15 mL) at room temperature. Subsequently, O-(7-azabenzotriazol-1-yl)-N,N,N,N-tetramethyluronium hexafluorophosphate (85.21 mg, 224.11 µmol) and N,N-diisopropylethylamine (77.24 mg, 597.62 µmol, 104.10 µL) were added sequentially. The reaction mixture was stirred to react at room temperature for 10 hours. After the reaction was completed, the reaction solution was added to water (50 mL) and extracted with ethyl acetate (40 mL×2). The organic phases were combined, washed with saturated brine (40 mL), dried over anhydrous sodium sulfate, and filtered. The filtrate was concentrated under reduced pressure to remove the solvent. The resulting residue was separated by preparative HPLC (mobile phase: acetonitrile/water; neutral system: 0.05% NH₄HCO₃) to give the title compound **WX017.** MS-ESI *m*/*z:* 740.4 [M+H]⁺. ¹H NMR (400 MHz, DMSO_*d*₆) δ: 10.93 (s, 1H), 9.60 (s, 1H), 8.50 (s, 1H), 8.18 (t, *J* = 5.4 Hz, 1H), 8.11 (dd, *J* = 2.8, 6.8 Hz, 1H), 8.07 (br d, *J* = 9.2 Hz, 1H), 7.95 (s, 1H), 7.82 (s, 1H), 7.81-7.76 (m, 1H), 7.74-7.70 (m, 2H), 7.51 (d, *J* = 2.8 Hz, 1H), 7.44 (t, *J* = 9.2 Hz, 1H), 7.23-7.18 (m, 2H), 4.61 (br dd, *J* = 4.6, 12.2 Hz, 1H), 4.18-4.08 (m, 4H), 3.92 (s, 3H), 3.55-3.47 (m, 2H), 2.94-2.81 (m, 1H), 2.70-2.57 (m, 1H), 2.44-2.31 (m, 1H), 2.29-2.20 (m, 3H), 1.90-1.79 (m, 2H), 1.78-1.67 (m, 2H).

### Example 18

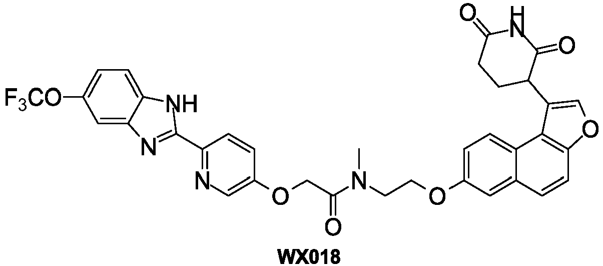

### Synthetic route:

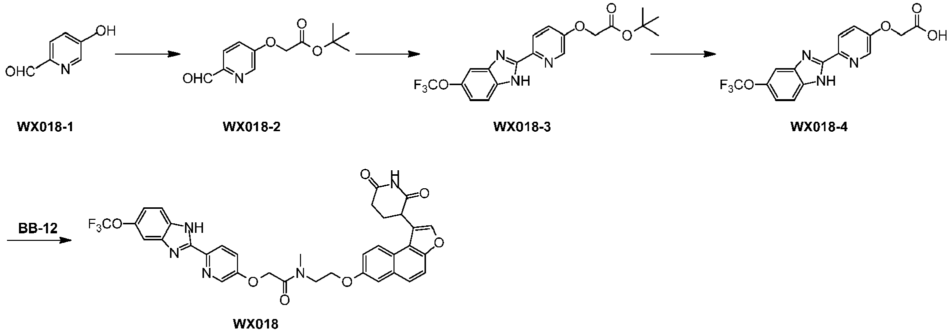

### Step 1: Synthesis of intermediate WX018-2

Compound **WX018-1** (1 g, 8.12 mmol) and tert-butyl bromoacetate (1.58 g, 8.12 mmol) were dissolved in N,N-dimethylformamide (10 mL) at room temperature under nitrogen, and potassium carbonate (4.49 g, 32.49 mmol) was then added. The reaction mixture was heated to 70°C and stirred to react for 12 hours. After the reaction was completed, the mixture was cooled down to room temperature. The reaction solution was filtered and the filtrate was poured into half-saturated brine (80 mL). The mixture was extracted with ethyl acetate (50 mL×4). The organic phases were combined, washed with saturated brine (10 mL×2), dried over anhydrous sodium sulfate, and filtered. The filtrate was concentrated under reduced pressure to remove the solvent. The resulting residue was separated by column chromatography (eluent: petroleum ether/ethyl acetate = 1/0-5/1, v/v) to give the intermediate **WX018-2.** ¹H NMR (400 MHz, CDCl₃) δ: 10.01 (d, *J* = 0.8 Hz, 1H), 8.45 (d, *J* = 2.4 Hz, 1H), 7.97 (d, *J* = 8.8 Hz, 1H), 7.30-7.26 (m, 1H), 4.66 (s, 2H), 1.50 (s, 9H).

### Step 2: Synthesis of intermediate WX018-3

Intermediate **WX018-2** (500 mg, 2.11 mmol), 2-nitro-4-(trifluoromethoxy)aniline (468.11 mg, 2.11 mmol) and dimethyl sulfoxide (8.23 mg, 105.37 µmol) were dissolved in ethanol (5 mL) at room temperature under nitrogen, and a solution of sodium dithionite (1.10 g, 6.32 mmol) in water (1.5 mL) was then added. The reaction mixture was heated to 85°C and stirred to react for 12 hours. After the reaction was completed, the reaction solution was cooled down to room temperature. The reaction solution was directly concentrated under reduced pressure to remove the solvent, and water (50 mL) was added to the residue. The pH was adjusted to 7 with ammonia, and the mixture was extracted with ethyl acetate (50 mL×4). The organic phases were combined, washed with saturated brine (10 mL×2), dried over anhydrous sodium sulfate, and filtered. The filtrate was concentrated under reduced pressure to remove the solvent. The resulting residue was separated by column chromatography (eluent: dichloromethane/methanol = 1/0, v/v) to give the intermediate **WX018-3.**

### Step 3: Synthesis of intermediate WX018-4

Intermediate **WX018-3** (450 mg, 1.10 mmol) was dissolved in trifluoroacetic acid (7.70 g, 67.53 mmol, 5 mL) at room temperature under nitrogen, and the reaction mixture was stirred to react at room temperature for 3 h. After the reaction was completed, the reaction solution was poured into water (50 mL) and the pH was adjusted to 7 with 1 N aqueous sodium hydroxide. The mixture was extracted with ethyl acetate (50 mL×5). The organic phases were combined, washed with saturated brine (50 mL×2), dried over anhydrous sodium sulfate, and filtered. The filtrate was concentrated under reduced pressure to remove the solvent to give the intermediate **WX018-4.**

### Step 11: Synthesis of WX018

Intermediate **WX018-4** (100 mg, 283.08 µmol) was dissolved in N,N-dimethylformamide (2 mL) at room temperature under nitrogen. Subsequently, 1-(3-dimethylaminopropyl)-3-ethylcarbodiimide hydrochloride (81.40 mg, 424.63 µmol), 1-hydroxy benzotriazole (57.38 mg, 424.63 µmol) and diisopropylethylamine (91.47 mg, 707.71 µmol) were added sequentially. The reaction mixture was cooled to 0 °C and stirred to react at 0°C for 20 min. Intermediate **BB-12** (99.75 mg, 283.08 µmol, hydrochloride) was then added. The reaction mixture was warmed up to room temperature and stirred for another 12 hours. After the reaction was completed, the reaction solution was filtered and the filtrate was separated by preparative HPLC (mobile phase: acetonitrile/water; acidic system: 0.04% HCl), and the resulting product was separated by preparative HPLC (mobile phase: acetonitrile/water; acidic system: 0.04% HCl) to give the title compound **WX018.** MS-ESI *m*/*z:* 688.2 [M+H]⁺. ¹H NMR (400 MHz, DMSO_*d*₆) δ: 10.94 (s, 1H), 8.52 (s, 1H), 8.36 (dd, *J* = 2.8, 9.0 Hz, 1H), 8.12 (dd, *J* = 9.2, 15.2 Hz, 1H), 7.96 (d, *J* = 9.6 Hz, 1H), 7.79-7.68 (m, 3H), 7.67-7.48 (m, 3H), 7.39-7.19 (m, 2H), 5.29-5.12 (m, 2H), 4.65 (dd, *J* = 4.2, 11.8 Hz, 1H), 4.42-4.35 (m, 1H), 4.23 (t, *J* = 5.4 Hz, 1H), 3.89-3.72 (m, 2H), 3.21-2.95 (m, 3H), 2.92-2.81 (m, 1H), 2.69-2.57 (m, 1H), 2.43-2.32 (m, 1H), 2.31-2.22 (m, 1H).

### Example 19

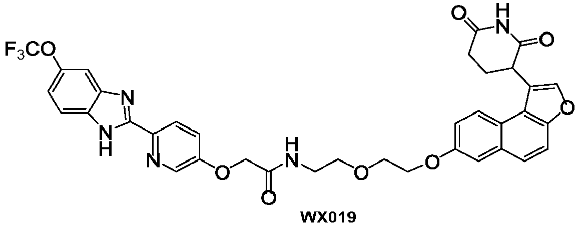

### Synthetic route:

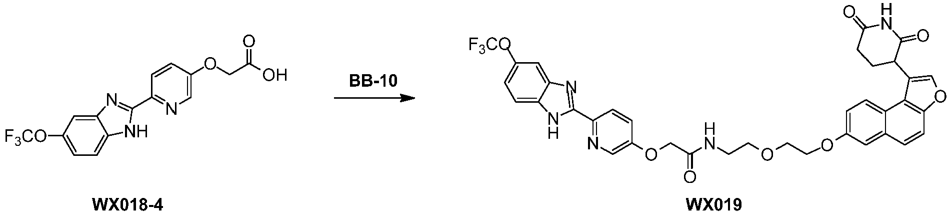

Intermediate **WX018-4** (100 mg, 283.08 µmol) was dissolved in N,N-dimethylformamide (2 mL) at room temperature under nitrogen. Subsequently, 1-(3-dimethylaminopropyl)-3-ethylcarbodiimide hydrochloride (81.40 mg, 424.63 µmol), 1-hydroxy benzotriazole (57.38 mg, 424.63 µmol) and diisopropylethylamine (91.47 mg, 707.71 µmol) were added sequentially. The reaction mixture was cooled to 0 °C and stirred at 0°C for 30 min. Intermediate **BB-10** (108.25 mg, 283.08 µmol, hydrochloride) was then added. The reaction mixture was warmed up to room temperature and stirred to react for another 12 h. After the reaction was completed, the reaction solution was filtered and the filtrate was separated by preparative HPLC (mobile phase: acetonitrile/water; acidic system: 0.04% HCl) to give the title compound **WX019.** MS-ESI *m*/*z:* 718.2 [M+H]⁺. ¹H NMR (400 MHz, DMSO_*d*₆) δ: 10.94 (s, 1H), 8.54 (d, *J* = 2.8 Hz, 1H), 8.42 (d, *J* = 8.8 Hz, 1H), 8.35 (t, *J* = 5.2 Hz, 1H), 8.07 (d, *J* = 9.2 Hz, 1H), 7.95 (s, 1H), 7.77 (d, *J* = 8.8 Hz, 1H), 7.72-7.69 (m, 2H), 7.68-7.63 (m, 2H), 7.48 (d, *J* = 2.8 Hz, 1H), 7.37 (d, *J* = 8.8 Hz, 1H), 7.23 (dd, *J* = 2.8, 9.2 Hz, 1H), 4.76 (s, 2H), 4.63 (dd, *J* = 4.0, 11.6 Hz, 1H), 4.25-4.18 (m, 2H), 3.86-3.79 (m, 2H), 3.63-3.55 (m, 2H), 3.42-3.34 (m, 2H), 2.95-2.83 (m, 1H), 2.69-2.58 (m, 1H), 2.44-2.37 (m, 1H), 2.30-2.20 (m, 1H).

### Example 20

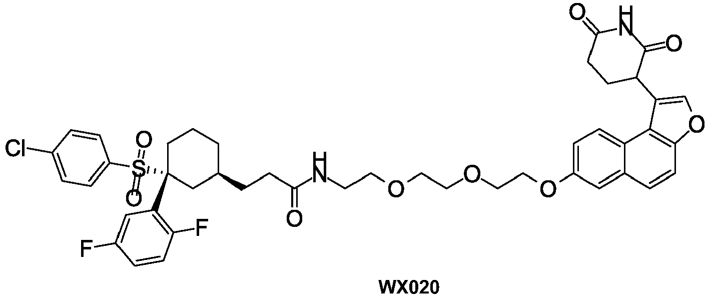

### Synthetic route:

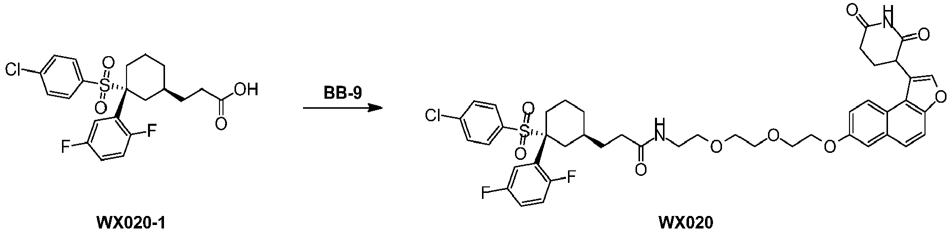

**WX020-1** (50 mg, 112.89 µmol) was dissolved in N,N-dimethylformamide (1 mL) at 0°C under nitrogen. Subsequently, O-(7-azabenzotriazol-1-yl)-N,N,N,N-tetramethyluronium hexafluorophosphate (85.85 mg, 225.78 µmol) and diisopropylethylamine(58.36 mg, 451.57 µmol) were added sequentially. The reaction mixture was stirred to react at 0°C for 30 min, then intermediate **BB-9** (52.26 mg, 112.89 µmol, hydrochloride) was added, and the reaction mixture was warmed up to 15 °C and stirred for another16 h. After the reaction was completed, the reaction solution was filtered and the filtrate was separated directly by preparative HPLC (mobile phase: acetonitrile/water; acidic system: 0.04% HCl) to give the title compound **WX020.** MS-ESI *m*/*z:* 851.2 [M+H]⁺. ¹H NMR(400 MHz, DMSO_*d*₆) δ: 10.94 (s, 1H), 8.09 (d, *J* = 8.8 Hz, 1H), 7.97 (s, 1H), 7.88 (t, *J* = 5.6 Hz, 1H), 7.77-7.70 (m, 2H), 7.60 (d, *J* = 8.4 Hz, 2H), 7.52 (d, *J* = 2.4 Hz, 1H), 7.36-7.27 (m, 3H), 7.24 (dd, *J* = 2.4, 9.2 Hz, 1H), 7.20-7.08 (m, 2H), 4.64 (dd, *J* = 4.2, 11.8 Hz, 1H), 4.26-4.18 (m, 2H), 3.84-3.77 (m, 2H), 3.66-3.60 (m, 2H), 3.59-3.51 (m, 2H), 3.42-3.40 (m, 2H), 3.25-3.15 (m, 2H), 2.93-2.82 (m, 1H), 2.70-2.58 (m, 1H), 2.45-2.32 (m, 3H), 2.30-2.12 (m, 3H), 2.11-2.01 (m, 2H), 1.68-1.52 (m, 4H), 1.49-1.40 (m, 1H), 1.36-1.21 (m, 2H).

### Example 21

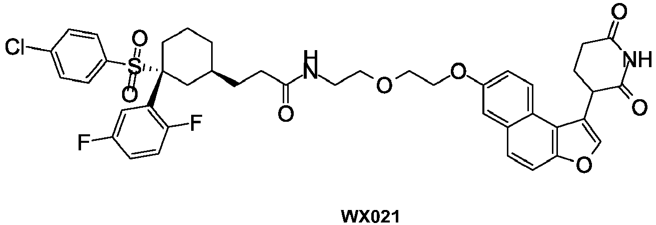

### Synthetic route:

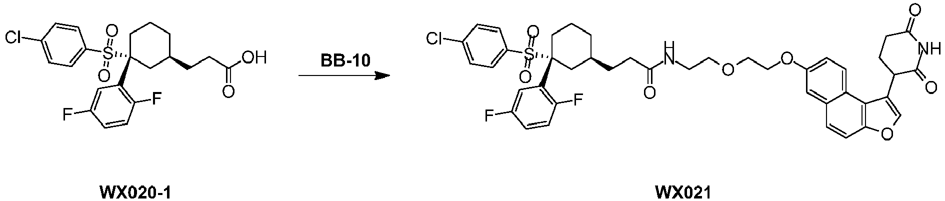

**WX020-1** (100 mg, 225.78 µmol) was dissolved in N,N-dimethylformamide (2 mL) at 0°C under nitrogen. Subsequently, 1-(3-dimethylaminopropyl)-3-ethylcarbodiimide hydrochloride (64.92 mg, 338.67 µmol), 1-hydroxy benzotriazole (45.76 mg, 338.67 µmol) and N,N-diisopropylethylamine(102.13 mg, 790.24 µmol) were added sequentially. The reaction mixture was stirred to react at 0°C for 30 min, then intermediate **BB-10** (94.57 mg, 225.78 µmol, hydrochloride) was added, and the reaction mixture was warmed up to 15 °C and stirred to react for another 16 h. After the reaction was completed, the reaction solution was filtered and the filtrate was separated directly by preparative HPLC (mobile phase: acetonitrile/water; acidic system: 0.04% HCl) to give the title compound **WX021.** MS-ESI *m*/*z*: 807.3 [M+H]⁺. ¹H NMR (400 MHz, DMSO_*d*₆) δ: 10.94 (s, 1H), 8.08 (d, *J* = 8.8 Hz, 1H), 7.96 (s, 1H), 7.92 (t, *J* = 5.2 Hz, 1H), 7.76-7.71 (m, 2H), 7.59 (d, *J* = 8.8 Hz, 2H), 7.50 (d, *J* = 2.8 Hz, 1H), 7.37-7.25 (m, 3H), 7.22 (dd, *J* = 2.4, 9.2 Hz, 1H), 7.19-7.07 (m, 2H), 4.63 (dd, *J* = 4.2, 11.8 Hz, 1H), 4.30-4.13 (m, 2H), 3.87-3.71 (m, 2H), 3.57-3.43 (m, 2H), 3.28-3.20 (m, 2H), 2.93-2.80 (m, 1H), 2.69-2.57 (m, 1H), 2.43-2.31 (m, 3H), 2.29-2.12 (m, 3H), 2.11-1.98 (m, 2H), 1.67-1.50 (m, 4H), 1.50-1.39 (m, 1H), 1.38-1.20 (m, 2H).

### Example 22

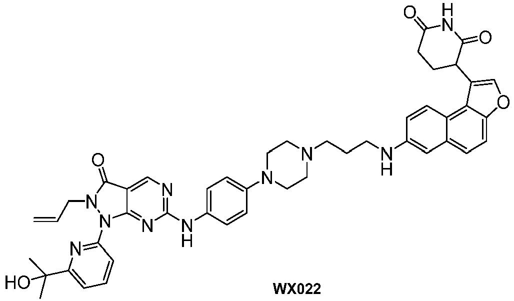

### Synthetic route:

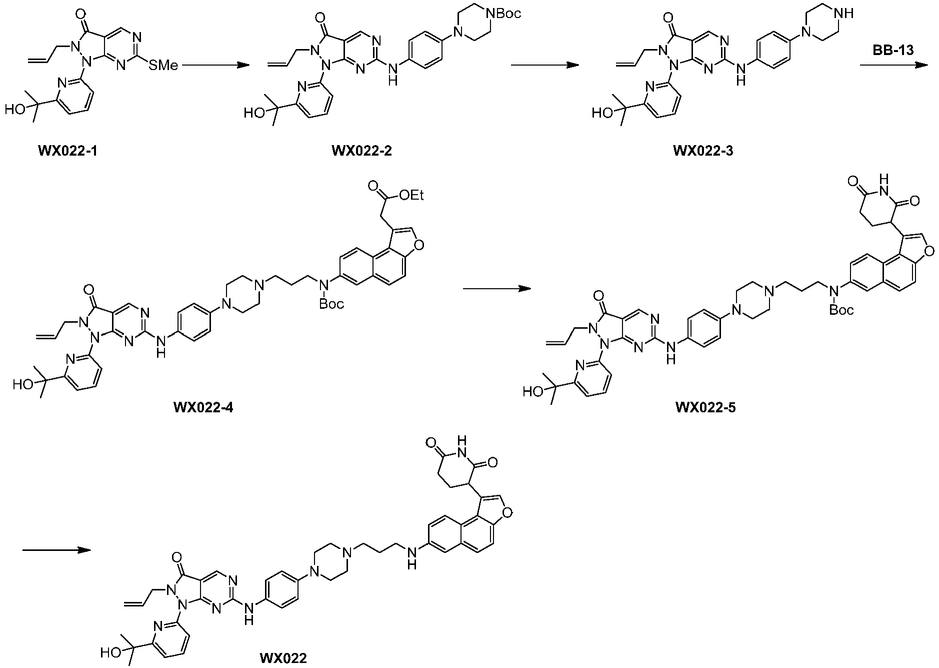

### Step 1: Synthesis of intermediate WX022-2

**WX022-1** (2.13 g, 5.96 mmol) was added to toluene (30 mL) at room temperature under nitrogen, and m-chloroperoxybenzoic acid (1.33 g, 6.56 mmol, purity: 85%) was then added. The reaction solution was stirred to react at room temperature for 1 h. N,N-diisopropylethylamine (1.54 g, 11.92 mmol, 2.08 mL) and 1-tert-butoxycarbonyl-4-(4-aminophenyl)piperazine (1.98 g, 7.15 mmol) were added to the reaction solution sequentially, and the reaction mixture was stirred at room temperature for another 14 hours. After the reaction was completed, saturated aqueous sodium bicarbonate solution (20 mL) and saturated sodium sulfite solution (30 mL) were added to the reaction solution, and the mixture was extracted with ethyl acetate (50 mL×3). The organic phases were combined, washed with saturated brine (100 mL×2), dried over anhydrous sodium sulfate, and filtered. The solvent was removed from the filtrate under reduced pressure. The resulting residue was separated by column chromatography (eluent: petroleum ether/ethyl acetate = 3/1-10/1, v/v) to give the intermediate **WX022-2.** MS-ESI *m*/*z:* 587.3 [M+H]⁺. ¹H NMR (400 MHz, DMSO_*d*₆) δ: 10.16 (br s, 1H), 8.83 (s, 1H), 8.05 (br t, *J* = 7.4 Hz, 1H), 7.75 (br d, *J* = 8.0 Hz, 1H), 7.62-7.60 (m, 3H), 6.95 (br d, *J* = 9.2 Hz, 2H), 5.71-5.61 (m, 1H), 5.32 (s, 1H), 4.99 (br d, *J* = 10.4 Hz, 1H), 4.83 (br d, *J* = 16.8 Hz, 1H), 4.68 (br d, *J* = 5.6 Hz, 2H), 3.47-3.46 (m, 4H), 3.06-3.05 (m, 4H), 1.46 (s, 6H), 1.42 (s, 9H).

### Step 2: Synthesis of intermediate WX022-3 trifluoroacetate

Trifluoroacetic acid (5.02 g, 44.01 mmol, 3.26 mL) was added dropwise to a solution of intermediate **WX022-2** (2.59 g, 4.40 mmol) in dichloromethane (30 mL) at room temperature under nitrogen, and the reaction mixture was stirred to react at room temperature for 14 hours. After the reaction was completed, the reaction solution was directly concentrated under reduced pressure to remove the solvent to give the intermediate **WX022-3** trifluoroacetate. ¹H NMR (400 MHz, DMSO_*d*₆) δ: 10.24 (br s, 1H), 9.39 (br s, 2H), 8.85 (s, 1 H), 8.04 (br t, *J* = 8.0 Hz, 1H), 7.74 (br d, *J* = 8.0 Hz, 1H), 7.68-7.57 (m, 3H), 7.04 (br d, *J* = 8.8 Hz, 2H), 5.71-5.59 (m, 1H), 4.99 (br d, *J* = 10.4 Hz, 1H), 4.82 (br d, *J* = 17.2 Hz, 1H), 4.75-4.73 (m, 2H), 3.38-3.37 (m, 4H), 3.30-3.18 (m, 4H), 1.46 (s, 6H).

### Step 3: Synthesis of intermediate WX022-4

Intermediate **WX022-3** (280.06 mg, 466.31 µmol, trifluoroacetate) and intermediate **BB-13** (247 mg, 466.31 µmol) were added to N,N-dimethylformamide (5 mL) at room temperature under nitrogen. Subsequently, potassium carbonate (322.24 mg, 2.33 mmol) and potassium iodide (77.41 mg, 466.31 µmol) were added sequentially. The reaction mixture was heated to 80°C and stirred to react for 24 hours. After the reaction was completed, the mixture was cooled down to room temperature. The reaction solution was added to water (30 mL) and extracted with ethyl acetate (30 mL×3). The organic phases were combined, washed with saturated brine (50 mL×2), dried over anhydrous sodium sulfate, and filtered. The solvent was removed from the filtrate under reduced pressure. The resulting residue was separated by column (eluent: dichloromethane/methanol = 1/0-50/1, v/v) to give the intermediate **WX022-4** (purity: 83.58%). MS-ESI *m*/*z:* 896.4 [M+H]⁺. ¹H NMR (400 MHz, CDCl₃) δ: 8.86-8.82 (m, 1H), 8.20 (d, *J* = 8.8 Hz, 1H), 8.15-8.07 (m, 1H), 7.85 (br t, *J* = 7.6 Hz, 1H), 7.79-7.74 (m, 3H), 7.70-7.65 (m, 1H), 7.59-7.54 (m, 1H), 7.48-7.41 (m, 3H), 7.38-7.31 (m, 2H), 6.90 (br d, *J* = 9.2 Hz, 2H), 5.05 (br d, *J* = 10.0 Hz, 1H), 4.95 (br d, *J* = 16.4 Hz, 1H), 4.75 (br d, *J* = 6.0 Hz, 2H), 4.27-4.20 (m, 2H), 4.07 (s, 2H), 3.97-3.86 (m, 2H), 3.83 (br t, *J* = 7.4 Hz, 2H), 3.21-3.10 (m, 4H), 2.65-2.50 (m, 4H), 1.91-1.81 (m, 2H), 1.57 (br s, 6H), 1.45 (br s, 9H), 1.29-1.27 (m, 3H).

### Step 4: Synthesis of intermediate WX022-5

Intermediate **WX022-4** (152 mg, 141.78 µmol, purity: 83.58%) was added to N,N-dimethylformamide (10 mL) at 0°C under nitrogen, and potassium tert-butoxide (15.91 mg, 141.78 µmol) and acrylamide (10.08 mg, 141.78 µmol) were then added. The reaction mixture was stirred to react at 0°C for 2 h. After the reaction was completed, the reaction solution was warmed up to room temperature. Water (30 mL) was added and the mixture was extracted with ethyl acetate (30 mL×3). The organic phases were combined, washed with saturated brine (50 mL×2), dried over anhydrous sodium sulfate, and filtered. The solvent was removed from the filtrate under reduced pressure to give the intermediate **WX022-5** (purity: 63.53%). MS-ESI *m*/*z:* 921.3 [M+H]⁺.

### Step 5: Synthesis of compound WX022

Intermediate **WX022-5** (124 mg, 85.53 µmol, purity: 63.53%) was added to a solution of hydrochloric acid in ethyl acetate (4 M, 15 mL) at room temperature under nitrogen, and the reaction mixture was stirred to react at room temperature for 3 h. After the reaction was completed, the solvent was removed from the reaction solution under reduced pressure. The resulting residue was separated by preparative HPLC (mobile phase: acetonitrile/water; acidic system: 0.05% HCl) to give the title compound **WX022.** MS-ESI *m*/*z:* 821.3 [M+H]⁺. ¹H NMR (400 MHz, DMSO_*d*₆) δ: 10.94 (s, 1H), 10.21 (br s, 1H), 8.84 (s, 1H), 8.14-8.00 (m, 2H), 7.97 (s, 1H), 7.79-7.69 (m, 3H), 7.67-7.59 (m, 3H), 7.57-7.49 (m, 1H), 7.43-7.34 (m, 1H), 7.00 (br d, *J* = 8.4 Hz, 2H), 5.72-5.60 (m, 1H), 4.99 (br d, *J* = 10.4 Hz, 1H), 4.82 (br d, *J* = 16.8 Hz, 1H), 4.68 (br d, *J* = 4.0 Hz, 2H), 4.66-4.59 (m, 1H), 3.81-3.68 (m, 4H), 3.43-3.36 (m, 2H), 3.35-3.28 (m, 2H), 3.27-3.05 (m, 4H), 2.93-2.81 (m, 1H), 2.68-2.59 (m, 1H), 2.44-2.34 (m, 2H), 2.25-2.15 (m, 2H), 1.46 (s, 6H).

### Example 23

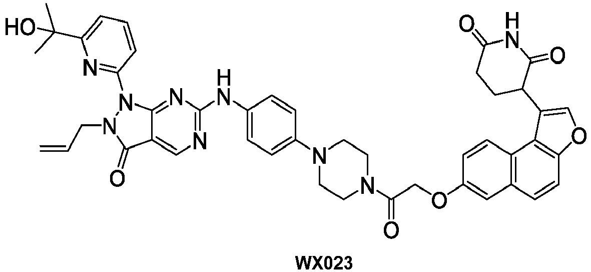

### Synthetic route:

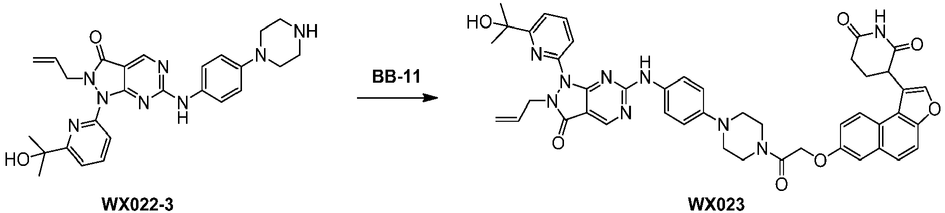

Intermediate **BB-11** (101.99 mg, 169.82 µmol) and intermediate **WX022-3** (60 mg, 169.82 µmol, trifluoroacetate) were added to N,N-dimethylformamide (5 mL) at room temperature under nitrogen. Subsequently, O-(7-azabenzotriazol-1-yl)-N,N,N,N-tetramethyluronium hexafluorophosphate (64.57 mg, 169.82 µmol) and N,N-diisopropylethylamine (65.84 mg, 509.45 µmol, 88.74 µL) were added sequentially. The reaction mixture was stirred to react at room temperature for 14 hours. After the reaction was completed, water (30 mL) was added to the reaction solution and the mixture was extracted with ethyl acetate (30 mL×3). The organic phases were combined, washed with saturated brine (50 mL×2), dried over anhydrous sodium sulfate, and filtered. The solvent was removed from the filtrate under reduced pressure. The resulting residue was separated by preparative HPLC (mobile phase: acetonitrile/water; acidic system: 0.05% HCl) to give the title compound **WX023.** MS-ESI *m*/*z:* 822.3 [M+H]⁺. ¹H NMR (400 MHz, DMSO_*d*₆) δ: 10.94 (s, 1H), 10.19 (br s, 1H), 8.85 (s, 1H), 8.16-8.01 (m, 2H), 7.97 (s, 1H), 7.78-7.73 (m, 3H), 7.68-7.59 (m, 3H), 7.52 (d, *J* = 2.4 Hz, 1H), 7.29 (dd, *J* = 2.6, 9.4 Hz, 1H), 7.07 (br d, *J* = 8.0 Hz, 2H), 5.72-5.61 (m, 1H), 5.05-4.97 (m, 3H), 4.82 (dd, *J* = 1.4, 17.0 Hz, 1H), 4.68 (br d, *J* = 5.6 Hz, 2H), 4.66-4.61 (m, 1H), 3.73-3.69 (m, 4H), 3.30-3.16 (m, 4H), 2.94-2.82 (m, 1H), 2.69-2.63 (m, 1H), 2.43-2.39 (m, 1H), 2.22-2.30 (m, 1H), 1.46 (s, 6H).

### Example 24

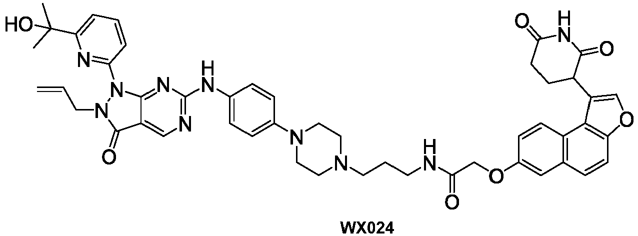

### Synthetic route:

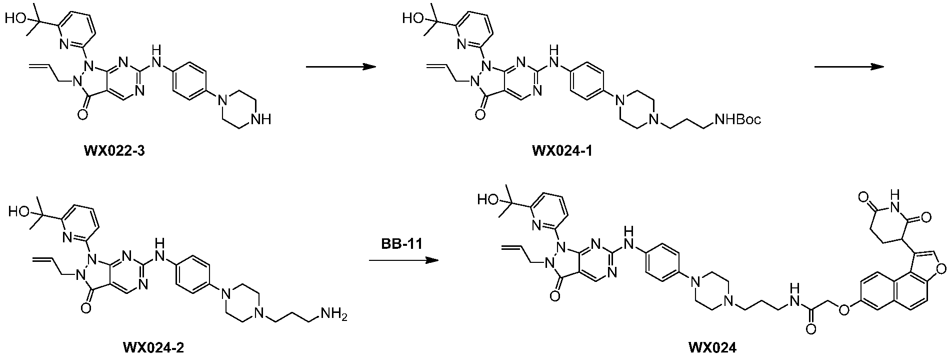

### Step 1: Synthesis of intermediate WX024-1

Compound **WX022-3** (600 mg, 999.02 µmol, trifluoroacetate) and N-Boc-3-aminopropyl bromide (261.68 mg, 1.10 mmol) were added to N,N-dimethylformamide (10 mL) at room temperature under nitrogen, and potassium carbonate (276.15 mg, 2.00 mmol) was then added. The reaction mixture was heated to 60°C and stirred to react for 8 hours. After the reaction was completed, the mixture was cooled down to room temperature. Water (30 mL) was added to the reaction solution and the mixture was extracted with ethyl acetate (30 mL×3). The organic phases were combined, washed with saturated brine (50 mL×2), dried over anhydrous sodium sulfate, and filtered. The solvent was removed from the filtrate under reduced pressure. The resulting residue was separated by column chromatography (eluent: petroleum ether/ethyl acetate = 1/0-100/1, v/v) to give the intermediate **WX024-1.** MS-ESI *m*/*z:* 644.3 [M+H]⁺. ¹H NMR (400 MHz, DMSO_*d*₆) δ: 10.14 (br s, 1H), 8.82 (s, 1H), 8.08-8.00 (m, 1H), 7.75 (br d, *J =* 7.6 Hz, 1H), 7.64-7.54 (m, 3H), 6.92 (br d, *J* = 8.8 Hz, 2H), 6.85-6.77 (m, 1H), 5.73-5.60 (m, 1H), 5.32 (s, 1H), 4.99 (br d, *J* = 10.0 Hz, 1H), 4.82 (br d, *J* = 17.6 Hz, 1H), 4.71-4.64 (m, 2H), 3.32-3.26 (m, 4H), 3.15-3.04 (m, 4H), 2.99-2.94 (m, 2H), 2.36-2.28 (m, 2H), 1.60-1.54 (m, 2H), 1.46 (s, 6H), 1.38 (s, 9H).

### Step 2: Synthesis of intermediate WX024-2 hydrochloride

Intermediate **WX024-1** (500.47 µmol) was added to a solution of hydrochloric acid in ethyl acetate (4 M, 50 mL) at room temperature under nitrogen. The reaction mixture was stirred to react at room temperature for 14 hours. After the reaction was completed, the solvent was removed from the reaction solution under reduced pressure to give the intermediate **WX024-2** hydrochloride. ¹H NMR (400 MHz, DMSO_*d*₆) δ: 11.27 (br s, 1H), 10.24 (br s, 1H), 8.85 (s, 1H), 8.14 (br s, 3H), 8.04 (br t, *J =* 8.0 Hz, 1H), 7.75 (br d, *J =* 8.4 Hz, 1H), 7.66-7.60 (m, 2H), 7.01 (br d, *J =* 9.2 Hz, 2H), 5.72-5.60 (m, 1H), 4.99 (br d, *J =* 10.0 Hz, 1H), 4.82 (br d, *J* = 17.2 Hz, 1H), 4.73-4.64 (m, 2H), 3.82-3.73 (m, 2H), 3.60-3.49 (m, 2H), 3.24-3.17 (m, 4H), 3.17-3.10 (m, 2H), 2.97-2.90 (m, 2H), 2.13-2.06 (m, 2H), 1.59 (s, 6H).

### Step 3: Synthesis of compound WX024 hydrochloride

Intermediate **WX024-2** (98.51 mg, 169.82 µmol, hydrochloride) and intermediate **BB-11** (60 mg, 169.82 µmol) were added to N,N-dimethylformamide (5 mL) at room temperature under nitrogen. Subsequently, O-(7-azabenzotriazol-1-yl)-N,N,N,N-tetramethyluronium hexafluorophosphate (64.57 mg, 169.82 µmol) and N,N-diisopropylethylamine (65.84 mg, 509.45 µmol, 88.74 µL) were added sequentially. The reaction mixture was stirred to react at room temperature for 14 hours. After the reaction was completed, water (30 mL) was added to the reaction solution and the mixture was extracted with ethyl acetate (30 mL×3). The organic phases were combined, washed with saturated brine (50 mL×2), dried over anhydrous sodium sulfate, and filtered. The solvent was removed from the filtrate under reduced pressure. The resulting residue was separated by preparative HPLC (mobile phase: acetonitrile/water; acidic system: 0.05% HCl) to give the title compound **WX024** hydrochloride. MS-ESI *m*/*z:* 879.4 [M+H]⁺. ¹H NMR (400 MHz, DMSO_*d*₆) δ: 10.94 (s, 1H), 10.38 (br s, 1H), 10.21 (br s, 1H), 8.84 (s, 1H), 8.41 (br t, *J* = 5.2 Hz, 1H), 8.15 (br d, *J* = 9.6 Hz, 1H), 8.04 (br t, *J* = 7.4 Hz, 1H), 7.97 (s, 1H), 7.80-7.72 (m, 3H), 7.67-7.58 (m, 3H), 7.53 (br d, *J* = 2.0 Hz, 1H), 7.35 (br d, *J* = 8.4 Hz, 1H), 6.97 (br d, *J* = 8.8 Hz, 2H), 5.73-5.59 (m, 1H), 4.99 (br d, *J* = 10.0 Hz, 1H), 4.82 (br d, *J* = 17.6 Hz, 1H), 4.72-4.62 (m, 5H), 3.71 (br d, *J* = 8.0 Hz, 2H), 3.36-3.21 (m, 2H), 3.15-2.93 (m, 8H), 2.93-2.81 (m, 1H), 2.70-2.58 (m, 1H), 2.43-2.37 (m, 1H), 2.29-2.22 (m, 1H), 1.98-1.86 (m, 2H), 1.46 (s, 6H).

### Example 25

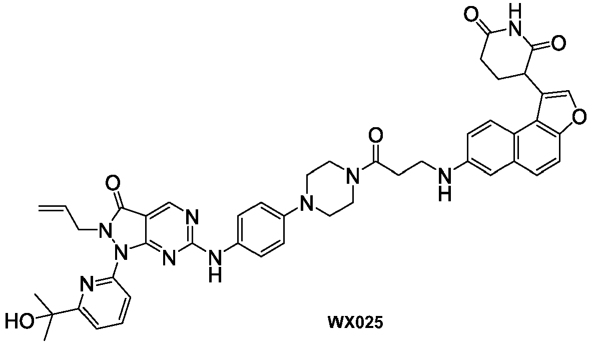

### Synthetic route:

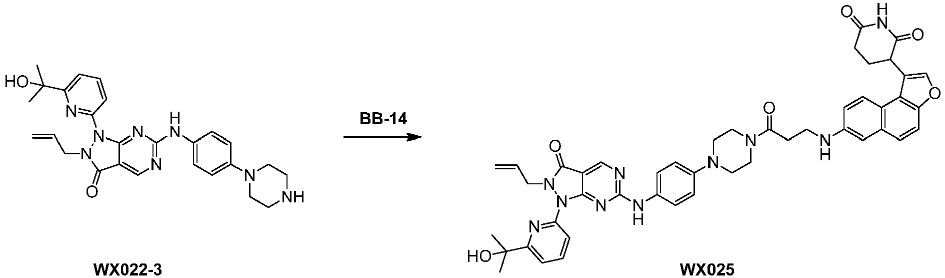

Intermediate **WX022-3** (81.97 mg, 136.48 µmol, trifluoroacetate) and intermediate **BB-14** (50 mg, 136.48 µmol) were added to N,N-dimethylformamide (5 mL) at room temperature under nitrogen. Subsequently, O-(7-azabenzotriazol-1-yl)-N,N,N,N-tetramethyluronium hexafluorophosphate (51.89 mg, 136.48 µmol) and N,N-diisopropylethylamine (52.92 mg, 409.43 µmol, 71.31 µL) were added sequentially. The reaction mixture was stirred to react at room temperature for 14 hours. After the reaction was completed, water (30 mL) was added to the reaction solution and the mixture was extracted with ethyl acetate (30 mL×3). The organic phases were combined, washed with saturated brine (50 mL×2), dried over anhydrous sodium sulfate, and filtered. The solvent was removed from the filtrate under reduced pressure. The resulting residue was separated by preparative HPLC (mobile phase: acetonitrile/water; acidic system: 0.05% HCl) to give the title compound **WX025.** MS-ESI *m*/*z:* 835.4 [M+H]⁺. ¹H NMR (400 MHz, DMSO_*d*₆) δ: 10.96 (s, 1H), 10.34 (br s, 1H), 8.89 (s, 1H), 8.23 (br d, *J* = 8.4 Hz, 1H), 8.08-8.01 (m, 2H), 7.93 (br s, 1H), 7.88-7.81 (m, 2H), 7.78-7.71 (m, 3H), 7.65-7.56 (m, 2H), 7.46-7.33 (m, 2H), 5.72-5.61 (m, 1H), 4.99 (dd, *J =* 1.0, 10.2 Hz, 1H), 4.82 (br dd, *J =* 1.0, 17.0 Hz, 1H), 4.72-4.63 (m, 3H), 3.92-3.74 (m, 4H), 3.61 (br t, *J* = 6.8 Hz, 2H), 3.44-3.28 (m, 4H), 2.94 (br t, *J* = 6.4 Hz, 2H), 2.91-2.81 (m, 1H), 2.68-2.59 (m, 1H), 2.45-2.37 (m, 1H), 2.31-2.22 (m, 1H), 1.46 (s, 6H).

### Example 26

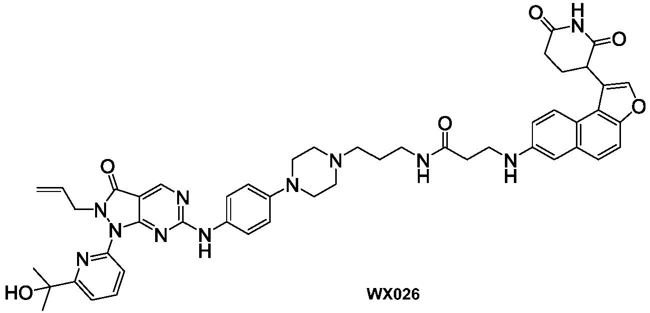

### Synthetic route:

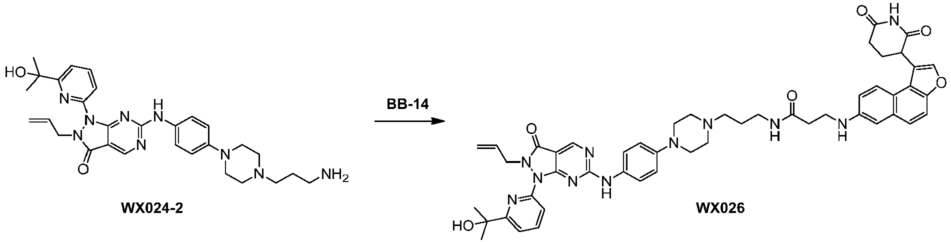

Intermediate **WX024-2** (79.18 mg, 136.48 µmol, hydrochloride) and intermediate **BB-14** (50 mg, 136.48 µmol) were added to N,N-dimethylformamide (5 mL) at room temperature under nitrogen. Subsequently, O-(7-azabenzotriazol-1-yl)-N,N,N,N-tetramethyluronium hexafluorophosphate (51.89 mg, 136.48 µmol) and N,N-diisopropylethylamine (17.64 mg, 136.48 µmol, 23.77 µL) were added sequentially. The reaction mixture was stirred to react at room temperature for 14 hours. After the reaction was completed, water (30 mL) was added to the reaction solution and the mixture was extracted with ethyl acetate (30 mL×3). The organic phases were combined, washed with saturated brine (50 mL×2), dried over anhydrous sodium sulfate, and filtered. The solvent was removed from the filtrate under reduced pressure. The resulting residue was separated by preparative HPLC (mobile phase: acetonitrile/water; acidic system: 0.05% HCl) to give the title compound **WX026.** MS-ESI *m*/*z:* 892.4 [M+H]⁺. ¹H NMR (400 MHz, DMSO_*d*₆) δ: 10.96 (s, 1H), 10.89 (br s, 1H), 10.23 (br s, 1H), 8.85 (s, 1H), 8.32 (br t, *J* = 5.6 Hz, 1H), 8.27-8.21 (m, 1H), 8.08-8.01 (m, 2H), 7.94 (br s, 1H), 7.88-7.82 (m, 2H), 7.75 (br d, *J* = 8.0 Hz, 1H), 7.67-7.57 (m, 4H), 6.99 (br d, *J* = 8.8 Hz, 2H), 5.72-5.60 (m, 1H), 4.99 (dd, *J* = 1.0, 10.2 Hz, 1H), 4.82 (br dd, *J* = 1.2, 17.2 Hz, 1H), 4.73-4.64 (m, 3H), 3.80-3.68 (m, 2H), 3.57 (br t, *J* = 7.2 Hz, 2H), 3.54-3.46 (m, 2H), 3.20-3.06 (m, 8H), 2.92-2.83 (m, 1H), 2.69-2.60 (m, 3H), 2.45-2.37 (m, 1H), 2.31-2.21 (m, 1H), 1.93-1.82 (m, 2H), 1.46 (s, 6H).

### Example 27

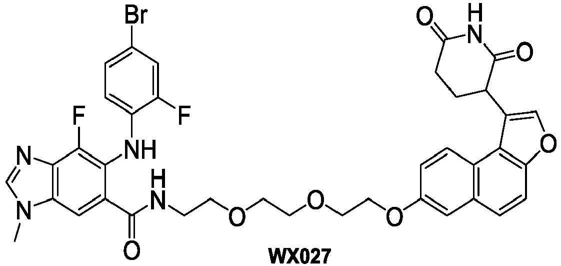

### Synthetic route:

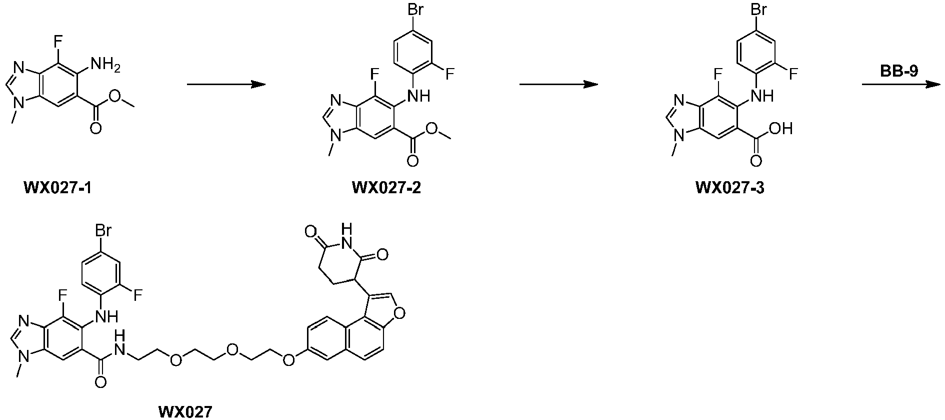

### Step 1: Synthesis of intermediate WX027-2

**WXF027-1** (2.00 g, 8.96 mmol) and 1-bromo-3-fluoro-4-iodobenzene (3.24 g, 10.75 mmol) were dissolved in a mixture of toluene (20 mL) and dioxane (20 mL) at room temperature. Subsequently, 4,5-bis(diphenylphosphino)-9,9-dimethylxanthene (1.04 g, 1.79 mmol), tris(dibenzylideneacetone)dipalladium (820.52 mg, 896.04 µmol) and cesium carbonate (8.76 g, 26.88 mmol) were added sequentially. The reaction mixture was heated to 100°C and stirred to react for 12 hours. After the reaction was completed, the mixture was cooled down to room temperature. Water (30 mL) was added to the resulting reaction solution, and the mixture was extracted with ethyl acetate (30 mL×3). The organic phases were sequentially combined, washed with saturated brine (100 mL), dried over anhydrous sodium sulfate, and filtered. The filtrate was concentrated under reduced pressure to remove the solvent. The resulting residue was separated by column chromatography (eluent: dichloromethane/methanol = 1/0-20/1, v/v) to give the intermediate **WX027-2.** ¹H NMR (400 MHz, DMSO_*d*₆) δ: 8.44 (s, 1H), 8.09-8.00 (m, 2H), 7.46 (dd, *J* = 2.4, 11.2 Hz, 1H), 7.14 (d, *J* = 8.8 Hz, 1H), 6.50-6.41 (m, 1H), 3.92 (s, 3H), 3.81 (s, 3H).

### Step 2: Synthesis of intermediate WX027-3

Intermediate **WX027-2** (1.10 g, 2.78 mmol) was dissolved in methanol (30 mL) at room temperature, and sodium hydroxide (444.20 mg, 11.11 mmol) was then added. The reaction mixture was stirred to react at room temperature for 12 hours. After the reaction was completed, the reaction solution was adjusted to pH 5-6 with 2 M dilute aqueous hydrochloric acid. The mixture was filtered, and the filter cake was collected and washed with water (15 mL) to give the intermediate **WX027-3.** ¹H NMR (400 MHz, DMSO_*d*₆) δ: 8.44 (s, 1H), 8.39 (s, 1H), 8.08 (s, 1H), 7.47 (dd, *J* = 1.8, 11.0 Hz, 1H), 7.17 (d, *J* = 8.0 Hz, 1H), 6.57-6.52 (m, 1H), 3.92 (s, 3H).

### Step 3: Synthesis of WX027

Intermediate **WX027-3** (50.00 mg, 130.84 µmol) was dissolved in dimethylformamide (10 mL) at room temperature. Subsequently, O-(7-azabenzotriazol-1-yl)-N,N,N,N-tetramethyluronium hexafluorophosphate (74.62 mg, 196.25 µmol) and N,N-diisopropylethylamine (84.55 mg, 654.18 µmol) were added sequentially. The reaction mixture was stirred to react at room temperature for half an hour. Intermediate **BB-9** (60.59 mg, 120.53 µmol, hydrochloride) was then added. The reaction mixture was stirred to react at room temperature for another 12 hours. After the reaction was completed, water (40 mL) was added to the reaction solution and the resulting mixture was extracted with ethyl acetate (20 mL×3). The organic phases were combined, washed with saturated brine (80 mL), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX027.** MS-ESI *m*/*z:* 790.1 [M+H]⁺, 792.2 [M+2+H]⁺. ¹H NMR (400 MHz, DMSO_*d*₆) δ: 10.93 (s, 1H), 8.79 (t, *J* = 5.4 Hz, 1H), 8.71 (s, 1H), 8.44 (s, 1H), 8.06 (d, *J* = 8.8 Hz, 1H), 7.95 (s, 1H),7.92 (s, 1H), 7.77-7.67 (m, 2H), 7.50-7.41 (m, 2H), 7.19 (dd, *J* = 2.2, 9.0 Hz, 1H), 7.12 (d, *J* = 8.8 Hz, 1H), 6.52-6.47 (m, 1H), 4.62 (dd, *J* = 4.0, 11.6 Hz, 1H), 4.25-4.10 (m, 2H), 3.92 (s, 3H), 3.77-3.76 (m, 2H), 3.51-3.47 (m, 5H), 3.42-3.39 (m, 3H), 2.91-2.83 (m, 1H), 2.64-2.59 (m, 1H), 2.44-2.35 (m, 1H), 2.27-2.20 (m, 1H).

### Example 28

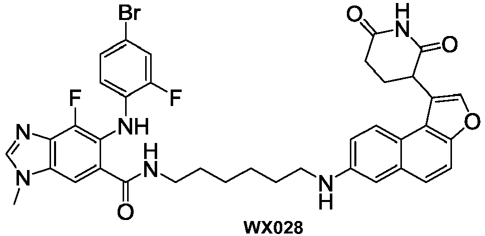

### Synthetic route:

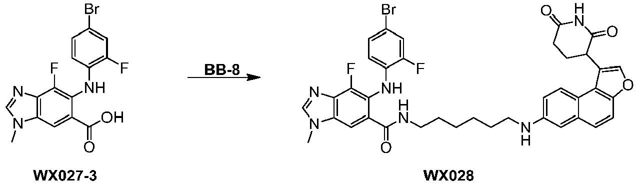

Intermediate **WX027-3** (50.00 mg, 130.84 µmol) was dissolved in dimethylformamide (10 mL) at room temperature. Subsequently, O-(7-azabenzotriazol-1-yl)-N,N,N,N-tetramethyluronium hexafluorophosphate (74.62 mg, 196.25 µmol) and N,N-diisopropylethylamine (84.55 mg, 654.18 µmol) were added sequentially. The reaction mixture was stirred to react at room temperature for half an hour and intermediate **BB-8** (56.25 mg, 130.84 µmol, hydrochloride) was then added. The reaction mixture was stirred to react at room temperature for another 12 hours. After the reaction was completed, water (40 mL) was added to the reaction solution and the resulting mixture was extracted with ethyl acetate (20 mL×3). The organic phases were sequentially combined, washed with saturated brine (80 mL), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX028.** MS-ESI *m*/*z:* 757.1 [M+H]⁺, 759.1 [M+2+H]⁺. ¹H NMR (400 MHz, DMSO_*d*₆) δ: 10.96 (s, 1H), 8.79-8.73 (m, 2H), 8.29 (d, *J* = 7.6 Hz, 1H), 8.13 (s, 1H), 8.06 (s, 1H), 7.96 (s, 1H), 7.91-7.85 (m, 2H), 7.71 (d, *J* = 8.8 Hz, 1H), 7.44 (dd, *J* = 2.0, 10.8 Hz, 1H), 7.12 (d, *J* = 8.4 Hz, 1H), 6.53-6.47 (m, 1H), 4.69 (dd, *J* = 3.8, 12.2 Hz, 1H), 3.95 (s, 3H), 3.35-3.31 (m, 2H), 3.24-3.19 (m, 2H), 2.92-2.83 (m, 1H), 2.66-2.62 (m, 1H), 2.45-2.39 (m, 1H), 2.33-2.26 (m, 1H), 1.76-1.67 (m, 2H), 1.48-1.44 (m, 2H), 1.36-1.33 (m, 2H), 1.30-1.28 (m, 2H).

### Example 29

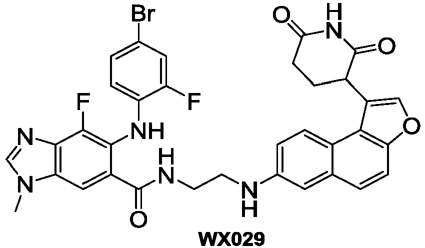

### Synthetic route:

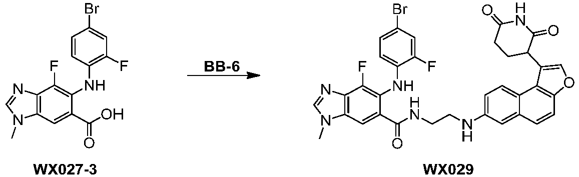

Intermediate **WX027-3** (50.00 mg, 130.84 µmol) was dissolved in dimethylformamide (10 mL) at room temperature. Subsequently, O-(7-azabenzotriazol-1-yl)-N,N,N,N-tetramethyluronium hexafluorophosphate (74.62 mg, 196.25 µmol) and N,N-diisopropylethylamine (84.55 mg, 654.18 µmol) were added sequentially. The reaction mixture was stirred to react at room temperature for half an hour, and intermediate **BB-6** (48.91 mg, 130.84 µmol, hydrochloride) was then added. The reaction mixture was stirred to react at room temperature for another 12 hours. After the reaction was completed, water (40 mL) was added to the reaction solution and the resulting mixture was extracted with ethyl acetate (20 mL×3). The organic phases were combined, washed with saturated brine (80 mL), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX029.** MS-ESI *m*/*z:* 701.3 [M+H]⁺, 703.3 [M+2+H]⁺. ¹H NMR (400 MHz, DMSO_*d*₆) δ: 10.94 (s, 1H), 9.05 (t, *J* = 5.2 Hz, 1H), 8.75 (s, 1H), 8.14-8.02 (m, 2H), 7.96 (s, 1H), 7.74-7.67 (m, 2H), 7.55 (s, 1H), 7.45 (dd, *J* = 2.4, 11.2 Hz, 1H), 7.36 (d, *J =* 9.6 Hz, 1H), 7.16 (d, *J* = 10.0 Hz, 1H), 6.59-6.53 (m, 1H), 4.62 (dd, *J* = 4.2, 11.8 Hz, 1H), 3.92 (s, 3H), 3.61-3.52 (m, 2H), 3.44-3.43 (m, 2H), 2.90-2.82 (m, 1H), 2.67-2.59 (m, 1H), 2.44-2.36 (m, 1H), 2.30-2.19 (m, 1H).

### Example 30

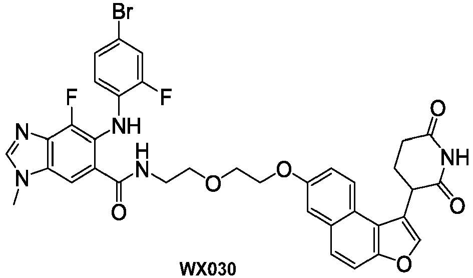

### Synthetic route:

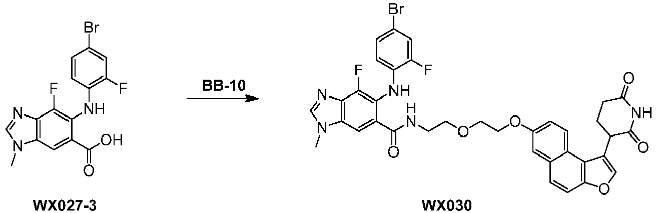

Intermediate **WX027-3** (50.00 mg, 130.84 µmol) was dissolved in dimethylformamide (10 mL) at room temperature. Subsequently, O-(7-azabenzotriazol-1-yl)-N,N,N,N-tetramethyluronium hexafluorophosphate (74.62 mg, 196.25 µmol) and N,N-diisopropylethylamine (84.55 mg, 654.18 µmol) were added sequentially. The reaction mixture was stirred to react at room temperature for half an hour, and intermediate **BB-10** (48.91 mg, 130.84 µmol, hydrochloride) was then added. The reaction mixture was stirred to react at room temperature for another 12 hours. After the reaction was completed, water (40 mL) was added to the reaction solution and the resulting mixture was extracted with ethyl acetate (20 mL×3). The organic phases were combined, washed with saturated brine (80 mL), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX030.** MS-ESI m/z: 746.2 [M+H]⁺, 748.2 [M+2+H]⁺. ¹H NMR (400 MHz, DMSO_*d*₆) δ: 10.93 (s, 1H), 8.83 (t, *J =* 5.6 Hz, 1H), 8.75 (s, 1H), 8.05 (d, *J =* 8.8 Hz, 1H), 7.96 (s, 1H), 7.93 (s, 1H), 7.75-7.68 (m, 2H), 7.50-7.42 (m, 2H), 7.19 (dd, *J* = 2.6, 9.4 Hz, 1H), 7.14 (dd, *J =* 1.2, 8.8 Hz, 1H), 6.55-6.49 (m, 1H), 4.64-4.60 (m, 1H), 4.23-4.13 (m, 2H), 3.93 (s, 3H), 3.83-3.73 (m, 2H), 3.59 (t, *J* = 5.8 Hz, 2H), 3.47-3.43 (m, 2H), 2.92-2.83 (m, 1H), 2.67-2.59 (m, 1H), 2.45-2.34 (m, 1H), 2.28-2.25 (m, 1H).

### Example 31

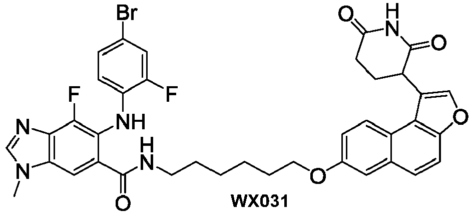

### Synthetic route:

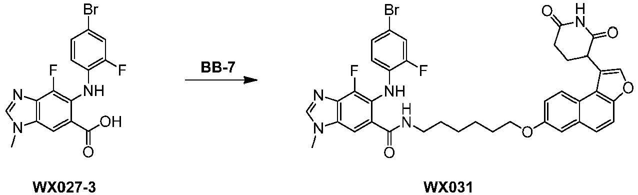

Intermediate **WX027-3** (50.00 mg, 130.84 µmol) was dissolved in dimethylformamide (10 mL) at room temperature. Subsequently, O-(7-azabenzotriazol-1-yl)-N,N,N,N-tetramethyluronium hexafluorophosphate (74.62 mg, 196.25 µmol) and N,N-diisopropylethylamine (84.55 mg, 654.18 µmol) were added sequentially. The reaction mixture was stirred to react at room temperature for half an hour, and intermediate **BB-7** (51.61 mg, 130.84 µmol, hydrochloride) was then added. The reaction mixture was stirred to react at room temperature for another 12 hours. After the reaction was completed, water (40 mL) was added to the reaction solution and the resulting mixture was extracted with ethyl acetate (20 mL×3). The organic phases were combined, washed with saturated brine (80 mL), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX031.** MS-ESI *m*/*z:* 758.2 [M+H]⁺, 760.2 [M+2+H]⁺. ¹H NMR (400 MHz, DMSO_*d*₆) δ: 10.93 (s, 1H), 8.68-8.65 (m, 2H), 8.06 (d, *J =* 8.8 Hz, 1H), 7.96 (s, 1H), 7.88 (s, 1H), 7.77-7.69 (m, 2H), 7.48-7.43 (m, 2H), 7.20 (dd, *J* = 2.8, 9.2 Hz, 1H), 7.14 (d, *J* = 8.4 Hz, 1H), 6.51-6.45 (m, 1H), 4.63 (dd, *J* = 4.4, 12.0 Hz, 1H), 4.07 (t, *J* = 6.4 Hz, 2H), 3.93 (s, 3H), 3.27-3.22 (m, 2H), 2.92-2.83 (m, 1H), 2.67-2.59 (m, 1H), 2.45-2.33 (m, 1H), 2.28-2.25 (m, 1H), 1.79-1.67 (m, 2H), 1.55-1.38 (m, 4H), 1.36-1.28 (m, 2H).

### Example 32

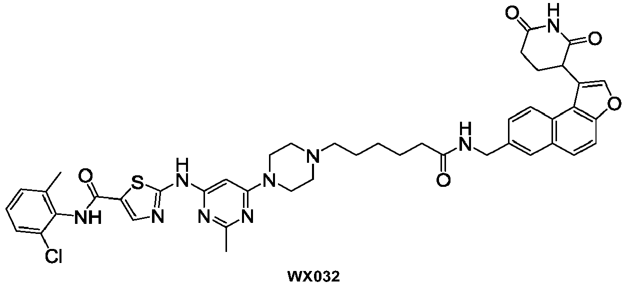

### Synthetic route:

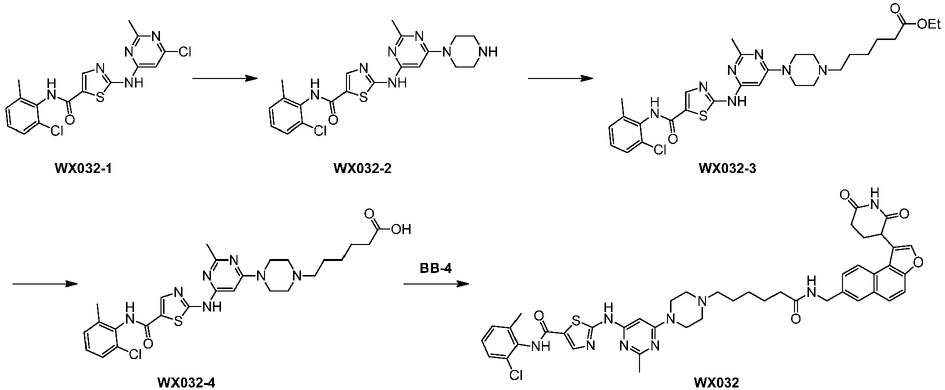

### Step 1: Synthesis of intermediate WX032-2

**WX032-1** (4 g, 10.15 mmol) and anhydrous piperazine (1.31 g, 15.22 mmol) were dissolved in anhydrous dioxane (40 mL) at room temperature, and diisopropylethylamine (3.53 mL, 20.29 mmol) was then added. The reaction mixture was heated to 100°C and stirred to react for 8 hours. After the reaction was completed, the mixture was cooled down to room temperature, and filtered. The resulting filter cake was added to ethyl acetate (50 mL), and the mixture was stirred at room temperature for 1 h, and filtered. The solvent was removed under reduced pressure to give the intermediate **WX032-2.** ¹H NMR (400 MHz, DMSO_*d*₆) δ: 9.89 (s, 1H), 8.23 (s, 1H), 7.40 (br d, *J =* 6.8 Hz, 1H), 7.33-7.22 (m, 2H), 6.05 (s, 1H), 3.53-3.47 (m, 4H), 2.85-2.79 (m, 4H), 2.41 (s, 3H), 2.24 (s, 3H).

### Step 2: Synthesis of intermediate WX032-3

Intermediate **WX032-2** (500 mg, 1.13 mmol) and ethyl 6-bromohexanoate (299.14 µL, 1.69 mmol) were dissolved in N,N-dimethylformamide (10 mL) at room temperature. Subsequently, sodium bicarbonate (189.23 mg, 2.25 mmol) and potassium iodide (93.48 mg, 563.12 µmol) were added sequentially. The reaction mixture was heated to 80°C and stirred to react for 8 hours. After the reaction was completed, the mixture was cooled down to room temperature. The reaction solution was added to saturated brine (120 mL) and extracted with ethyl acetate (40 mL×4). The organic phases were combined, washed with saturated brine (200 mL), dried over anhydrous sodium sulfate, and filtered. The filtrate was concentrated under reduced pressure to remove the solvent. Petroleum ether (30 mL) and ethyl acetate (5 mL) were added to the resulting residue, and the mixture was stirred at room temperature for 30 min, and filtered. The solvent was removed under reduced pressure to give the intermediate **WX032-3.**

### Step 3: Synthesis of intermediate WX032-4

**WX032-3** (440 mg, 751.93 µmol) was dissolved in methanol (10 mL) at room temperature. Subsequently, sodium hydroxide (150.38 mg, 3.76 mmol) and water (10 mL) were added sequentially. The reaction mixture was heated to 80°C and stirred to react for 2 hours. After the reaction was completed, the mixture was cooled down to room temperature. Saturated brine (40 mL) was added to the reaction solution, and the pH was adjusted to 5 with solid citric acid trihydrate. The mixture was extracted with ethyl acetate (20 mL×4). The organic phases were combined, washed with saturated brine (100 mL×2), dried over anhydrous sodium sulfate, and filtered. The filtrate was concentrated under reduced pressure to remove the solvent to give the intermediate **WX032-4.**

### Step 4: Synthesis of WX032

**WX032-4** (60 mg, 107.51 µmol) and diisopropylethylamine (74.90 µL, 430.04 µmol) were dissolved in N,N-dimethylformamide (2 mL) at room temperature, and O-(7-azabenzotriazol-1-yl)-N,N,N,N-tetramethyluronium hexafluorophosphate (44.97 mg, 118.26 µmol) was then added. The reaction mixture was stirred to react at room temperature for 1 hour, and then intermediate **BB-4** (40.77 mg, 118.26 µmol, hydrochloride) was added. The reaction mixture was stirred to react at room temperature for another 2 hours. After the reaction was completed, the reaction solution was diluted to 6 mL with methanol, and filtered to remove insoluble matter. The filtrate was directly separated by preparative HPLC (mobile phase: acetonitrile/water; neutral system: 0.05% NH₄HCO₃) to give the title compound **WX032.** MS-ESI *m*/*z:* 848.4 [M+H]⁺. ¹H NMR (400 MHz, DMSO_*d*₆) δ: 11.47 (br s, 1H), 10.95 (s, 1H), 9.88 (br s, 1H), 8.42 (t, *J* = 5.6 Hz, 1H), 8.22 (s, 1H), 8.13 (br d, *J* = 8.0 Hz, 1H), 7.99 (s, 1H), 7.87 (s, 1H), 7.83-7.70 (m, 2H), 7.48 (br d, *J* = 8.0 Hz, 1H), 7.40 (br d, *J* = 7.2 Hz, 1H), 7.34-7.20 (m, 2H), 6.04 (br s, 1H), 4.66 (br dd, *J* = 3.8, 11.8 Hz, 1H), 4.44 (br d, *J* = 5.6 Hz, 2H), 3.60-3.40 (m, 2H), 2.96-2.79 (m, 2H), 2.69-2.55 (m, 3H), 2.43-2.27 (m, 10H), 2.24 (s, 3H), 2.19 (br t, *J* = 7.0 Hz, 2H), 1.63-1.54 (m, 2H), 1.52-1.41 (m, 2H), 1.35-1.25 (m, 2H).

### Example 33

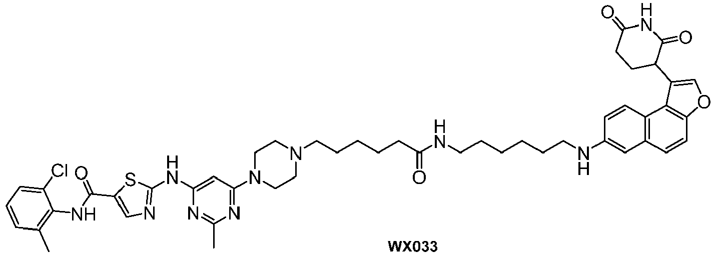

### Synthetic route:

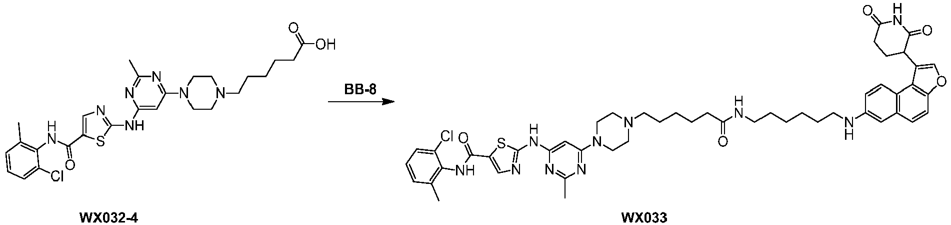

Intermediate **WX032-4** (70 mg, 125.43 µmol) and diisopropylethylamine (87.39 µL, 501.71 µmol) were dissolved in N,N-dimethylformamide (2 mL) at room temperature, and O-(7-azabenzotriazol-1-yl)-N,N,N,N-tetramethyluronium hexafluorophosphate (52.46 mg, 137.97 µmol) was then added. The reaction mixture was stirred to react at room temperature for 1 hour, and then Intermediate **BB-8** (59.32 mg, 137.97 µmol, hydrochloride) was added. The reaction mixture was stirred to react at room temperature for another 2 hours. After the reaction was completed, the reaction solution was filtered to remove insoluble matter. The filtrate was diluted to 5 mL with methanol, and then directly separated by preparative HPLC (mobile phase: acetonitrile/water; neutral system: 0.05% NH₄HCO₃) to give the title compound **WX033.** MS-ESI *m*/*z:* 933.5 [M+H]⁺. ¹H NMR (400 MHz, DMSO_*d*₆) δ: 11.47 (br s, 1H), 10.93 (s, 1H), 9.88 (s, 1H), 8.22 (s, 1H), 7.90-7.80 (m, 2H), 7.75 (br t, *J* = 5.8 Hz, 1H), 7.60-7.48 (m, 2H), 7.40 (br d, *J* = 7.6 Hz, 1H), 7.32-7.21 (m, 2H), 7.02 (dd, *J* = 2.0, 9.2 Hz, 1H), 6.88 (d, *J =* 2.0 Hz, 1H), 6.04 (s, 1H), 5.75 (br t, *J* = 5.0 Hz, 1H), 4.56 (br dd, *J* = 4.2, 11.8 Hz, 1H), 3.60-3.43 (m, 3H), 3.14-2.97 (m, 4H), 2.91-2.79 (m, 1H), 2.70-2.56 (m, 2H), 2.45-2.36 (m, 6H), 2.35-2.30 (m, 2H), 2.30-2.18 (m, 6H), 2.05 (br t, *J* = 7.4 Hz, 2H), 1.67-1.57 (m, 2H), 1.55-1.47 (m, 2H), 1.46-1.36 (m, 6H), 1.35-1.29 (m, 2H), 1.27-1.18 (m, 2H).

### Example 34

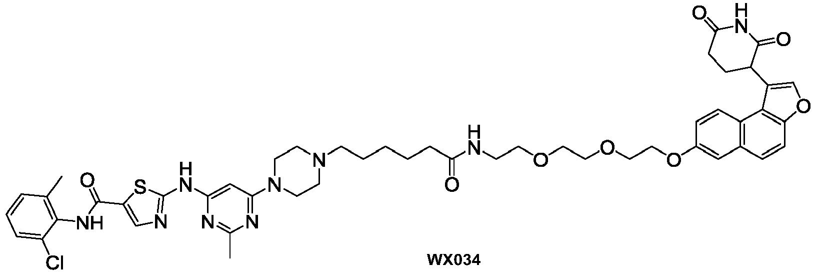

### Synthetic route:

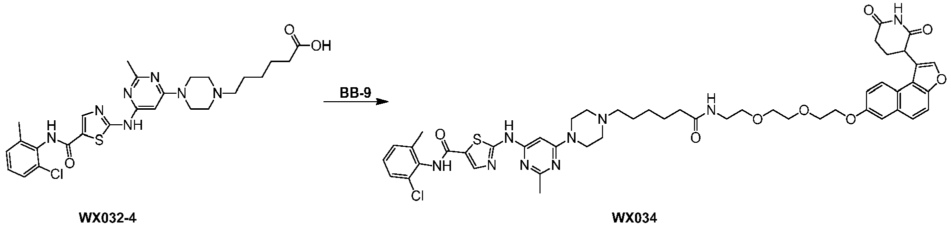

Intermediate **WX032-4** (70 mg, 125.43 µmol) and diisopropylethylamine (87.39 µL, 501.71 µmol) were dissolved in N,N-dimethylformamide (2 mL) at room temperature, and O-(7-azabenzotriazol-1-yl)-N,N,N,N-tetramethyluronium hexafluorophosphate (52.46 mg, 137.97 µmol) was then added. The reaction mixture was stirred to react at room temperature for 1 hour, and then Intermediate **BB-9** (63.87 mg, 137.97 µmol, hydrochloride) was added. The reaction mixture was stirred to react at room temperature for another 2 hours. After the reaction was completed, the reaction solution was filtered to remove insoluble matter. The filtrate was diluted to 5 mL with methanol, and then directly separated by preparative HPLC (mobile phase: acetonitrile/water; neutral system: 0.05% NH₄HCO₃) to give the title compound **WX034.** MS-ESI *m*/*z:* 966.4 [M+H]⁺. ¹H NMR (400 MHz, DMSO_*d*₆) δ: 11.48 (br s, 1H), 10.94 (s, 1H), 9.89 (br s, 1H), 8.22 (s, 1H), 8.09 (br d, *J =* 9.2 Hz, 1H), 7.96 (s, 1H), 7.84 (t, *J =* 5.0 Hz, 1H), 7.77-7.68 (m, 2H), 7.51 (br d, *J* = 2.0 Hz, 1H), 7.40 (br d, *J* = 7.2 Hz, 1H), 7.33-7.19 (m, 3H), 6.04 (br s, 1H), 4.63 (dd, *J* = 4.0, 12.0 Hz, 1H), 4.28-4.15 (m, 2H), 3.87-3.75 (m, 2H), 3.66-3.59 (m, 2H), 3.58-3.52 (m, 2H), 3.52-3.45 (m, 3H), 3.45-3.39 (m, 4H), 3.23-3.12 (m, 2H), 2.96-2.81 (m, 1H), 2.70-2.57 (m, 2H), 2.43-2.35 (m, 6H), 2.35-2.19 (m, 6H), 2.06 (br t, *J* = 7.2 Hz, 2H), 1.59-1.34 (m, 4H), 1.30-1.13 (m, 2H).

### Example 35

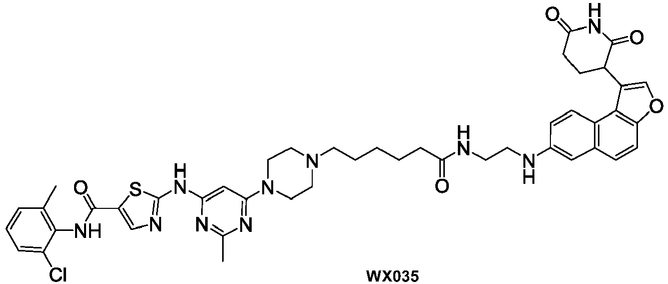

### Synthetic route:

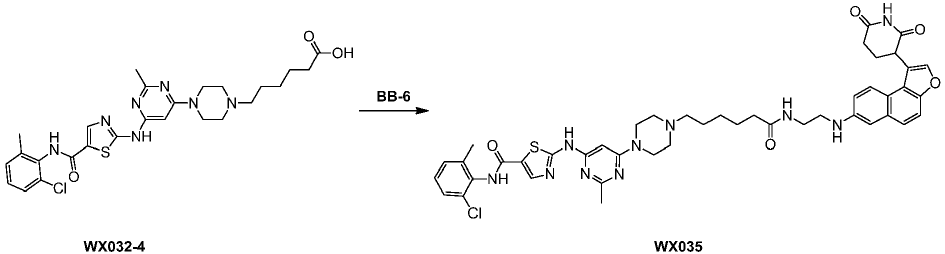

Intermediate **WX032-4** (70 mg, 125.43 µmol) and diisopropylethylamine (87.39 µL, 501.71 µmol) were dissolved in N,N-dimethylformamide (2 mL) at room temperature, and O-(7-azabenzotriazol-1-yl)-N,N,N,N-tetramethyluronium hexafluorophosphate (52.46 mg, 137.97 µmol) was then added. The reaction mixture was stirred to react at room temperature for 1 hour, and then Intermediate **BB-6** (51.58 mg, 137.97 µmol, hydrochloride) was added. The reaction mixture was stirred to react at room temperature for another 2 hours. After the reaction was completed, the reaction solution was filtered to remove insoluble matter. The filtrate was diluted to 5 mL with methanol, and then directly separated by preparative HPLC (mobile phase: acetonitrile/water; acidic system: 0.05% HCl) to give the title compound **WX035.** MS-ESI *m*/*z*: 877.4 [M+H]⁺. ¹H NMR (400 MHz, DMSO_*d*₆) δ: 10.94 (s, 1H), 9.97 (s, 1H), 8.26 (s, 1H), 8.12 (br t, *J* = 5.0 Hz, 1H), 8.03 (br d, *J* = 8.0 Hz, 1H), 7.94 (s, 1H), 7.73-7.65 (m, 2H), 7.45-7.36 (m, 2H), 7.32-7.22 (m, 3H), 6.16 (s, 1H), 4.60 (br dd, *J* = 4.0, 11.6 Hz, 1H), 4.34 (br d, *J* = 12.0 Hz, 2H), 3.37-3.28 (m, 4H), 3.10-2.93 (m, 4H), 2.92-2.79 (m, 1H), 2.70-2.57 (m, 1H), 2.45 (s, 3H), 2.41-2.31 (m, 2H), 2.30-2.19 (m, 5H), 2.13 (br t, *J* = 7.2 Hz, 2H), 1.74-1.61 (m, 2H), 1.59-1.48 (m, 2H), 1.35-1.19 (m, 4H).

### Example 36

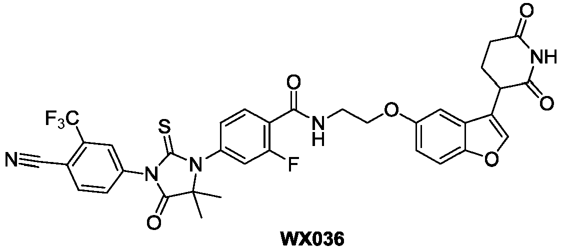

### Synthetic route:

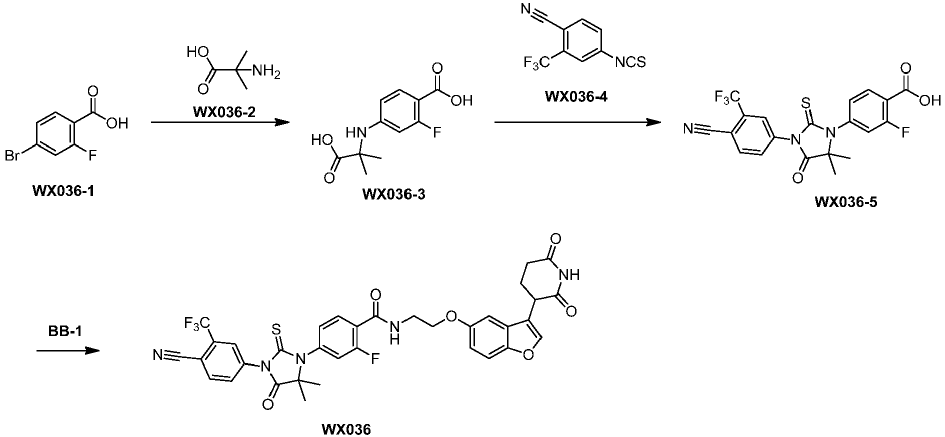

### Step 1: Synthesis of intermediate WX036-3

**WX036-1** (10 g, 45.66 mmol) and compound **WX036-2** (14.13 g, 136.98 mmol) were added to a mixture of N,N-dimethylformamide (100 mL) and water (10 mL), and cuprous iodide (1.74 g, 9.14 mmol), copper powder (580.00 mg, 9.13 mmol), potassium carbonate (31.55 g, 228.30 mmol) and N,N-dimethylglycine (2.35 g, 22.83 mmol) were slowly added to the reaction sequentially. The mixture was purged three times with nitrogen and then heated to 110°C and stirred for 12 hours. After the reaction was completed, the reaction solution was cooled down to room temperature. To the mixture was added 200 mL of ice water. The mixture was adjusted to pH of 4-5 with 6 M hydrochloric acid, and extracted with ethyl acetate (500 mL x 2). The combined organic phases were washed with saturated brine (300 mL x 2), dried over anhydrous sodium sulfate, and filtered. The filtrate was rotary-evaporated to dryness under reduced pressure. The crude product was added to 15 mL of dichloromethane and the mixture was stirred for 1 h. The mixture was filtered, and the filter cake was washed with dichloromethane (5 mL x 2). The solid was collected and rotary-evaporated to dryness under reduced pressure to give the title compound **WX036-3.** ¹H NMR (400MHz, DMSO-*d*₆) δ: 12.56 (br s, 1H), 7.60 (t, J = 8.9 Hz, 1H), 6.96 (br s, 1H), 6.34 (dd, J = 2.3, 8.8 Hz, 1H), 6.16 (dd, J = 2.0, 14.6 Hz, 1H), 1.46 (s, 6H).

### Step 2: Synthesis of intermediate WX036-5

Intermediate **WX036-3** (0.1 g, 414.57 µmol) and compound **WX036-4** (141.90 mg, 621.85 µmol) were added to ethanol (2 mL), and triethylamine (142.63 mg, 1.41 mmol, 196.19 µL) was slowly added to the reaction mixture. The mixture was stirred at 80 °C for 12 h. After the reaction was completed, the reaction solution was cooled to room temperature, and rotary-evaporated to dryness under reduced pressure to remove the solvent. 20 mL of water was added, and the mixture was adjusted to pH of 3-4 with 1 M hydrochloric acid. The mixture was extracted with ethyl acetate (20 mL x 2), washed with saturated brine (20 mL x 2), dried over anhydrous sodium sulfate, and filtered. The filtrate was rotary-evaporated to dryness. The crude product was purified by column chromatography (eluent: dichloromethane:methanol = 1:0-10:1, v/v) to give the title compound **WX036-5.**

MS-ESI *m*/*z:* 452.1 [M+H]⁺. ¹H NMR (400 MHz, DMSO_*d*₆) δ: 10.04 (s, 1H), 8.41 (d, *J* = 8.3 Hz, 1H), 8.30 (d, *J* = 1.3 Hz, 1H), 8.11 - 8.05 (m, 2H), 7.47 (dd, *J* = 1.6, 11.2 Hz, 1H), 7.38 (dd, *J* = 1.8, 8.3 Hz, 1H), 1.56 (s, 6H).

### Step 3: Synthesis of Example WX036

Intermediate **WX036-5** (150 mg, 235.17 µmol) and intermediate **BB-1(81.36** mg, 282.21 µmol) were added to *N*,*N*-dimethylformamide (2 mL), and O-(7-azabenzotriazol-1-yl)-N,N,N,N-tetramethyluronium hexafluorophosphate(178.84 mg, 470.34 µmol) and triethylamine (71.39 mg, 705.51 µmol, 98.20 µL) were slowly added to the reaction mixture. The reaction solution was stirred at 20°C under nitrogen for 12 hours. After the reaction was completed, 20 mL of water and 30 mL of ethyl acetate were added to the reaction solution and the organic phase was separated. The organic phase was washed with water (20 mL x 2), and the combined organic phases were dried over anhydrous sodium sulfate, and filtered. The filtrate was rotary-evaporated to dryness under reduced pressure to give the crude product. The crude product was separated by preparative HPLC (mobile phase: acetonitrile/water; acidic system: 0.05% HCl) to give the title compound **WX036.** MS-ESI m/z: 722.1 [M+H]⁺. ¹H NMR (400 MHz, DMSO_*d*₆) δ: 10.88 (s, 1H), 8.76 (br t, *J* = 5.1 Hz, 1H), 8.41 (d, *J* = 8.3 Hz, 1H), 8.30 (d, *J* = 1.8 Hz, 1H), 8.09 (dd, *J* = 1.8, 8.3 Hz, 1H), 7.86 (s, 1H), 7.80 (t, *J* = 8.0 Hz, 1H), 7.51 - 7.41 (m, 2H), 7.34 (dd, J = 1.8, 8.3 Hz, 1H), 7.16 (d, *J* = 2.5 Hz, 1H), 6.99 - 6.91 (m, 1H), 4.19-4.07 (m, 3H), 3.68 (q, J = 5.5 Hz, 2H), 2.80 - 2.66 (m, 1H), 2.63-2.53 (m, 1H), 2.43-2.29 (m, 1H), 2.15-2.04 (m, 1H), 1.55 (s, 6H).

### Example 37

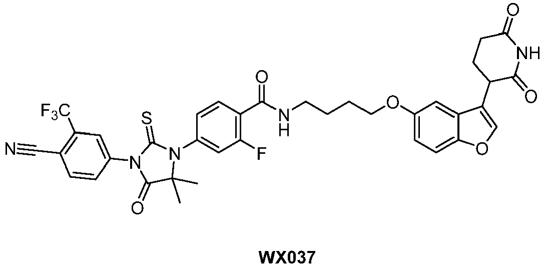

### Synthetic route:

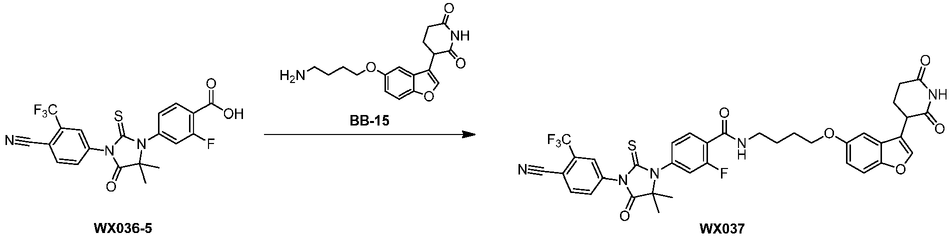

Intermediate **WX036-5** (150 mg, 277.11 µmol) and intermediate **BB-15** (332.53 µmol, hydrochloride) were added to *N*,*N*-dimethylformamide (2 mL), and O-(7-azabenzotriazol-1-yl)-N,N,N,N-tetramethyluronium hexafluorophosphate (210.73 mg, 554.22 µmol) and triethylamine (84.12 mg, 831.33 µmol, 115.71 µL) were slowly added to the reaction mixture. The reaction solution was stirred at 20°C under nitrogen for 12 hours. After the reaction was completed, 20 mL of water and 30 mL of ethyl acetate were added to the reaction solution and the organic phase was separated. The organic phase was washed with water (20 mL x 2), and the combined organic phases were dried over anhydrous sodium sulfate, and filtered. The filtrate was rotary-evaporated to dryness under reduced pressure to give the crude product. The crude product was separated by preparative HPLC (mobile phase: acetonitrile/water; acidic system: 0.05% HCl) to give the title compound **WX037.** MS-ESI *m*/*z:* 750.2[M+H]⁺. ¹H NMR (400 MHz, DMSO_*d*₆) δ: 10.89 (s, 1H), 8.59 (t, *J =* 5.5 Hz, 1H), 8.41 (d, *J* = 8.3 Hz, 1H), 8.30 (d, *J* = 1.8 Hz, 1H), 8.09 (d, *J* = 8.3 Hz, 1H), 7.89-7.82 (m, 1H), 7.77 (t, *J* = 8.0 Hz, 1H), 7.51-7.40 (m, 2H), 7.33 (dd, *J* = 1.8, 8.0 Hz, 1H), 7.12 (d, *J* = 2.5 Hz, 1H), 6.92 (dd, *J* = 2.4, 8.9 Hz, 1H), 4.12 (dd, *J* = 4.8, 11.8 Hz, 1H), 4.06 - 3.98 (m, 2H), 2.81-2.64 (m, 1H), 2.63-2.52 (m, 3H), 2.35 (dq, *J* = 4.5, 12.4 Hz, 1H), 2.15-2.07 (m, 1H), 1.86-1.76 (m, 2H), 1.75-1.65 (m, 2H), 1.55 (s, 6H).

### Example 38

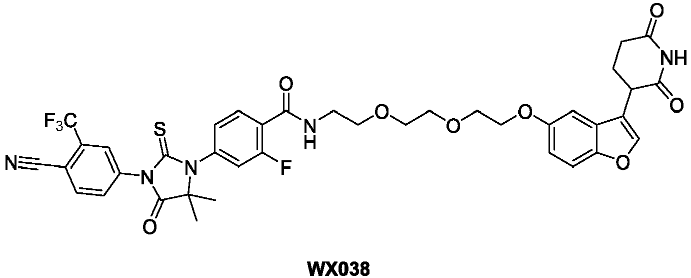

### Synthetic route:

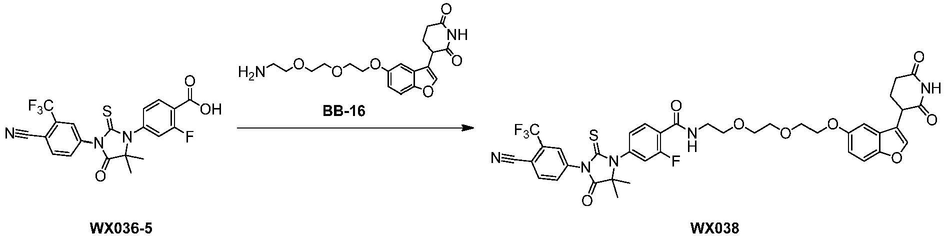

Intermediate **WX036-5** (150 mg, 277.11 µmol) and intermediate **BB-16**(125.17 mg, 332.53 µmol) were added to DMF (2 mL), and O-(7-azabenzotriazol-1-yl)-N,N,N,N-tetramethyluronium hexafluorophosphate(210.73 mg, 554.22 µmol) and triethylamine (84.12 mg, 831.33 µmol, 115.71 µL) were slowly added to the reaction mixture. The reaction solution was stirred at 20°C under nitrogen for 12 hours. After the reaction was completed, 20 mL of water and 30 mL of ethyl acetate were added to the reaction solution and the organic phase was separated. The organic phase was washed with water (20 mL x 2), and the combined organic phases were dried over anhydrous sodium sulfate, and filtered. The filtrate was rotary-evaporated to dryness under reduced pressure to give the crude product. The crude product was separated by preparative HPLC (mobile phase: acetonitrile/water; acidic system: 0.05% HCl) to give the title compound **WX038.** MS-ESI *m*/*z:* 810.2[M+H]⁺. ¹H NMR (400 MHz, DMSO_*d*₆) δ: 10.88 (s, 1H), 8.60 - 8.49 (m, 1H), 8.41 (d, *J* = 8.0 Hz, 1H), 8.30 (d, *J* = 1.8 Hz, 1H), 8.09 (dd, *J* = 1.5, 8.3 Hz, 1H), 7.85 (s, 1H), 7.78 (t, *J* = 8.0 Hz, 1H), 7.51-7.37 (m, 2H), 7.35-7.26 (m, 1H), 7.16-7.10 (m, 1H), 6.92 (dd, *J* = 2.5, 8.8 Hz, 1H), 4.10 (sxt, *J* = 5.3 Hz, 3H), 3.77 (t, *J* = 4.6 Hz, 2H), 3.66-3.52 (m, 6H), 3.44 (q, *J* = 5.9 Hz, 2H), 2.82 - 2.65 (m, 1H), 2.62-2.54 (m, 1H), 2.35 (dq, *J* = 4.4, 12.5 Hz, 1H), 2.16-1.96 (m, 1H), 1.53 (s, 6H).

### Example 39

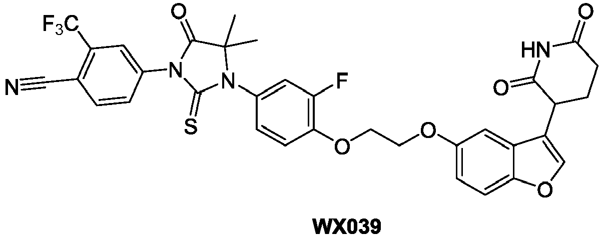

### Synthetic route:

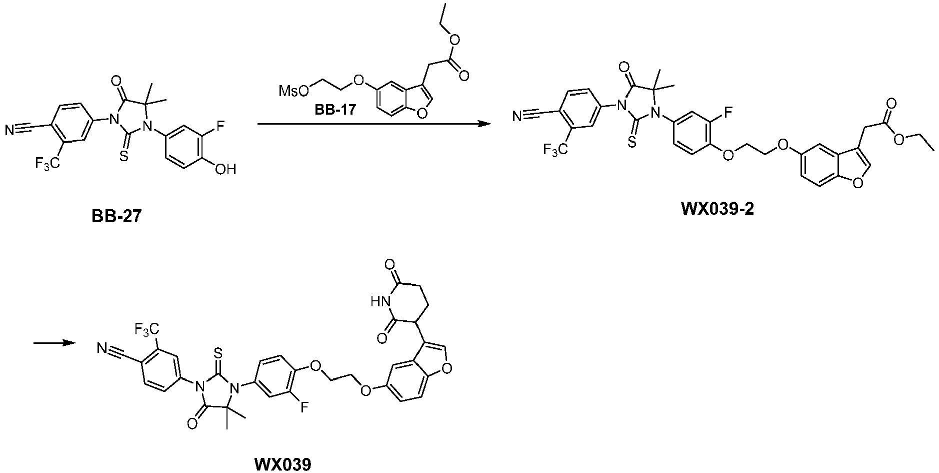

### Step 1: Synthesis of compound WX039-2

Intermediate **BB-27** (360 mg, 1.05 mmol) was dissolved in *N*,*N*-dimethylformamide (8 mL) at room temperature under nitrogen, and intermediate **BB-17**(404.72 mg, 955.92 µmol) and potassium carbonate (396.34 mg, 2.87 mmol) were added. The reaction system was stirred to react at 50 °C for 12 hours. After the reaction was completed, the reaction mixture was cooled to room temperature. The mixture was diluted with saturated brine (20 mL) and ethyl acetate (30 mL), and the layers were separated. The organic phase was collected, and the aqueous phase was extracted with ethyl acetate (30 mL x 3). The organic phases were combined, washed with saturated brine (30 mL x 3), dried over anhydrous sodium sulfate, and filtered. The filtrate was concentrated under reduced pressure to remove the solvent. The resulting residue was separated by column chromatography (eluent: petroleum ether/ethyl acetate = 1/0-2/3, v/v) to give the crude product. The crude product was further purified by thin layer chromatography on silica gel plate (developing solvent: petroleum ether:ethyl acetate = 1:1) to give the intermediate **WX039-2.** ¹H NMR (400 MHz, DMSO_*d*₆) *δ:* 8.39 (d, *J* = 8.0 Hz, 1H), 8.28 (s, 1H), 8.07 (d, *J* = 6.8 Hz, 1H), 7.87 (s, 1H), 7.57-7.38 (m, 2H), 7.34 (dd, *J* = 2.4, 11.6 Hz, 1H), 7.26-7.15 (m, 2H), 6.96 (dd, *J* = 2.4, 8.8 Hz, 1H), 4.52-4.50 (m, 2H), 4.40-4.38 (m, 2H), 4.18-4.05 (m, 2H), 3.76 (s, 2H), 1.52 (s, 6H), 1.20 (t, *J* = 7.2 Hz, 3H).

### Step 2: Synthesis of compound WX039

Intermediate **WX039-2** (136 mg, 203.09 µmol) was dissolved in dry anhydrous tetrahydrofuran (3 mL) at room temperature under nitrogen. Subsequently, acrylamide (28.87 mg, 406.19 µmol) and a solution of potassium tert-butoxide in tetrahydrofuran (1 M, 406.19 µL) were added sequentially. The reaction mixture was stirred to react at 25°C for 1 hour. After the reaction was completed, the reaction solution was diluted with saturated brine (20 mL) and 2-methyltetrahydrofuran (20 mL), and the layers were separated. The aqueous phase was extracted with 2-methyltetrahydrofuran (30 mL x 3). The organic phases were sequentially combined, washed with saturated brine (20 mL x 2), dried over anhydrous sodium sulfate, and filtered. The filtrate was concentrated under reduced pressure to remove the solvent. The resulting residue was separated by preparative HPLC (mobile phase: acetonitrile/water; acidic system: HCl) to give the title compound **WX039.** MS-ESI *m*/*z:* 695.2 [M+H]⁺. ¹H NMR (400MHz, DMSO_*d*₆) δ: 10.88 (s, 1H), 8.39 (d, *J* = 8.0 Hz, 1H), 8.28 (s, 1H), 8.07 (d, *J* = 7.6 Hz, 1H), 7.86 (s, 1H), 7.55-7.38 (m, 2H), 7.34 (dd, *J* = 2.0, 11.8 Hz, 1H), 7.20 (d, *J* = 2.4 Hz, 2H), 6.96 (dd, *J* = 2.0, 8.8 Hz, 1H), 4.61-4.28 (m, 4H), 4.11 (dd, *J* = 4.8, 12.0 Hz, 1H), 2.79-2.65 (m, 1H), 2.62-2.53 (m, 1H), 2.43-2.30 (m, 1H), 2.16-2.06 (m, 1H), 1.52 (s, 6H).

### Example 40

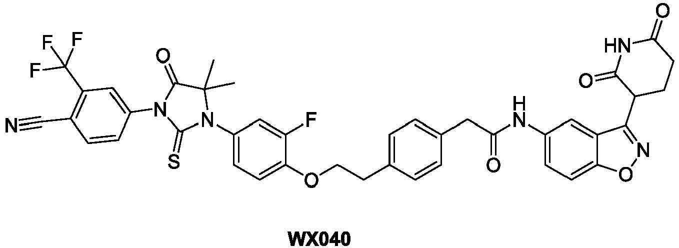

### Synthetic route:

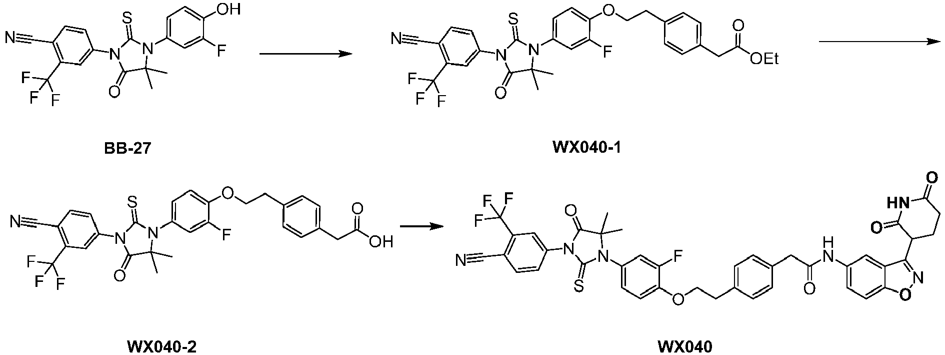

### Step 1: Synthesis of compound WX040-1

Intermediate **BB-27** (479.06 mg, 1.13 mmol) and ethyl 2-[4-(2-methylsulfonyloxyethyl)phenyl]acetate (0.27 g, 942.92 µmol, 1 eq) were added to dimethyl sulfoxide (5 mL), and potassium carbonate (260.64 mg, 1.89 mmol) was slowly added to the reaction. The mixture was stirred at 80°C under nitrogen for 10 h. After the reaction was completed, the mixture was diluted with ethyl acetate (50 mL). The organic phase was washed with water (50 mL x 2) and saturated brine (30 mL), dried over anhydrous sodium sulfate, and filtered. The filtrate was rotary-evaporated under reduced pressure. The crude product was purified by column chromatography (eluent: petroleum ether:ethyl acetate = 1:0-3:1, v/v) to give the title compound **WX040-1.** ¹H NMR (400MHz, CDCl₃) δ: 8.14-7.93 (m, 2H), 7.85 (dd, *J* = 1.8, 8.3 Hz, 1H), 7.35 - 7.22 (m, 4H), 7.14-6.98 (m, 3H), 4.22-4.08 (m, 2H), 3.62 (s, 2H), 3.17 (t, *J* = 6.9 Hz, 2H), 1.59 (s, 6H), 1.28 (t, *J* = 7.2 Hz, 5H).

### Step 2: Synthesis of compound WX040-2

**WX040-1** (0.16 g, 225.21 µmol) was added to ethanol (3 mL) and water (0.3 mL), and lithium hydroxide monohydrate (18.90 mg, 450.41 µmol) was slowly added to the reaction mixture. The mixture was purged three times with nitrogen and then stirred at 25°C for 10 h. Most of the organic solvent was rotary-evaporated under reduced pressure, and 20 mL of water was added. The aqueous phase was adjusted to pH of 2 with 2 M hydrochloric acid, and extracted with ethyl acetate (50 mLx2). The combined organic phases were washed with saturated brine (30 mL), dried over anhydrous sodium sulfate, and filtered. The filtrate was rotary-evaporated under reduced pressure. The crude product was separated by column chromatography (eluent: dichloromethane:methanol = 100:1-20:1, v/v) to give the compound **WX040-2.** MS-ESI *m*/*z:* 586.3 [M+H]⁺.

### Step 3: Synthesis of compound WX040

**WX040-2** (23.37 mg, 85.39 µmol) and 3-(5-aminobenzo[d]isoxazol-3-yl)piperidine-2,6-dione (23.37 mg, 85.39 µmol) were added to N,N-dimethylformamide (2 mL), and triethylamine (17.28 mg, 170.77 µmol, 23.77 µL) and O-(7-azabenzotriazol-1-yl)-N,N,N,N-tetramethyluronium hexafluorophosphate (48.70 mg, 128.08 µmol) were slowly added to the reaction mixture. The mixture was purged with nitrogen 3 times and stirred at 25°C for 2 hours. After the reaction was completed, the reaction solution was poured into 20 mL of water and 30 mL of ethyl acetate. The organic phase was washed with water (20 mL x 2), dried over anhydrous sodium sulfate, and filtered. The filtrate was rotated-evaporated to dryness under reduced pressure. The crude product was purified by preparative HPLC (mobile phase: acetonitrile/water; acidic system: HCl) to give the title compound **WX040.** MS-ESI *m*/*z:* 813.2 [M+H]⁺. ¹H NMR (400MHz, DMSO-*d₆*) δ: 10.80 (s, 1H), 10.08 (s, 1H), 8.39 (d, *J* = 8.3 Hz, 1H), 8.28 (d, *J* = 1.5 Hz, 1H), 8.06 (dd, *J* = 1.8, 8.3 Hz, 1H), 7.41 - 7.21 (m, 6H), 7.15 (br d, *J* = 8.5 Hz, 1H), 6.81 - 6.64 (m, 2H), 6.20 (dd, *J* = 1.9, 11.9 Hz, 1H), 4.39 - 4.21 (m, 3H), 3.59 (s, 2H), 3.08 (br t, *J* = 6.9 Hz, 2H), 2.79 - 2.65 (m, 1H), 2.64 - 2.53 (m, 1H), 2.17 - 2.00 (m, 1H), 1.97 - 1.81 (m, 1H), 1.50 (s, 6H).

### Example 41

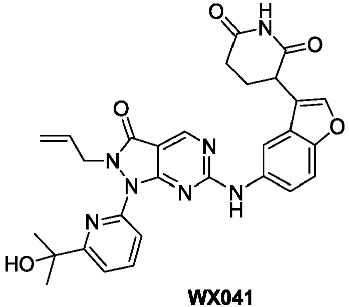

### Synthetic route:

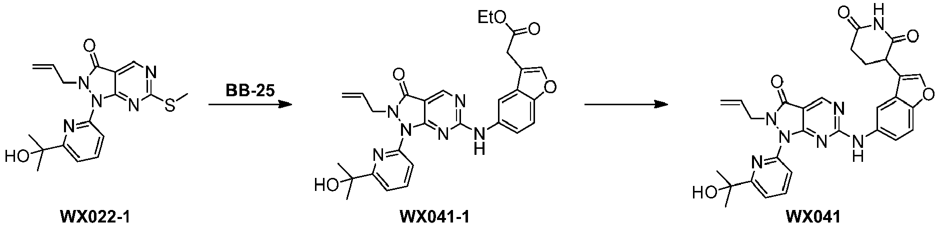

### Step 1: Synthesis of intermediate WX041-1

**WX022-1** (250.00 mg, 699.44 µmol) was dissolved in toluene (20 mL) at room temperature, and m-chloroperoxybenzoic acid (181.05 mg, 839.33 µmol, purity: 80%) was then added. The reaction solution was stirred at 20 °C for 1 h. Subsequently, N,N-diisopropylethylamine (451.98 mg, 3.50 mmol) and intermediate **BB-25** (196.73 mg, 769.38 µmol, hydrochloride) were added to the above reaction solution. The reaction mixture was stirred at 20 °C under nitrogen for another 12 h. After the reaction was completed, water (50 mL) was added to the reaction solution and the mixture was extracted with ethyl acetate (50 mL×3). The organic phases were sequentially combined, washed with saturated brine (50 mL×3), dried over anhydrous sodium sulfate, and filtered. The filtrate was concentrated under reduced pressure to remove the solvent. The resulting residue was separated by column chromatography (eluent: petroleum ether/ethyl acetate = 511-111, v/v) to give the intermediate **WX041-1.** ¹H NMR (400 MHz, CDCl₃) δ: 8.88 (s, 1H), 7.88 (t, *J* = 7.8 Hz, 1H), 7.80-7.74 (m, 2H), 7.69 (s, 1H), 7.46 (s, 2H), 7.36 (d, *J* = 8.0 Hz, 1H), 5.77-5.66 (m, 1H), 5.05 (d, *J* = 10.8 Hz, 1H), 4.94 (d, *J* = 16.8 Hz, 1H), 4.76 (d, *J* = 6.4 Hz, 2H), 4.20 (q, *J* = 7.2 Hz, 2H), 3.68 (s, 2H), 1.59 (s, 6H), 1.28 (t, *J* = 7.0 Hz, 3H).

### Step 2: Synthesis of WX041

**WX041-1** (280.00 mg, 529.74 µmol) was dissolved in N,N-dimethylformamide (20 mL) at 0°C under nitrogen. To the mixture were added separately potassium tert-butoxide (65.39 mg, 582.72 µmol) and acrylamide (37.65 mg, 529.74 µmol). The reaction mixture was stirred to react at 0°C for 1 h. After the reaction was completed, water (30 mL) was added to the reaction solution and the resulting mixture was extracted with ethyl acetate (30 mL×3). The organic phases were sequentially combined, washed with saturated brine (30 mL×3), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX041.** MS-ESI *m*/*z:* 554.1 [M+H]⁺. ¹H NMR (400 MHz, DMSO_*d*₆) δ: 10.93 (s, 1H), 10.25 (br s, 1H), 8.87 (s, 1H), 7.99-7.86 (m, 2H), 7.78 (s, 1H), 7.74 (d, *J* = 7.2 Hz, 1H), 7.67 (d, *J* = 8.8 Hz, 1H), 7.60-7.53 (m, 2H), 5.72-5.57 (m, 1H), 4.99 (d, *J* = 10.0 Hz, 1H), 4.82 (d, *J* = 16.8 Hz, 1H), 4.68 (d, *J =* 5.6 Hz, 2H), 4.08-4.03 (m, 1H), 2.81-2.67 (m, 1H), 2.64-2.53 (m, 1H), 2.38-2.09 (m, 2H), 1.44 (s, 6H).

### Example 42

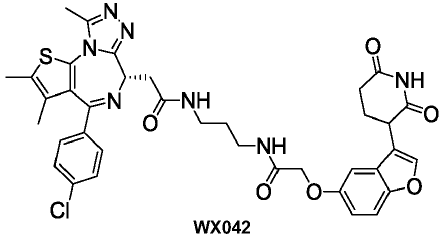

### Synthetic route:

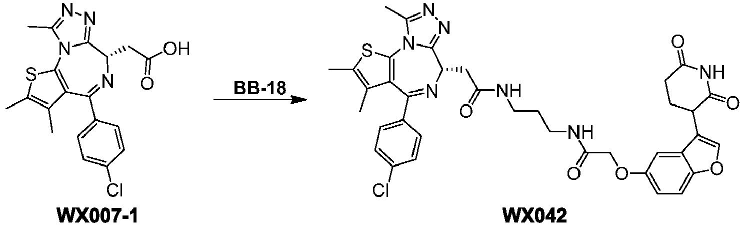

**WX007-1** (50.00 mg, 124.73 µmol) was dissolved in N,N-dimethylformamide (10 mL) at room temperature, and N,N-diisopropylethylamine (80.60 mg, 623.63 µmol) and O-(7-azabenzotriazol-1-yl)-N,N,N,N-tetramethyluronium hexafluorophosphate(71.14 mg, 187.09 µmol) were added separately. The mixture solution was stirred at room temperature for 30 min. Intermediate **BB-18** (49.37 mg, 124.73 µmol, hydrochloride) was then added. The reaction mixture was stirred to react at room temperature for another 12 hours. After the reaction was completed, water (40 mL) was added to the reaction solution and the resulting mixture was extracted with ethyl acetate (40 mL×3). The organic phases were sequentially combined, washed with saturated brine (40 mL×2), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX042.** MS-ESI *m*/*z:* 742.5 [M+H]⁺. ¹H NMR (400 MHz, DMSO_*d*₆) δ: 10.88 (s, 1H), 8.25 (t, *J* = 5.2 Hz, 1H), 8.16 (t, *J* = 5.6 Hz, 1H), 7.86 (s, 1H), 7.51-7.37 (m, 5H), 7.17 (d, *J* = 2.0 Hz, 1H), 7.00 (dd, *J* = 2.4, 8.8 Hz, 1H), 4.56-4.50 (m, 1H), 4.49-4.39 (m, 2H), 4.09 (dd, *J* = 4.8, 12.0 Hz, 1H), 3.31-3.05 (m, 6H), 2.79-2.64 (m, 1H), 2.60 (s, 3H), 2.58-2.55 (m, 1H), 2.39 (s, 3H), 2.36-2.27 (m, 1H), 2.11-2.03 (m, 1H), 1.67-1.60 (m, 2H), 1.59 (s, 3H).

### Example 43

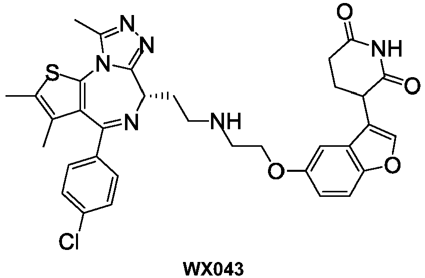

### Synthetic route:

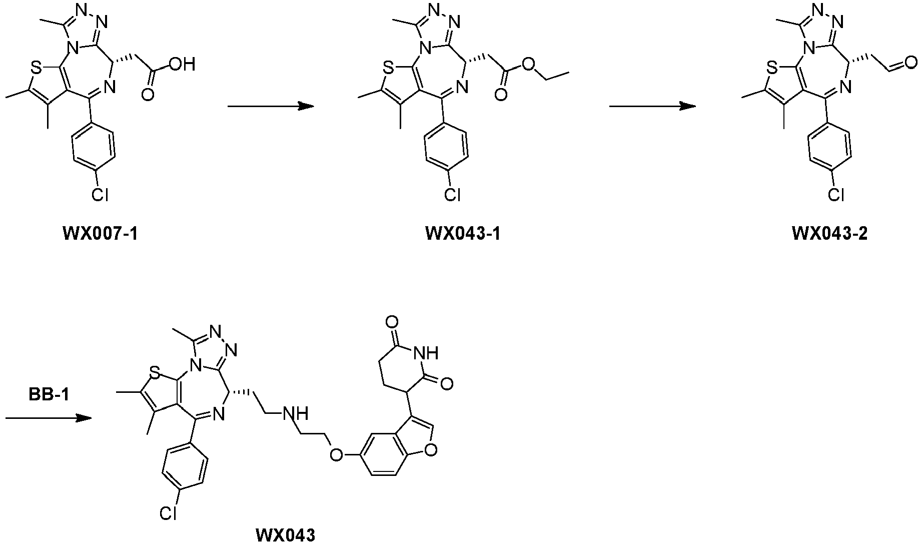

### Step 1: Synthesis of intermediate WX043-1

**WX007-1** (0.4 g, 997.80 µmol, 1 eq) was dissolved in ethanol (5 mL) at room temperature under nitrogen, and concentrated sulfuric acid (368.00 mg, 3.68 mmol, 0.2 mL, purity: 98%) was then added. The reaction mixture was heated to 80 °C and refluxed to react for 12 h. After the reaction was completed, the mixture was cooled down to room temperature. The solvent was removed by concentration under vacuum, and the resulting residue was diluted with aqueous potassium carbonate (1 M, 30 mL). The mixture was extracted with dichloromethane (20 mL×3). The organic phases were combined, washed with saturated brine (30 mL), dried over anhydrous sodium sulfate, and filtered. The filtrate was concentrated under reduced pressure to remove the solvent to give the intermediate **WX043-1.** ¹H NMR (400MHz, CDCl₃) δ: 7.45-7.37 (m, 2H), 7.36-7.30 (m, 2H), 4.66-4.58 (m, 1H), 4.24 (t, *J =* 7.1 Hz, 2H), 3.68-3.58 (m, 2H), 2.68 (s, 3H), 2.42 (s, 3H), 1.70 (s, 3H), 1.33 (t, *J* = 7.0 Hz, 3H).

### Step 2: Synthesis of intermediate WX043-2

Intermediate **WX043-1** (0.21 g, 489.59 µmol) was dissolved in dichloromethane (5 mL) at -78 °C under nitrogen, and DIBAL-H (1 M, 735.00 µL, 1.50 eq) (1 M in toluene) was then added. The reaction mixture was stirred to react at -78 °C for 2 h. After the reaction was completed, the reaction solution was quenched by adding saturated ammonium chloride solution (5 mL) and the solvent was removed by concentration under vacuum. The resulting residue was diluted with water (30 mL). The mixture was extracted with ethyl acetate (20 mL x 3). The organic phases were combined, washed sequentially with water (50 mL) and saturated brine (50 mL), dried over anhydrous sodium sulfate, and filtered. The filtrate was concentrated under reduced pressure to remove the solvent to give the intermediate **WX043-2.** ¹H NMR (400MHz, CDCl₃) δ: 10.10 (s, 1H), 7.43-7.39 (m, 2H), 7.36-7.32 (m, 2H), 4.69 (t, *J* = 6.4 Hz, 1H), 3.80-3.75 (m, 2H), 2.69 (s, 3H), 2.43 (s, 3H), 1.71 (s, 3H).

### Step 3: Synthesis of WX043

Intermediate **WX043-2** (0.05 g, 129.91 µmol, 1 eq) was dissolved in dichloromethane (5 mL) at room temperature under nitrogen. Subsequently, intermediate **BB-1** (155.89 µmol, 1.2 eq, hydrochloride), triethylamine (39.44 mg, 389.73 µmol, 54.25 µL, 3 eq) and sodium triacetoxyborohydride (63.33 mg, 298.79 µmol, 2.3 eq) were added sequentially. The reaction mixture was stirred to react at room temperature for 2 hours. After the reaction was completed, the reaction solution was diluted with water (20 mL) and extracted with dichloromethane (10 mL×3). The organic phases were combined, washed with water (20 mL×2), dried over anhydrous sodium sulfate, and filtered. The filtrate was concentrated under reduced pressure to remove the solvent. The resulting residue was separated by preparative HPLC (mobile phase: acetonitrile/water; neutral system: 10 mM NH₄HCO₃) to give the title compound **WX043.** MS-ESI *m*/*z:* 657.3 [M+H]⁺. ¹H NMR (400 MHz, DMSO_*d*₆) δ: 10.87 (br s, 1H), 7.84 (s, 1H), 7.49-7.40 (m, 5H), 7.10 (d, *J* = 1.6 Hz, 1H), 6.88 (dd, *J* = 2.6, 9.0 Hz, 1H), 4.22 (br t, *J* = 6.6 Hz, 1H), 4.14-4.06 (m, 1H), 4.06-3.94 (m, 2H), 3.05-2.87 (m, 4H), 2.78-2.65 (m, 1H), 2.59 (s, 3H), 2.62-2.56 (m, 1H), 2.56-2.52 (m, 2H), 2.39 (s, 3H), 2.36-2.26 (m, 1H), 2.12-2.02 (m, 1H), 1.59 (s, 3H).

### Example 44

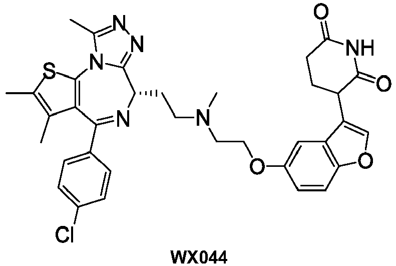

### Synthetic route:

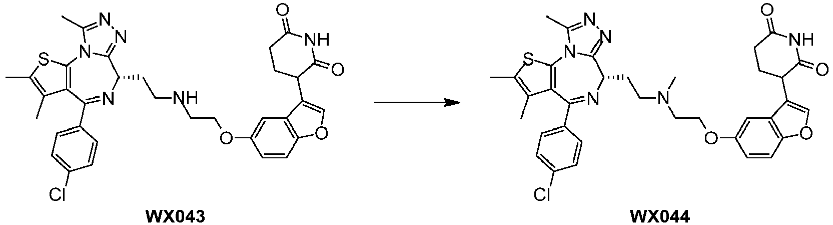

**WX043** (0.045 g, 68.47 µmol, 1 eq) was dissolved in dichloromethane (5 mL) at room temperature under nitrogen. Subsequently, aqueous formaldehyde solution (27.78 mg, 342.37 µmol, 25.49 µL, 5 eq, purity: 37%), magnesium sulfate (0.4 g, 3.32 mmol, 48.53 eq) and sodium triacetoxyborohydride (43.54 mg, 205.42 µmol, 3 eq) were added sequentially. The reaction mixture was stirred at room temperature for 1 hour. After the reaction was completed, the reaction solution was diluted with water (20 mL) and extracted with dichloromethane (10 mL×3). The organic phases were combined, washed with water (20 mL×2), dried over anhydrous sodium sulfate, and filtered. The filtrate was concentrated under reduced pressure to remove the solvent. The resulting residue was separated by preparative HPLC (mobile phase: acetonitrile/water; neutral system: 10 mM NH₄HCO₃) to give the title compound **WX044.** MS-ESI *m*/*z:* 671.3 [M+H]⁺. ¹H NMR (400 MHz, DMSO_*d*₆) δ: 10.87 (s, 1H), 7.83 (s, 1H), 7.43 - 7.35 (m, 5H), 7.09-7.03 (m, 1H), 6.76 (dd, *J* = 2.0, 8.8 Hz, 1H), 4.21 - 4.15 (m, 1H), 4.12 - 4.03 (m, 1H), 4.03 - 3.93 (m, 2H), 2.88 - 2.80 (m, 1H), 2.79 - 2.65 (m, 4H), 2.61-2.57 (m, 1H), 2.56 (s, 3H), 2.55-2.52 (m, 2H), 2.35 (s, 3H), 2.34-2.30 (m, 1H), 2.29 (s, 3H), 2.11-2.00 (m, 1H), 1.54 (d, *J* = 4.0 Hz, 3H).

### Example 45

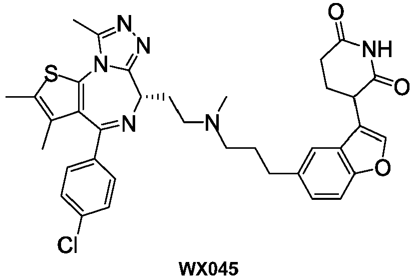

### Synthetic route:

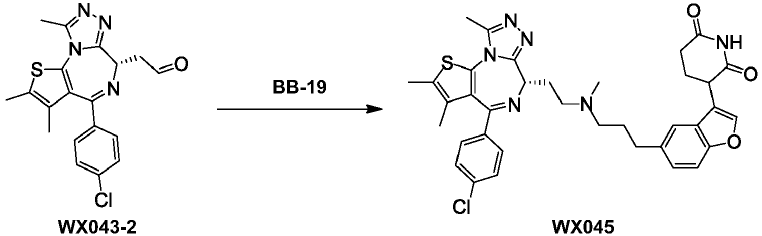

Intermediate **WX043-2** (0.04 g, 103.93 µmol, 1 eq) was dissolved in dichloromethane (5 mL) at room temperature under nitrogen. Subsequently, intermediate **BB-19** (0.04 g, 118.76 µmol, 1.14 eq, crude hydrochloride), triethylamine (21.03 mg, 207.86 µmol, 28.93 µL, 2 eq) and sodium triacetoxyborohydride (44.05 mg, 207.86 µmol, 2 eq) were added sequentially. The reaction mixture was stirred at room temperature for 2 hours. After the reaction was completed, the reaction solution was concentrated under reduced pressure to remove the solvent, and the resulting residue was diluted with water (50 mL) and extracted with ethyl acetate (30 mL×3). The organic phases were combined, washed with saturated brine (50 mL×2), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX045** hydrochloride. MS-ESI *m*/*z:* 669.2 [M+H]⁺. ¹H NMR (400MHz, DMSO_*d*₆) δ: 10.87 (s, 1H), 10.80 (br s, 1H), 7.86 (s, 1H), 7.52-7.42 (m, 6H), 7.24-7.19 (m, 1H), 4.39-4.31 (m, 1H), 4.12 (dd, *J* = 4.6, 11.8 Hz, 1H), 3.47-3.28 (m, 2H), 3.26-3.07 (m, 2H), 2.83 (s, 3H), 2.80-2.66 (m, 5H), 2.64 (s, 3H), 2.59-2.53 (m, 1H), 2.40 (s, 3H), 2.35-2.27 (m, 1H), 2.15-2.03 (m, 3H), 1.61 (d, *J* = 8.4 Hz, 3H).

### Example 46

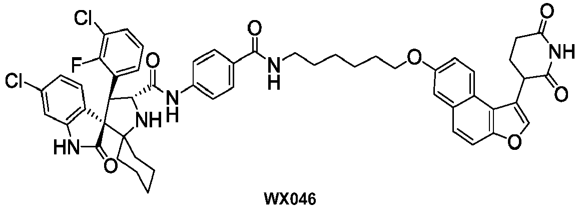

### Synthetic route:

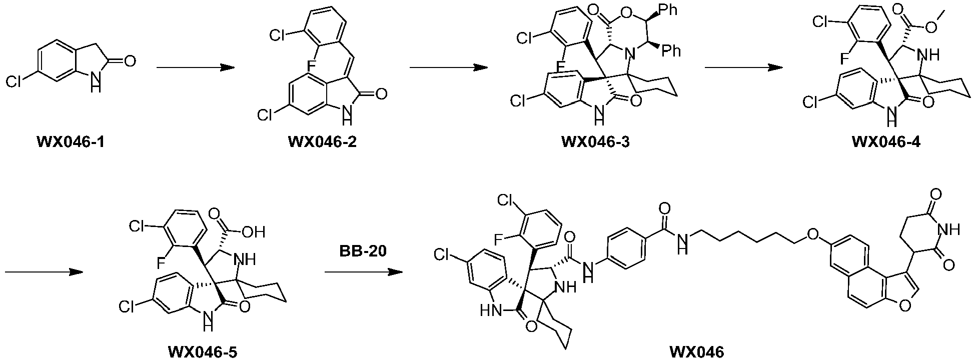

### Step 1: Synthesis of intermediate WX046-2

**WX046-1** (10 g, 59.67 mmol) and 3-chloro-2-fluorobenzaldehyde (10.88 g, 68.62 mmol) were dissolved in ethanol (100 mL), and then the atmosphere was replaced three times with nitrogen. Piperidine (5.08 g, 59.67 mmol, 5.89 mL) was added dropwise under nitrogen and the reaction mixture was stirred to react at 80°C for 2 hours. After the reaction was completed, the reaction solution was cooled to 4°C, and filtered. The filter cake was dried under reduced pressure to remove the solvent to give the intermediate **WX046-2.** ¹H NMR (400 MHz, DMSO_*d*₆) δ: 10.87 (s, 1H), 7.77-7.68 (m, 2H), 7.55 (s, 1H), 7.38 (t, *J* = 8.0 Hz, 1H), 7.17 (d, *J* = 8.4 Hz, 1H), 6.93 (dd, *J* = 1.8, 8.2 Hz, 1H), 6.90 (d, *J* = 1.8 Hz, 1H).

### Step 2: Synthesis of intermediate WX046-3

Intermediate **WX046-2** (10 g, 32.45 mmol) was dissolved in toluene (200 mL) and the atmosphere was replaced three times with nitrogen. (5R,6S)-5,6-di-phenylmorpholin-2-one (9.86 g, 38.94 mmol) and cyclohexanone (6.37 g, 64.91 mmol, 6.73 mL) were added sequentially under nitrogen, and the reaction mixture was stirred to react at 140 °C for 2 h. Cyclohexanone (3.19 g, 32.45 mmol, 3.36 mL) was added under nitrogen and the reaction mixture was stirred to react at 140 °C for 5 h. The reaction solution was filtered, and the filtrate was concentrated under reduced pressure to remove the solvent. The resulting residue was separated by column chromatography (eluent: petroleum ether/ethyl acetate = 1/0-3/1, v/v) to give the intermediate **WX046-3.** ¹H NMR (400 MHz, DMSO_*d*₆) δ: 10.78 (s, 1H), 7.94 (s, 1H), 7.45-7.36 (m, 1H), 7.29-7.23 (m, 4H), 7.15-7.10 (m, 4H), 7.04-6.99 (m, 2H), 6.83 (d, *J* = 2.0 Hz, 1H), 6.82-6.76 (m, 2H), 6.65 (dd, *J =* 2.0, 8.2 Hz, 1H), 6.34 (d, *J* = 8.4 Hz, 1H), 5.44 (d, *J* = 11.6 Hz, 1H), 4.90 (d, *J* = 2.6 Hz, 1H), 4.57 (d, *J* = 11.2 Hz, 1H), 2.38 (d, *J* = 13.2 Hz, 1H), 2.15 (d, *J* = 11.2 Hz, 1H), 1.77-1.73 (m, 5H), 1.69-1.55 (m, 3H).

### Step 3: Synthesis of intermediate WX046-4

Compound **WX046-3** (5.5 g, 8.57 mmol) was dissolved in methanol (60 mL) at room temperature and concentrated sulfuric acid (1.68 g, 17.15 mmol, 913.95 µL) was added. The mixture was then heated to 80 °C and reacted for 16 h. After the reaction was completed, the two batches were combined and the reaction solution was concentrated under reduced pressure to remove methanol. The residue was diluted with ethyl acetate (300 mL), and then adjusted to pH of 8 with saturated sodium bicarbonate solution. The organic phase was separated. The organic phase was dried over anhydrous sodium sulfate, and filtered. The filtrate was concentrated under reduced pressure to give the crude product. The crude product was separated by column chromatography (eluent: petroleum ether/ethyl acetate = 5/1-0/1, v/v) to give the intermediate **WX046-4.**

¹H NMR (400 MHz, CD₃OD) δ: 7.61 (t, *J* = 7.6 Hz, 1H), 7.42 (dd, *J* = 2.4, 8.4 Hz, 1H), 7.23 (t, *J* = 8.4 Hz 1H), 7.04 (t, *J* = 7.6 Hz, 2H), 6.73 (d, *J* = 2.0 Hz, 1H), 4.84 (d, *J* = 9.6 Hz, 1H), 4.63 (d, *J* = 9.6 Hz, 1H), 3.66 (s, 3H), 2.24-2.14 (m, 1H), 1.97-1.84 (m, 2H), 1.80-1.67 (m, 2H), 1.64-1.57 (m, 2H), 1.51-1.47 (m, 1H), 1.12-0.97 (m, 2H).

### Step 4: Synthesis of intermediate WX046-5 trifluoroacetate

Intermediate **WX046-4** (8.57 mmol) was added to tetrahydrofuran (50 mL) and water (50 mL), and lithium hydroxide monohydrate (2.64 g, 62.85 mmol) and methanol (10 mL) were added. The mixture was reacted at room temperature for 16 h. After the reaction was completed, the reaction solution was concentrated under reduced pressure to remove tetrahydrofuran and methanol, and then diluted with ethyl acetate (150 mL). The solution was adjusted to pH of 4-5 with trifluoroacetic acid, and stirred for 20 h. The organic phase was separated, dried over anhydrous sodium sulfate, and concentrated under reduced pressure to remove the solvent. The residue was purified by preparative HPLC (mobile phase: acetonitrile/water; acidic system: 0.1% TFA) to give the intermediate **WX046-5** trifluoroacetate. ¹H NMR (400 MHz, CD₃OD) δ: 7.64 (t, *J* = 6.8 Hz, 1H), 7.55 (dd, *J* = 2.4, 8.0 Hz, 1H), 7.32 (t, *J* = 8.0 Hz, 1H), 7.16-7.08 (m, 2H), 6.76 (d, *J* = 1.6 Hz, 1H), 4.99 (d, *J* = 10.8 Hz, 1H), 4.80 (d, *J* = 10.4 Hz, 1H), 2.56 (d, *J* = 8.4 Hz, 1H), 2.13 (d, *J* = 12.4 Hz, 1H), 2.02-1.84 (m, 3H), 1.82-1.73 (m, 2H), 1.66-1.50 (m, 1H), 1.32-1.09 (m, 2H).

### Step 5: Synthesis of WX046 hydrochloride

Intermediate **BB-20** (139.77 mg, 259.81 µmol, hydrochloride) and intermediate **WX046-5** (150 mg, 259.81 µmol, trifluoroacetate) were dissolved in N,N dimethylformamide (2 mL) at room temperature under nitrogen, and a solution of tri-1-propyl cyclophosphonic anhydride 50% in ethyl acetate (330.66 mg, 519.61 µmol, 309.03 µL), and N,N-diisopropylethylamine (134.31 mg, 1.04 mmol, 181.01 µL) were added at 0°C and stirred at 0°C for 30 min. The reaction mixture was warmed up to 15 °C and stirred to react under nitrogen for 16 h. The reaction solution was filtered through a filter membrane to obtain a clarified solution, which was separated by preparative HPLC (mobile phase: acetonitrile/water; acidic system: 0.04% HCl) to give the title compound **WX046** hydrochloride. MS-ESI *m*/*z*: 958.3 [M+H]⁺. ¹H NMR (400MHz, DMSO_*d₆*) δ: 10.96 (s, 1H), 10.61 (s, 1H), 10.24 (s, 1H), 8.39 (t, *J* = 5.2 Hz, 1H), 8.09 (d, *J* = 8.4 Hz, 1H), 7.98 (s, 1H), 7.83 (d, *J* = 8.8 Hz, 2H), 7.75 (s, 2H), 7.70 (d, *J =* 8.8 Hz, 2H), 7.64 (t, *J* = 6.8 Hz, 1H), 7.50 (d, *J* = 2.4 Hz, 1H), 7.48 (dd, *J* = 2.4, 8.4 Hz, 1H), 7.38 (t, *J* = 8.0 Hz, 1H), 7.23 (dd, *J* = 2.8, 9.9 Hz, 1H), 7.16 (t, *J* = 7.6 Hz, 1H), 7.06 (dd, *J* = 2.0, 8.4 Hz, 1H), 6.70 (d, *J* = 2.0 Hz, 1H), 4.80-4.75 (m, 1H), 4.75-4.62 (m, 2H), 4.11 (t, *J* = 6.4 Hz, 2H), 3.74-3.67 (m, 1H), 3.31-3.23 (m, 2H), 2.94-2.84 (m, 1H), 2.68-2.59 (m, 1H), 2.43-2.40 (m, 1H), 2.31-2.23 (m, 1H), 2.11-1.99 (m, 1H), 1.89-1.77 (m, 3H), 1.73-1.47 (m, 10H), 1.46-1.34 (m, 3H), 1.05-0.93 (m, 1H), 0.91- 0.79 (m, 1H).

### Example 47

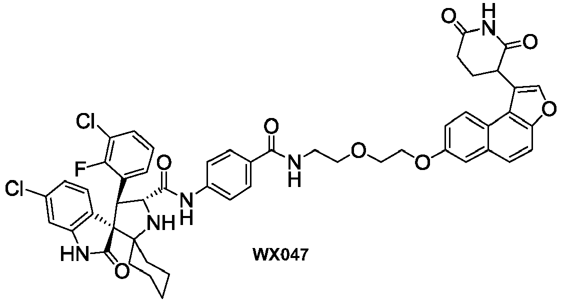

### Synthetic route:

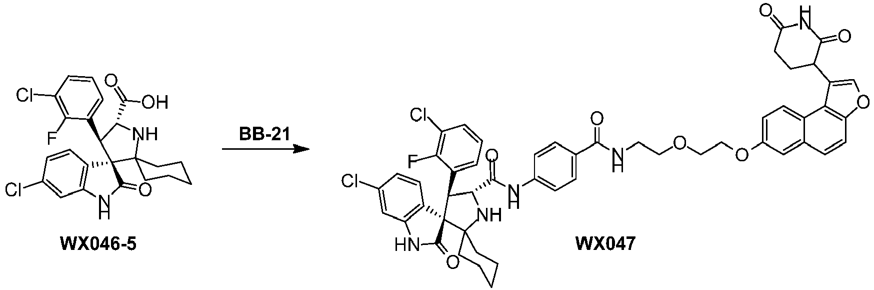

Intermediate **BB-21** (139.77 mg, 259.81 µmol, hydrochloride) and intermediate **WX046-5** (150 mg, 259.81 µmol, trifluoroacetate) were dissolved in N,N dimethylformamide (2 mL), and a solution of tri-1-propyl cyclophosphonic anhydride 50% in ethyl acetate (330.66 mg, 519.61 µmol, 309.03 µL), and N,N-diisopropylethylamine (134.31 mg, 1.04 mmol, 181.01 µL) were added at 0°C. The reaction mixture was warmed up to 15 °C and stirred to react under nitrogen for 16 h. The reaction solution was filtered through a filter membrane to obtain a clarified solution, which was separated by preparative HPLC (mobile phase: acetonitrile/water; acidic system: 0.04% HCl) to give the title compound **WX047** hydrochloride. MS-ESI m/z: 946.3 [M+H]⁺. ¹H NMR (400 MHz, CD₃OD) δ: 8.00 (d, *J* = 9.6 Hz, 1H), 7.80 (d, *J* = 5.2 Hz, 1H), 7.76-7.67 (m, 3H), 7.64 (d, *J* = 8.8 Hz, 1H), 7.60 (d, *J* = 8.4 Hz, 1H), 7.58-7.49 (m, 3H), 7.43-7.32 (m, 2H), 7.24 (dd, *J* = 2.8, 9.2 Hz, 1H), 7.18 (t, *J* = 7.6 Hz, 1H), 7.15-7.08 (m, 1H), 6.80 (s, 1H), 5.01-4.91 (m, 1H), 4.63-4.55 (m, 1H), 4.28 (d, *J* = 4.4 Hz, 2H), 3.96-3.90 (m, 2H), 3.81-3.74 (m, 2H), 3.64-3.57 (m, 2H), 3.31-3.17 (m, 1H), 2.95-2.82 (m, 1H), 2.79-2.70 (m, 1H), 2.51-2.38 (m, 2H), 2.29-2.04 (s, 1H), 2.03-1.82 (m, 3H), 1.82-1.62 (m, 3H), 1.62-1.46 (m, 1H), 1.28-1.09 (m, 2H).

### Example 48

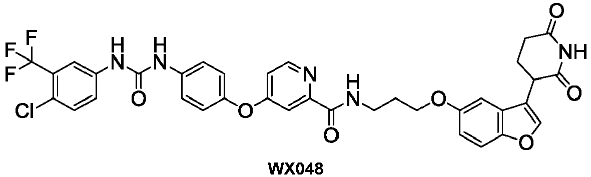

### Synthetic route:

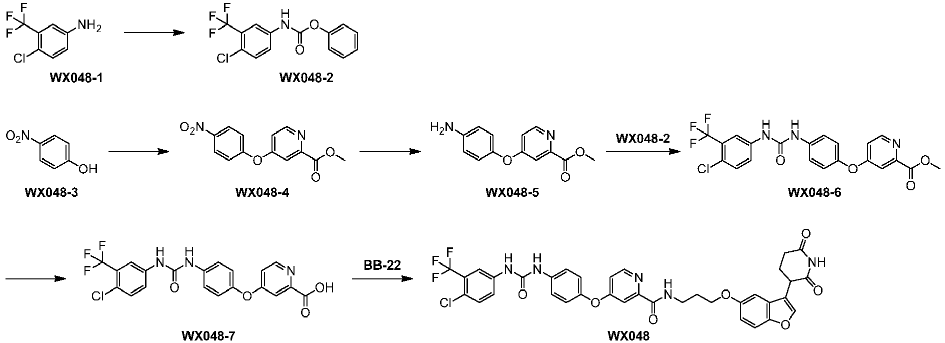

### Step 1: Synthesis of intermediate WX048-2

Compound **WX048-1** (4.9 g, 25.05 mmol, 1 eq) was dissolved in dichloromethane (50 mL) at room temperature. Phenyl chloroformate (3.92 g, 25.05 mmol, 3.14 mL, 1 eq) was dissolved in dichloromethane (10 mL) and the mixture was added to the above reaction solution. The reaction mixture was stirred to react at room temperature for 1 h. After the reaction was completed, the reaction solution was poured into water (50 mL), and dilute hydrochloric acid (1 M, 50 mL) was added. The mixture was extracted with dichloromethane (50 mL). The organic phase was sequentially washed with dilute hydrochloric acid solution (1 M, 50 mL×3), dried over anhydrous sodium sulfate, and filtered. The filtrate was concentrated under reduced pressure to remove the solvent to give the intermediate **WX048-2.** ¹H NMR (400MHz, CDC1₃) δ: 7.80 (d, *J* = 1.6 Hz, 1H), 7.59 (dd, *J* = 2.0, 8.8 Hz, 1H), 7.47-7.38 (m, 3H), 7.30-7.25 (m, 1H), 7.19 (d, *J* = 7.6 Hz, 2H).

### Step 2: Synthesis of intermediate WX048-4

Compound **WX048-3** (10.00 g, 71.89 mmol) was dissolved in chlorobenzene (100 mL) at room temperature under nitrogen, and methyl 4-chloropyridine-2-carboxylate (9.25 g, 53.91 mmol) was added. The reaction mixture was stirred at 130°C for 12 hours. After the reaction was completed, the reaction system was cooled down to room temperature. The reaction solution was poured into petroleum ether (100 mL), stirred for 15 min, and allowed to stand. The upper clear layer was poured out, and the remaining black oily substance was dissolved with dichloromethane (500 mL). The mixture was adjusted to pH of about 9 with sodium hydroxide solution (1N). The mixture was diluted with water (100 mL), and the layers were separated. The organic phase was collected, and the aqueous phase was extracted with dichloromethane (250 mL × 3). The organic phases were combined, dried over anhydrous sodium sulfate, and filtered. The filtrate was concentrated under reduced pressure to remove the solvent. The resulting residue was separated by column chromatography (eluent: petroleum ether/ethyl acetate = 1/0-1/1, v/v) to give the intermediate **WX048-4.** ¹H NMR (400MHz, CDC1₃) δ: 8.71 (d, *J* = 5.6 Hz, 1H), 8.46-8.23 (m, 2H), 7.76 (d, *J* = 2.4 Hz, 1H), 7.26-7.20 (m, 2H), 7.14 (dd, *J* = 2.4, 5.6 Hz, 1H), 4.02 (s, 3H).

### Step 3: Synthesis of intermediate WX048-5

Wet palladium on carbon (0.40 g) was dissolved in methanol (20 mL) at room temperature under nitrogen, and intermediate **WX048-4** (1.67 g, 6.09 mmol) was added. The reaction mixture was purged three times with nitrogen, and then purged three times with hydrogen. The reaction system was stirred at 25°C under hydrogen atmosphere for 12 hours. After the reaction was completed, the reaction solution was filtered through celite, and the filter cake was rinsed with methanol (50 mL×3). The organic phases were combined, and concentrated under reduced pressure to remove the solvent to give the crude intermediate **WX048-5.**

### Step 4: Synthesis of intermediate WX048-6

Intermediate **WX048-5** (500.00 mg, 2.05 mmol) and intermediate **WX048-2** (646.22 mg, 2.05 mmol) were dissolved in N,N-dimethylformamide (4 mL) at room temperature under nitrogen, and triethylamine (0.42 mL, 3.07 mmol) was added to the reaction system. The reaction mixture was stirred at 25°C for 2 hours, and then half-saturated brine (20 mL) was added to quench the reaction. The mixture was diluted with ethyl acetate (30 mL), and the layers were separated. The organic phases were collected. The aqueous phase was extracted with ethyl acetate (30 mL×3). The organic phases were sequentially combined, washed with saturated brine (50 mL×3), dried over anhydrous sodium sulfate, and filtered. The filtrate was concentrated under reduced pressure to remove the solvent. The resulting residue was separated by column chromatography (eluent: petroleum ether/ethyl acetate = 1/0-2/1, v/v) to give the intermediate **WX048-6.**

### Step 5: Synthesis of intermediate WX048-7

Intermediate **WX048-6** (0.80 g, 1.72 mmol) was dissolved in methanol (6 mL) at room temperature under nitrogen, and lithium hydroxide monohydrate (288.28 mg, 6.87 mmol) was added. Water (6 mL) was added and the reaction mixture was stirred at 25 °C for 1 h. After the reaction was completed, the reaction solution was adjusted to pH of 5-6 by adding dilute hydrochloric acid (1 M), then concentrated under reduced pressure, and filtered. The filter cake was rinsed with ethyl acetate (20 mL×3). The filter cake was collected and dried under reduced pressure to remove the residual solvent to give the crude intermediate **WX048-7.** ¹H NMR (400 MHz, DMSO_*d₆*) δ: 9.22 (s, 1H), 9.00 (s, 1H), 8.57 (d, *J* = 5.6 Hz, 1H), 8.12 (s, 1H), 7.71-7.55 (m, 3H), 7.40 (d, *J* = 2.4 Hz, 1H), 7.19 (d, *J* = 9.0 Hz, 2H).

### Step 6: Synthesis of WX048

Intermediate **WX048-7** (105.00 mg, 232.41 µmol) was dissolved in N,N-dimethylformamide (2 mL) at room temperature under nitrogen, and O-(7-azabenzotriazol-1-yl)-N,N,N,N-tetramethyluronium hexafluorophosphate (176.74 mg, 464.82 µmol), N,N-diisopropylethylamine (121.45 µL, 697.24 µmol), and intermediate **BB-22** (157.48 mg, 464.82 µmol, hydrochloride) were added sequentially. The reaction mixture was stirred at 25 °C for 12 h. After the reaction was completed, the reaction mixture was quenched by adding saturated brine (20 mL), and diluted with ethyl acetate (30 mL). The layers were separated. The organic phase was collected, and thethe aqueous phase was extracted with ethyl acetate (30 mL×3). The organic phases were combined, washed with saturated saline (20 mL×3), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX048.** MS-ESI *m*/*z*: 736.2 [M+H] ⁺. ¹H NMR (400MHz, DMSO_*d₆*) δ: 10.86 (s, 1H), 9.34 (s, 1H), 9.10 (s, 1H), 8.96 (t, *J* = 6.0 Hz, 1H), 8.52 (d, *J* = 5.6 Hz, 1H), 8.12 (d, *J* = 2.2 Hz, 1H), 7.84 (s, 1H), 7.69-7.55 (m, 4H), 7.46 (d, *J* = 9.0 Hz, 1H), 7.41 (d, *J* = 2.6 Hz, 1H), 7.21-7.14 (m, 3H), 7.10 (d, *J* = 2.4 Hz, 1H), 6.92 (dd, *J* = 2.4, 8.8 Hz, 1H), 4.09 (dd, *J* = 4.8, 12.0 Hz, 1H), 4.06-3.99 (m, 2H), 3.46 (q, *J* = 6.4 Hz, 2H), 2.78-2.65 (m, 1H), 2.62-2.55 (m, 1H), 2.39-2.26 (m, 1H), 2.14-2.07 (m, 1H), 2.00 (q, *J* = 6.4 Hz, 2H).

### Example 49

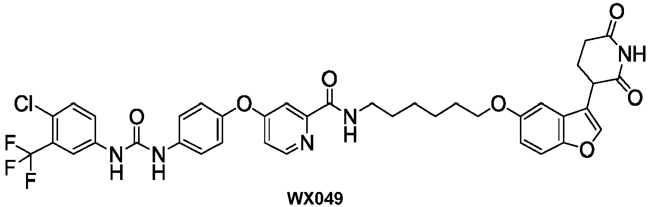

### Synthetic route:

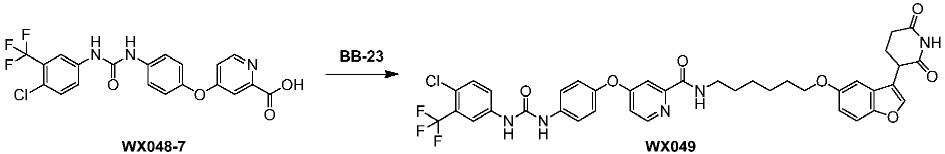

Intermediate **WX048-7** (133.00 mg, 294.39 µmol) was dissolved in N,N-dimethylformamide (3 mL) at room temperature under nitrogen, and O-(7-azabenzotriazol-1-yl)-N,N,N,N-tetramethyluronium hexafluorophosphate (223.87 mg, 588.78 µmol), N,N-diisopropylethylamine (114.14 mg, 883.17 µmol, 153.83 µL) and intermediate **BB-23** (224.25 mg, 588.78 µmol, hydrochloride) were added sequentially. The reaction system was stirred at 15°C for 12 hours. After the reaction was completed, the reaction mixture was quenched by adding saturated brine (20 mL). The mixture was diluted with ethyl acetate (30 mL), and the layers were separated. The organic phase was collected, and the aqueous phase was extracted with ethyl acetate (30 mL × 3). The organic phases were sequentially combined, washed with saturated brine (20 mL×3), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX049.** MS-ESI *m*/*z*. 778.2 [M+H] ⁺. ¹H NMR (400MHz, DMSO_*d₆*) δ: 10.86 (s, 1H), 9.29 (s, 1H), 9.06 (s, 1H), 8.80 (t, *J* = 6.0 Hz, 1H), 8.51 (d, *J* = 5.6 Hz, 1H), 8.12 (d, *J* = 2.2 Hz, 1H), 7.83 (s, 1H), 7.70-7.55 (m, 4H), 7.46-7.36 (m, 2H), 7.21-7.13 (m, 3H), 7.08 (d, *J* = 2.4 Hz, 1H), 6.88 (dd, *J* = 2.4, 8.8 Hz, 1H), 4.10 (dd, *J* = 4.8, 12.4 Hz, 1H), 3.99-3.88 (m, 2H), 3.27 (q, *J* = 6.4 Hz, 2H), 2.77-2.66 (m, 1H), 2.60-2.54 (m, 1H), 2.38-2.28 (m, 1H), 2.13-2.07 (m, 1H), 1.76-1.67 (m, 2H), 1.58-1.51 (m, 2H), 1.49-1.40 (m, 2H), 1.39-1.30 (m, 2H).

### Example 50

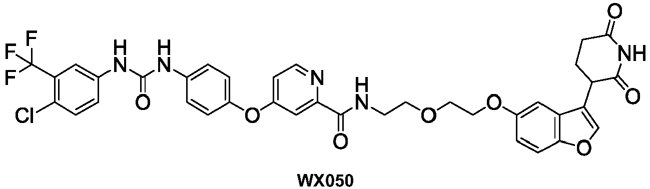

### Synthetic route:

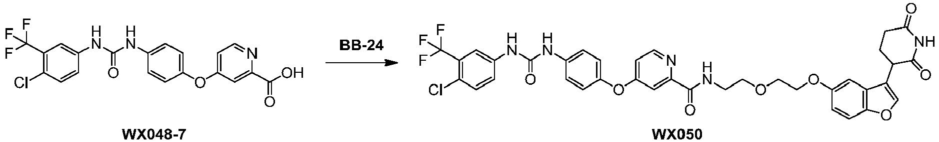

Intermediate **WX048-7** (125.00 mg, 276.68 µmol) was dissolved in N,N-dimethylformamide (2 mL) at 25°C under nitrogen, and O-(7-azabenzotriazol-1-yl)-N,N,N,N-tetramethyluronium hexafluorophosphate (210.41 mg, 553.36 _{f.l}mo1), N,N-diisopropylethylamine (144.58 µL, 830.05 µmol) and intermediate **BB-24** (204.09 mg, 553.36 µmol, hydrochloride) were added sequentially. The reaction system was stirred at 25°C for 4 hours. After the reaction was completed, the reaction solution was filtered and the filtrate was separated by preparative HPLC (mobile phase: acetonitrile/water; acidic system: 0.04% HCl) to give the title compound **WX050.** MS-ESI m/z: 766.2 [M+H]⁺. ¹H NMR (400MHz, CD₃OD) δ: 8.43 (s, 1H), 8.00 (d, *J* = 2.4 Hz, 1H), 7.71-7.55 (m, 5H), 7.51 (d, *J* = 8.4 Hz, 1H), 7.32 (d, *J* = 8.8 Hz, 1H), 7.17-6.98 (m, 4H), 6.89 (d, *J* = 7.8 Hz, 1H), 4.20-4.12 (m, 2H), 4.11-4.04 (m, 1H), 3.89-3.81 (m, 2H), 3.74 (d, *J* = 4.4 Hz, 2H), 3.61 (s, 2H), 2.79-2.70 (m, 2H), 2.43-2.31 (m, 1H), 2.29-2.23 (m, 1H).

### Example 51

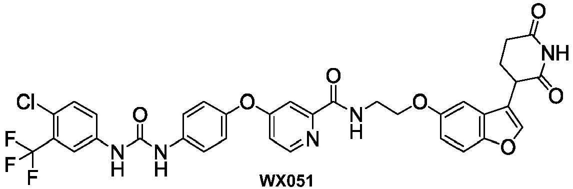

### Synthetic route:

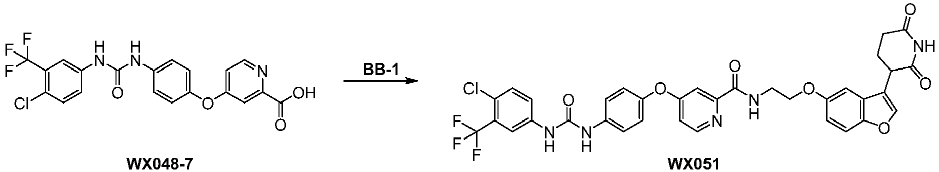

Intermediate **WX048-7** (125.00 mg, 276.68 µmol) was dissolved in N,N-dimethylformamide (2 mL) at 25°C under nitrogen, and O-(7-azabenzotriazol-1-yl)-N,N,N,N-tetramethyluronium hexafluorophosphate (210.41 mg, 553.36 _{f.l}mo1), N,N-diisopropylethylamine(144.58 µL, 830.04 µmol) and intermediate **BB-1** (159.53 mg, 553.36 µmol, hydrochloride) were added sequentially. The reaction system was stirred at 25°C for 1 hour. After the reaction was completed, the reaction mixture was quenched by adding saturated brine (20 mL). The mixture was diluted with ethyl acetate (30 mL), and the layers were separated. The organic phase was collected, and the aqueous phase was extracted with ethyl acetate (30 mL × 3). The organic phases were combined, washed with saturated brine (20 mL×3), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX051.** MS-ESI m/z: 722.2 [M+H]⁺. ¹H NMR (400MHz, DMSO_*d₆*) δ: 10.86 (s, 1H), 9.28 (s, 1H), 9.05 (s, 1H), 8.95 (s, 1H), 8.53 (d, *J* = 5.6 Hz, 1H), 8.12 (d, *J* = 2.2 Hz, 1H), 7.84 (s, 1H), 7.68-7.57 (m, 4H), 7.45 (d, *J* = 8.8 Hz, 1H), 7.42 (s, 1H), 7.20-7.15 (m, 3H), 7.14 (d, *J* = 2.6 Hz, 1H), 6.91 (dd, *J* = 2.4, 9.0 Hz, 1H), 4.15-4.06 (m, 3H), 3.73-3.64 (m, 2H), 2.77-2.67 (m, 1H), 2.57 (d, *J* = 4.0 Hz, 1H), 2.40-2.29 (m, 1H), 2.12-2.04 (m, 1H).

### Example 52

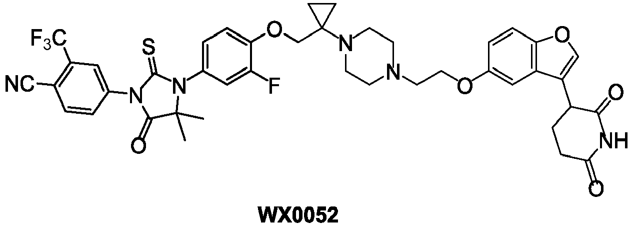

### Synthetic route:

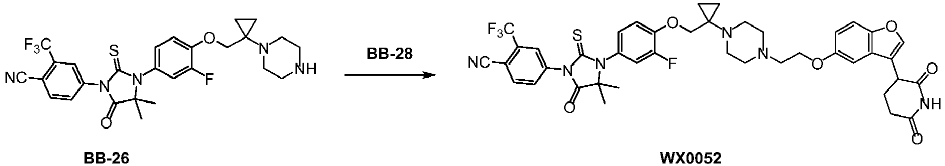

Compound **BB-28** (0.1 g, 348.11 µmol) was dissolved in 1,2-dichloroethane (3 mL), and compound **BB-26** hydrochloride (138.79 mg, 232.07 µmol) and sodium acetate (38.07 mg, 464.14 µmol) were added. The mixture was stirred for 30 min, and sodium triacetoxyborohydride (98.37 mg, 464.14 µmol) was added. The mixture was reacted at 25°C for 12 h. After the reaction was completed, water (5 mL) and dichloromethane (10 mL) were added to the reaction solution, and the organic phase was separated. The aqueous phase was extracted with dichloromethane (20 mL×3). The organic phases were combined, washed with saturated brine (20 mL×2), dried over anhydrous sodium sulfate, and filtered. The filtrate was concentrated under reduced pressure to remove the solvent. The residue was purified by HPLC (mobile phase: acetonitrile/water; acidic system: 0.04% HCl) to give the title compound **WX052.** MS-ESI *m*/*z*: 833.4[M+H]⁺. ¹H NMR (400MHz, CD₃OD) δ: 8.16-8.14 (m, 2H), 7.99-7.96 (m, 1H), 7.72 (s, 1H), 7.42 (d, J = 8.8 Hz, 1H), 7.29-7.24 (m, 2H), 7.17-7.16 (m, 2H), 7.03-7.00 (m, 1H), 4.43-4.35 (m, 2H), 4.30-4.22 (m, 2H), 4.11 (dd, *J* = 4.8 Hz, 8.0 Hz, 1H), 3.68-3.59 (m, 4H), 3.46 (s, 4H), 3.31-3.30 (m, 2H), 2.85-2.68 (m, 2H), 2.45-2.34 (m, 1H), 2.30-2.23 (m, 1H), 1.56 (s, 6H), 1.10-0.93 (m, 4H).

### Example 53

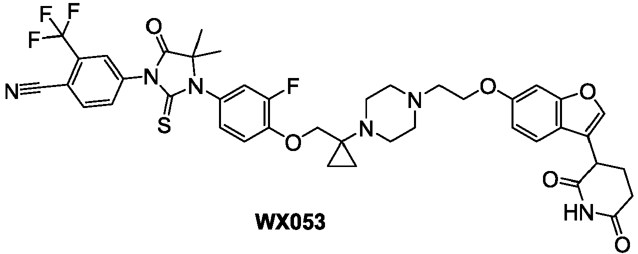

### Synthetic route:

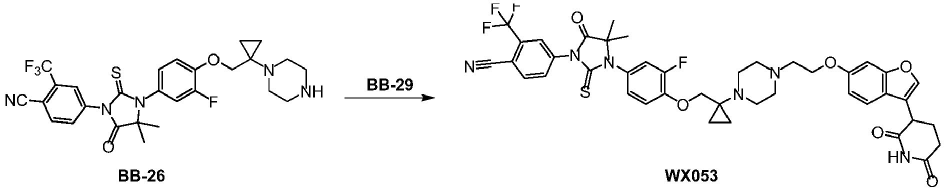

Compound **BB-29** (0.1 g, 348.11 µmol) was dissolved in 1,2-dichloroethane (3 mL) at room temperature under nitrogen, and compound **BB-26** hydrochloride (138.79 mg, 232.07 µmol) and sodium acetate (38.08 mg, 464.14 µmol) were added. The mixture was stirred for 30 min, and then sodium triacetoxyborohydride (98.37 mg, 464.14 µmol) was added. The mixture was reacted at 25 °C for 12 h. After the reaction was completed, water (5 mL) and dichloromethane (10 mL) were added to the reaction solution. The organic phase was separated, and the aqueous phase was extracted with dichloromethane (20 mL×3). The organic phase was washed with saturated brine (20 mL×2), dried over anhydrous sodium sulfate, and filtered. The filtrate was concentrated under reduced pressure to remove the solvent. The residue was separated by HPLC (mobile phase: acetonitrile/water; acidic system: 0.04% HCl) to give the title compound WX053. MS-ESI *m*/*z*: 833.4 [M+H]⁺. ¹H NMR (400MHz, CD₃OD) δ: 8.16-8.14 (m, 2H), 7.98-7.96 (m, 1H), 7.65 (s, 1H), 7.49-7.47 (m, 1H), 7.29-7.25 (m, 2H), 7.19-7.16 (m, 2H), 6.99-6.97 (m, 1H), 4.46-4.42(m, 2H), 4.27 (s, 2H), 4.11-4.07 (m, 1H), 3.66-3.65 (m, 4H), 3.51-3.45 (m, 4H), 3.31-3.30 (m2H), 2.82-2.66 (m, 2H), 2.40-2.23 (m, 2H), 1.55 (s, 6H), 1.09-0.97 (m, 4H).

### Example 54

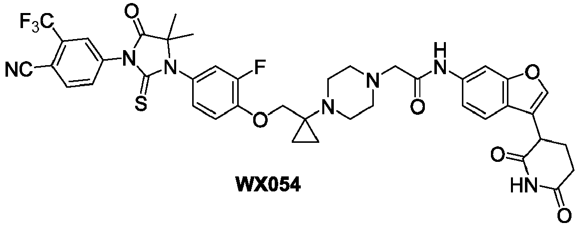

### Synthetic route:

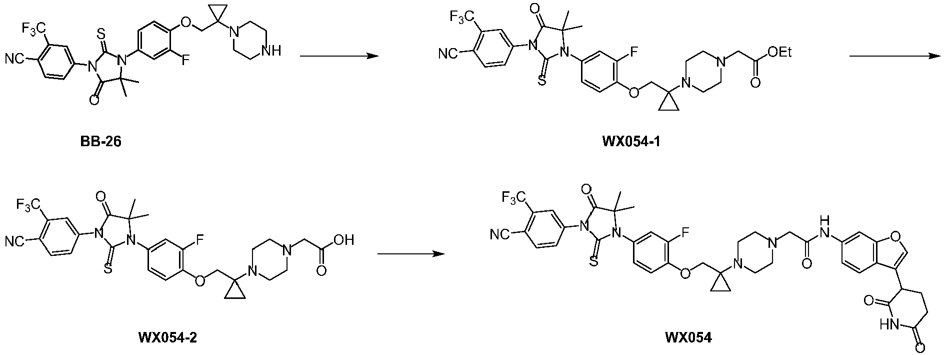

### Step 1: Synthesis of compound WX054-1

Compound **BB-26** hydrochloride (0.4 g, 668.84 µmol) was dissolved in tetrahydrofuran (4 mL), and the solution was cooled down to 0 °C under nitrogen. Triethylamine (169.20 mg, 1.67 mmol, 232.73 µL) was added dropwise, and then a solution of ethyl bromoacetate (122.87 mg, 735.72 µmol, 81.37 µL) in tetrahydrofuran (1 mL) was added dropwise. The mixture was slowly heated to 20 °C and stirred for 15 hours. Water (10 mL) was added to the reaction solution, and the mixture was extracted with ethyl acetate (10 mL × 2). The organic phase was washed with saturated brine (20 mL), dried over anhydrous sodium sulfate, and filtered. The solvent was removed under reduced pressure to give the compound **WX054-1.**

### Step 2: Synthesis of compound WX054-2

Compound **WX054-1** (0.4 g, 617.59 µmol) was dissolved in a mixture of tetrahydrofuran (8 mL) and water (2 mL), and the mixture was cooled down to 0 °C. Lithium hydroxide monohydrate (129.58 mg, 3.09 mmol) was added and then the mixture was stirred at 20 °C for 2 h. The reaction solution was adjusted to pH of 4-5 with 1N dilute hydrochloric acid, and extracted with dichloromethane (50 mL×3). The organic phase was washed with saturated brine (30 mL), dried over anhydrous sodium sulfate, and filtered. The solvent was removed under reduced pressure to give the compound **WX054-2.**

### Step 3: Synthesis of compound WX054

Compound **WX054-2** (0.08 g, 129.11 µmol) was placed in N,N-dimethylformamide (2 mL). N,N-diisopropylethylamine (50.06 mg, 387.33 µmol, 67.47 µL) and O-(7-azabenzotriazol-1-yl)-N,N,N,N-tetramethyluronium hexafluorophosphate (98.18 mg, 258.22 µmol) were added, and compound **BB-30** (34.69 mg, 142.02 µmol) was then added. The mixture was stirred at 20 °C for 12 h. The reaction solution was concentrated under reduced pressure to remove the solvent, and the resulting residue was purified by HPLC (mobile phase: acetonitrile/water; acidic system: 0.04% HCl) to give the title compound **WX054.** ¹H NMR (400 MHz, CD₃OD) δ: 8.17-8.14 (m, 2 H), 8.03 (d, *J* = 2.0 Hz, 1 H), 8.00-7.97 (m, 1 H), 7.72 (s, 1 H), 7.52 (d, *J* = 8.4 Hz, 1 H), 7.29-7.25 (m, 3 H), 7.20-7.17 (m, 1 H), 4.26 (s, 2 H), 4.13-4.09 (m, 3 H), 3.49-3.35 (m, 8 H), 2.84-2.67 (m, 2 H), 2.43-2.33 (m, 1 H), 2.30-2.23 (m, 1 H), 1.57 (s, 6 H), 1.00-0.91 (m, 4 H).

### Example 55

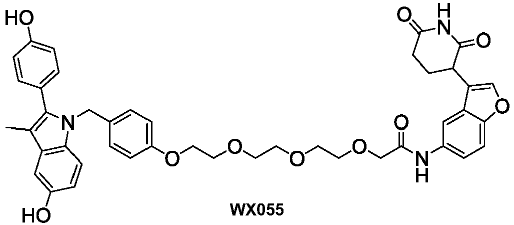

### Synthetic route:

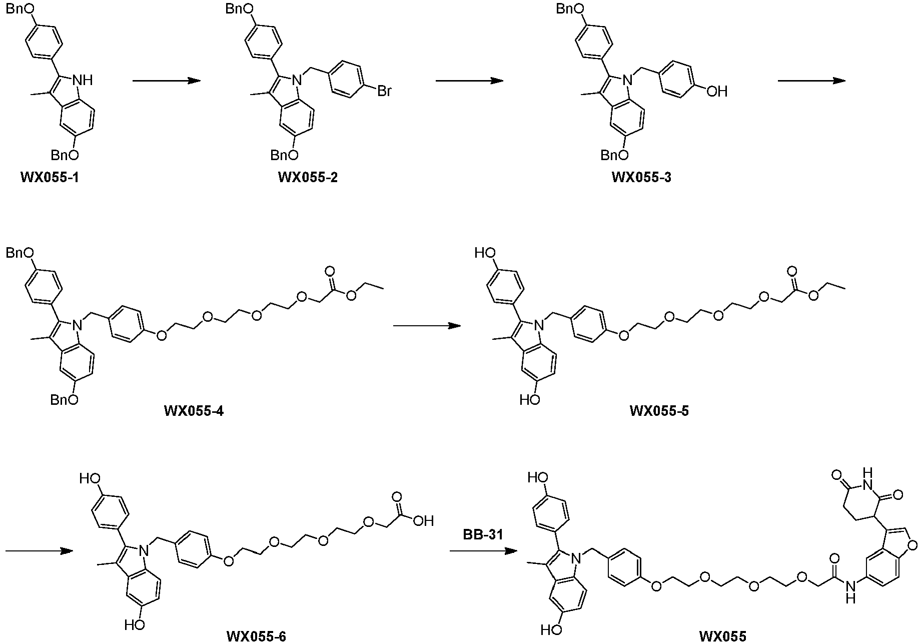

### Step 1: Synthesis of intermediate WX055-2

**WX055-1** (20 g, 47.67 mmol) was dissolved in N,N-dimethylformamide (200 mL) at 0°C. Sodium hydride (3.81 g, 95.35 mmol, 60% content) was added slowly in batches, and the reaction mixture was stirred to react at 0°C for 10 min. 1-bromo-4-(bromomethyl)benzene (11.92 g, 47.67 mmol) was then added, and the reaction mixture was reacted with stirring at 0°C for another 30 min. After the reaction was completed, water (600 mL) was added to the reaction solution and the resulting mixture was extracted with ethyl acetate (200 mL×3). The organic phases were combined, washed with saturated brine (200 mL×3), dried over anhydrous sodium sulfate, and filtered. The solvent was removed from the filtrate under reduced pressure. The resulting residue was separated by column chromatography (eluent: petroleum ether/ethyl acetate = 10/1-1/1, v/v) to give the intermediate **WX055-2.** ¹H NMR (400 MHz, DMSO_d₆) δ: 7.51-7.45 (m, 4H), 7.43-7.31 (m, 8H), 7.28 (d, *J* = 8.8 Hz, 2H), 7.19 (d, *J* = 8.8 Hz, 1H), 7.15 (d, *J* = 2.4 Hz, 1H), 7.11 (d, *J* = 8.4 Hz, 2H), 6.83 (dd, *J* = 2.2, 9.0 Hz, 1H), 6.75 (d, *J* = 8.4 Hz, 2H), 5.21 (s, 2H), 5.12 (s, 4H), 2.17 (s, 3H).

### Step 2: Synthesis of intermediate WX055-3

Intermediate **WX055-2** (5.71 g, 9.70 mmol) and boracic acid (1.20 g, 19.40 mmol) were added to 1-methyl-2-pyrrolidinone (100 mL) at room temperature under nitrogen. Palladium acetate (217.82 mg, 970.21 _{f.l}mo1), di-tert-butyl-(2,4,6-triisopropyl-3,6-dimethoxybiphenyl-2-yl)phosphine (940.50 mg, 1.94 mmol) and cesium carbonate (6.32 g, 19.40 mmol) were added sequentially, and the reaction mixture was stirred to react at 100 °C under nitrogen for 12 h. After the reaction was completed, the mixture was cooled down to room temperature. The reaction solution was poured into water (200 mL) and the mixture was extracted with ethyl acetate (100 mL × 3). The organic phases were combined, washed with saturated brine (100 mL×3), dried over anhydrous sodium sulfate, and filtered. The solvent was removed from the filtrate under reduced pressure. The resulting residue was separated by column chromatography (eluent: petroleum ether/ethyl acetate = 10/1-5/1, v/v) to give the intermediate **WX055-3.** ¹H NMR (400 MHz, CDC1₃) δ: 7.53-7.32 (m, 10H), 7.26-7.22 (m, 2H), 7.15 (d, *J* = 2.4 Hz, 1H), 7.09 (d, *J* = 8.8 Hz, 1H), 7.05-7.00 (m, 2H), 6.90 (dd, *J* = 2.4, 8.8 Hz, 1H), 6.81 (d, *J* = 8.4 Hz, 2H), 8.71-6.66 (m, 2H), 5.15 (s, 2H), 5.11 (s, 4H), 2.26 (s, 3H).

### Step 3: Synthesis of intermediate WX055-4

Intermediate **WX055-3** (2 g, 3.80 mmol) was dissolved in N,N-dimethylformamide (30 mL) at room temperature under nitrogen, and cesium carbonate (2.48 g, 7.61 mmol) and ethyl 2-(2-(2-(2-(p-toluenesulfonyloxy)ethoxy)ethoxy)ethoxy)acetate (1.78 g, 4.57 mmol) were added sequentially. The reaction mixture was stirred to react at 80 °C under nitrogen for 12 hours. After the reaction was completed, the mixture was cooled down to room temperature. Water (150 mL) was added to the reaction solution and the resulting mixture was extracted with ethyl acetate (70 mL×3). The organic phases were combined, washed with saturated brine (70 mL×3), dried over anhydrous sodium sulfate, and filtered. The solvent was removed from the filtrate under reduced pressure. The resulting residue was separated by column chromatography (eluent: petroleum ether/ethyl acetate = 3/1-1/1, v/v) to give the intermediate **WX055-4.** ¹H NMR (400 MHz, CDC1₃) δ: 7.53-7.30 (m, 10H), 7.26-7.21 (m, 2H), 7.16 (d, *J* = 2.0 Hz, 1H), 7.08 (d, *J* = 8.8 Hz, 1H), 7.05-6.99 (m, 2H), 6.90 (dd, *J* = 2.8, 8.8 Hz, 1H), 6.86 (d, *J* = 8.8 Hz, 2H), 6.80-6.75 (m, 2H), 5.15 (s, 2H), 5.12 (s, 2H), 5.11 (s, 2H), 4.21 (q, *J* = 7.2 Hz, 2H), 4.15 (s, 2H), 4.09-4.05 (m, 2H), 3.85-3.81 (m, 2H), 3.76-3.66 (m, 8H), 2.26 (s, 3H), 1.28 (t, *J* = 7.2 Hz, 3H).

### Step 4: Synthesis of intermediate WX055-5

Intermediate **WX055-4** (1.5 g, 2.02 mmol) was dissolved in methanol (40 mL) at 20°C, and wet palladium on carbon (3 g, 10% content) was then added under nitrogen. The reaction system was purged three times with hydrogen, and the reaction mixture was stirred to react at 20°C under hydrogen (15 Psi) for 4 hours. After the reaction was completed, the reaction solution was filtered and the filtered palladium on carbon was poured into the corresponding catalyst recovery drum. The solvent was removed from the filtrate under reduced pressure to give the intermediate **WX055-5.** ¹H NMR (400 MHz, CDC1₃) δ: 7.09 (d, *J* = 8.4 Hz, 2H), 6.99 (d, *J* = 2.4 Hz, 1H), 6.97 (d, *J* = 8.8 Hz, 1H), 6.85 (d, *J* = 8.4 Hz, 2H), 6.77 (d, *J* = 8.4 Hz, 2H), 6.73-6.67 (m, 3H), 5.03 (s, 2H), 4.17 (q, *J* = 7.1 Hz, 2H), 4.11 (s, 2H), 4.03-3.97 (m, 2H), 3.80-3.76 (m, 2H), 3.72-3.63 (m, 8H), 2.14 (s, 3H), 1.23 (t, *J* = 7.0 Hz, 3H).

### Step 5: Synthesis of intermediate WX055-6

Intermediate **WX055-5** (650 mg, 1.15 mmol) was dissolved in a mixture of methanol (30 mL) and water (6 mL) at room temperature. Then sodium hydroxide solid (138.38 mg, 3.46 mmol) was added and the reaction mixture was stirred to react at 40 °C under nitrogen for 2 hours. After the reaction was completed, the mixture was cooled down to room temperature. The solvent was removed from the reaction solution under reduced pressure, and water (50 mL) was added to the residue. The solution was adjusted to pH of 4-5 with dilute hydrochloric acid (2M), and extracted with ethyl acetate (50 mL × 3). The organic phases were combined, washed with saturated brine (50 mL×3), dried over anhydrous sodium sulfate, and filtered. The solvent was removed from the filtrate under reduced pressure to give the intermediate **WX055-6.** ¹H NMR (400 MHz, DMSO_*d₆*) δ: 7.15 (d, *J* = 8.4 Hz, 2H), 7.06 (d, *J* = 8.8 Hz, 1H), 6.85 (d, *J* = 8.4 Hz, 2H), 6.80 (d, *J* = 2.0 Hz, 1H), 6.75 (s, 4H), 6.57 (dd, *J* = 2.2, 8.6 Hz, 1H), 5.10 (s, 2H), 3.99 (s, 2H), 3.99-3.95 (m, 2H), 3.71-3.64 (m, 2H), 3.58-3.48 (m, 8H), 2.10 (s, 3H).

### Step 6: Synthesis of compound WX055

Intermediate **WX055-6** (50 mg, 93.36 µmol) was dissolved in N,N-dimethylformamide (3 mL) at room temperature under nitrogen, and HATU (46.15 mg, 121.36 µmol), N,N-diisopropylethylamine (40.65 µ L, 233.39 µmol) and intermediate **BB-31** (22.80 mg, 93.36 µmol, hydrochloride) were added sequentially. The reaction mixture was stirred to react at room temperature under nitrogen for 1 hour. After the reaction was completed, water (50 mL) was added to the reaction solution and the resulting mixture was extracted with ethyl acetate (50 mL×3). The organic phases were combined, washed with saturated brine (50 mL×3), dried over anhydrous sodium sulfate, and filtered. The solvent was removed from the filtrate under reduced pressure. The resulting residue was separated by preparative HPLC (mobile phase: acetonitrile/water; acidic system: 0.05% HCl) to give the title compound **WX055.** MS-ESI m/z: 762.3 [M+H]⁺. ¹H NMR (400 MHz, DMSO_d₆) δ: 10.93 (s, 1H), 9.67 (s, 1H), 9.64 (s, 1H), 8.70 (s, 1H), 7.86 (s, 2H), 7.54-7.47 (m, 2H), 7.15 (d, *J* = 8.4 Hz, 2H), 7.05 (d, *J* = 8.8 Hz, 1H), 6.85 (d, *J* = 8.4 Hz, 2H), 6.80 (d, *J* = 2.0 Hz, 1H), 6.76-6.68 (m, 4H), 6.57 (dd, *J* = 2.0, 8.4 Hz, 1H), 5.09 (s, 2H), 4.09 (dd, *J* = 5.0, 12.2 Hz, 1H), 4.06 (s, 2H), 3.96-3.91 (m, 2H), 3.68-3.63 (m, 4H), 3.62-3.58 (m, 2H), 3.56 (s, 4H), 2.81-2.70 (m, 1H), 2.61-2.57 (m, 1H), 2.31-2.19 (m, 1H), 2.13-2.05 (m, 4H).

### Example 56

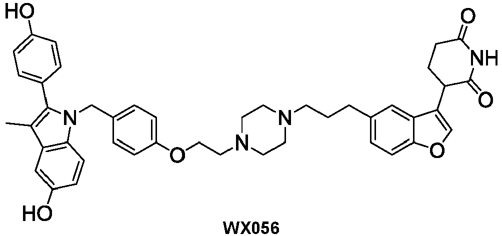

### Synthetic route:

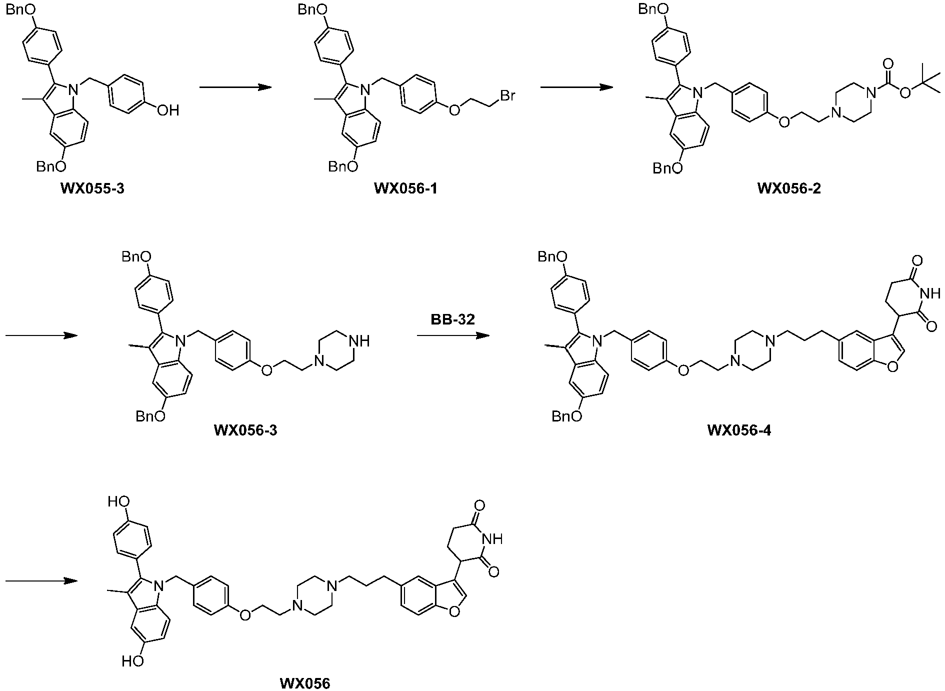

### Step 1: Synthesis of intermediate WX056-1

Intermediate **WX055-3** (4.2 g, 7.99 mmol) was dissolved in toluene (100 mL) at room temperature under nitrogen, and potassium carbonate (3.31 g, 23.97 mmol), 18-crown-6 (21.12 g, 79.90 mmol) and 1,2-dibromoethane (3.01 mL, 39.95 mmol) were added. The reaction mixture was stirred to react at 110 °C under nitrogen for 12 h. After the reaction was completed, the mixture was cooled down to room temperature. The solvent was removed by concentration under reduced pressure, and water (150 mL) was added to the residue. The mixture was extracted with ethyl acetate (100 mL×3). The organic phases were combined, washed with saturated brine (100 mL×3), dried over anhydrous sodium sulfate, and filtered. The solvent was removed from the filtrate under reduced pressure. The resulting residue was separated by column chromatography (eluent: petroleum ether/ethyl acetate = 20/1, v/v) to give the intermediate **WX056-1.** ¹H NMR (400MHz, DMSO_*d₆*) δ: 7.50-7.27 (m, 14H), 7.21 (d, *J* = 8.8 Hz, 1H), 7.15-7.10 (m, 3H), 6.81 (dd, *J* = 2.4, 8.8 Hz, 1H), 6.78-6.75 (m, 2H), 5.17 (s, 2H), 5.15 (s, 2H), 5.12 (s, 2H), 4.23-4.19 (m, 2H), 3.76-3.71 (m, 2H), 2.16 (s, 3H).

### Step 2: Synthesis of intermediate WX056-2

Intermediate **WX056-1** (1.20 g, 1.90 mmol) and 1-tert-butoxycarbonyl-piperazine (423.98 mg, 2.28 mmol) were dissolved in acetonitrile (20 mL) at room temperature. Potassium carbonate (524.36 mg, 3.79 mmol) was added. The reaction mixture was stirred to react at 80 °C under nitrogen for 12 h. After the reaction was completed, the mixture was cooled down to room temperature. Water (50 mL) was added to the resulting residue and the mixture was extracted with ethyl acetate (50 mL × 3). The organic phases were sequentially combined, washed with saturated brine (50 mL×3), dried over anhydrous sodium sulfate, and filtered. The filtrate was concentrated under reduced pressure to remove the solvent. The resulting residue was separated by column chromatography (eluent: petroleum ether/ethyl acetate = 1/0-1/1, v/v) to give the intermediate **WX056-2.** ¹H NMR (400 MHz, CDC1₃) δ: 7.51 (d, *J* = 7.2 Hz, 2H), 7.49-7.45 (m, 2H), 7.45-7.30 (m, 6H), 7.25 (d, *J* = 8.4 Hz, 2H), 7.17 (d, *J* = 2.0 Hz, 1H), 7.08 (d, *J* = 8.8 Hz, 1H), 7.03 (d, *J* = 8.8 Hz, 2H), 6.90 (dd, *J* = 2.2, 9.0 Hz, 1H), 6.88 (d, *J* = 8.8 Hz, 2H), 6.77 (d, *J* = 8.8 Hz, 2H), 5.15 (s, 2H), 5.13 (s, 2H), 5.11 (s, 2H), 4.06 (t, *J* = 5.2 Hz, 2H), 3.53-3.40 (m, 4H), 2.86-2.74 (m, 2H), 2.59-2.45 (m, 4H), 2.27 (s, 3H), 1.48 (s, 9H).

### Step 3: Synthesis of Intermediate WX056-3 hydrochloride

**WX056-2** (1.30 g, 1.76 mmol) was dissolved in ethyl acetate (10 mL) at room temperature. A solution of hydrogen chloride in ethyl acetate (4M, 4.40 mL) was added. The reaction mixture was stirred to react at room temperature for 12 hours. After the reaction was completed, the reaction solution was concentrated under reduced pressure to remove the solvent to give the intermediate **WX056-3** hydrochloride.

### Step 4: Synthesis of intermediate WX056-4

Intermediate **WX056-3** (500.00 mg, 741.54 µmol, hydrochloride) was dissolved in dichloromethane (10 mL) at room temperature. N,N-diisopropylethylamine (191.67 mg, 1.48 mmol) and intermediate **BB-32** (211.56 mg, 741.54 µmol) were added separately. The reaction mixture was stirred to react at room temperature under nitrogen for 3 hours. Sodium triacetoxyborohydride (157.16 mg, 741.54 µmol) was then added and the reaction mixture was stirred to react at room temperature for 10 hours.After the reaction was completed, water (30 mL) was added to the reaction solution and the resulting mixture was extracted with dichloromethane (30 mL×3). The organic phases were sequentially combined, washed with saturated brine (30 mL×3), dried over anhydrous sodium sulfate, and filtered. The filtrate was concentrated under reduced pressure to remove the solvent. The resulting residue was separated by column chromatography (eluent: dichloromethane/methanol = 1/0-20/1, v/v) to give the intermediate **WX056-4.** ¹H NMR (400 MHz, DMSO_*d₆*) δ: 10.89 (s, 1H), 7.85 (s, 1H), 7.53-7.28 (m, 14H), 7.21 (d, *J* = 8.8 Hz, 1H), 7.16-7.09 (m, 4H), 6.82 (dd, *J* = 2.0, 8.8 Hz, 1H), 6.75 (s, 4H), 5.16 (s, 2H), 5.15 (s, 2H), 5.13 (s, 2H), 4.11 (dd, *J* = 4.8, 12.0 Hz, 1H), 3.96 (br t, *J* = 5.6 Hz, 2H), 3.34 (s, 4H), 2.79-2.69 (m, 1H), 2.68-2.55 (m, 5H), 2.47-2.43 (m, 2H), 2.38-2.25 (m, 5H), 2.17 (s, 3H), 2.12-2.05 (m, 2H), 1.79-1.67 (m, 2H).

### Step 5: Synthesis of WX056

Intermediate **WX056-4** (200.00 mg, 220.48 µmol) was dissolved in a mixture of methanol (40 mL) and ethyl acetate (5 mL) at room temperature. To the mixture were added hydrochloric acid solution (12 M, 91.87 µL) and Raney nickel (30 mg). The reaction mixture was purged with hydrogen three times and stirred to react at room temperature under hydrogen (45 psi) for 12 hours. After the reaction was completed, the reaction solution was filtered. The filter cake was washed with methanol (30 mL×3) and the filtrate was concentrated under reduced pressure to remove the solvent. The resulting residue was separated by thin layer chromatography (developing agent: dichloromethane/methanol = 10/1, v/v) to give the title compound **WX056.** MS-ESI m/z: 727.3 [M+H]⁺. ¹H NMR (400 MHz, DMSO_*d₆*) δ: 10.87 (s, 1H), 9.66 (s, 1H), 8.69 (s, 1H), 7.84 (s, 1H), 7.44 (d, *J* = 8.8 Hz, 1H), 7.38 (s, 1H), 7.17-7.11 (m, 3H), 7.05 (d, *J* = 8.4 Hz, 1H), 6.85 (d, *J* = 8.0 Hz, 2H), 6.80 (d, *J* = 2.4 Hz, 1H), 6.75 (s, 4H), 6.57 (dd, *J* = 2.0, 8.8 Hz, 1H), 5.10 (s, 2H), 4.11 (dd, *J* = 5.0, 12.2 Hz, 1H), 3.95 (t, *J* = 5.8 Hz, 2H), 2.79-2.55 (m, 10H), 2.38-2.28 (m, 4H), 2.23 (br t, *J* = 7.2 Hz, 3H), 2.09 (s, 3H), 2.15-2.05 (m, 1H), 1.76-1.66 (m, 2H).

### Example 57

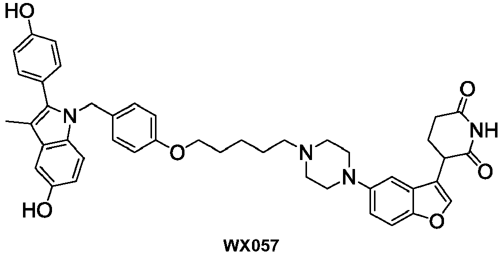

### Synthetic route:

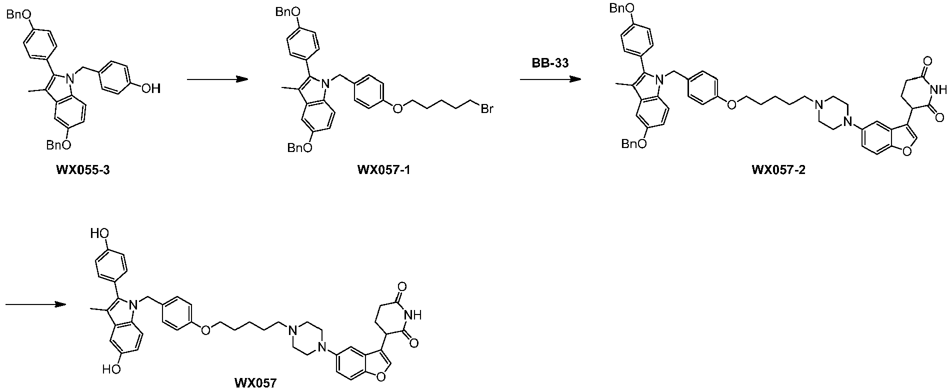

### Step 1: Synthesis of intermediate WX057-1

Intermediate **WX055-3** (770 mg, 1.46 mmol) was dissolved in N,N-dimethylformamide (20 mL) at room temperature under nitrogen, and potassium carbonate (404.91 mg, 2.93 mmol) and 1,5-dibromopentane (990.70 µL, 7.32 mmol) were added. The reaction mixture was stirred to react at 80 °C under nitrogen for 12 hours. After the reaction was completed, the mixture was cooled down to room temperature. Water (100 mL) was added to the reaction solution and the resulting mixture was extracted with ethyl acetate (50 mL×3). The organic phases were combined, washed with saturated brine (50 mL×3), dried over anhydrous sodium sulfate, and filtered. The solvent was removed from the filtrate under reduced pressure. The resulting residue was separated by column chromatography (eluent: petroleum ether/ethyl acetate = 20/1-10/1, v/v) to give the intermediate **WX057-1.** MS-ESI *m*/*z*: 674.0 [M+H]⁺, 676.0 [M+2+H]⁺.

### Step 2: Synthesis of intermediate WX057-2

Intermediate **WX057-1** (428.35 µmol) was dissolved in acetonitrile (10 mL) at room temperature under nitrogen, and sodium bicarbonate (179.93 mg, 2.14 mmol) and intermediate **BB-33** (172.43 mg, 471.19 µmol, purity: 95.59%) were added. The reaction mixture was stirred to react at 80 °C under nitrogen for 12 hours. After the reaction was completed, the mixture was cooled down to room temperature. The solvent was removed from the reaction solution under reduced pressure. Water (50 mL) was added to the resulting residue and the mixture was extracted with ethyl acetate (50 mL×3). The organic phases were combined, washed with water (50 mL×2), dried over anhydrous sodium sulfate, and filtered. The solvent was removed from the filtrate under reduced pressure. The resulting residue was separated by column chromatography (eluent: dichloromethane/methanol = 20/1, v/v) to give the intermediate **WX057-2.** MS-ESI *m*/*z*: 907.5 [M+H]⁺.

### Step 3: Synthesis of WX057

Intermediate **WX057-2** (153.21 µmol) was dissolved in a mixture of methanol (5 mL) and ethyl acetate (5 mL) at room temperature under nitrogen. Hydrochloric acid (63.84 µL, 766.05 µmol) and Raney nickel (13.13 mg, 153.21 µmol) were added and the reaction system was purged three times with hydrogen. The reaction mixture was stirred to react at room temperature under hydrogen (45 Psi) for 12 h. After the reaction was completed, the reaction solution was filtered and the solvent was removed from the filtrate under reduced pressure. The resulting residue was separated by thin layer chromatography (developing agent: dichloromethane/methanol = 20/1, v/v) to give the title compound **WX057.** MS-ESI *m*/*z*: 727.4 [M+H]⁺. ¹H NMR (400 MHz, DMSO_d₆) δ: 10.85 (s, 1H), 9.66 (s, 1H), 8.69 (s, 1H), 7.77 (s, 1H), 7.40 (d, *J* = 9.2 Hz, 1H), 7.15 (d, *J* = 8.8 Hz, 2H), 7.06 (d, *J* = 8.8 Hz, 1H), 7.03 (d, *J* = 2.0 Hz, 1H), 6.98 (dd, *J* = 2.2, 9.0 Hz, 1H), 6.85 (d, *J* = 8.4 Hz, 2H), 6.80 (d, *J* = 2.0 Hz, 1H), 6.74 (br s, 4H), 6.57 (dd, *J* = 2.4, 8.8 Hz, 1H), 5.09 (s, 2H), 4.09 (dd, *J* = 4.8, 11.6 Hz, 1H), 3.86 (t, *J* = 6.4 Hz, 2H), 3.10-3.06 (m, 4H), 2.95-2.87 (m, 1H), 2.77-2.69 (m, 1H), 2.69-2.65 (m, 2H), 2.35-2.29 (m, 4H), 2.21-2.15 (m, 1H), 2.09 (s, 3H), 2.08-2.04 (m, 1H), 1.70-1.63 (m, 2H), 1.53-1.44 (m, 2H), 1.43-1.35 (m, 2H).

### Example 58

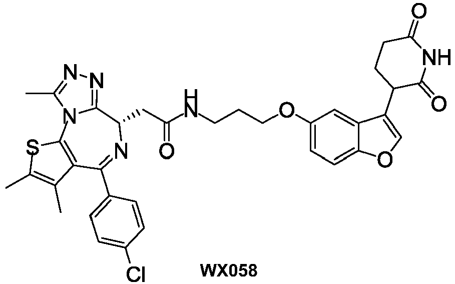

### Synthetic route:

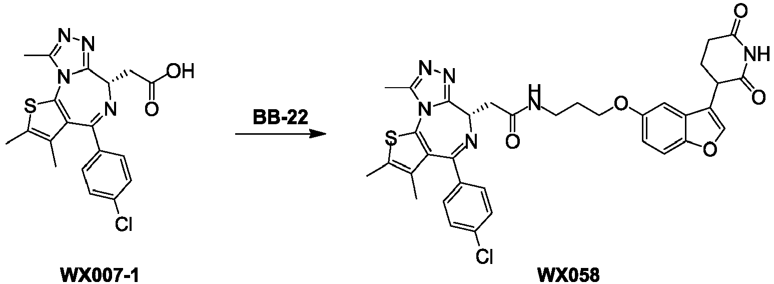

**WX007-1** (50 mg, 124.73 µmol) was dissolved in N,N-dimethylformamide (20 mL) at 20 °C, and O-(7-azabenzotriazol-1-yl)-N,N,N,N-tetramethyluronium hexafluorophosphate (61.65 mg, 162.14 µmol) and N,N-diisopropylethylamine (64.48 mg, 498.90 µmol) were then added. The reaction mixture was stirred to react at 20 °C under nitrogen for 0.5 h, and intermediate **BB-22** (66.18 mg, 137.20 µmol, hydrochloride) was then added. The reaction mixture was stirred to react at 20 °C under nitrogen for 2 h. After the reaction was completed, the reaction solution was cooled down to room temperature, poured into water (50 mL), and extracted with ethyl acetate (10 mL×3). The organic phases were combined, washed with saturated brine (50 mL×2), dried over anhydrous sodium sulfate, and filtered. The solvent was removed from the filtrate under reduced pressure. The resulting residue was separated by preparative HPLC (mobile phase: acetonitrile/water; acidic system: 0.05% HCl) to give the title compound **WX058.** ¹H NMR (400 MHz, DMSO_*d₆*) δ: 10.87 (s, 1H), 8.37 (br t, *J* = 5.2 Hz, 1H), 7.84 (s, 1H), 7.47-7.38 (m, 5H), 7.12 (d, *J* = 2.0 Hz, 1H), 6.91 (dd, *J* = 2.6, 9.0 Hz, 1H), 4.56 (t, *J* = 7.0 Hz, 1H), 4.13-4.07 (m, 1H), 4.07-3.99 (m, 2H), 3.35-3.19 (m, 4H), 2.77-2.65 (m, 1H), 2.63 (s, 3H), 2.60-2.52 (m, 1H), 2.41 (s, 3H), 2.38-2.26 (m, 1H), 2.13-2.04 (m, 1H), 1.96-1.87 (m, 2H), 1.59 (s, 3H).

### Example 59

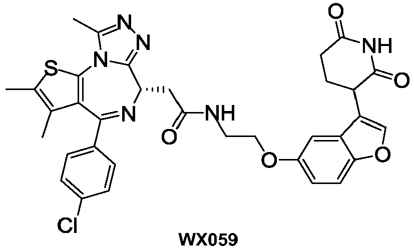

### Synthetic route:

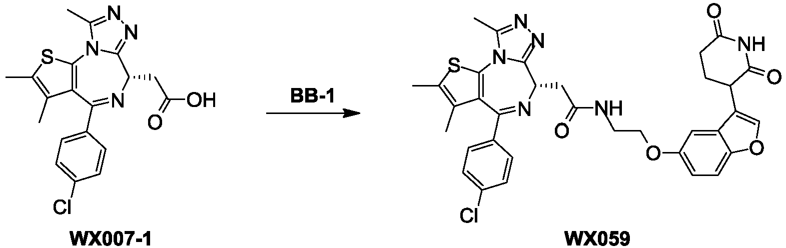

**WX007-1** (50 mg, 124.73 µmol) was dissolved in N,N-dimethylformamide (20 mL) at 20 °C, and O-(7-azabenzotriazol-1-yl)-N,N,N,N-tetramethyluronium hexafluorophosphate (61.65 mg, 162.15 µmol) and N,N-diisopropylethylamine (64.48 mg, 498.92 µmol) were then added. The reaction mixture was stirred to react at 20 °C under nitrogen for 0.5 h, and intermediate **BB-1** (57.13 mg, 137.20 µmol, hydrochloride) was then added. The reaction mixture was stirred to react at 20 °C under nitrogen for another 14 h. After the reaction was completed, the reaction solution was cooled down to room temperature, poured into water (50 mL), and extracted with ethyl acetate (10 mL×3). The organic phases were combined, washed with saturated brine (50 mL×2), dried over anhydrous sodium sulfate, and filtered. The solvent was removed from the filtrate under reduced pressure. The resulting residue was separated by preparative HPLC (mobile phase: acetonitrile/water; acidic system: 0.05% HCl) to give the title compound WX059. ¹H NMR (400 MHz, DMSO_*d₆*) δ: 10.86 (d, *J* = 4.4 Hz, 1H), 8.54 (br t, *J* = 5.4 Hz, 1H), 7.86 (s, 1H), 7.48 (d, *J* = 8.8 Hz, 1H), 7.38-7.33 (m, 2H), 7.31-7.26 (m, 2H), 7.17 (d, *J* = 2.4 Hz, 1H), 6.94 (br d, *J* = 9.2 Hz, 1H), 4.53 (dd, *J* = 5.8, 8.2 Hz, 1H), 4.10 (dd, *J* = 4.8, 11.6 Hz, 1H), 4.07-4.01 (m, 2H), 3.62-3.53 (m, 1H), 3.52-3.45 (m, 1H), 3.35-3.29 (m, 1H), 3.20-3.16 (m, 1H), 2.76-2.64 (m, 1H), 2.59 (s, 3H), 2.58-2.53 (m, 1H), 2.41 (s, 3H), 2.37-2.27 (m, 1H), 2.12-2.02 (m, 1H), 1.60 (s, 3H).

### Example 60

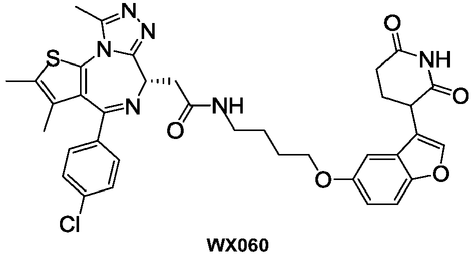

### Synthetic route:

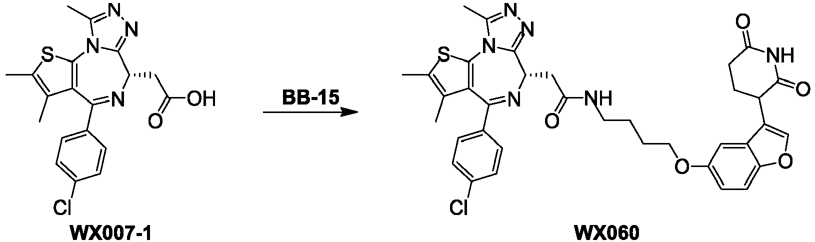

**WX007-1** (50 mg, 124.73 µmol) was dissolved in N,N-dimethylformamide (20 mL) at 20 °C, and O-(7-azabenzotriazol-1-yl)-N,N,N,N-tetramethyluronium hexafluorophosphate (61.65 mg, 162.15 µmol) and N,N-diisopropylethylamine (64.48 mg, 498.92 µmol) were then added. The reaction mixture was stirred to react at 20 °C under nitrogen for 0.5 h, and intermediate **BB-15** (45.61 mg, 123.02 µmol, hydrochloride) was then added. The reaction mixture was stirred to react at 20 °C under nitrogen for another 14 h. After the reaction was completed, the reaction solution was cooled down to room temperature, poured into water (50 mL), and extracted with ethyl acetate (10 mL×3). The organic phases were combined, washed with saturated brine (50 mL×2), dried over anhydrous sodium sulfate, and filtered. The solvent was removed from the filtrate under reduced pressure. The resulting residue was separated by preparative HPLC (mobile phase: acetonitrile/water; acidic system: 0.05% HCl) to give the title compound **WX060.** ¹H NMR (400 MHz, DMSO_*d₆*) δ: 10.87 (s, 1H), 8.27 (br t, *J* = 5.8 Hz, 1H), 7.83 (s, 1H), 7.48-7.40 (m, 5H), 7.09 (s, 1H), 6.88 (dd, *J* = 2.2, 9.0 Hz, 1H), 4.55 (dd, *J* = 6.2, 8.2 Hz, 1H), 4.14-4.06 (m, 1H), 4.03-3.95 (m, 2H), 3.32-3.12 (m, 4H), 2.77-2.65 (m, 1H), 2.62 (s, 3H), 2.60-2.53 (m, 1H), 2.41 (s, 3H), 2.38-2.25 (m, 1H), 2.13-2.04 (m, 1H), 1.82-1.72 (m, 2H), 1.67-1.61 (m, 2H), 1.61 (s, 3H).

### Example 61

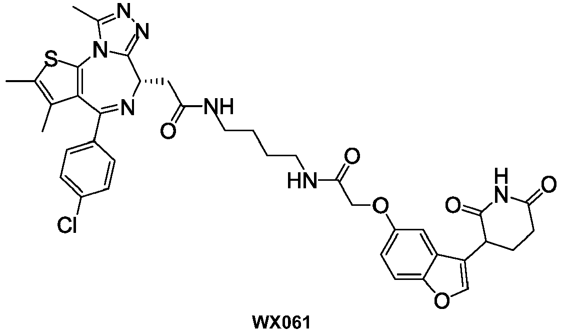

### Synthetic route:

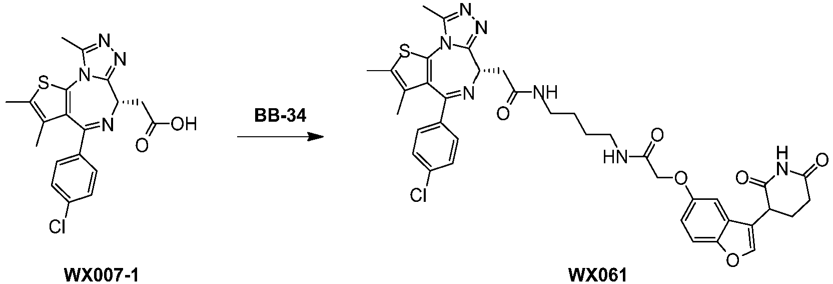

**WX007-1** (50.00 mg, 124.73 µmol) was dissolved in N,N-dimethylformamide (10 mL) at room temperature. N,N-diisopropylethylamine (80.60 mg, 623.63 µmol) and O-(7-azabenzotriazol-1-yl)-N,N,N,N-tetramethyluronium hexafluorophosphate (71.14 mg, 187.09 µmol) were added separately. The mixture solution was reacted with stirring at room temperature for half an hour. Intermediate **BB-34** (51.12 mg, 124.73 µmol, hydrochloride) was then added. The reaction mixture was stirred to react at room temperature for 12 hours. After the reaction was completed, water (40 mL) was added to the reaction solution and the resulting mixture was extracted with ethyl acetate (20 mL×3). The organic phases were sequentially combined, washed with saturated brine (40 mL×2), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the title compound **WX061.** MS-ESI *m*/*z*: 756.5 [M+H]⁺. ¹H NMR (400 MHz, DMSO_*d₆*) δ: 10.89 (s, 1H), 8.21 (s, 1H), 8.12 (s, 1H), 7.86 (s, 1H), 7.54-7.37 (m, 5H), 7.16 (s, 1H), 6.99 (d, *J* = 8.4 Hz, 1H), 4.52 (t, *J* = 7.0 Hz, 1H), 4.46 (s, 2H), 4.09 (dd, *J* = 4.0, 11.6 Hz, 1H), 3.30-3.01 (m, 6H), 2.82-2.65 (m, 1H), 2.65-2.55 (m, 4H), 2.40 (s, 3H), 2.37-2.27 (m, 1H), 2.14-2.03 (m, 1H), 1.61 (s, 3H), 1.55-1.37 (m, 4H).

### Example 62

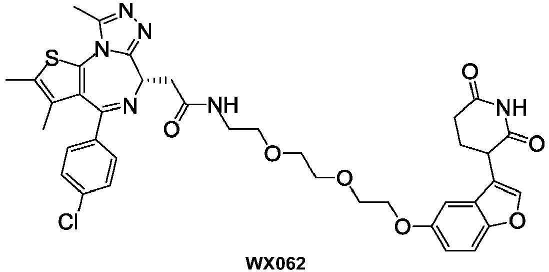

### Synthetic route:

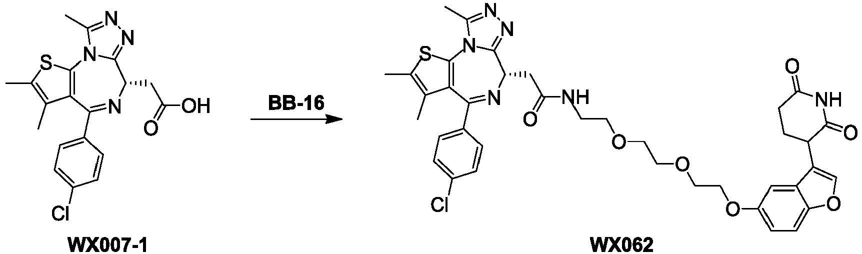

**WX007-1** (50 mg, 124.73 µmol) was dissolved in N,N-dimethylformamide (20 mL) at 20 °C, and O-(7-azabenzotriazol-1-yl)-N,N,N,N-tetramethyluronium hexafluorophosphate (61.65 mg, 162.15 µmol) and N,N-diisopropylethylamine (64.48 mg, 498.92 µmol) were then added. The reaction mixture was stirred to react at 20 °C under nitrogen for 0.5 h, and intermediate **BB-16** (57.12 mg, 124.73 µmol, hydrochloride) was then added. The reaction mixture was stirred to react at 20 °C under nitrogen for another 14 h. After the reaction was completed, the reaction solution was cooled down to room temperature, poured into water (50 mL), and extracted with ethyl acetate (10 mL×3). The organic phases were combined, washed with saturated brine (50 mL×2), dried over anhydrous sodium sulfate, and filtered. The solvent was removed from the filtrate under reduced pressure. The resulting residue was separated by preparative HPLC (mobile phase: acetonitrile/water; acidic system: 0.05% HCl) to give the title compound **WX062.** ¹H NMR (400 MHz, DMSO_*d₆*) δ: 10.87 (s, 1H), 8.31 (br t, *J* = 5.6 Hz, 1H), 7.84 (s, 1H), 7.51-7.40 (m, 5H), 7.11 (d, *J* = 2.4 Hz, 1H), 6.91 (dd, *J* = 2.4, 8.8 Hz, 1H), 4.53 (t, *J* = 7.0 Hz, 1H), 4.15-4.04 (m, 3H), 3.76 (t, *J* = 4.6 Hz, 2H), 3.65-3.56 (m, 4H), 3.48-3.40 (m, 2H), 3.34-3.17 (m, 4H), 2.77-2.65 (m, 1H), 2.61 (s, 3H), 2.59-2.53 (m, 1H), 2.40 (s, 3H), 2.38-2.28 (m, 1H), 2.13-2.04 (m, 1H), 1.61 (s, 3H).

### Example 63

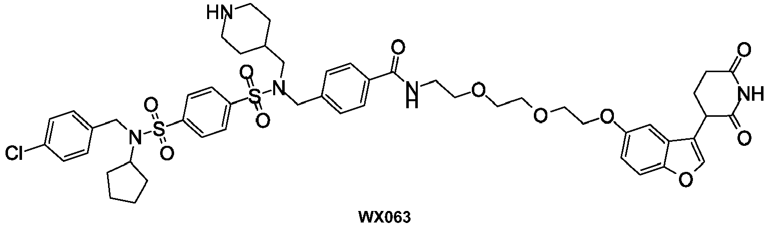

### Synthetic route:

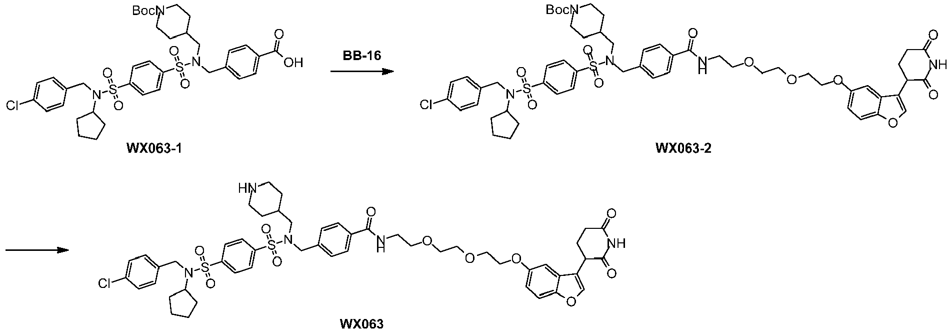

### Step 1: Synthesis of intermediate WX063-2

Intermediate **BB-16** (200 mg, 484.42 µmol, hydrochloride), compound **WX063-1** (368.33 mg, 484.42 _{f.l}mo1), and N,N-diisopropylethylamine (187.82 mg, 1.45 mmol, 253.13 µL) were dissolved in N,N-dimethylformamide (5 mL). The atmosphere was then replaced three times with nitrogen. The mixture was cooled down to 0 °C and O-(7-azabenzotriazol-1-yl)-N,N,N,N-tetramethyluronium hexafluorophosphate (368.38 mg, 968.84 µmol) was added. The reaction mixture was stirred at 25 °C for 2 h. After the reaction was completed, the reaction solution was diluted with ethyl acetate (50 mL) and half-saturated brine (20 mL). The organic phase was separated, dried over anhydrous sodium sulfate, and filtered. The filtrate was concentrated under reduced pressure to remove the solvent to give the intermediate **WX063-2**.

### Step 2: Synthesis of compound WX063 hydrochloride

Intermediate **WX063-2** (380 mg, 339.67 µmol) was added to hydrochloric acid/ethyl acetate solution (4 M, 20 mL) and the mixture was stirred at 20 °C for 30 min. After the reaction was completed, the reaction solution was concentrated under reduced pressure to give a crude product, which was separated by preparative HPLC (mobile phase: acetonitrile/water; acidic system: 0.04% HCl) to give the title compound **WX063** hydrochloride. MS-ESI m/z: 1018.3 [M+H]⁺. ¹H NMR (400 MHz, DMSO_*d₆*) δ: 10.88 (s, 1H), 8.73 (d, *J* = 9.6 Hz, 1H), 8.55 (br t, *J* = 5.6 Hz, 1H), 8.47 (d, *J* = 10.0 Hz, 1H), 8.12-8.02 (m, 4H), 7.84 (s, 1H), 7.81 (d, *J* = 8.4 Hz, 2H), 7.48-7.38 (m, 5H), 7.32 (d, *J* = 8.0 Hz, 2H), 7.11 (d, *J* = 2.4 Hz, 1H), 6.89 (dd, *J* = 2.6, 9.0 Hz, 1H), 4.40 (s, 4H), 4.34-4.22 (m, 1H), 4.15-4.02 (m, 3H), 3.74 (t, *J* = 4.6 Hz, 2H), 3.64-3.49 (m, 6H), 3.46-3.37 (m, 2H), 3.21-3.11 (m, 2H), 3.06 (d, *J* = 7.2 Hz, 2H), 2.79-2.68 (m, 1H), 2.67-2.53 (m, 3H), 2.41-2.28 (m, 1H), 2.14-2.04 (m, 1H), 1.71-1.56 (m, 3H), 1.53-1.41 (m, 4H), 1.40-1.29 (m, 2H), 1.25-1.08 (m, 4H).

### Example 64

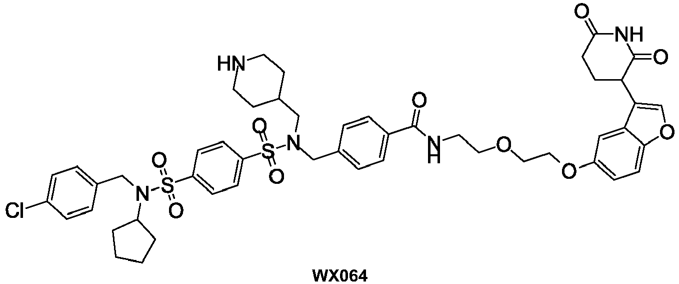

### Synthetic route:

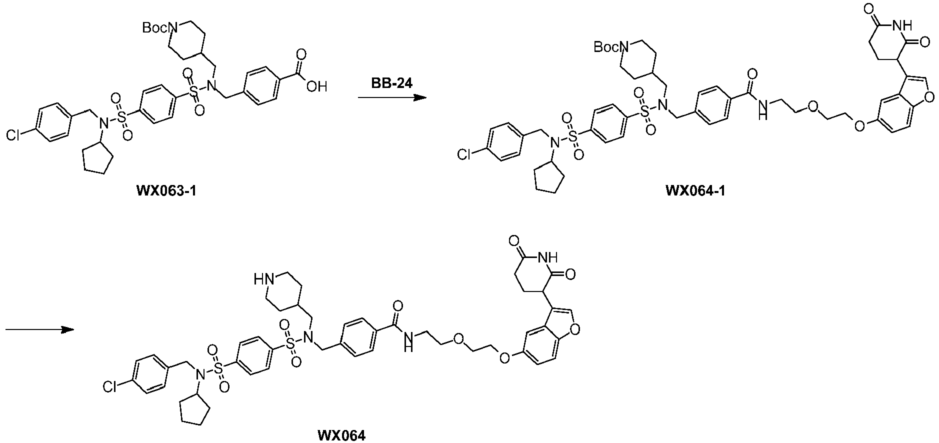

### Step 1: Synthesis of intermediate WX064-1

Compound **WX063-1** (100 mg, 131.52 µmol) and N,N-diisopropylethylamine (50.99 mg, 394.55 µmol, 68.72 µL) were dissolved in N,N-dimethylformamide (1 mL). The atmosphere was replaced three times with nitrogen. The mixture was cooled down to 0 °C and O-(7-azabenzotriazol-1-yl)-N,N,N,N-tetramethyluronium hexafluorophosphate (75.01 mg, 197.28 µmol) was added. The reaction mixture was warmed up to 20 °C and stirred for 30 min. Intermediate **BB-24** (48.50 mg, 131.52 µmol, hydrochloride) was added, and the mixture was stirred for another 3h. After the reaction was completed, water (1 mL) and ethyl acetate (5 mL) were added to the reaction solution. The layers were separated. The organic phase was dried over anhydrous sodium sulfate, and filtered. The filtrate was concentrated under reduced pressure to give the intermediate **WX064-1.**

### Step 2: Synthesis of compound WX064 hydrochloride

Hydrochloric acid /ethyl acetate (4 M, 20 mL) was added to intermediate **WX064-1** (100 mg, 93.05 µmol), and the mixture was stirred at 20 °C for 2 h. After the reaction was completed, the reaction solution was concentrated under reduced pressure. The resulting residue was separated by preparative HPLC (mobile phase: acetonitrile/water; acidic system: 0.05% HCl) to give the title compound **WX064** hydrochloride. MS-ESI *m*/*z*: 974.3 [M+H]⁺. ¹H NMR (400 MHz, DMSO_*d₆*) δ: 10.88 (s, 1H), 8.66 (br s, 1H), 8.57 (br t, *J* = 5.6 Hz, 1H), 8.39 (br s, 1H), 8.12-8.04 (m, 4H), 7.85 (s, 1H), 7.81 (d, *J* = 8.4 Hz, 2H), 7.47-7.38 (m, 5H), 7.33 (d, *J* = 8.4 Hz, 2H), 7.11 (d, *J* = 2.4 Hz, 1H), 6.89 (dd, *J* = 2.4, 9.2 Hz, 1H), 4.40 (s, 4H), 4.33-4.22 (m, 1H), 4.16-4.03 (m, 3H), 3.77 (t, *J* = 4.6 Hz, 2H), 3.65-3.56 (m, 2H), 3.49-3.39 (m, 2H), 3.21-3.11 (m, 2H), 3.05 (br d, *J* = 7.2 Hz, 2H), 2.78-2.66 (m, 1H), 2.65-2.51 (m, 3H), 2.41-2.28 (m, 1H), 2.13-2.04 (m, 1H), 1.72-1.55 (m, 3H), 1.54-1.41 (m, 4H), 1.40-1.30 (m, 2H), 1.23-1.09 (m, 4H).

### Example 65

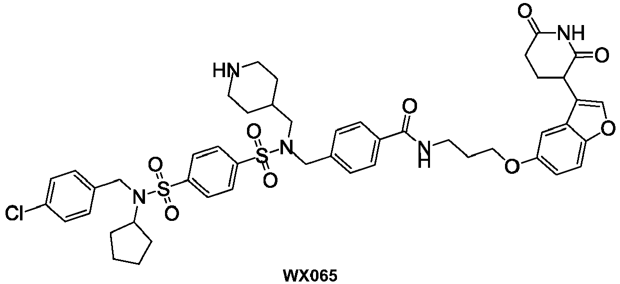

### Synthetic route:

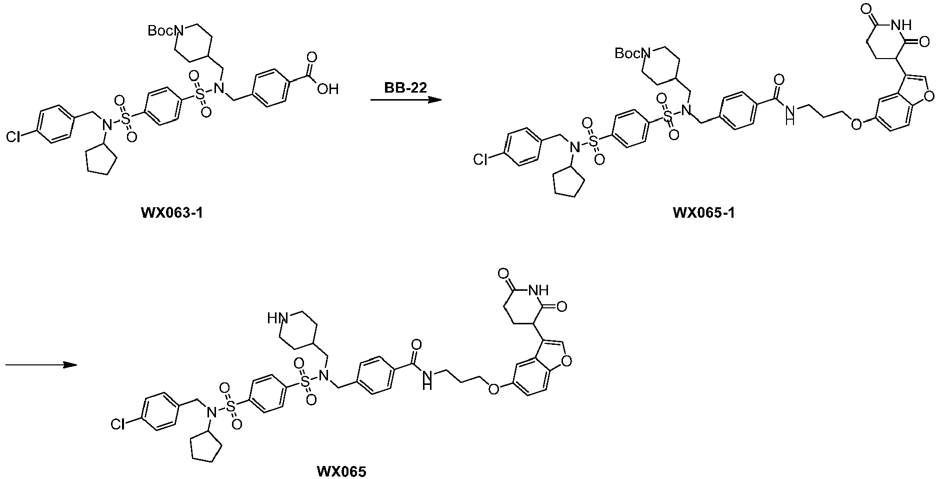

### Step 1: Synthesis of intermediate WX065-1

Compound **WX063-1** (112.22 mg, 147.59 µmol) and N,N-diisopropylethylamine (57.22 mg, 442.76 µmol, 77.12 µL,) were dissolved in N,N-dimethylformamide (1 mL). The atmosphere was replaced three times with nitrogen. The mixture was cooled down to 0 °C and O-(7-azabenzotriazol-1-yl)-N,N,N,N-tetramethyluronium hexafluorophosphate (112.23 mg, 295.17 µmol) was added. The reaction mixture was warmed up to 20 °C and stirred for 30 min. Intermediate **BB-22** (50 mg, 147.59 µmol, hydrochloride) was added, and the mixture was stirred for another 3h. After the reaction was completed, water (1 mL) and ethyl acetate (5 mL) were added to the reaction solution. The layers were separated. The organic phase was dried over anhydrous sodium sulfate, and filtered. The filtrate was concentrated under reduced pressure to give the intermediate **WX065-1.**

### Step 2: Synthesis of compound WX065 hydrochloride

Hydrochloric acid/ethyl acetate (4 M, 20 mL) was added to intermediate **WX065-1** (120 mg, 114.87 µmol), and the mixture was stirred at 20 °C for 2 h. After the reaction was completed, the reaction solution was concentrated under reduced pressure. The resulting residue was separated by preparative HPLC (mobile phase: acetonitrile/water; acidic system: 0.05% HCl) to give the title compound **WX065** hydrochloride. MS-ESI mlz: 944.3 [M+H]⁺. ¹H NMR (400 MHz, DMSO_*d₆*) δ: 10.87 (s, 1H), 8.67 (br d, *J* = 9.2 Hz, 1H), 8.60 (br t, *J* = 5.4 Hz, 1H), 8.40 (br d, *J* = 10.0 Hz, 1H), 8.12-8.05 (m, 4H), 7.85 (s, 1H), 7.81 (d, *J* = 8.4 Hz, 2H), 7.46 (d, *J* = 8.8 Hz, 1H), 7.44-7.38 (m, 4H), 7.33 (d, *J* = 8.0 Hz, 2H), 7.11 (d, *J* = 2.4 Hz, 1H), 6.91 (dd, *J* = 2.2, 9.0 Hz, 1H), 4.41 (s, 2H), 4.40 (s, 2H), 4.33-4.22 (m, 1H), 4.13-4.00 (m, 3H), 3.48-3.40 (m, 2H), 3.22-3.12 (m, 2H), 3.06 (d, *J* = 7.2 Hz, 2H), 2.79-2.52 (m, 4H), 2.40-2.27 (m, 1H), 2.14-2.04 (m, 1H), 2.03-1.94 (m, 2H), 1.73-1.57 (m, 3H), 1.54-1.41 (m, 4H), 1.41-1.29 (m, 2H), 1.25-1.09 (m, 4H).

### Example 66

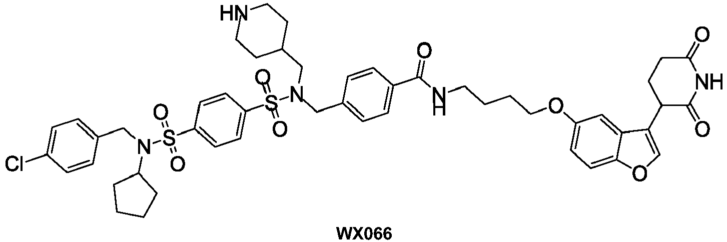

### Synthetic route:

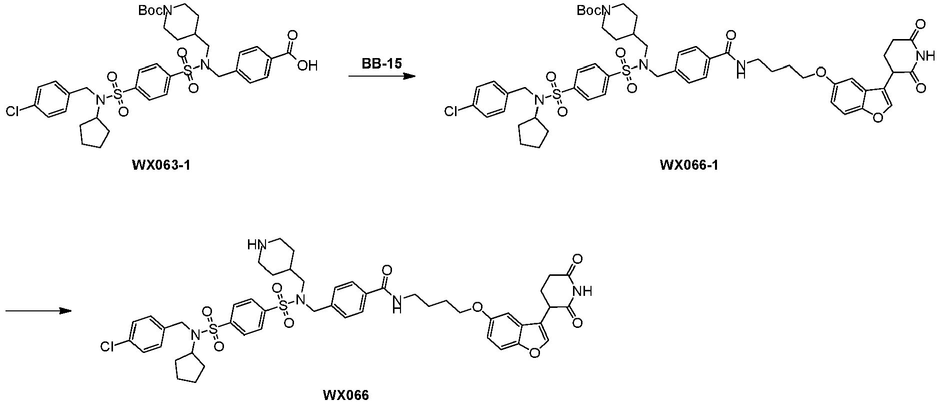

### Step 1: Synthesis of intermediate WX066-1

Intermediate **BB-15** (50 mg, 158.05 µmol, hydrochloride), compound **WX063-1** (120.18 mg, 158.05 µmol) and N,N-diisopropylethylamine (61.28 mg, 474.16 µmol, 82.59 µL) were dissolved in N,N-dimethylformamide (1 mL). The mixture was protected under nitrogen and cooled down to 0 °C. O-(7-azabenzotriazol-1-yl)-N,N,N,N-tetramethyluronium hexafluorophosphate (120.19 mg, 316.10 µmol) was added, and the mixture was stirred at 25 °C for 2 h. After the reaction was completed, the reaction solution was diluted with ethyl acetate (50 mL) and water (20 mL), and the organic phase was separated. The organic phase was dried over anhydrous sodium sulfate, and filtered. The filtrate was concentrated under reduced pressure to give the intermediate **WX066-1.**

### Step 4: Synthesis of compound WX066 hydrochloride

Intermediate **WX066-1** (158.05 µmol) was dissolved in hydrochloric acid/ethyl acetate solution (4 M, 5 mL), and the mixture was stirred at 20 °C for 1 h. After the reaction was completed, the reaction solution was concentrated under reduced pressure and the resulting crude product was separated by preparative HPLC (mobile phase: acetonitrile/water; acidic system: 0.05% HCl) to give the title compound **WX066** hydrochloride. MS-ESI *m*/*z*: 958.3 [M+H]⁺. ¹H NMR (400 MHz, DMSO_*d₆*) δ: 10.88 (s, 1H), 8.63 (d, *J* = 9.2 Hz, 1H), 8.53 (br t, *J* = 5.4 Hz, 1H), 8.43 (br d, *J* = 8.8 Hz, 1H), 8.12-8.02 (m, 4H), 784 (s, 1H), 7.80 (d, *J* = 8.0 Hz, 2H), 7.48-7.37 (m, 5H), 7.32 (d, *J* = 8.0 Hz, 2H), 7.09 (d, *J* = 2.4 Hz, 1H), 6.89 (dd, *J* = 2.2, 9.0 Hz, 1H), 4.41 (s, 2H), 4.40 (s, 2H), 4.33-4.22 (m, 1H), 4.10 (dd, *J* = 4.8, 12.0 Hz, 1H), 4.05-3.93 (m, 2H), 3.39-3.34 (m, 2H), 3.22-3.13 (m, 2H), 3.06 (d, *J* = 7.2 Hz, 2H), 2.79-2.52 (m, 4H), 2.40-2.26 (m, 1H), 2.14-2.04 (m, 1H), 1.82-1.57 (m, 7H), 1.54-1.41 (m, 4H), 1.41-1.29 (m, 2H), 1.25-1.08 (m, 4H).

### Example 67

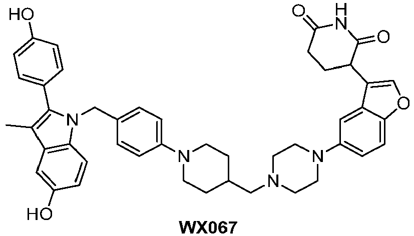

### Synthetic route:

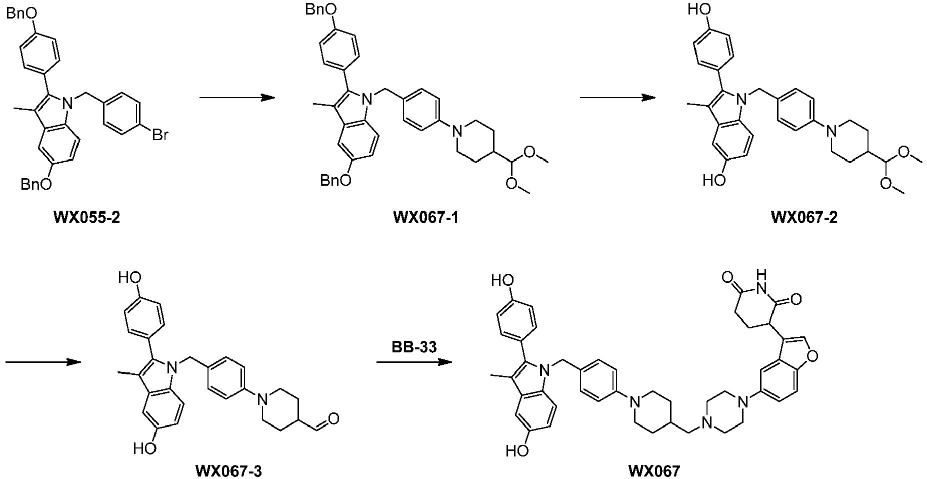

### Step 1: Synthesis of intermediate WX067-1

Intermediate **WX055-2** (700 mg, 1.19 mmol) and 4-(dimethoxymethyl)piperidine (284.07 mg, 1.78 mmol, 19.64 µL) were dissolved in toluene (10 mL). Subsequently, potassium tert-butoxide (400.39 mg, 3.57 mmol), 2-dicyclohexylphosphino-2,4,6-triisopropylbiphenyl (113.40 mg, 237.88 µmol) and palladium acetate (40.05 mg, 178.41 µmol) were added sequentially. The reaction mixture was reacted at 90 °C for 16 h. After the reaction was completed, the reaction solution was diluted with ethyl acetate (50 mL) and water (20 mL), and the organic phase was separated. The organic phase was dried over anhydrous sodium sulfate, and filtered. The filtrate was concentrated under reduced pressure to remove the solvent. The resulting residue was separated by column chromatography (eluent: petroleum ether/ethyl acetate = 1/0-1/1, v/v) to give the intermediate **WX067-1.**

### Step 2: Synthesis of intermediate WX067-2

Wet palladium on carbon (0.3 g, purity: 10%) was added to methanol (5 mL), and intermediate **WX067-1** (450 mg, 674.82 µmol) was then added. The mixture was reacted at 20 °C under hydrogen (15 Psi) for 16 h. After the reaction was completed, the reaction solution was filtered through celite. The filter cake was washed with methanol (50 mL×2), and the filtrate was concentrated under reduced pressure to remove the solvent. The resulting residue was separated by column chromatography (eluent: petroleum ether/ethyl acetate = 1/0-0/1, v/v) to give the intermediate **WX067-2.**

### Step 3: Synthesis of intermediate WX067-3

Intermediate **WX067-2** (220 mg, 452.12 µmol) was dissolved in tetrahydrofuran (9 mL), and sulfuric acid (2 M, 9.04 mL) was then added. The reaction solution was reacted at 20 °C for 12 h. After the reaction was completed, the reaction solution was adjusted to pH of 7 with saturated sodium bicarbonate and extracted with ethyl acetate (50 mL×2). The organic phases were combined, dried over anhydrous sodium sulfate, and filtered. The filtrate was concentrated under reduced pressure to remove the solvent to give the intermediate **WX067-3.**

### Step 4: Synthesis of compound WX067

Intermediate **BB-33** (196.13 mg, 560.69 µmol, hydrochloride) was dissolved in methanol (2 mL) and dichloromethane (2 mL), and sodium acetate (70.76 mg, 862.59 µmol) was then added. The reaction mixture was stirred at 20 °C for 0.5 h. Subsequently, intermediate **WX067-3** (190 mg, 431.30 µmol) and sodium cyanoborohydride (81.31 mg, 1.29 mmol) were added, and the reaction solution was stirred at 20 °C for another 12 h. After the reaction was completed, the reaction solution was adjusted to pH of 6-7 by adding saturated aqueous sodium bicarbonate solution, and extracted with ethyl acetate (20 mL×2). The organic phases were combined and concentrated under reduced pressure to remove the solvent. The resulting residue was separated by thin layer chromatography (developing agent: dichloromethane/methanol = 18/1-15/1, v/v) to give the title compound **WX067.** MS-ESI *m*/*z*: 738.3 [M+H]⁺. ¹H NMR (400 MHz, DMSO_*d₆*) δ: 10.86 (s, 1H), 9.66 (s, 1H), 8.67 (s, 1H), 7.78 (s, 1H), 7.40 (d, *J* = 8.8 Hz, 1H), 7.17 (d, *J* = 8.8 Hz, 2H), 7.06 (d, *J* = 8.8 Hz, 1H), 7.04 (d, *J* = 2.0 Hz, 1H), 6.99 (dd, *J* = 2.2, 9.0 Hz, 1H), 6.86 (d, *J* = 8.4 Hz, 2H), 6.79 (d, *J* = 2.0 Hz, 1H), 6.77-6.67 (m, 4H), 6.57 (dd, *J* = 2.2, 8.6 Hz, 1H), 5.06 (s, 2H), 4.08 (dd, *J* = 4.8, 12.0 Hz, 1H), 3.63-3.51 (m, 2H), 3.16-2.99 (m, 4H), 2.79-2.65 (m, 1H), 2.64-2.53 (m, 5H), 2.41-2.26 (m, 3H), 2.24-2.15 (m, 2H), 2.13-2.03 (m, 4H), 1.81-1.71 (m, 2H), 1.70-1.58 (m, 1H), 1.22-1.09 (m, 2H).

### Example 68

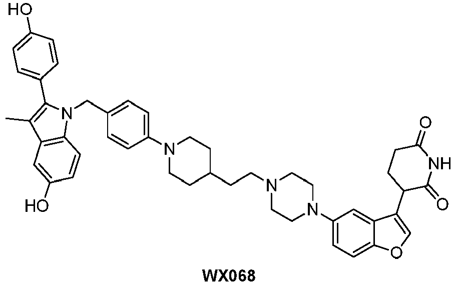

### Synthetic route:

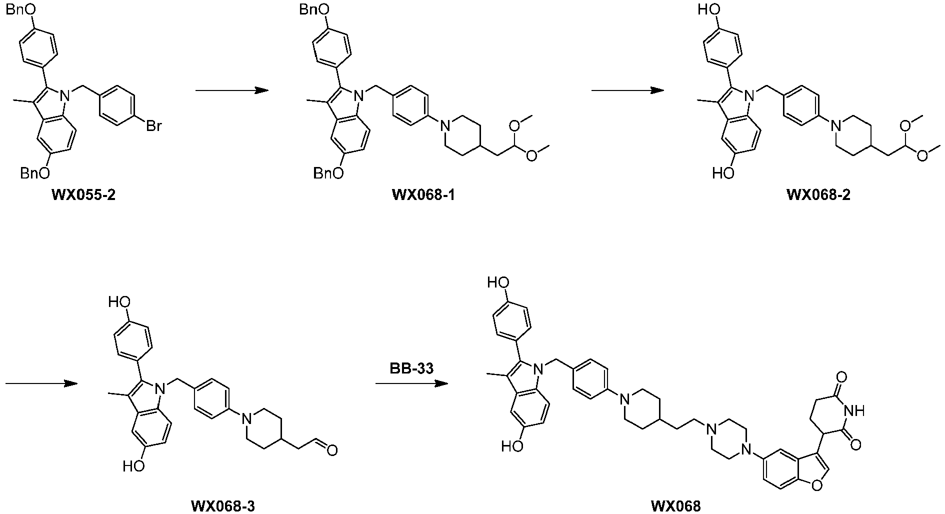

### Step 1: Synthesis of intermediate WX068-1

Intermediate **WX055-2** (500 mg, 849.57 µmol) was dissolved in toluene (10 mL), and 4-(2,2-dimethoxyethyl)piperidine (171.02 mg, 849.57 µmol), potassium tert-butoxide (285.99 mg, 2.55 mmol), palladium acetate (28.61 mg, 127.44 µmol), and 2-dicyclohexylphosphino-2,4,6-triisopropylbiphenyl (81.00 mg, 169.91 µmol) were added sequentially. The atmosphere was replaced three times with nitrogen. The reaction solution was heated to 90 °C and stirred under nitrogen for 16 h. After the reaction was completed, the reaction solution was cooled down to room temperature, diluted with ethyl acetate (10 mL), and filtered through celite. The filter cake was rinsed with ethyl acetate (5 mL×2), and the filtrate was collected, and concentrated under reduced pressure to remove the solvent. The resulting crude product was separated by column chromatography (eluent: petroleum ether/ethyl acetate = 1/0-1/1, v/v) to give the intermediate **WX068-1.**

### Step 2: Synthesis of intermediate WX068-2

Intermediate **WX068-1** (500 mg, 705.29 µmol) and wet palladium on carbon (0.5 g, 10% content) were dissolved in methanol (5 mL) and tetrahydrofuran (10 mL). The atmosphere was replaced three times with hydrogen and the reaction mixture was stirred at 15 °C under hydrogen (15 Psi) for 15 hours. After the reaction was completed, the reaction solution was filtered through celite. The filter cake was rinsed with tetrahydrofuran (10 mL×2), and the filtrate was collected, and concentrated under reduced pressure to remove the solvent. The resulting residue was separated by column chromatography (eluent: petroleum ether/ethyl acetate = 1/0-1/1, v/v) to give the intermediate **WX068-2.**

### Step 3: Synthesis of intermediate WX068-3

Intermediate **WX068-2** (50 mg, 94.58 µmol) was dissolved in tetrahydrofuran (2 mL) and sulfuric acid (2 M, 1.89 mL) was then added. The reaction solution was reacted at 20 °C for 1h. After the reaction was completed, the reaction solution was adjusted to pH of 7 with saturated sodium bicarbonate and extracted with ethyl acetate (10 mL×3). The organic phases were combined, washed with saturated brine (30 mL), dried over anhydrous sodium sulfate, and filtered. The filtrate was concentrated under reduced pressure to remove the solvent to give the intermediate **WX068-3.**

### Step 4: Synthesis of compound WX068

Intermediate **BB-33** (40.02 mg, 114.40 µmol, hydrochloride) was dissolved in methanol (1 mL) and dichloromethane (1 mL), and sodium acetate (14.44 mg, 175.99 µmol) was then added. The reaction mixture was stirred at 20 °C for 0.5 h. Subsequently, intermediate WX068-3 (40 mg, 88.00 µmol) and sodium cyanoborohydride (16.59 mg, 263.99 µmol) were added sequentially, and the reaction solution was stirred to react for another 12 h. After the reaction was completed, the reaction solution was concentrated under reduced pressure to remove the solvent, and a mixed solution of dichloromethane/methanol (5 mL, 10/1, v/v) was added for dilution. The reaction solution was adjusted to pH of 6-7 by dropwise adding saturated aqueous sodium bicarbonate solution, and extracted with ethyl acetate (5 mL×3). The organic phases were combined, washed with saturated brine (10 mL), dried over anhydrous sodium sulfate, and filtered. The filtrate was concentrated under reduced pressure to remove the solvent. The resulting residue was separated by thin layer chromatography (developing agent: dichloromethane/methanol = 18:1-15:1, v/v) to give the title compound **WX068.** MS-ESI m/z: 752.3 [M+H]⁺. ¹H NMR (400 MHz, DMSO_*d₆*) δ: 10.85 (s, 1H), 9.66 (s, 1H), 8.66 (s, 1H), 7.77 (s, 1H), 7.40 (d, *J* = 9.2 Hz, 1H), 7.17 (d, *J* = 8.4 Hz, 2H), 7.06 (d, *J* = 8.4 Hz, 1H), 7.03 (d, *J* = 2.0 Hz, 1H), 6.99 (dd, *J* = 2.0, 9.2 Hz, 1H), 6.86 (d, *J* = 8.4 Hz, 2H), 6.79 (d, *J* = 2.0 Hz, 1H), 6.76-6.66 (m, 4H), 6.57 (dd, *J* = 2.6, 8.6 Hz, 1H), 5.05 (s, 2H), 4.09 (dd, *J* = 4.2, 11.8 Hz, 1H), 3.60-3.51 (m, 2H), 3.11-3.03 (m, 4H), 2.78-2.68 (m, 1H), 2.60-2.54 (m, 5H), 2.42-2.29 (m, 5H), 2.14-2.04 (m, 4H), 1.75-1.67 (m, 2H), 1.45-1.36 (m, 3H), 1.22-1.13 (m, 2H).

### Assay Example 1: In vitro assaying of ALK protein levels and phosphorylation levels thereof in human lung cancer NCI-H2228 cells

### Purpose of the assay

WB (western blot) method was used to study the regulation of ALK protein levels and phosphorylation levels (p-ALK) thereof in human lung cancer cell NCI-H2228 (EML4-ALK fusion mutation) by compounds at different concentration conditions.

### Protocol of the assay

1) NCI-H2228 cells were thawed and passaged 2 times;
2) NCI-H2228 cells were inoculated at 6 × 10⁵ cells per well in a 6-well plate, adhered overnight, and then treated with a certain concentration of assay compounds;
3) After 24 hours of treatment, the supernatant of cultured cell samples was discarded, and the samples were washed twice with DPBS (Dulbecco's Phosphate Buffer). The cells were then lysed with a certain amount of 2% SDS lysis buffer pre-heated at 100°C, collected, and then denatured at 100°C for 15 min;
4) After being denatured and cooled down, the above lysate was subjected to protein quantification assay (Pierce BCA Protein Assay Kit, Thermo), diluted to a volume with the same protein concentration using 5 times the loading buffer (containing dithiothreitol (DDT), Beyotime), and then reduced and denatured at 100°C for 10 minutes;
5) The above samples (10 to 20 µg protein) were separated by SDS-PAGE and transferred to PVDF membrane (Biorad);
6) The bands were cut according to the molecular weight of the target protein, blocked with a blocking solution (5% bovine serum albumin in TBS-T solution, wherein TBS-T solution is Tris-HCl buffer containing 0.2% of Tween-20) for 1 hour, and incubated with primary antibodies (anti-ALK (#3633, CST), anti-p-ALK (#6941, CST) and anti-β-actin (#4970, CST), formulated by diluting with a blocking solution at 1:1000, 1:1000, and 1:2000, respectively) at 4°C overnight;
7) Finally, the membrane was incubated with HRP-conjugated secondary antibody (anti-rabbit IgG (#7074, CST), formulated by diluting with a blocking solution at 1:2000) at room temperature for 1 h, and then the bands on the membrane were detected with a chemiluminescent substrate (Clarity ECL, Biorad).

### Assay results

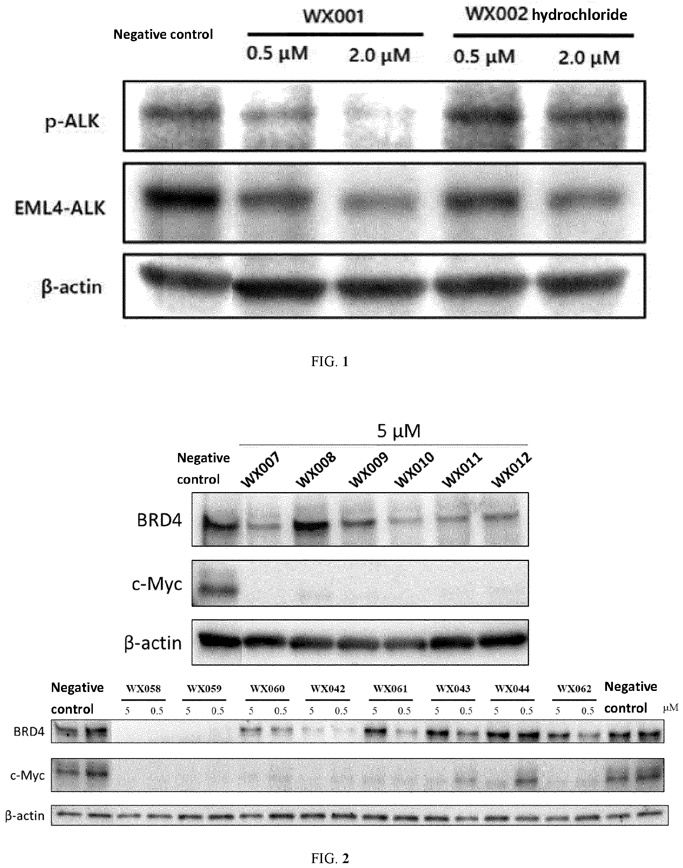

The assay results are shown in FIG. 1.

### Conclusion

The compounds of the present disclosure can reduce ALK protein levels and phosphorylation levels in human lung cancer cell NCI-H2228.

### Assay Example 2: Inhibitory activity of compounds against ALK kinase

### Purpose of the assay

The inhibitory activity of the compounds on ALK kinase was detected by time-resolved fluorescence resonance energy transfer method, that is, the ratio of fluorescence signals at 665 nm/615 nm was used to represent the activity of the enzyme.

### Reagents of the assay

Assay buffer solution: 50 mM 2-[4-(2-hydroxyethyl)-1-piperazinyl]ethanesulfonic acid buffer solution (HEPES) (pH 7.5), 10 mM MgCl₂, 1 mM EDTA 0.01%, polyoxyethylene lauryl ether (Brij 35), 2 mM dithiothreitol (DTT).

Enzyme: Recombinant human-derived ALK (gene accession number: BAG10812.1; 1058-1620 (terminal) amino acids) was expressed with a N-terminal GST fusion protein (90 kDa) using a baculovirus expression system.

Enzyme substrate: 50 nM ULight-poly GT polypeptide.

Zymolyte: 30 µM ATP.

Detection: europium-labeled anti-phosphotyrosine (PT66) antibody.

### Steps of the assay

1. Compounds dissolved in 100% DMSO were added to 384-well plates by acoustic technique;
2. Recombinant human-derived ALK enzyme and enzyme substrate (50 nM ULight-poly GT peptide) or zymolyte (30 µM ATP) were dissolved in freshly prepared assay buffer;
3. The above ALK enzyme and enzyme substrate in the buffer solution were transferred to reaction wells;
4. Then, the zymolyte in the buffer solution was added to the above reaction wells to initiate the reaction;
5. The plate was incubated at room temperature for 1 hour;
6. The detection mixture (PT66 antibody and EDTA) was added to terminate the reaction, and the mixture was incubated at room temperature for 60 minutes;
7. Then, the plate was read using a microplate reader to obtain the ratio of fluorescence signals at 665 nm/615 nm.

Data analysis: Emission ratio (665 nm/615 nm) in the original data was involved in the calculation of the inhibition rate. The concentration and inhibition rate were analyzed using XLfit software 205 mode (4 Parameters Logistic Model) to obtain IC₅₀ value and curve.

### Assay results

The assay results are shown in Table 2.

**Table 2. Assay results of activity of compounds of the present disclosure on ALK kinase**

| Compound | IC₅₀ (nM) | Compound | IC₅₀ (nM) |
|---|---|---|---|
| WX001 | 21.8 | WX003 | 33.8 |
| WX002 hydrochloride | 15.6 | WX006 | 12.2 |

### Conclusion

Compounds of the present disclosure exhibit excellent inhibitory effects on ALK kinase.

### Assay Example 3: Evaluation of anti-proliferative effect in human lung cancer cell NCI-H2228

### Purpose of the assay

In this assay, the inhibitory effect of assay compounds on cell proliferation was assayed in human lung cancer cell NCI-H2228.

### Materials of the assay:

### 1. Cell line and culture method

| Cell line | Tumor type | Growth characteristics | Culture method |
|---|---|---|---|
| NCI-H2228 | Lung cancer | Adhered | RPMI-1640+10% FBS |

### 2. Culture medium and reagents

| Culture medium and reagents | Manufacturer | Cat. No. |
|---|---|---|
| RPMI 1640 | GIBCO | 22400-089 |
| Dulbecco's PBS | Hyclone | SH30256.01 |
| FBS | Hyclone | SY30087.03 |
| Antibiotic-antimycotic | GIBCO | 15240-062 |
| 0.25% Trypsin | GIBCO | 25200072 |
| DMSO | SIGMA | D2650 |
| 2-mercaptoethanol | SIGMA | 60-24-2 |

### 3. Multiwell Plate

Greiner CELLSTAR^{®} 96-well plate, flat-bottom chalkboard (with lid and clear bottom), # 655090.

4. Reagents and instruments used in cell viability assay
(1) Promega CellTiter-Glo luminescent cell viability assay kit (Promega-G7573).
(2) 2104 EnVision^{®} plate reader, PerkinElmer.

### Protocol of the assay

### 1. Cell Culture

The tumor cell line was cultured in a 37°C, 5% CO₂ incubator according to the above-mentioned culture conditions. Cells were passaged periodically, and cells in logarithmic growth phase were used for plating.

### 2. Cell Plating

(1). Cells were stained with trypan blue and viable cells were counted;
(2). The cell concentration was adjusted to an appropriate concentration;

| **Cell line** | **Density (per 96-well)** |
|---|---|
| NCI-H2228 | 7000 |

(3). 90 µL of cell suspension was added to each well of the culture plate as shown in the table above, and cell-free culture medium was added to the blank control well;
(4). The plate was incubated in an incubator at 37°C, 5% CO₂, and 100% relative humidity overnight.

### 3. Preparation of a compound storage plate

Preparation of a mother solution storage plate with 400 times the starting compound concentration: the compounds were diluted serially with DMSO from the highest concentration to the lowest concentration. The plate was prepared freshly every time it was used.

4. Preparation of a working solution with 10 times the starting compound concentration and treatment of cells with the compounds
(1). 78 µL of cell culture medium was added to a 96-well plate with a V-shaped bottom. 2 µL of the compound was pipetted from the mother solution storage plate with 400 times the starting compound concentration into the cell culture medium in the 96-well plate. 2 µL of DMSO was added to the vehicle control and the blank control. After the addition of compound or DMSO, the well content was mixed evenly by pipetting with a multi-channel pipette.
(2). Addition of a drug: 10 µL of the working solution with 10 times the starting compound concentration was added to the cell culture plate. 10 µL of a mixture of DMSO and the cell culture medium was added to the vehicle control and the blank control.
(3). The 96-well cell plate was placed back into the incubator and cultured for 3 days.

### 5. Assay of cell viability by CellTiter-Glo luminescence

The following steps were performed according to the instruction of Promega CellTiter-Glo luminescent cell viability assay kit (Promega-G7573).
(1). The CellTiter-Glo buffer was thawed and allowed to stand to reach room temperature;
(2). The CellTiter-Glo substrate was allowed to stand to reach room temperature;
(3). 10 mL of the CellTiter-Glo buffer was added to the CellTiter-Glo substrate in a bottle to dissolve the substrate to formulate the CellTiter-Glo working solution;
(4). The working solution was vortexed and shaken slowly for full dissolution;
(5). The cell culture plate was taken out and allowed to stand for 30 minutes to equilibrate to room temperature;
(6). 50 µL (equal to half the volume of cell culture medium in each well) of Cell Titer-Glo working solution was added into each well. The cell plate was wrapped with aluminum foil to protect the cell plate from light;
(7). The culture plate was shaken on an orbital shaker for 2 minutes to induce cell lysis;
(8). The culture plate was left at room temperature for 10 minutes to stabilize the luminescence signal;
(9). The luminescent signal was detected on a 2104 EnVision plate reader.

### 6. Data Analysis

The following formula was used to calculate the inhibition rate (IR) of the assay compounds: IR (%) = (RLU of the vehicle control - RLU of the compound)/(RLU of the vehicle control - RLU of the blank control)^{∗} 100%. The inhibition rates of the compounds at different concentrations were calculated in Excel, and then GraphPad Prism software was used to graph the inhibition curves and calculate the relevant parameters, including the minimum inhibition rate, maximum inhibition rate, and IC₅₀.

### Assay results:

The assay results are shown in Table **3.**

**Table 3. Inhibitory effect of the compounds of the present disclosure on cell proliferation in NCI-H2228 cell line**

| Compound | NCI-H2228 IC50 (nM) |
|---|---|
| WX001 | 91 |
| WX002 hydrochloride | 160 |

**Conclusion:** The compounds of the present disclosure exhibit excellent inhibitory effect on cell proliferation in human lung cancer cell NCI-H2228.

### Assay Example 4: Evaluation of pharmacokinetic of the compound in mice

### Purpose of the assay

In this study, C57BL male mice were selected as assay animals, and the LC/MS/MS method was used to quantitatively determine the compound concentration at different time points in the plasma of mice administered intravenously or orally with the assay compound, so as to evaluate pharmacokinetic profile of the assay compound in mice.

### Materials of the assay

C57BL/6 (C57) mice (male, 17-20 g, 7-10 weeks old, from Shanghai lingchang).

### Procedure of the assay

A clear solution of the assay compound was intravenously injected into C57 mice via tail (fasted overnight) or administered to C57 mice by gavage (fasted overnight). 200 µL of blood was collected from the saphenous vein at 0.0833, 0.25, 0.5, 1, 2, 4, 8 and 24 h after intravenous administration, and placed in an anticoagulant tube (Jiangsu Kangjian Medical Co., Ltd.) supplemented with EDTA-K2. The mixture was thoroughly vortexed at 4°C and centrifuged at 3200 g for 10 min. Blood was collected from the saphenous vein at 0.25, 0.5, 1, 2, 4, 6, 8 and 24 h after oral gavage administration, and placed in an anticoagulant tube (Jiangsu Kangjian Medical Co., Ltd.) supplemented with EDTA-K2. The mixture was thoroughly vortexed and centrifuged at 3200 g for 10 min. Blood drug concentration was determined by LC-MS/MS, and relevant pharmacokinetic parameters were calculated with the non-compartmental model Linear/log trapezoidal method using Phoenix WinNonlin 6.3 pharmacokinetic software.

### Assay results

The assay results are shown in Table **4.**

**Table 4. Pharmacokinetic parameters of the compound of the present disclosure in mice**

| Pharmacokinetic parameters in mice | Intravenous injection (2 mg/kg) | | | Oral (10 mg/kg) | | | |
|---|---|---|---|---|---|---|---|
| | Plasma clearance (mL/min/kg) | Half-life (h) | Area under the curve (0-inf, µM·h) | Peak concentration (µM) | Time to peak (h) | Area under the curve (0-inf, µM·h) | Bioavailability F (%) |
| WX001 | 10.3 | 1.74 | 3.69 | 0.68 | 0.75 | 2.33 | 11.7 |

### Conclusion

The compound of the present disclosure has low plasma clearance and acceptable oral gavage bioavailability in mice.

### Assay Example 5: In vivo pharmacodynamic study of the compound in a subcutaneous xenograft tumor NOD SCID mouse model of human lung cancer NCI-H2228 cells

### Cell culture

Human lung cancer cell NCI-H2228 (ATCC^{®} CRL-5935^{™}) was cultured in vitro in an adherent monolayer in RPMI 1640 medium supplemented with 10% fetal bovine serum, 100 U/mL penicillin and 100 µg/mL streptomycin in an incubator at 37°C and 5% CO₂. Cells were passaged by routine digestion with trypsin-EDTA twice a week. When the cell saturation was 80%-90% and the number met the requirement, the cells are collected, counted, and inoculated.

### Animals of the assay

NOD SCID mice, female, 6-8 weeks old, weighing 18-22 grams.

### Protocol of the assay

0.2 mL (10×10⁶ cells) of NCI-H2228 cells (with matrigel, v/v 1:1) were subcutaneously inoculated into the right back of each mouse. The administration by group was started when the average tumor volume reached 171 mm³. One administration cycle comprised seven days, with administration once a day. The assay compounds were administered by subcutaneous injection for a total of four cycles. The subcutaneous injection doses of the assay compounds WX001 and WX002 were both 50 mg/kg. Tumor diameter was measured twice a week with a vernier caliper, and tumor volume was measured in cubic millimeters and calculated by the following formula: V = 0.5a × b², where a and b are the long and short diameters of the tumor, respectively. The tumor-inhibitory efficacy of the assay compounds was evaluated by using TGI (%). TGI (%) reflects a tumor growth inhibition rate. TGI(%) = [1 - (Average tumor volume at the end of administration of a treatment group - Average tumor volume at the beginning of administration of the treatment group) / (Average tumor volume at the end of treatment of a vehicle control group - Average tumor volume at the beginning of treatment of the vehicle control group)] × 100%.

### Assay results

The assay results are shown in Table **5.**

**Table 5. Assay results of the assay compounds in a subcutaneous xenograft tumor NOD SCID model of human lung cancer cell NCI-H2228 cells**

| Group | Dosage | Tumor volume (mm³) (Day 1) | Tumor volume (mm³) (Day 28) | TGI(%) (Day 28) |
|---|---|---|---|---|
| Vehicle control | / | 171±13 | 789±88 | / |
| WX001 | 50 mg/kg | 172±12 | 102±5 | 111.22 |
| WX002 hydrochloride | 50 mg/kg | 172±11 | 169±30 | 100.38 |

### Conclusion

The compounds of the present disclosure exhibit a tumor-reducing effect in a subcutaneous xenograft tumor model of human lung cancer NCI-H2228 cells.

### Assay Example 6: In vitro assay of BRD4 protein level and c-Myc protein level of human acute myeloid leukemia cell MV4-11

### Purpose of the assay

WB (western blot) method was used to study the effects of the compounds at different concentrations on BRD4 protein level and downstream c-Myc expression in human acute myeloid leukemia cell MV4-11.

### Materials of the assay

Cell line and culture method

| Cell Line | Source (Cat. No.) | Tumor Type | Growth Characteristics | Culture Method |
|---|---|---|---|---|
| MV4-11 | ATCC(CRLP9591) | Human acute myeloid leukemia | Suspension | 1640+10%FBS |

Culture medium and reagents

| Culture medium and reagents | Manufacturer | Cat. No. |
|---|---|---|
| RMPI-1640 | GIBCO | 22400089 |
| FBS | Hyclone | RB35950 |

### Protocol of the assay

1) MV4-11 cells were recovered and cultured to a suitable state;
2) MV4-11 cells were inoculated at 1.5×10⁶ cells per well in a 6-well plate, adhered overnight, and then treated with a certain concentration of the assay compounds;
3) After 24 hours of treatment, the supernatant of cultured cell samples was discarded, and the samples was washed twice with DPBS (Dulbecco's Phosphate Buffer, 21-031-CVR, CORNING). The cells were lysed with a certain amount of 2% SDS lysis buffer (SDS Lysis Buffer, P0013G, Beyotime) pre-heated at 100°C, collected, and then denatured at 100°C for 20 min;
4) After being denatured and cooled down, the above lysate was subjected to protein quantification assay (BCA Protein Quantitation Kit, P0011, Beyotime), diluted to a volume with the same protein concentration using 5 times the loading buffer (containing reducing buffer, NP0009, Thermo), and then reduced and denatured at 100°C for 10 min;
5) The above sample (10 to 20 µg total protein) was separated by SDS-PAGE and transferred to PVDF membrane (Biorad);
6) The bands were cut according to the molecular weight of the target protein, blocked with a blocking solution (3% bovine serum albumin in TBS-T solution, wherein TBS-T solution is Tris-HCl buffer containing 0.2% of Tween-20) for 1 hour, and incubated with primary antibodies (BRD4 (#13440S, CST), c-Myc (#5605, CST) and (#3700, CST), formulated by diluting with a blocking solution at 1:1000, 1:1000, and 1:2000, respectively) at 4°C overnight;
7) Finally, the membrane was incubated with HRP-conjugated secondary antibodies (anti-rabbit IgG (#7074, CST), anti-mouse IgG (#7076, CST), formulated by diluting with a blocking solution at 1:2000) at room temperature for 1 h, and then the bands on the membrane were detected with a chemiluminescent substrate (Clarity ECL, Biorad).

### Assay results

The assay results are shown in FIG. **2****.**

### Conclusion

The compounds of the present disclosure can reduce the level of BRD4 protein and inhibit the expression of c-Myc in human acute myeloid leukemia cell MV4-11.

### Assay Example 7: Evaluation of anti-proliferative effect in human acute myeloid leukemia cell MV4-11

### Purpose of the assay

In this assay, the inhibitory effect of assay compounds on cell proliferation in human acute myeloid leukemia cell MV4-11 was detected by ATP fluorescence activity detection (CellTiter-Glo).

### Materials of the assay

### 1. Cell line and culture method

| Cell Line | Source (Cat. No.) | Tumor Type | Growth Characteristics | Culture Method |
|---|---|---|---|---|
| MV4-11 | ATCC(CRLP9591) | Human acute myeloid leukemia | Suspension | 1640+10%FBS |

### 2. Culture medium and reagents

| Culture medium and reagents | Manufacturer | Cat. No. |
|---|---|---|
| RMPI-1640 | GIBCO | 22400089 |
| FBS | HyClone | RB35945 |
| CTG | Promega | G7573 |

### 3. Multiwell Plate

Greiner CELLSTAR^{®} 96-well plate, flat-bottom chalkboard (with lid and clear bottom), # 655090.

### 4. Reagents and instruments used in cell viability assay

(3) Promega CellTiter-Glo luminescent cell viability assay kit (Promega-G7573).

(4) 2104 EnVision^{®} plate reader, PerkinElmer.

### Protocol of the assay

### 5. Cell Culture

The tumor cell line was cultured in a 37°C, 5% CO₂ incubator according to the above-mentioned culture conditions. Cells were passaged periodically, and cells in logarithmic growth phase were used for plating.

### 6. Cell Plating

(1). Cells were stained with trypan blue and viable cells were counted;
(2). The cell concentration was adjusted to an appropriate concentration;
(3). 90 µL of cell suspension was added to each well of the culture plate, and each well contained 6000 cells. In the blank group and other wells, no cells were inoculated, and only 90 µE of culture medium was added;
(4). The plate was incubated in an incubator at 37°C, 5% CO₂, and 100% relative humidity overnight.

### 7. Addition of the first batch of compounds:

(1) Formulation of 10 drug solution: A sterile round bottom 96-well plate was used, and the concentration of mother solutions of WX007, WX008, WX009, WX010, WX011 and WX012 was 10 mM. 5 µL of compound was added to the first well with 5 µL of 1640 medium, and mixed well to obtain a concentration of 5 mM. 5 µL of compound from the first well was added to the third well containing 45 µL of 1640 medium and mixed well to obtain a concentration of 500 µM. 5 µL of compound from the third well was added to the fourth well containing 45 µL of 1640 medium and mixed well to obtain a concentration of 50 µM. 20 µL of compound from the fourth well was added to the fifth well containing 180 µL of 1640 medium and mixed well to obtain 5 µM mother solution. Another sterile round bottom 96-well plate was used, and 60 µL of 5 µM compound was added to the first well. 30 µL of 5 µM compound was added to the second well with 60 µL of 1640 medium, and so forth. Compounds were serially diluted 3-fold to obtain a total of 9 concentrations. To the blank control was added an equal volume of DMSO in the same dilution way.
(2) Formulation of drug solution with the final concentration: 10 µL of the 10× drug solution as prepared above was added to 90 µL of culture medium in triplicate. To the control group was added an equal volume of DMSO solution, and the cell plate was flicked, so that the solution was mixed well. To the blank group was added 10 µL of 1640 medium. The plate was gently taped, so that the solution was mixed well. The plate was placed back in 37°C, 5% CO₂ incubator and incubated for 4 days.

### 8. Addition of the second batch of compounds:

(1) Formulation of the drug solution: A sterile round-bottom 96-well plate was used, and the stock concentration of each compound was 10 mM. 2 µL of compound in 18 µL of DMSO was added to the first well and the mixture was mixed well to obtain a concentration of 1 mM; 10 µL of compound from the first well was added to the third well containing 90 µL of 1640 medium and the mixture was mixed well to obtain a concentration of 100 µM;
(2) For WX058, WX059, WX060 and WX042, 12 µL of solution from the third well obtained above was added to the fourth well containing 108 µL of 1640 medium and the mixture was mixed well to obtain a drug with 10 µM maximum concentration.
(3) Another new sterile round bottom 96-well plate was used, and 60 µL of each drug with the maximum concentration in step (2) was placed in the first well. 60 µL of solution from the first well was added to the second well with 60 µL 1640 medium, and so forth. Compounds were serially diluted 2-fold to obtain a total of 9 concentrations.
(4) For the blank control group, an equal volume of DMSO was added in the same dilution way.
(5) 10 µL of the solution with gradient drug concentrations prepared as above was added to 90 µL of culture medium in triplicate. For the control group, an equal volume of DMSO solution was added. The cell plate was gently tapped, so that the solution was mixed well. For the blank control group, 10 µL of 1640 medium was added. The cell plate was gently tapped, so that the solution was mixed well. The plate was placed back in 37°C, 5% CO₂ incubator and incubated for 4 days.

### 9. CellTiter-Glo luminescent cell viability assay

The following steps were performed according to the instruction of Promega CellTiter-Glo luminescent cell viability assay kit (Promega-G7573), and the entire process was carried out in the dark.
(1). The CellTiter-Glo buffer was thawed and allowed to stand to reach room temperature;
(2). The CellTiter-Glo substrate was allowed to stand to reach room temperature;
(3). 10 mL of the CellTiter-Glo buffer was added to the CellTiter-Glo substrate in a bottle to dissolve the substrate to formulate the CellTiter-Glo working solution;
(4). The working solution was vortexed and shaken slowly for full dissolution;
(5). The cell culture plate was taken out and allowed to stand for 5 minutes to equilibrate to room temperature;
(6). 100 µL (equal to half the volume of cell culture medium in each well) of Cell Titer-Glo working solution was added into each well;
(7). The plate was shaken on an orbital shaker in the dark for 10 minutes;
(8). The luminescent signal was detected on a 2105 EnVision plate reader.

### 10. Data Analysis

The following formula was used to calculate the inhibition rate (IR) of the assay compounds: IR (%) = (RLU of the vehicle control - RLU of the compound)/(RLU of the vehicle control - RLU of the blank control)* 100%. The inhibition rates of the compounds at different concentrations were calculated in Excel, and then GraphPad Prism software was used to graph the inhibition curves and calculate the relevant parameters, including the minimum inhibition rate, maximum inhibition rate, and IC₅₀.

### Assay results:

The assay results are shown in Table **6.**

**Table 6. Inhibitory effect of the compounds of the present disclosure on cell proliferation in H358 cell line**

| Compound | MV4-11 IC₅₀ (nM) | Compound | MV4-11 IC₅₀ (nM) |
|---|---|---|---|
| **WX007** | 201.6 | **WX012** | 70.6 |
| **WX008** | 22.47 | **WX058** | 11.31 |
| **WX009** | 51.68 | **WX059** | 16.58 |
| **WX010** | 61.28 | **WX060** | 17.01 |
| **WX011** | 73.79 | **WX042** | 35.35 |

**Conclusion:** The compounds of the present disclosure exhibit excellent inhibitory effect on cell proliferation in human acute myeloid leukemia cell MV4-11.

### Assay Example 8: In vitro assay of PDEδ protein level in human non-small cell lung cancer cell H358

### Purpose of the assay

WB (Western Blot) method was used to study the effect of the compound at different concentrations on PDEδ protein level in non-small cell lung cancer cell H358.

### Materials of the assay:

### 1. Cell line and culture method

| Cell line | Source (Cat. No.) | Tumor Type | Growth Characteristics | Culture Method |
|---|---|---|---|---|
| H358 | ECACC (95111733) | Human non-small cell lung cancer | Adhered | 1640+10% FBS |

### 2. Culture medium and reagents

| Culture medium and reagents | Manufacturer | Cat. No. |
|---|---|---|
| RMPI-1640 | GIBCO | 22400089 |
| FBS | ExCell Bio | FSP500 |

### Protocol of the assay

1) H358 cells were recovered and cultured to a suitable state;
2) H358 cells were inoculated at 4.5×10⁵ cells per well in a 6-well plate, adhered overnight, and then treated with a certain concentration of the assay compounds;
3) After 8 hours of treatment, the supernatant of cultured cell samples was discarded, and the samples was washed twice with DPBS (Dulbecco's Phosphate Buffer, 21-031-CVR, CORNING). The cells were lysed with a certain amount of 2% SDS lysis buffer (SDS Lysis Buffer, P0013G, Beyotime) pre-heated at 100°C, collected, and then denatured at 100°C for 15 min;
4) After being denatured and cooled down, the above lysate was subjected to protein quantification assay (BCA Protein Quantitation Kit, P0011, Beyotime), diluted to a volume with the same protein concentration using 5 times the loading buffer (containing reducing buffer, NP0009, Thermo), and then reduced and denatured at 100°C for 10 min;
5) The above sample (10 to 20 µg total protein) was separated by SDS-PAGE and transferred to PVDF membrane (Biorad);
6) The bands were cut according to the molecular weight of the target protein, blocked with a blocking solution (3% bovine serum albumin in TBS-T solution, wherein TBS-T solution is Tris-HCl buffer containing 0.2% of Tween-20) for 1 hour, and incubated with primary antibodies (PDEδ (NBP2-38346, Novus) and (#4970, CST), formulated by diluting with a blocking solution at 1:1000 and 1:2000, respectively) at 4°C overnight;
7) Finally, the membrane was incubated with HRP-conjugated secondary antibody (anti-rabbit IgG (#7074, CST), formulated by diluting with a blocking solution at 1:2000) at room temperature for 1 h, and then the bands on the membrane were detected with a chemiluminescent substrate (Clarity ECL, Biorad).

### Assay results

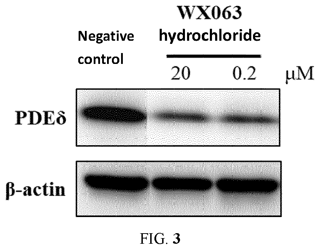

The assay results are shown in FIG. **3****.**

### Conclusion

The compound of the present disclosure can reduce the level of PDEδ protein in human non-small cell lung cancer cell H358.

## Claims

1. A compound of formula (I) or a pharmaceutically acceptable salt thereof,
PTM-L-ULM (I)
wherein
PTM is selected from a drug that binds to a targeted protein or a derivative thereof;
L is a chain connecting PTM and ULM;
ULM is selected from structures represented by formulae (III-1) and (III-2), 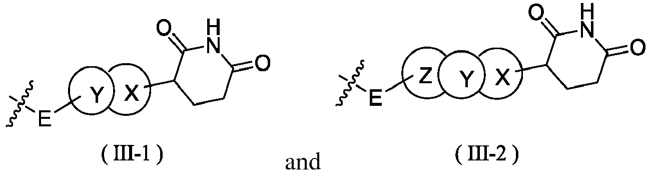
E is selected from a bond, -CH₂-, -NR₁-, -O-, -S-, -S(=O)-, -S(=O)₂-, -C(=O), and -C(=O)NR₂-;
R₁ and R₂ are each independently selected from H and C₁₋₃ alkyl;
Ring X, ring Y, and ring Z are each independently selected from phenyl, thienyl, furyl, triazolyl, oxazolyl, isoxazolyl, pyrrolyl, and pyridyl.

2. The compound according to claim 1 or a pharmaceutically acceptable salt thereof, wherein ULM is selected from structures represented by formulae (II-11), (11-12), (II-13), (II-1), (II-2), (II-3), (II-4), (III-21), (III-22), (III-23), and (III-24), 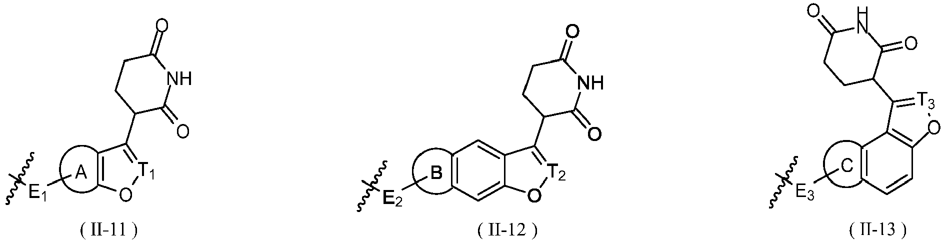 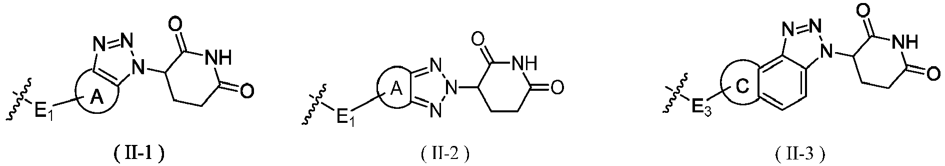 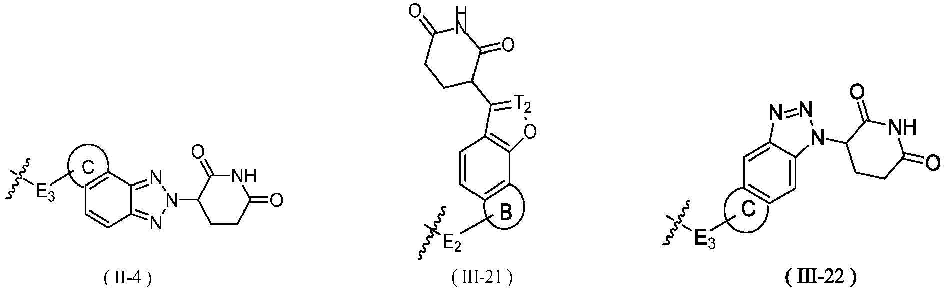 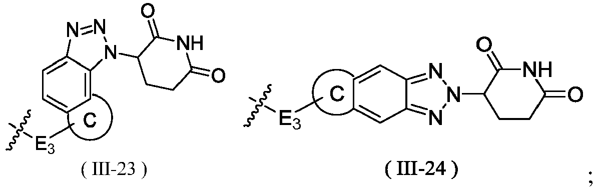
T₁, T₂, and T₃ are each independently selected from CH and N;
E₁, E₂, and E₃ are each independently selected from a bond, -CH₂-, -NR₁-, -O-, -S-, -S(=O)-, -S(=O)₂-, -C(=O), and -C(=O)NR₂-;
R₁ and R₂ are each independently selected from H and C₁₋₃ alkyl;
Ring A, ring B, and ring C are each independently selected from phenyl, thienyl, furyl, pyrrolyl, and pyridyl.

3. The compound according to claim 1 or a pharmaceutically acceptable salt thereof, wherein ring A is selected from phenyl and thienyl.

4. The compound according to any one of claim 1, 2, or 3, or a pharmaceutically acceptable salt thereof, wherein ULM is selected from structures represented by formulae (II-11-1), (II-11-2), (II-1-1), and (II-2-1), 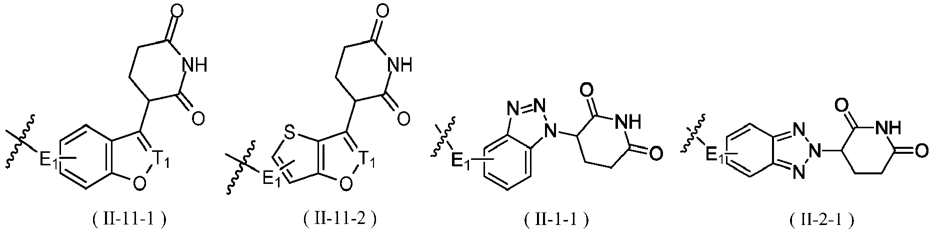 wherein T₁ and E₁ are as defined in claim 2.

5. The compound according to claim 2 or a pharmaceutically acceptable salt thereof, wherein the ring B is selected from phenyl.

6. The compound according to any one of claim 1, 2, or 5, or a pharmaceutically acceptable salt thereof, wherein ULM is selected from structures represented by formulae (II-12-1), and (III-21-1), 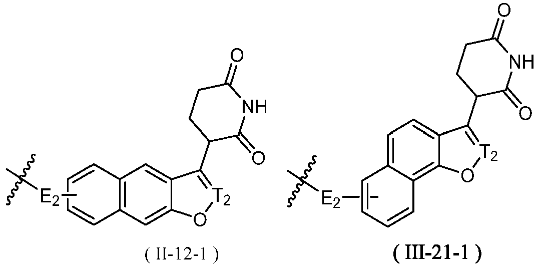 wherein T₂ and E₂ are as defined in claim 2.

7. The compound according to claim 2 or a pharmaceutically acceptable salt thereof, wherein the ring C is selected from phenyl.

8. The compound according to any one of claim 1, 2, or 7, or a pharmaceutically acceptable salt thereof, wherein ULM is selected from structures represented by formulae (II-13-1), (II-3-1), (II-4-2), (111-22-1), (111-23-1), and (111-24-1), 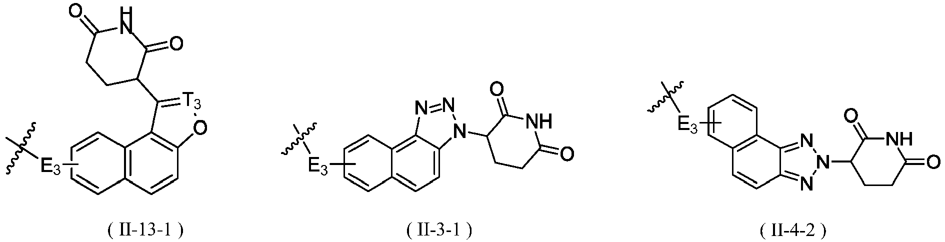 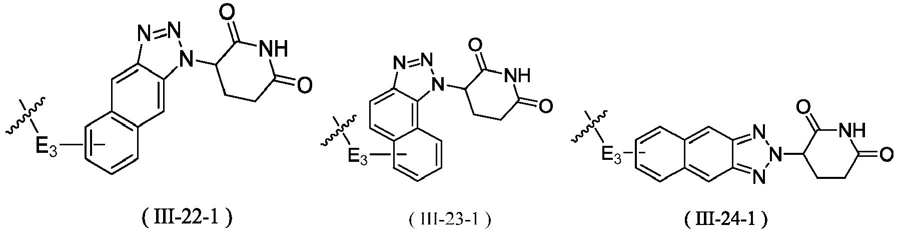 wherein T₃ and E₃ are as defined in claim 2.

9. The compound according to any one of claim 1, 2, 5, or 7, or a pharmaceutically acceptable salt thereof, wherein ULM is selected from

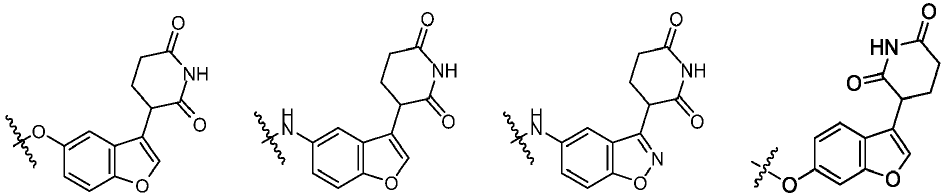

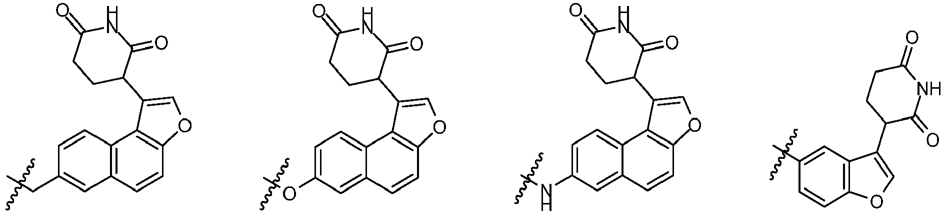

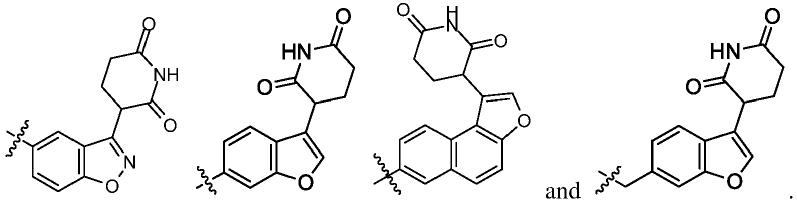

10. The compound according to claim 1 or a pharmaceutically acceptable salt thereof, wherein PTM is selected from drugs that act on ALK, BET, CDK, PARP, EGFR, γ-secretase, CBFβ-SMMHC, WEE1, MEK, BCR-ABL, MET, RAS, BTK, VEGFR, JAK, HER2, HDAC, Akt, PI3K, mTOR, AR, ER, PDEδ, SRC, MDM2, RAF, IRAK4, STAT3, and c-Myc, or derivatives thereof.

11. The compound according to claim 10 or a pharmaceutically acceptable salt thereof, wherein PTM is selected from drugs that act on ALK, BRD4, CDK4/6, CDK8, CDK9, PARP, EGFR, γ-secretase, CBFβ-SMMHC, WEE1, MEK, BCR-ABL, MET, KRAS, BTK, VEGFR, HER2, HDAC, Akt, PI3K, mTOR, AR, ER, PDEδ, SRC, JAK, MDM2, RAF, IRAK4, STAT3, and c-Myc, or derivatives thereof.

12. The compound according to claim 11 or a pharmaceutically acceptable salt thereof, wherein PTM is selected from 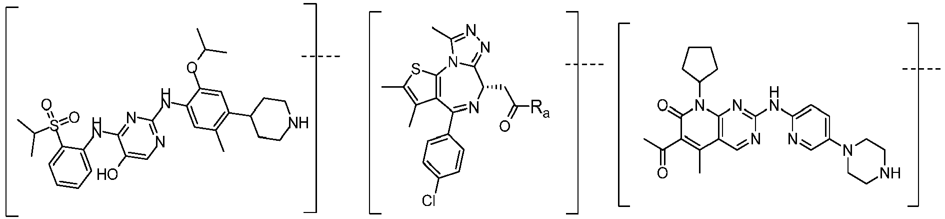 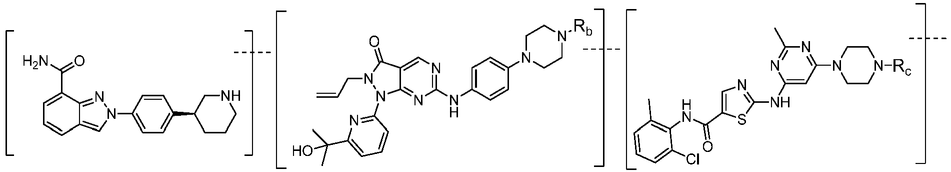 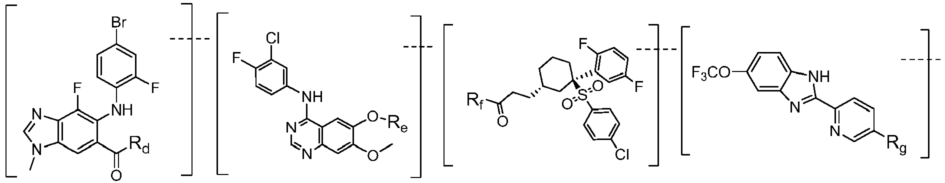 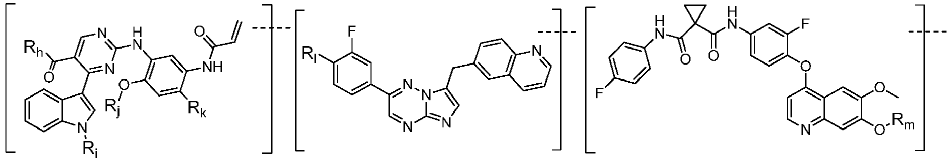 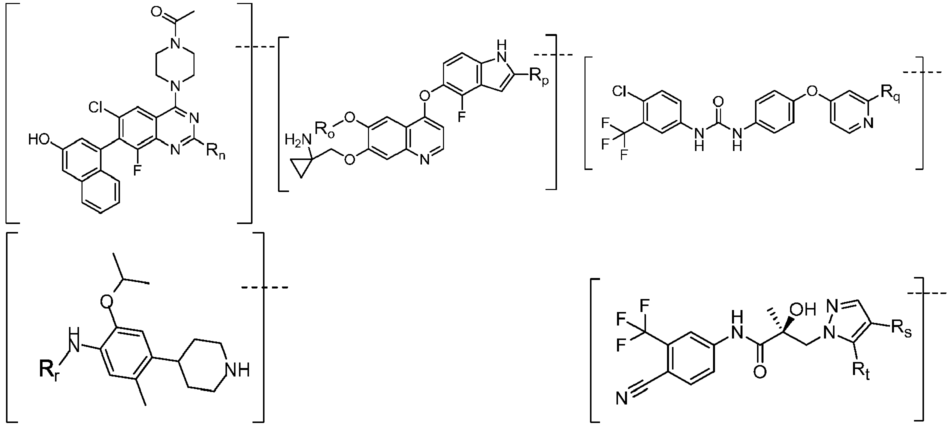 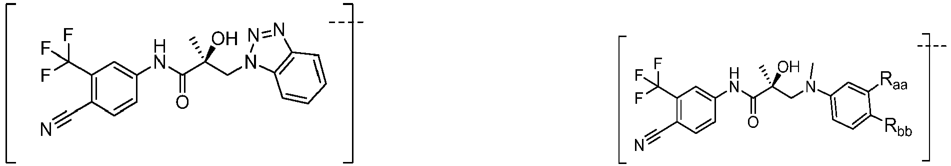 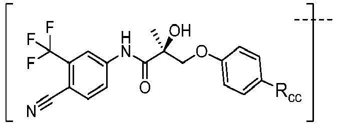 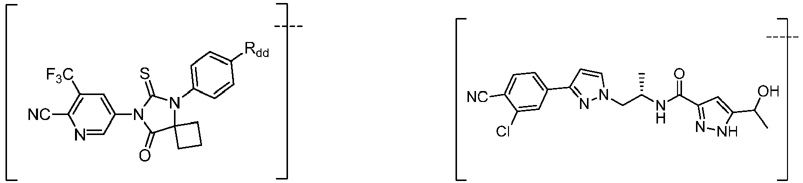 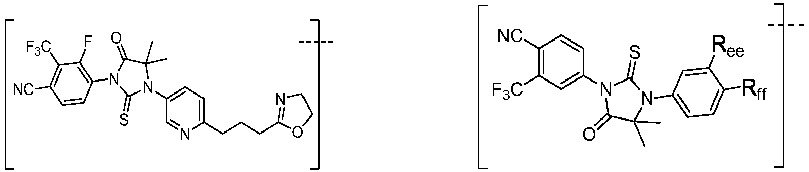 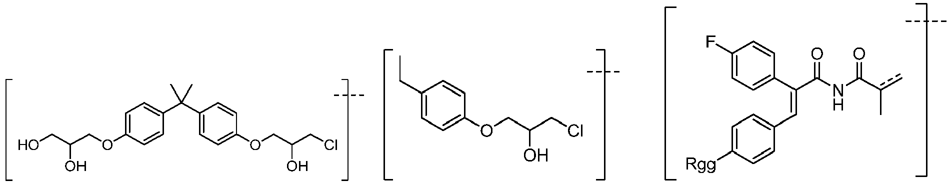 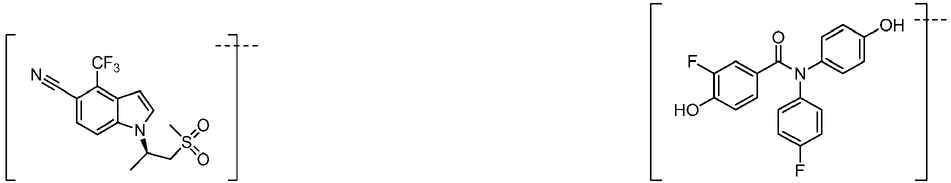 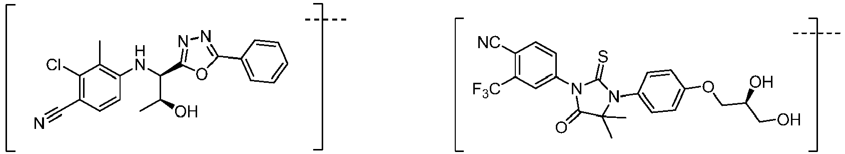 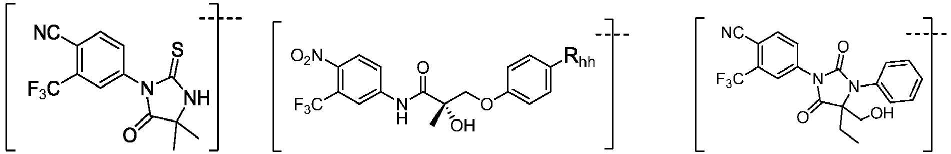 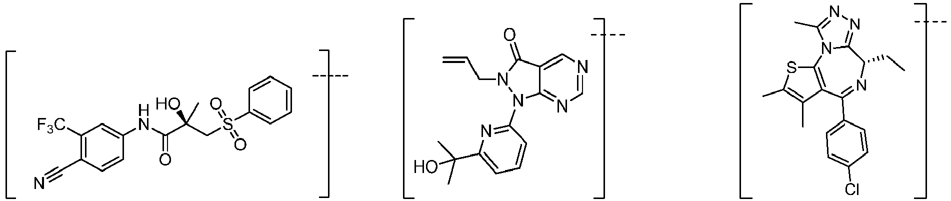 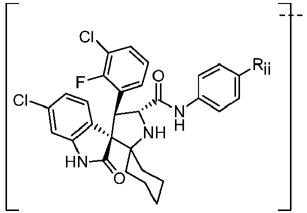 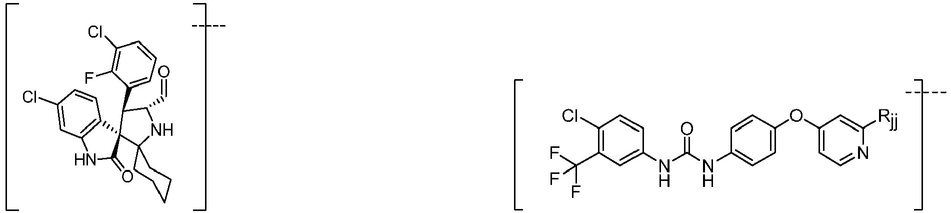 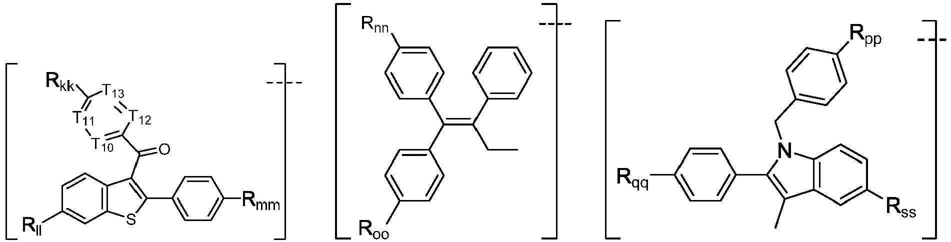 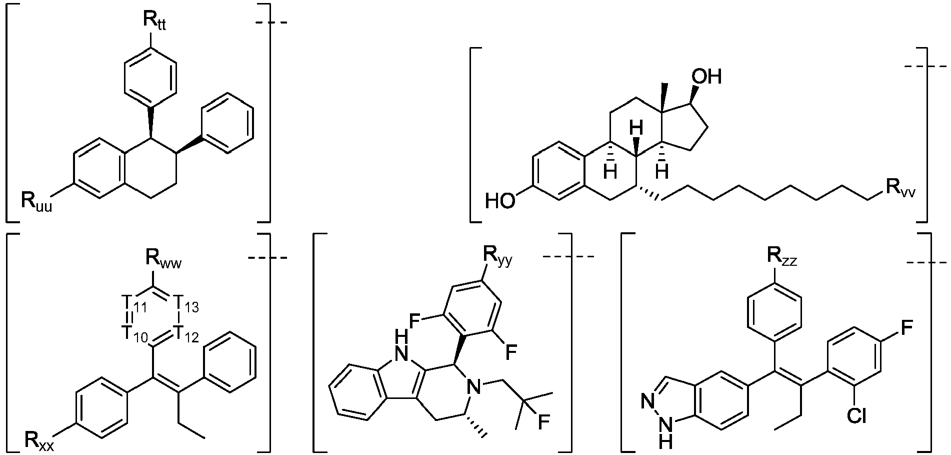 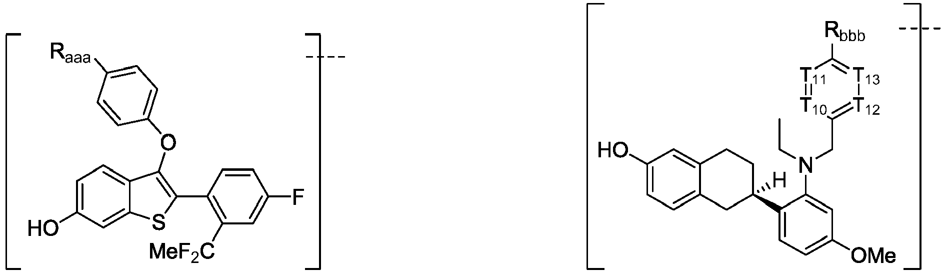 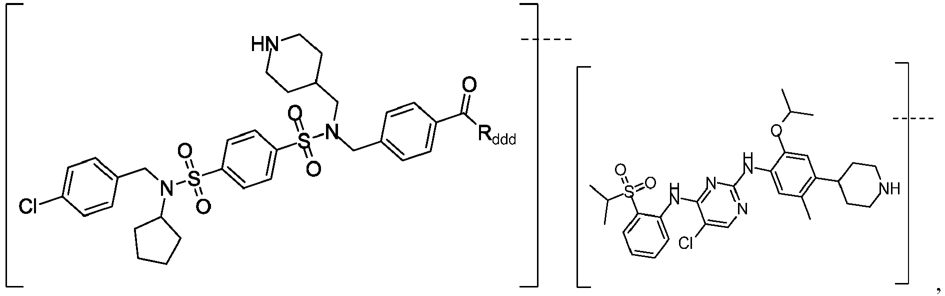 wherein 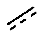
is selected from a single bond and a double bond;
T₁₀, T₁₁, T₁₂, and T₁₃ are each independently selected from N and CR_{ccc}, and at most two of T₁₀, T₁₁, T₁₂, and T₁₃ are selected from N;
Rₐ is selected from H, 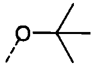 and NH₂;
R_{b} is selected from H and CH₃;
R_{c} is selected from H and 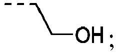 R_{d} is selected from H, NH₂ and 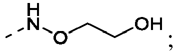
Rₑ is selected from H and 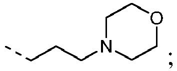
R_{f} is selected from H and OH;
R_{g} is selected from H and OH;
Rₕ is selected from H and 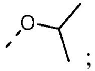
Rᵢ is selected from H and CH₃;
Rⱼ is selected from H and CH₃;
Rₖ is selected from H, NH₂, NHCH₃ and 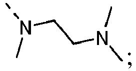
R₁ is selected from H, 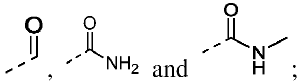
Rₘ is selected from H and 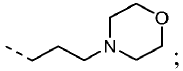
Rₙ is selected from H, NH₂, NHCH₂CH₃ and 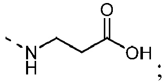
Rₒ is selected from H and CH₃;
Rₚ is selected from H and CH₃;
R_{q} is selected from H, 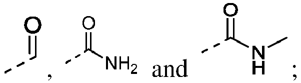
Rᵣ is selected from H and 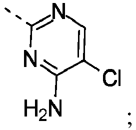
Rₛ is selected from H, F and Cl;
Rₜ is selected from H and Br;
Rₐₐ is selected from H and phenyl;
R_{bb} and R_{cc} are each independently selected from H and CN;
R_{dd}, R_{ff}, Rₕₕ, Rᵢᵢ, and Rⱼⱼ are each independently selected from H, OCH₃, 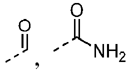 and 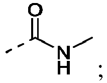
Rₑₑ is selected from H and F;
R_{gg} is selected from H and Cl;
Rₖₖ is selected from H, OH and 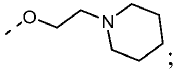
Rₗₗ and Rₘₘ are each independently selected from H, F, Cl, Br, I, OH, and OCH₃;
Rₙₙ is selected from H, OH and 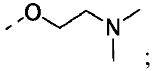
R.. is selected from H and OH;
Rₚₚ is selected from H, OH and 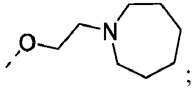
R_{qq} and Rₛₛ are each independently selected from H, F, Cl, Br, I, OH, and OCH₃;
Rₜₜ is selected from H, OH and 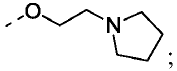
Rᵤᵤ is selected from H, F, Cl, Br, I, OH, and OCH₃;
Rᵥᵥ is selected from H and 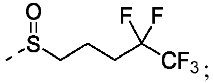
R_{ww} is selected from H and 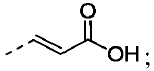
Rₓₓ is selected from H and OH;
R_{yy}, R_{zz}, and Rₐₐₐ are each independently selected from H and 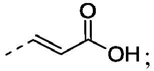
R_{bbb} is selected from H and 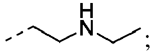
R_{ccc} is selected from H, F, Cl, Br, and I;
R_{ddd} is selected from H and NH₂.

13. The compound according to claim 12 or a pharmaceutically acceptable salt thereof, wherein PTM is selected from

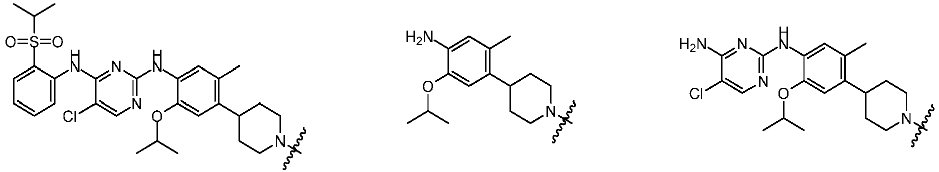

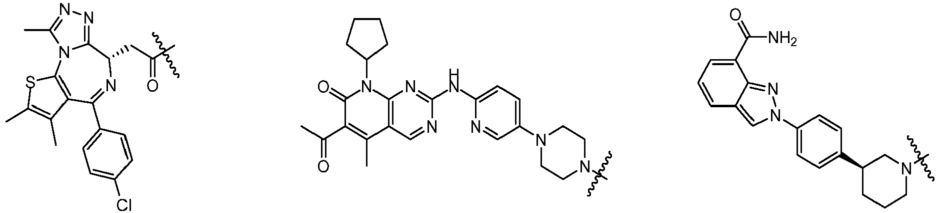

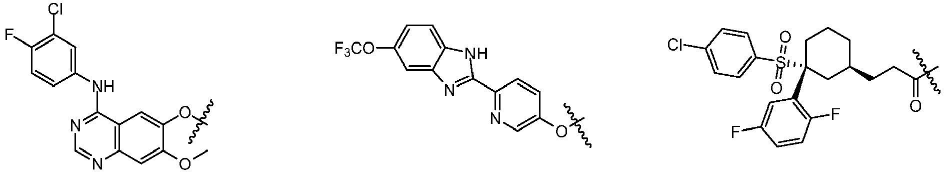

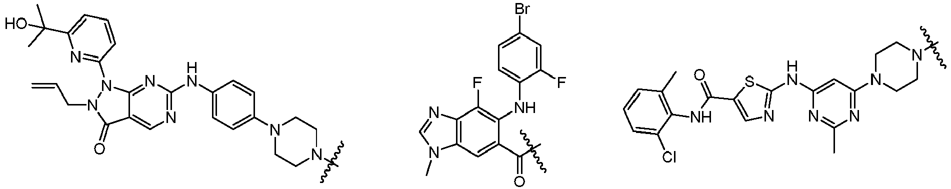

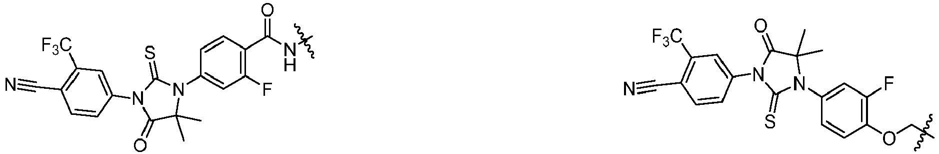

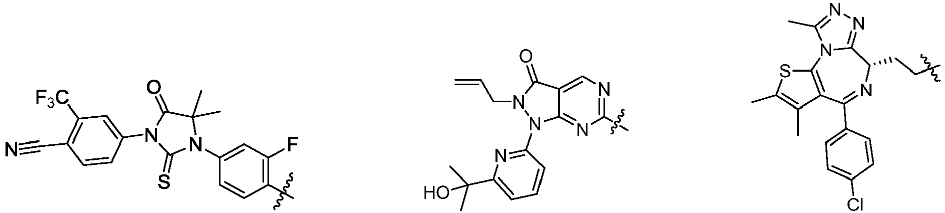

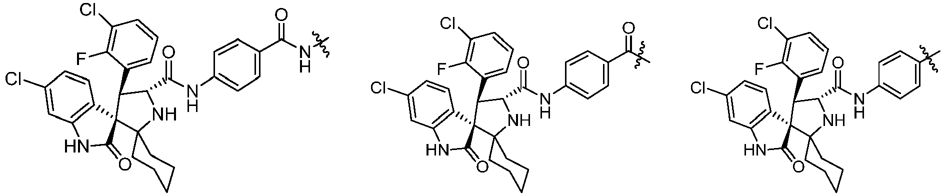

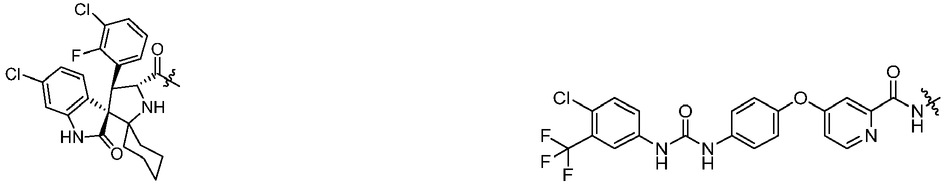

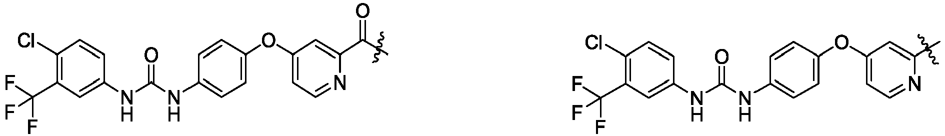

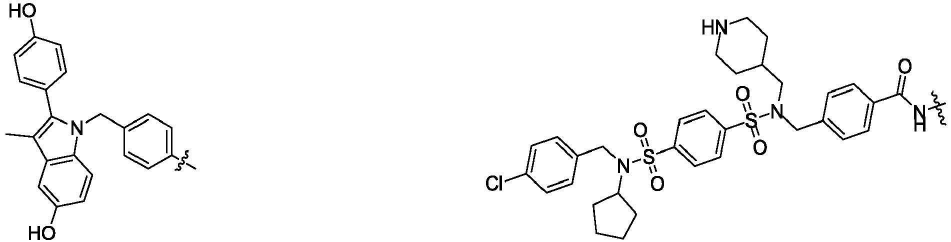

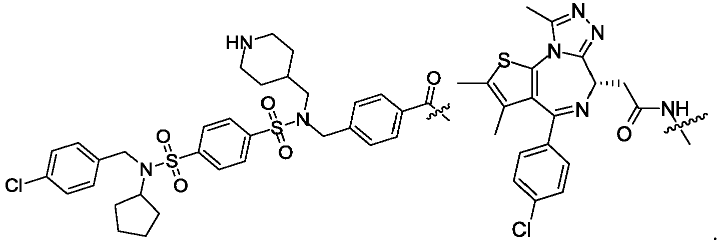

14. The compound according to claim 1 or a pharmaceutically acceptable salt thereof, wherein L is selected from C₁₋₂₀ alkyl; 1, 2 or 3 CH₂ on the L are replaced by cyclopropyl; 1, 2, 3, 4, 5 or 6 CH₂ on the L are optionally replaced by an atom or group selected from -NH-, =N-, -O-, -S-, -C(=O)-, -C(=O)O-, -NHC(=O)-, -NHC(=O)O-, -NHC(=O)NH-, -S(=O)-, -S(=O)₂-, -S(=O)₂NH-, =NO-, -P(=O)(OH)-, -P(=O)(R)-, -P(=O)(NHR)-, -P(=O)(NR₂)-, -P(=O)(R)NH-, C₂₋₄ alkenyl, C₂₋₄ alkynyl, C₆₋₁₂ aryl, 5- to 12-membered heteroaryl, C₃₋₁₄ cycloalkyl, and 3- to 14-membered heterocycloalkyl; and L is optionally substituted with 1, 2, 3, 4, 5 or 6 R, wherein R is selected from H, F, Cl, Br, I, OH, NH, CN, C₁₋₃ alkyl, C₆₋₁₂ aryl and C₅₋₁₀ heteroaryl.

15. The compound according to claim 14 or a pharmaceutically acceptable salt thereof, wherein L is selected from structures represented by formulae (II-5), (II-6), and (IV-1) 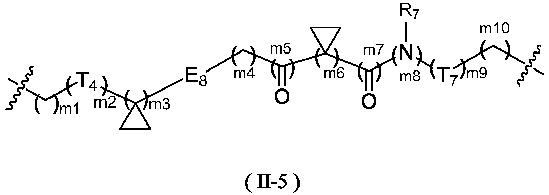 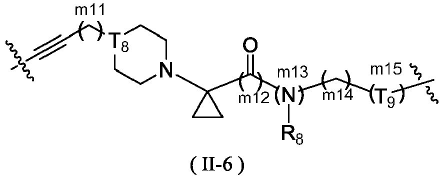 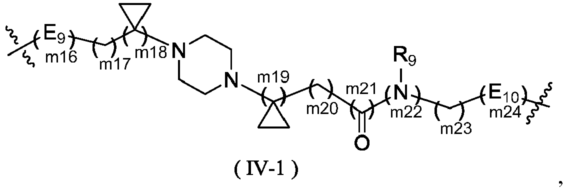 wherein
E₈ is selected from 3- to 8-membered monoheterocycloalkyl, 5- to 14-membered bridged heterocycloalkyl and 5- to 14-membered spiroheterocycloalkyl;
E₉ and E₁₀ are each independently selected from O and NH;
T₄, T₇, T₈, and T₉ are each independently selected from CH and N;
R₇, R₈, and R₉ are each independently selected from H and C₁₋₃ alkyl;
m2, m3, m5, m6, m7, m8 and m9 are each independently selected from 0 or 1;
m1, m4 and m10 are each independently selected from 0 to 15;
and at least one of m1, m2, m3, m4, m5, m6, m7, m8, m9, and m10 is not 0;
and at least one of m3 and m6 is 1;
m12 and m13 are each independently selected from 0 or 1;
m11, m14 and m15 are each independently selected from 0 to 15;
and at least one of m11, m12, m13, m14, and m15 is not 0;
m17, m20 and m23 are each independently selected from 0 to 15;
m16, m18, m19, m21, m22 and m24 are each independently selected from 0 or 1;
and at least one of m16, m17, m18, m19, m20, m21, m22, m23, and m24 is not 0;
and at least one of m18 and m19 is 1.

16. The compound according to any one of claim 1, 14 or 15, or a pharmaceutically acceptable salt thereof, wherein L is selected from the structure represented by formula (IV-1-1) 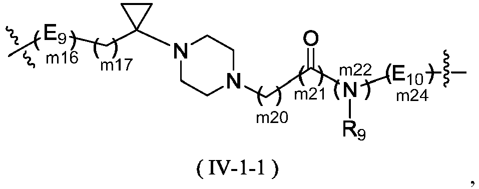 wherein
E₉ and E₁₀ are each independently selected from O and NH;
R₉ is selected from H and CH₃;
m16 is selected from 0 or 1;
m17 is selected from 0, 1, 2, or 3;
m20 is selected from 0, 1, 2, or 3;
m21 and m22 are each independently selected from 0 or 1;
m24 is selected from 0 or 1.

17. The compound according to claim 1 or a pharmaceutically acceptable salt thereof, wherein L is selected from structures represented by formulae (I-4), (I-5), (I-6), (II-7), (II-8), (IV-2), (P-1), and (P-2) 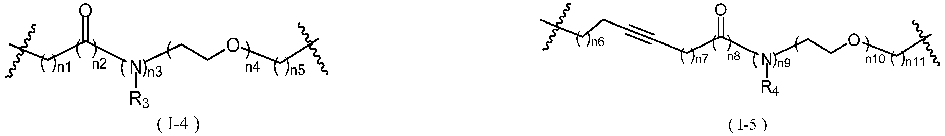 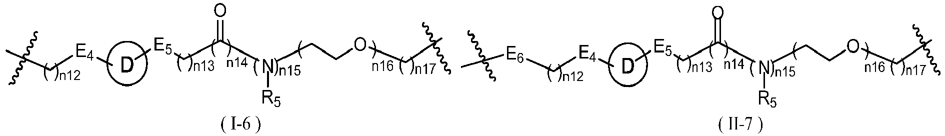 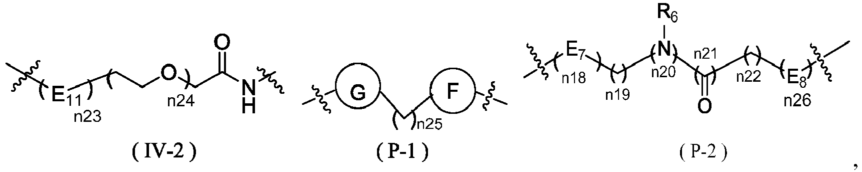 wherein
R₃, R₄, R₅, and R₆ are each independently selected from H and C₁₋₃ alkyl;
n1, n4 and n5 are each independently selected from 0 to 15, n2 and n3 are each independently selected from 0 or 1, and at least one of n1, n2, n3, n4 and n5 is not 0;
n6, n7, n10 and n11 are each independently selected from 0 to 15, n8 and n9 are each independently selected from 0 or 1, and at least one of n6, n7, n8, n9, n10 and n11 is not 0;
n12, n13, n16, and n17 are each independently selected from 0 to 15, n14 and n15 are each independently selected from 0 or 1, and at least one of n12, n13, n14, n15, n16, and n17 is not 0;
n19 and n22 are each independently selected from 0 to 15, n18, n20 and n21 are each independently selected from 0 or 1, and at least one of n18, n19, n20, n21 and n22 is not 0;
E₄ and E₅ are each independently selected from a bond, O, NH, and S(=O)₂;
E₆ and E₇ are each independently selected from O and NH; E₈ is selected from O and NH;
Ring D is selected from phenyl, piperidinyl, piperazinyl, 1,2,3-triazolyl, cyclobutyl and azetidinyl;
E₁₁ is selected from O and NH;
n23 is selected from 0 or 1, n24 is selected from 0 to 15, and at least one of n23 and n24 is not 0;
Ring F and ring G are each independently selected from piperidinyl and piperazinyl;
n25 is selected from 1 to 15;
n26 is selected from 0 and 1.

18. The compound according to claim 1 or 17, or a pharmaceutically acceptable salt thereof, wherein L is selected from NH,

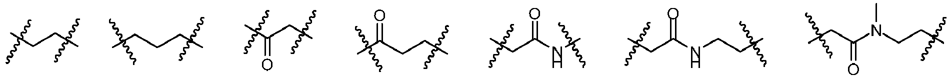

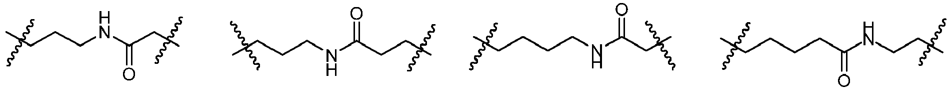

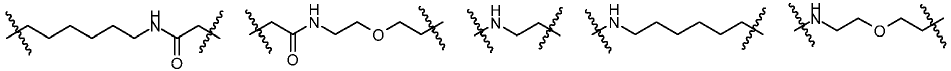

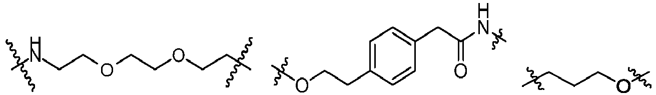

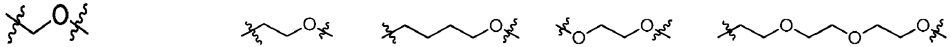

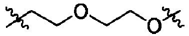

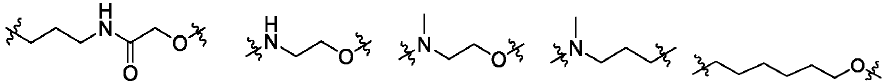

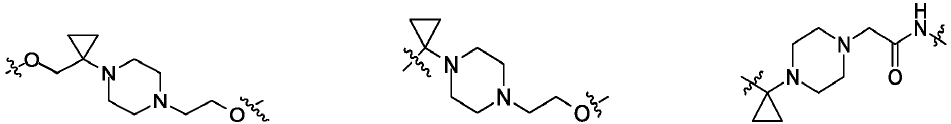

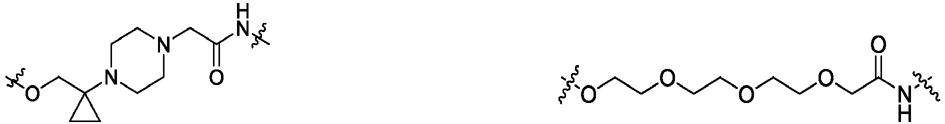

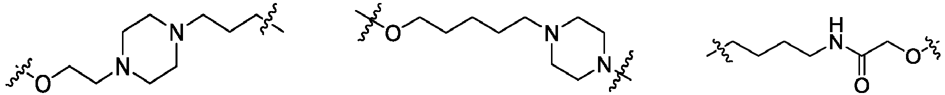

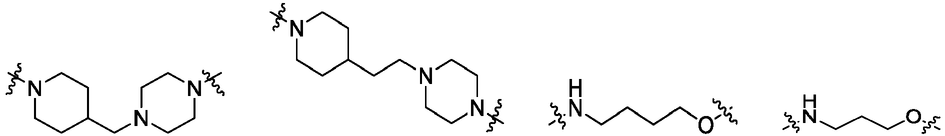

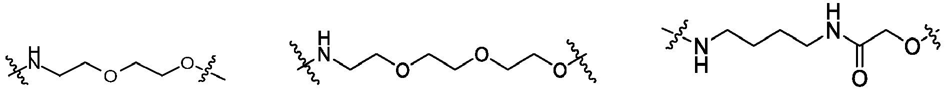

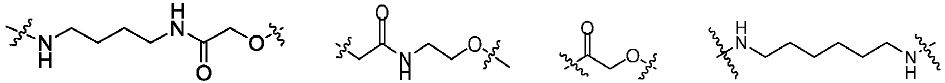

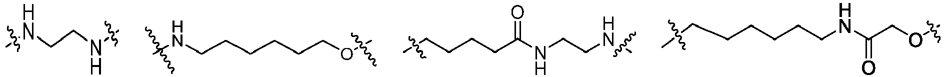

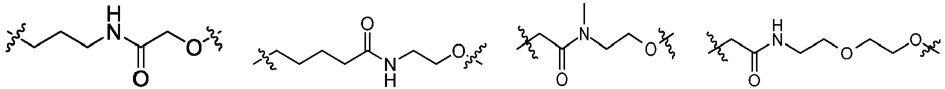

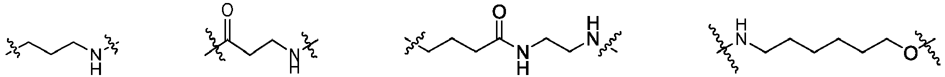

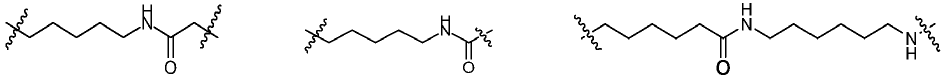

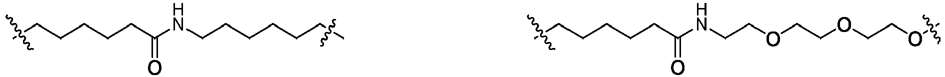

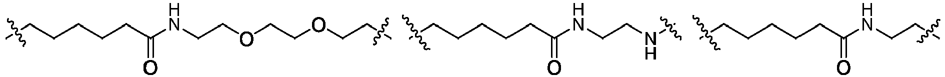

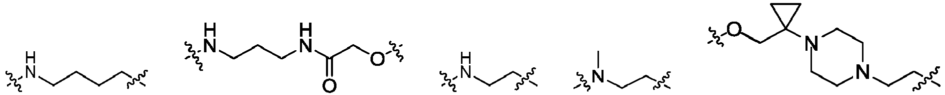

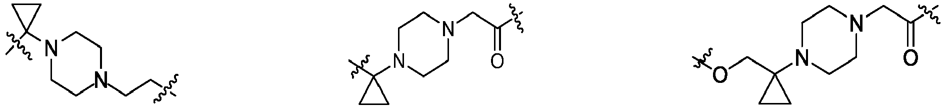

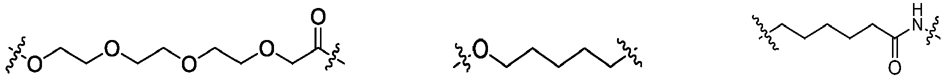

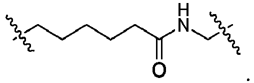

19. The compound of the following formula or a pharmaceutically acceptable salt thereof,

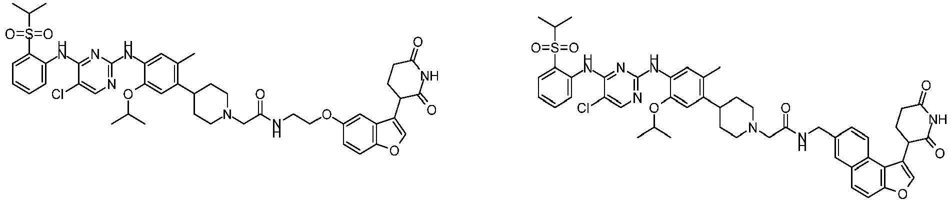

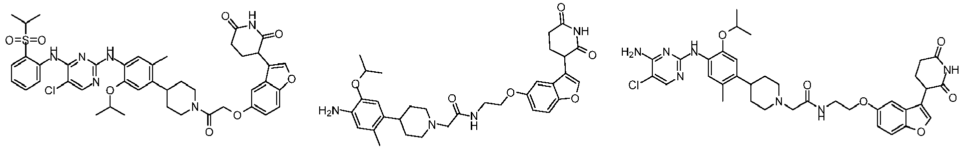

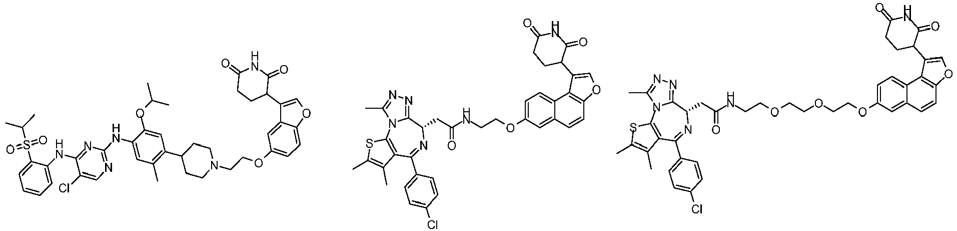

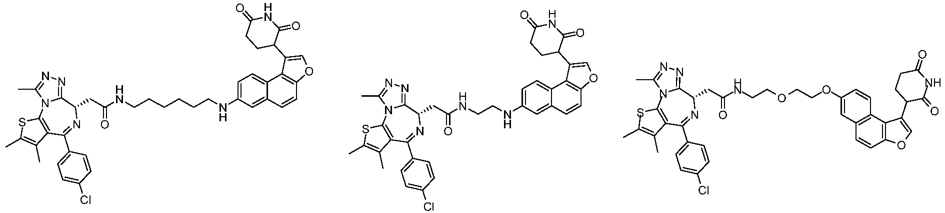

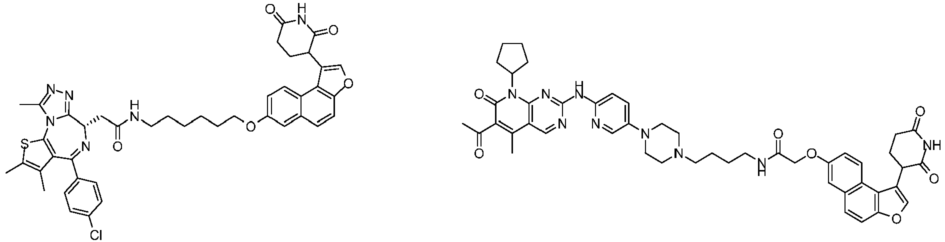

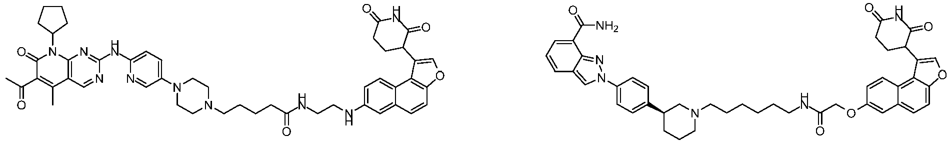

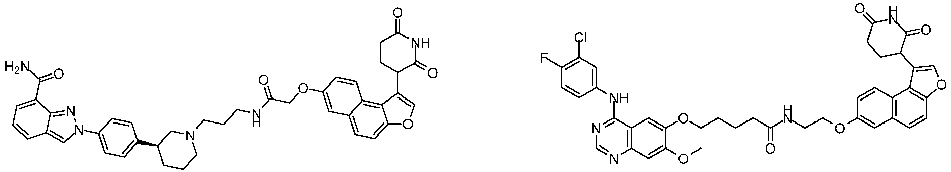

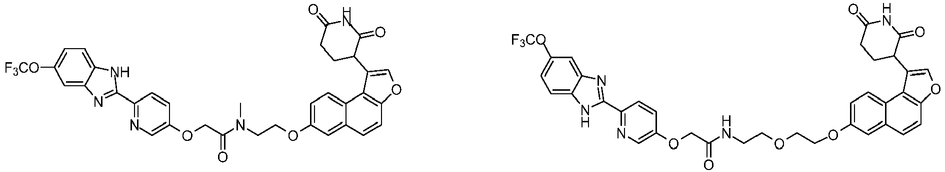

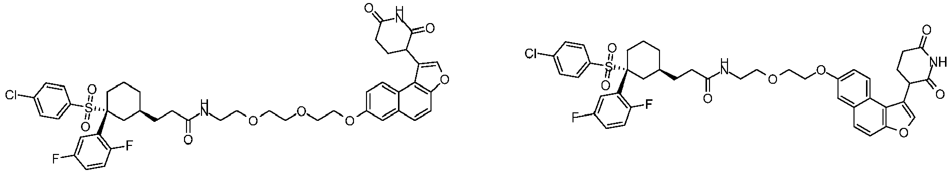

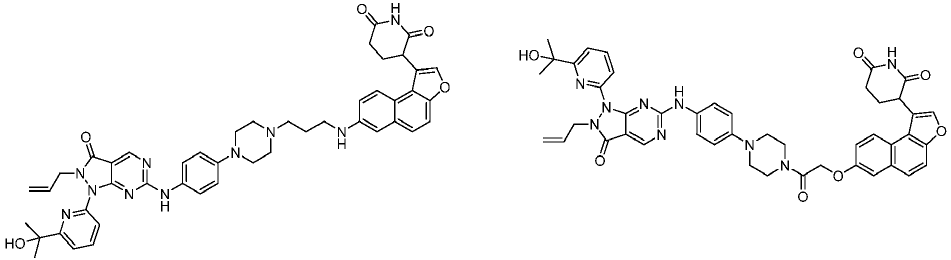

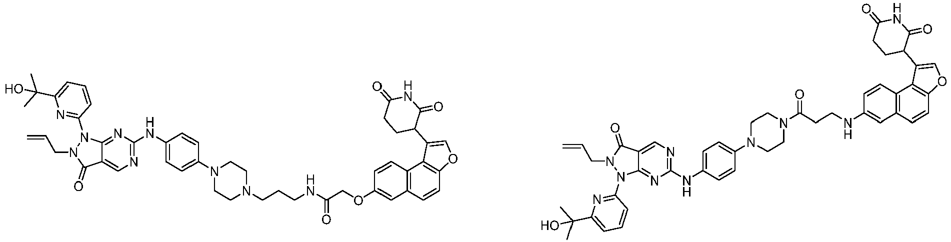

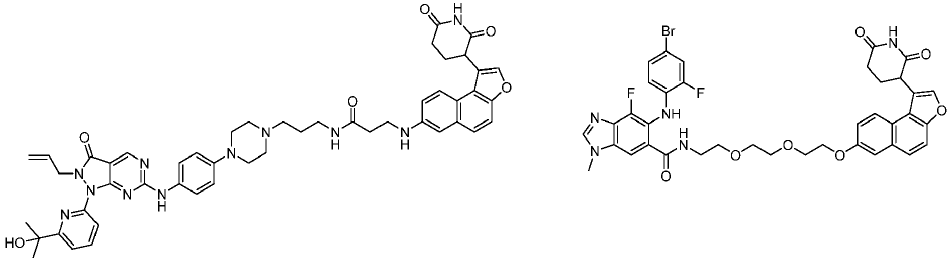

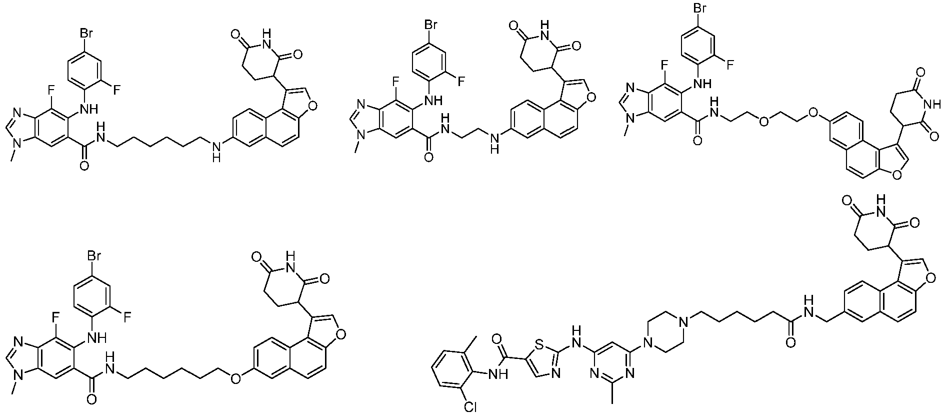

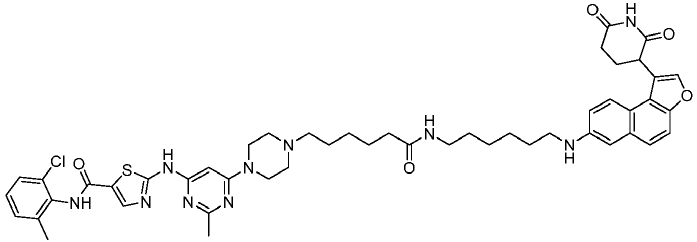

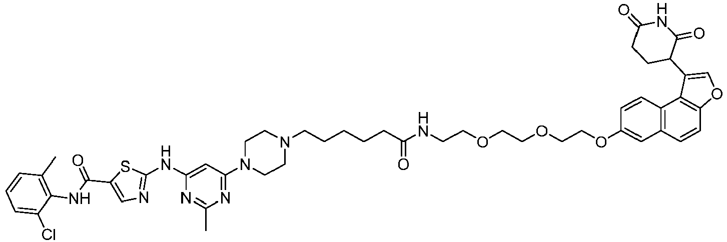

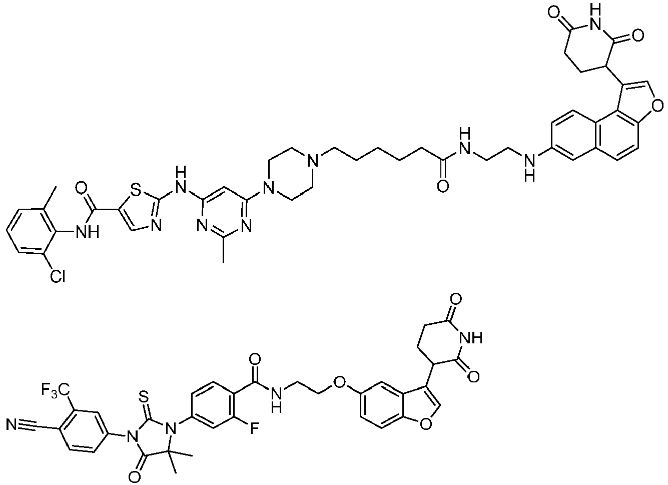

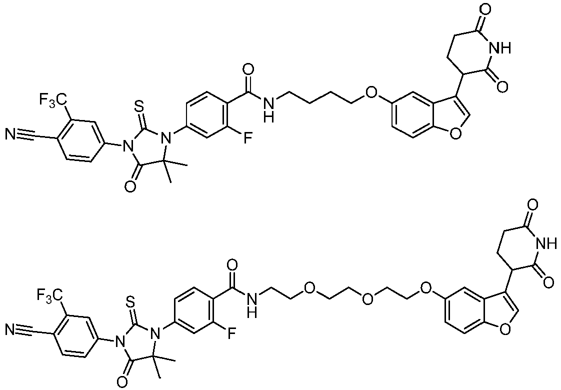

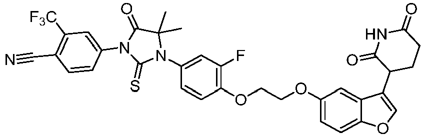

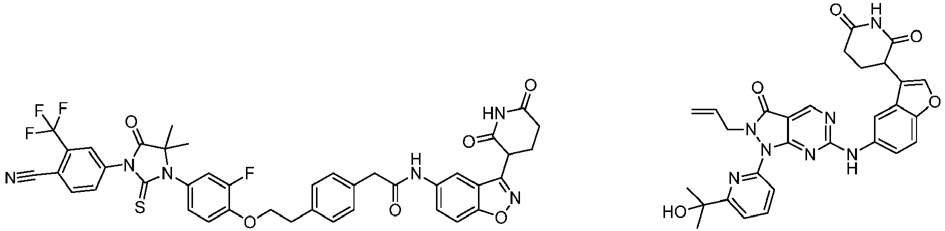

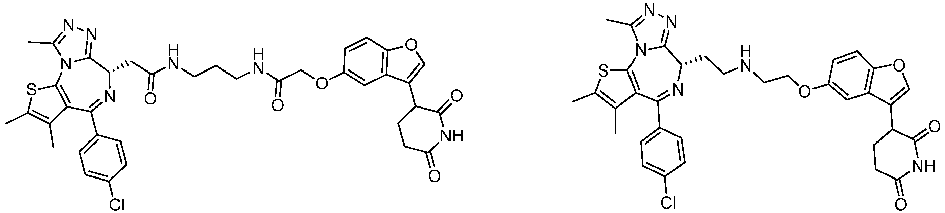

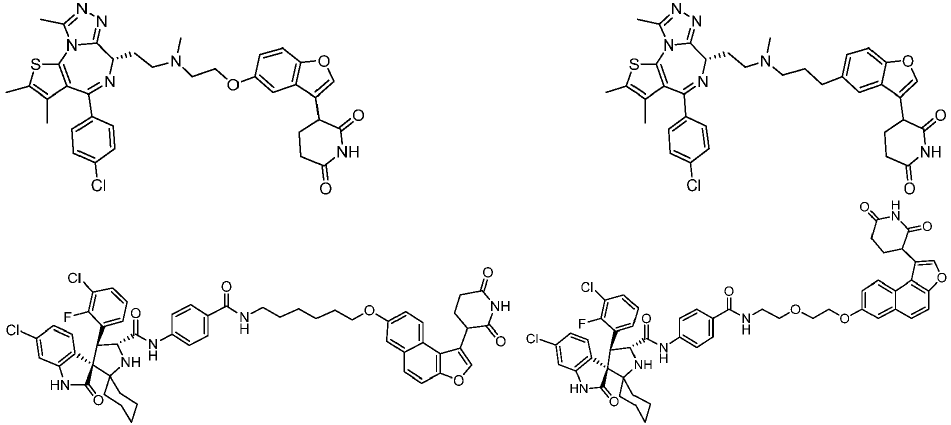

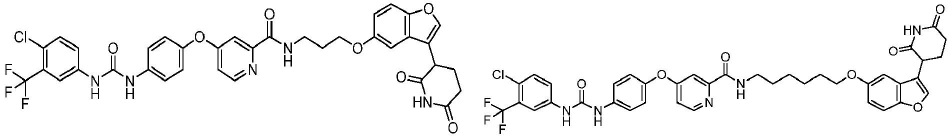

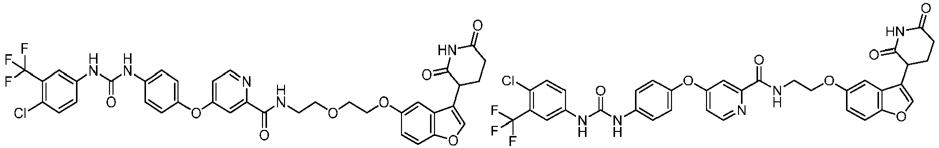

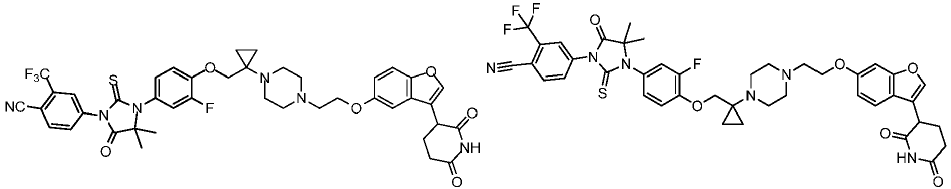

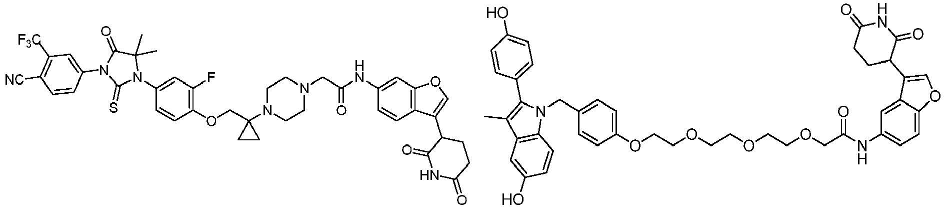

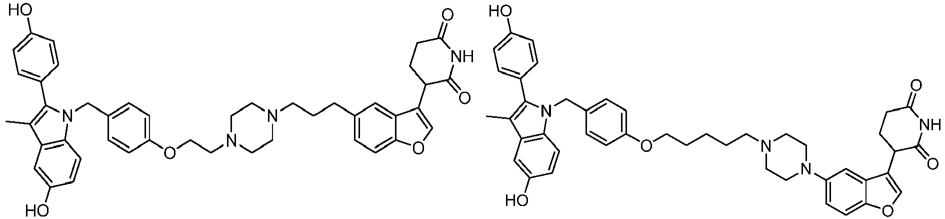

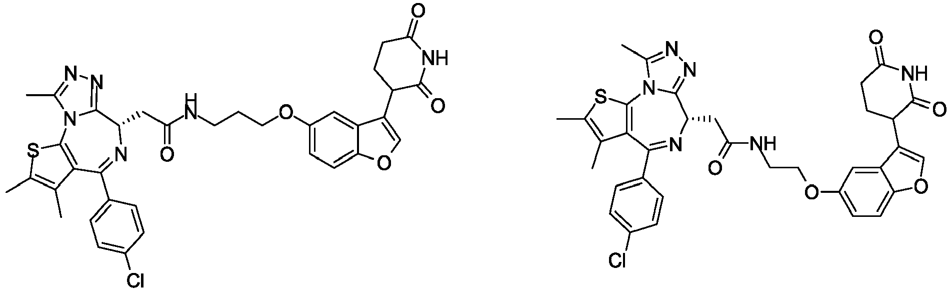

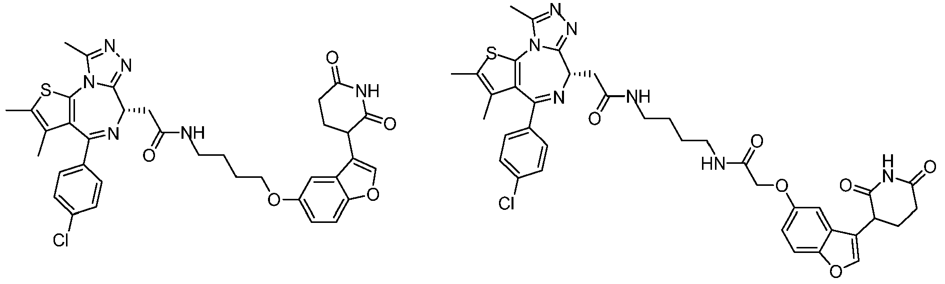

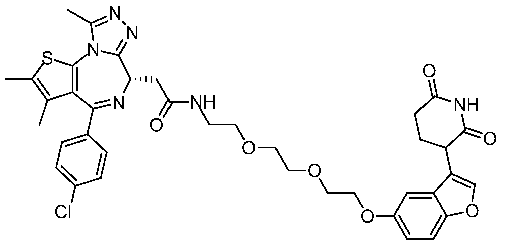

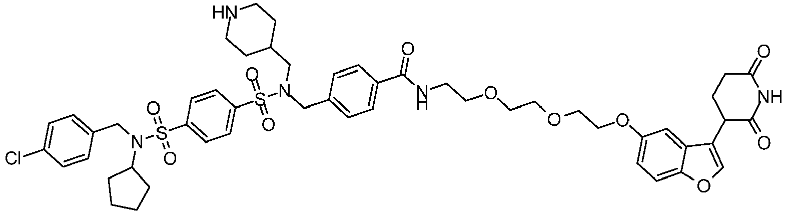

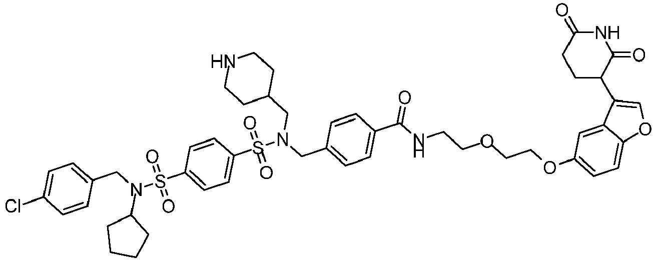

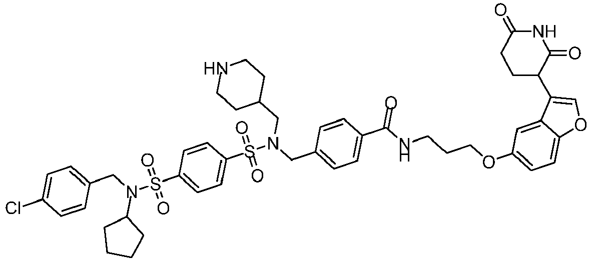

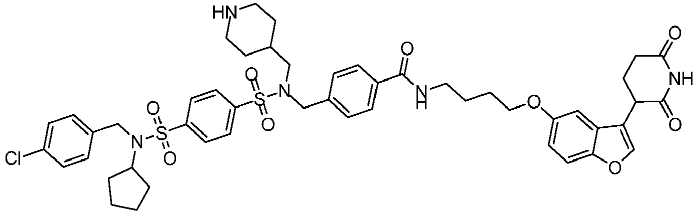

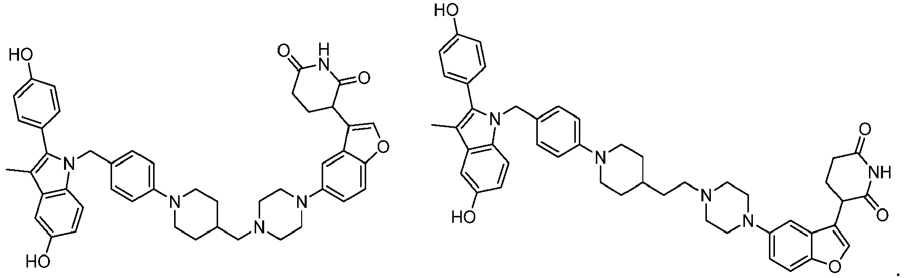

20. Use of the compound according to any one of claims 1 to 19 or a pharmaceutically acceptable salt thereof in the manufacture of a medicament for treating diseases associated with ALK, BET, CDK, PARP, EGFR, γ-secretase, CBFβ-SMMHC, WEE1, MEK, BCR-ABL, MET, RAS, BTK, VEGFR, JAK, HER2, HDAC, Akt, PI3K, mTOR, AR, ER, PDEδ, SRC, MDM2, RAF, IRAK4, STAT3 and c-Myc.
